# EUROPÄISCHE PATENTSCHRIFT

(11) **EP 1 216 225 B1**
(45) Veröffentlichungstag und Bekanntmachung des Hinweises auf die Patenterteilung: **06.07.2005**
(21) Anmeldenummer: 00958516.7
(22) Anmeldetag: 31.08.2000
(51) Int. Cl.: C07C 229/38, C07D 239/30, C07D 277/24, C07D 271/04, C07D 285/06, A61K 31/195, A61K 31/24, A61K 31/505, A61K 31/426, A61K 31/33

(54) **NEUARTIGE AMINODICARBONSÄUREDERIVATE MIT PHARMAZEUTISCHEN EIGENSCHAFTEN**
NOVEL DERIVATIVES OF DICARBOXYLIC ACID HAVING PHARMACEUTICAL PROPERTIES
NOUVEAUX DERIVES D'ACIDE AMINODICARBOXYLIQUE PRESENTANT DES PROPRIETES PHARMACEUTIQUES

(30) Priorität: 13.09.1999 DE 19943635
(43) Veröffentlichungstag der Anmeldung: 26.06.2002
(73) Patentinhaber: Bayer HealthCare AG, 51368 Leverkusen (DE)
(72) Erfinder: ALONSO-ALIJA, Cristina, D-42781 Haan (DE); HEIL, Markus, D-42799 Leichlingen (DE); FLUBACHER, Dietmar, D-40724 Hilden (DE); NAAB, Paul, D-42287 Wuppertal (DE); PERNERSTORFER, Josef, D-42103 Wuppertal (DE); STASCH, Johannes-Peter, D-42651 Solingen (DE); WUNDER, Frank, D-42115 Wuppertal (DE); DEMBOWSKY, Klaus, Boston, MA 02116 (US); PERZBORN, Elisabeth, D-42327 Wuppertal (DE); STAHL, Elke, D-51467 Bergisch Gladbach (DE)
(86) Internationale Anmeldenummer: PCT/EP2000/008469
(87) Internationale Veröffentlichungsnummer: WO 2001/019780

(56) Entgegenhaltungen:
- EP-A- 0 053 434
- DE-A- 19 642 255
- US-A- 3 646 145
- US-A- 4 154 837
- US-A- 4 483 867
- US-A- 4 629 737

## Beschreibung

Die vorliegende Erfindung betrifft neue chemische Verbindungen, welche die lösliche Guanylatcyclase auch über einen neuartigen, ohne Beteiligung der Häm-Gruppe des Enzyms verlaufenden Wirkmechanismus stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodoniumhexafluorophosphat (Pettibone et al., Eur. J. Pharmacol. 116 (1985), 307), Isoliquiritigenin (Yu et al., Brit J. Pharmacol. 114 (1995), 1587), sowie verschiedene substituierte Pyrazolderivate (WO 98/16223, WO 98/16507 und WO 98/23619).

Die bisher bekannten Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung (Gerzer et al, FEBS Lett. 132(1981), 71), oder über einen Häm-abhängigen Mechanismus, der unabhängig von NO ist, aber zu einer Potenzierung der stimulierenden Wirkung von NO oder CO führt (z.B. YC-1, Hoenicka et al., J. Mol. Med. (1999) 14; oder die in der WO 98/16223, WO 98/16507 und WO 98/23619 beschriebenen Pyrazolderivate).

Die in der Literatur behauptete stimulierende Wirkung von Isoliquiritigenin und von Fettsäuren, wie z. B. Arachidonsäure, Prostaglandinendoperoxide und Fettsäurehydroperoxide auf die lösliche Guanylatcyclase konnte nicht bestätigt werden (vgl. z.B Hoenicka et al., J. Mol. Med. 77 (1999), 14).

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben (Ignarro et al., Adv. Pharmacol. 26 (1994), 35). Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms fuhren dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird (Mülsch et al., Naunyn Schmiedebergs Arch. Pharmacol. 355, R47).

Bislang wurden somit keine Verbindungen beschrieben, welche die lösliche Guanylatcyclase unabhängig von der im Enzym befindlichen Häm-Gruppe stimulieren können.

Es war die Aufgabe der vorliegenden Erfindung, Arzneimittel zur Behandlung von Herz-Kreislauf erkrankungen oder anderen über eine Beeinflussung des cGMP-Signalweges in Organismen therapierbaren Erkrankungen zu entwickeln.

Überraschend wurde gefunden, dass es Verbindungen gibt, welche die lösliche Guanylatcyclase auch unabhängig von der im Enzym befindlichen Häm-Gruppe stimulieren können. Die biologische Aktivität dieser Stimulatoren beruht auf einem völlig neuen Mechanismus der Stimulierung der löslichen Guanylatcyclase. Im Gegensatz zu den vorstehend beschriebenen, aus dem Stand der Technik als Stimulatoren der löslichen Guanylatcyclase bekannten Verbindungen sind die erfindungsgemäßen Verbindungen in der Lage, sowohl die Häm-haltige als auch die Häm-freie Form der löslichen Guanylatcyclase zu stimulieren. Die Stimulierung des Enzyms verläuft bei diesen neuen Stimulatoren also über einen Häm-unabhängigen Weg, was auch dadurch belegt wird, dass die neuen Stimulatoren am Häm-haltigen Enzym einerseits keine synergistische Wirkung mit NO zeigen und andererseits sich die Wirkung dieser neuartigen Stimulatoren nicht durch den Häm-abhängigen Inhibitor der löslichen Guanylatcyclase, 1*H*-1,2,4-Oxadiazol-(4,3a)-chinoxalin-1-on (ODQ), blockieren lässt.

Dies stellt einen neuen Therapieansatz zur Behandlung von Herz-Kreislauferkrankungen und anderen über eine Beeinflussung des cGMP-Signalweges in Organismen therapierbaren Erkrankungen dar.

In der EP-A-0 345 068 ist unter anderem die Aminoalkancarbonsäure (1) als Zwischenprodukt bei der Synthese von GABA-Antagonisten beschrieben:

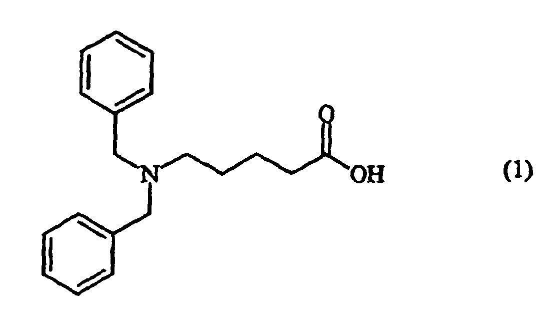

In der WO 93/00359 ist die Aminoalkancarbonsäure (2) als Intermediat in der Peptid-Synthese sowie dessen Verwendung als Wirkstoff zur Behandlung von Erkrankungen des zentralen Nervensystems beschrieben:

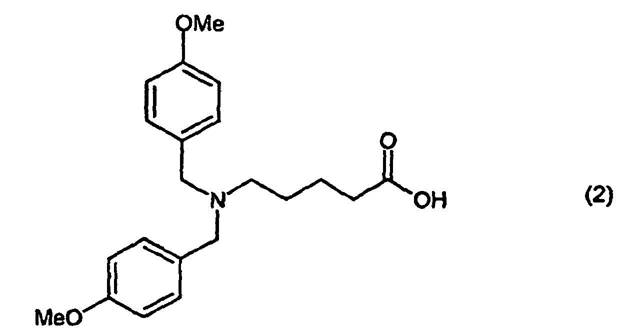

In keiner dieser beiden Schriften ist jedoch beschrieben, dass derartige Aminoalkancarbonsäuren einen von der im Enzym befindlichen Häm-Gruppe unabhängigen stimulierenden Effekt auf die lösliche Guanylatcyclase ausüben können.

Gemäß der vorliegenden Erfindung werden zur von der im Enzym befindlichen Häm-Gruppe unabhängigen Stimulation der löslichen Guanylatcyclase Aminoalkancarbonsäuren der Formel (I) eingesetzt: 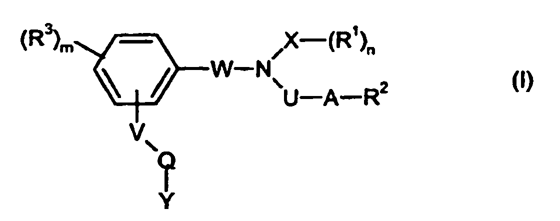 worin
- V: fehlt, O, S oder NR⁴ bedeutet,
worin
R⁴ Wasserstoff oder Methyl bedeutet,
- Q: fehlt, geradkettiges oder verzweigtes Alkylen mit bis zu 9 Kohlenstoffatomen oder geradkettiges oder verzweigtes Alkendiyl oder geradkettiges oder verzweigtes Alkindiyl mit bis zu 4 Kohlenstoffatomen bedeutet, die einfach durch Halogen substituiert sein können,
- Y: H, NR⁸R⁹, Cyclohexyl, Phenyl, Naphthyl oder einen Heterocyclus aus der Gruppe 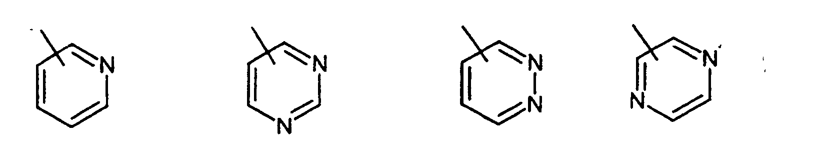 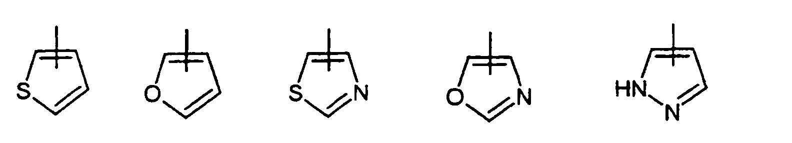 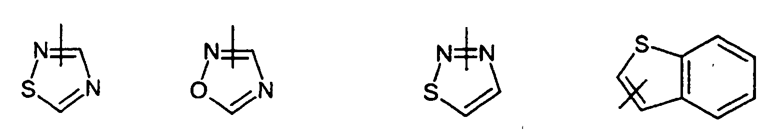 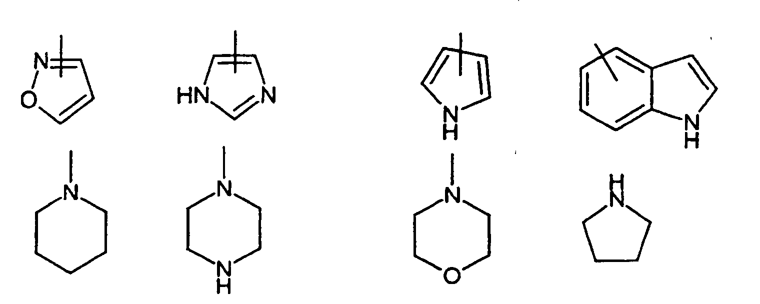 bedeutet, die auch über N gebunden sein können,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Alkoxyalkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 6 Kohlenstoffatomen, F, Cl, Br, I, NO₂, SR⁶, NR⁸R⁹, NR⁷COR¹⁰ oder CONR¹¹R¹² substituiert sein können,
worin
R⁶ Wasserstoff, geradkettiges oder verzweigtes Alkyl mit bis zu 8 Kohlenstoffatomen, oder geradkettiges oder verzweigtes Halogenalkyl mit bis zu 4 Kohlenstoffatomen bedeutet,
R⁷ Wasserstoff, oder geradkettiges oder verzweigtes Alkyl mit bis zu 4 Kohlenstoffatomen bedeutet,
R⁸, R⁹, R¹¹ und R¹² unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann,
oder zwei Substituenten aus R⁸ und R⁹ oder R¹¹ und R¹² miteinander unter Bildung eines fünf- oder sechsgliedrigen Ring verbunden sein können, der durch O oder N unterbrochen sein kann,
R¹⁰ Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann; und/oder die cyclischen Reste jeweils ein- bis dreifach durch Phenyl oder einen Heterocyclus aus der Gruppe 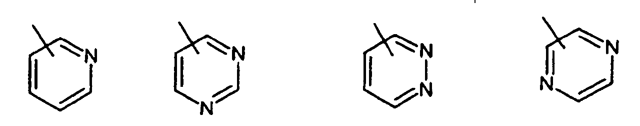 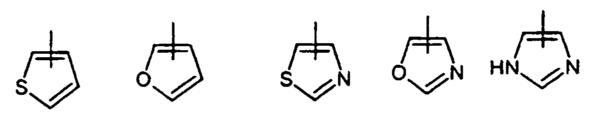 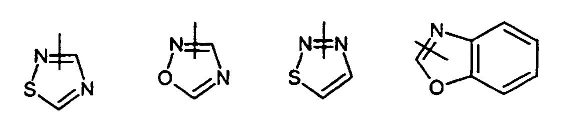 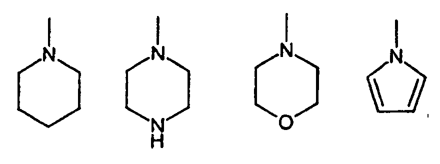 substituiert sein können,
welche direkt oder über eine Gruppe aus O, S, SO, SO₂, NR⁴, SO₂NR⁷, CONR⁷, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden sein können und ein- bis dreifach durch geradkettiges oder verzweigtes Alkyl, geradkettiges oder verzweigtes Alkoxy, geradkettiges oder verzweigtes Alkoxyalkoxy, geradkettiges oder verzweigtes Halogenalkyl oder geradkettiges oder verzweigtes Alkenyl mit jeweils bis zu 4 Kohlenstoffatomen, F, Cl, Br, I, CN, SCH₃, OCF₃, NO₂, NR⁸R⁹ oder NR¹⁴COR¹⁷ substituiert sein können,
worin
R¹⁴ Wasserstoff, geradkettiges oder verzweigtes Alkyl mit bis zu 8 Kohlenstoffatomen oder Cycloalkyl mit 3 bis 8 Kohlenstoffatomen bedeutet, und
R¹⁷ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylammo, NO₂, CF₃, OCF₃ oder CN substituiert sein können; und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
- R³: Wasserstoff oder Fluor bedeutet,
- m: eine ganze Zahl von 1 bis 4 bedeutet,
- W: CH₂, -CH₂CH₂-, CH₂CH₂CH₂, CH=CHCH₂ bedeutet,
- U: -CH₂- bedeutet,
- A: Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
- R²: COOR²⁴ bedeutet,
worin
R²⁴ Wasserstoff oder geradkettiges oder verzweigtes Alkyl mit bis zu 4 Kohlenstoffatomen bedeutet,
- X: geradkettiges oder verzweigtes Alkylen mit bis zu 8 Kohlenstoff atomen oder geradkettiges oder verzweigtes Alkendiyl mit bis zu 8 Kohlenstoffatomen bedeutet, die jeweils eine bis drei Gruppen aus Phenyl, Phenyloxy, O CO oder CONR³⁰ enthalten können,
worin
R³⁰ Wasserstoff, geradkettiges oder verzweigtes Alkyl mit bis zu 6 Kohlenstoffatomen oder Cycloalkyl mit 3 bis 6 Kohlenstoffatomen bedeutet,
- n: 1 oder 2 bedeutet;
- R¹: COOR³⁵ bedeutet,
worin
R³⁵ Wasserstoff oder geradkettiges oder verzweigtes Alkyl mit bis zu 6 Kohlenstoffatomen bedeutet.

Bevorzugt sind hierbei Verbindungen der Formel (I),
worin
- V: O bedeutet,
- Q: geradkettiges oder verzweigtes Alkylen mit bis zu 9 Kohlenstoffatomen oder geradkettiges oder verzweigtes Alkendiyl oder geradkettiges oder verzweigtes Alkindiyl mit bis zu 4 Kohlenstoffatomen bedeutet, die einfach durch Halogen substituiert sein können,
- Y: H, Cyclohexyl, Phenyl oder einen Heterocyclus aus der Gruppe 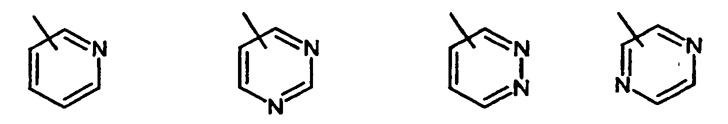 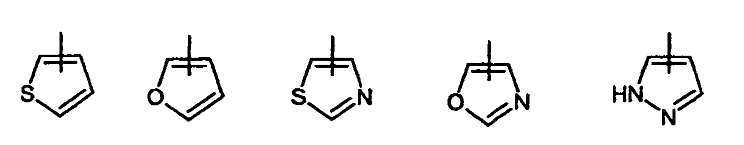 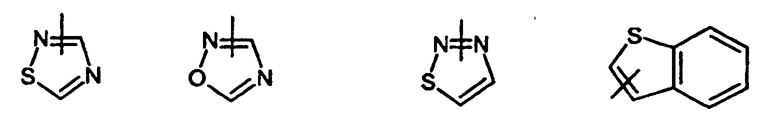 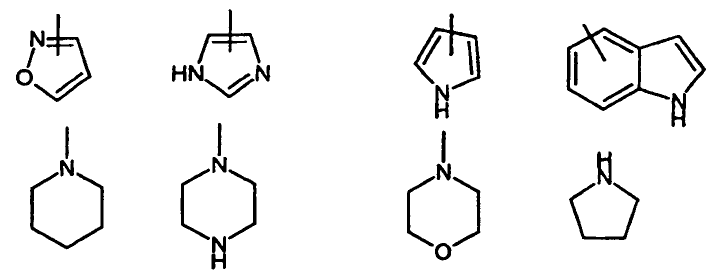 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Alkoxyalkory, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 6 Kohlenstoffatomen, F, Cl, Br, I, NO₂, SR⁶, NR⁸R⁹, NR⁷COR¹⁰ oder CONR¹¹R¹² substituiert sein können,
worin
R⁶ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁷ Wasserstoff, oder geradkettiges oder verzweigtes Alkyl mit bis zu 4 Kohlenstoffatomen bedeutet,
R⁸, R⁹, R¹¹ und R¹² unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s- Butyl, i- Butyl, t-Butyl, Methoxy, Ethoxy, Amino, Acetylamino, NO₂, CF₃, OCF₃ oder CN substituiert sein kann,
oder zwei Substituenten aus R⁸ und R⁹ oder R¹¹ und R¹² miteinander unter Bildung eines fünf- oder sechsgliedrigen Ring verbunden sein können, der durch O oder N unterbrochen sein kann,
R¹⁰ Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylammo, NO₂, CF₃, OCF₃ oder CN substituiert sein kann; und/oder die cyclischen Reste jeweils ein- bis dreifach durch Phenyl oder einen Heterocyclus aus der Gruppe 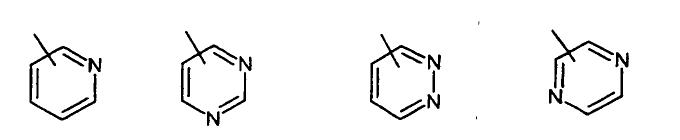 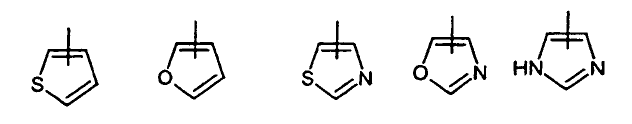 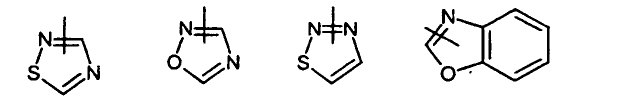 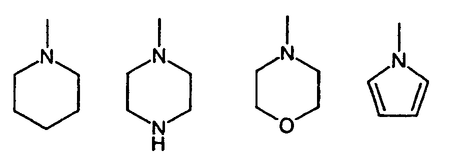 substituiert sein können,
welche direkt oder über eine Gruppe aus O, S, SO, SO₂, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden sein können und ein- bis dreifach durch geradkettiges oder verzweigtes Alkyl, geradkettiges oder verzweigtes Alkoxy, geradkettiges oder verzweigtes Alkoxyalkoxy, geradkettiges oder verzweigtes Halogenalkyl oder geradkettiges oder verzweigtes Alkenyl mit jeweils bis zu 4 Kohlenstoffatomen, F, Cl, Br, I, CN, SCH₃, OCF₃, NO₂, NR⁸R⁹ oder NR¹⁴COR¹⁷ substituiert sein können,
worin
R¹⁴ Wasserstoff, geradkettiges oder verzweigtes Alkyl mit bis zu 6 Kohlenstoffatomen oder Cycloalkyl mit 3 bis 6 Kohlenstoffatomen bedeutet, und
R¹⁷ Wasserstoff, geradkettiges oder verzweigtes Alkyl mit bis zu 6 Kohlenstoffatomen, geradkettiges oder verzweigtes Alkenyl mit bis zu 6 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 6 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können; und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
- R³: Wasserstoff oder Fluor bedeutet,
- m: eine ganze Zahl von 1 bis 2 bedeutet,
- W: -CH₂- oder -CH₂CH₂- bedeutet,
- U: -CH₂- bedeutet,
- A: Phenyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
- R²: COOR²⁴ bedeutet,
worin
R²⁴ Wasserstoff oder geradkettiges oder verzweigtes Alkyl mit bis zu 4 Kohlenstoffatomen bedeutet,
- X: geradkettiges oder verzweigtes Alkylen mit bis zu 6 Kohlenstoffatomen oder geradkettiges oder verzweigtes Alkendiyl mit bis zu 6 Kohlenstoffatomen bedeutet, die jeweils eine bis drei Gruppen aus Phenyloxy, O, CO oder CONR³⁰ enthalten können,
worin
R³⁰ Wasserstoff, geradkettiges oder verzweigtes Alkyl mit bis zu 6 Kohlenstoffatomen oder Cycloalkyl mit 3 bis 6 Kohlenstoffatomen bedeutet,
- n: 1 oder 2 bedeutet;
- R¹: COOR³⁵ bedeutet,
worin
R³⁵ Wasserstoff oder geradkettiges oder verzweigtes Alkyl mit bis zu 4 Kohlenstoffatomen bedeutet.

Erfindungsgemäß besonders bevorzugt sind Verbindungen der Formel (I), bei denen R¹ und R² jeweils COOH bedeuten.

Besonders bevorzugt sind gemäß der vorliegenden Erfindung Verbindungen, bei denen
- V: O bedeutet,
- Q: CH₂ bedeutet,
- Y: Phenyl bedeutet, das mit einem Rest substituiert ist, der aus der Gruppe, bestehend aus 2-Phenylethyl, Cyclohexyl, 4-Chlorphenyl, 4-Methoxyphenyl, 4-Trifluormethylphenyl, 4-Cyanophenyl, 4-Chlorphenoxy, 4-Methoxyphenoxy, 4-Trifluormethylphenoxy, 4-Cyanophenoxy, 4-Methylphenyl ausgewählt ist,
- R³: Wasserstoff oder Fluor bedeutet,
- m: eine ganze Zahl von 1 bis 2 bedeutet,
- W: -CH₂CH₂- bedeutet,
- U: -CH₂- bedeutet,
- A: Phenyl bedeutet,
- R²: COOH bedeutet, wobei R² in 4-Position zum Rest U angeordnet ist,
- X: (CH₂)₄ bedeutet,
- R¹: COOH bedeutet.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammomumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamm, Di- bzw. Triethanolamin, Dicyclohexylamin, Dimethylaminoethanol, Arginin, Lysin oder Ethylendiamm.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch Racematspaltung oder chromatographische Trennung, in die stereoisomer einheitlichen Bestandteile trennen. In den erfindungsgemäßen Verbindungen vorhandene Doppelbindungen können in der cis- oder trans- Konfiguration (Z- oder E-Form) vorliegen.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.
Alkylen steht im allgemeinen für eine geradkettige oder verzweigte Kohlenwasserstoffbrücke mit 1 bis 20 Kohlenstoffatomen. Beispielsweise seien Methylen, Ethylen, Propylen, α-Methylethylen, β-Methylethylen, α-Ethylethylen, β-Ethylethylen, Butylen, α-Methylpropylen, β-Methylpropylen, γ-Methylpropylen, α-Ethylpropylen, β-Ethylpropylen, γ-Ethylpropylen, Pentylen, Hexylen, Heptylen, Octylen, Nonylen, Decylen, Dodeylen und Eicosylen genannt.
Alkenyl steht im allgemeinen für einen geradkettigen oder verzweigten Kohlenwasserstoffrest mit 2 bis 20 Kohlenstoffatomen und einer oder mehreren, bevorzugt mit einer oder zwei Doppelbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl, Isooctenyl genannt.
Alkinyl steht im allgemeinen für einen geradkettigen oder verzweigten Kohlenwasserstoffrest mit 2 bis 20 Kohlenstoffatomen und einer oder mehreren, bevorzugt mit einer oder zwei Dreifachbindungen. Beispielsweise seien Ethinyl, 2-Butinyl, 2-Pentinyl und 2-Hexinyl benannt.
Alkendiyl steht im allgemeinen für eine geradkettige oder verzweigte Kohlenwasserstoffbrücke mit 2 bis 20 Kohlenstoffatomen und einer oder mehreren, bevorzugt mit einer oder zwei Doppelbindungen. Beispielsweise seien Ethen-1,2-diyl, Propen-1,3-diyl, Propen-1,2-diyl, 1-Buten-1,4-diyl, 1-Buten-1,3-diyl, 1-Buten-1,2-diyl, 2-Buten-1,4-diyl, 2-Buten-1,3-diyl, 2-Buten-2,3-diyl genannt.
Alkindiyl steht im allgemeinen für eine geradkettige oder verzweigte Kohlenwasserstoffbrücke mit 2 bis 20 Kohlenstoffatomen und einer oder mehreren, bevorzugt mit einer oder zwei Dreifachbindungen. Beispielsweise seien Ethin-1,2-diyl, Propin-1,3-diyl, 1-Butin-1,4-diyl, 1-Butin-1,3-diyl, 2-Buten-1,4-diyl genannt.
Acyl steht im allgemeinen für geradkettiges oder verzweigtes Niedrigalkyl mit 1 bis 9 Kohlenstoffatomen, das über eine Carbonylgruppe gebunden ist. Beispielsweise seien genannt: Acetyl, Ethylcarbonyl, Propylcarbonyl, Isopropylcarbonyl, Butylcarbonyl und Isobutylcarbonyl.
Alkoxy steht im allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.
Alkoxyalkyl steht im allgemeinen für einen Alkylrest mit bis zu 8 Kohlenstoffatomen, der durch einen Alkoxyrest mit bis zu 8 Kohlenstoffatomen substituiert ist.
Alkoxycarbonyl kann beispielsweise durch die Formel 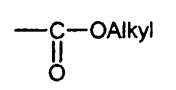 dargestellt werden.
Alkyl steht hierbei im allgemeinen für einen geradkettigen oder verzweigten Kohlenwasserstoffrest mit 1 bis 13 Kohlenstoffatomen. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.
Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Cycloalkoxy steht im Rahmen der Erfindung für einen Alkoxyrest, dessen Kohlenwasserstoffrest ein Cycloalkylrest ist. Der Cycloalkylrest hat im allgemeinen bis zu 8 Kohlenstoffatome. Als Beispiele seien genannt: Cyclopropyloxy und Cyclohexyloxy. Die Begriffe "Cycloalkoxy" und "Cycloalkyloxy" werden synonym verwendet.
Aryl steht im allgemeinen für einen aromatischen Rest mit 6 bis 10 Kohlenstoffatomen. Bevorzugte Arylreste sind Phenyl und Naphthyl.
Halogen steht im Rahmen der Erfindung für Fluor, Chlor, Brom und Iod.
Heterocyclus steht im Rahmen der Erfindung im allgemeinen für einen gesättigten, ungesättigten oder aromatischen 3- bis 10-gliedrigen, beispielsweise 5- oder 6-gliedrigen Heterocyclus, der bis zu 3 Heteroatome aus der Reihe S, N und/oder O enthalten kann und der im Fall eines Stickstoffatoms auch über dieses gebunden sein kann. Beispielsweise seien genannt: Oxadiazolyl, Thiadiazolyl, Pyrazolyl, Pyridyl, Pyrimidinyl, Pyridazinyl, Pyrazinyl, Thienyl, Furyl, Pyrrolyl, Pyrrolidinyl, Piperazinyl, Tetrahydropyranyl, Tetrahydrofuranyl, 1,2,3 Triazolyl, Thiazolyl, Oxazolyl, Imidazolyl, Morpholinyl oder Pipendyl. Bevorzugt sind Thiazolyl, Furyl, Oxazolyl, Pyrazolyl, Triazolyl, Pyridyl, Pyrimidinyl, Pyridazinyl und Tetrahydropyranyl Der Begriff "Heteroaryl" (bzw. "Hetaryl") steht für einen aromatischen heterocyclischen Rest.

Bei den in der vorliegenden Anmeldung gezeigten Heterocyclenstrukturen ist jeweils nur eine Bindung zur benachbarten Gruppe angedeutet, z.B. bei den Heterocyclenstrukturen, die für Y in Frage kommen, die Bindung zur Einheit Q. Unabhängig davon können diese Heterocyclenstrukturen jedoch wie angegeben weitere Substituenten tragen.
Die vorliegende Erfindung betrifft weiterhin ein Verfahren zur Herstellung der Verbindungen der Formel (I), dadurch gekennzeichnet, dass man
[A] Verbindungen der Formel (II) 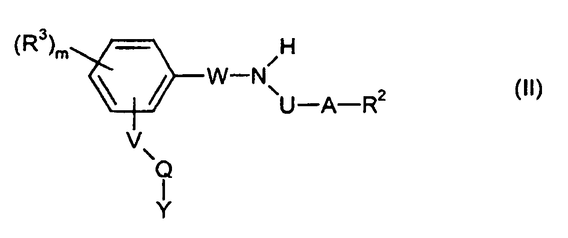 mit Verbindungen der Formel (III)

   E-X-R¹ (III)

   umsetzt,
   worin
   - R¹, R², R³, V, Q, Y, W, X, U, A und m: die gleichen Bedeutungen wie vorstehend definiert haben,
   - E: entweder eine Abgangsgruppe bedeutet, die in Gegenwart einer Base substituiert wird, oder eine gegebenenfalls aktivierte Hydroxyfunktion ist;
   oder
[B] Verbindungen der Formel (IV) 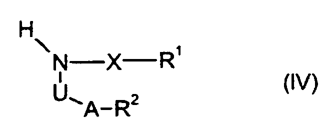 mit Verbindungen der Formel (V) 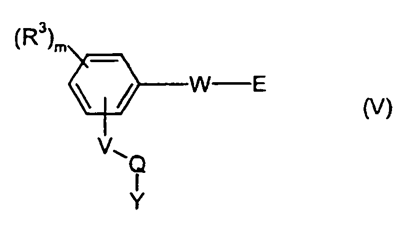 umsetzt,
   worin
   - R¹, R², R³, V, Q, Y, W, X, U, A und m: die gleichen Bedeutungen wie vorstehend definiert haben,
   - E: entweder eine Abgangsgruppe bedeutet, die in Gegenwart einer Base substituiert wird, oder eine gegebenenfalls aktivierte Hydroxyfunktion ist;
   oder
[C] Verbindungen der Formel (VI) 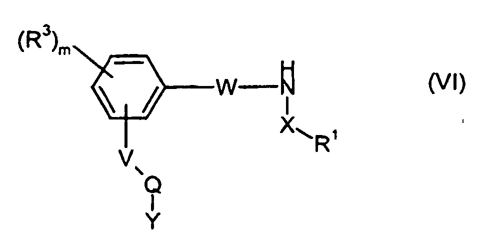 mit Verbindungen der Formel (VII)

   E-U-A-R² (VII)

   umsetzt,
   worin
   - R¹, R², R³, V, Q, Y, W, X, U, A und m: die gleichen Bedeutungen wie vorstehend definiert haben,
   - E: entweder eine Abgangsgruppe bedeutet, die in Gegenwart einer Base substituiert wird, oder eine gegebenenfalls aktivierte Hydroxyfunktion ist;
   oder
[D] Verbindungen der Formel (VIII), 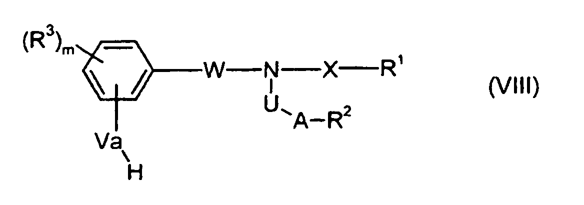 worin
   - Va: für O oder S steht und
   - R¹, R², R³, Y,Q,W,U, A, X und m: die vorstehend angegebene Bedeutung haben
   mit Verbindungen der Formel (IX) 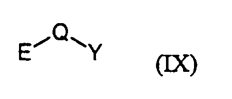 umsetzt,
   worin
   - Q, Y: die gleichen Bedeutungen wie vorstehend definiert haben,
   - E: entweder eine Abgangsgruppe bedeutet, die in Gegenwart einer Base substituiert wird, oder eine gegebenenfalls aktivierte Hydroxyfunktion ist;
   oder
[E] Verbindungen der Formel (X), 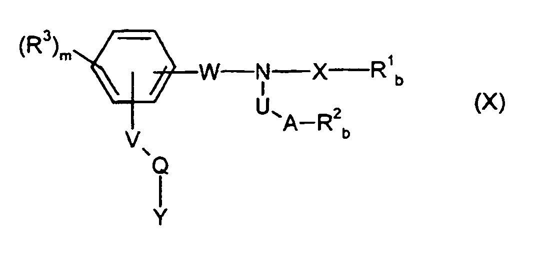 worin
   - R³, V, Q, Y, W, X, U, A und m: die gleichen Bedeutungen wie vorstehend definiert haben,
   - R¹ _{b} und R² _{b}: jeweils unabhängig für CN oder COOAlk stehen, wobei Alk für einen geradkettigen oder verzweigten Alkylrest mit bis zu 6 Kohlenstoffatomen steht,
   mit wässrigen Lösungen starker Säuren oder starker Basen in die entsprechenden freien Carbonsäuren überführt.
   oder
[F] Verbindungen der Formel (XI) 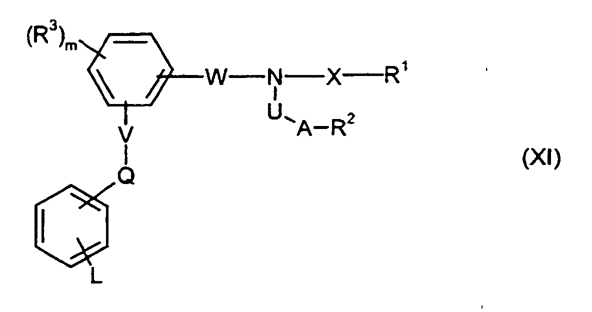 worin
   - R¹, R², R³, V, Q, X, W, U, A und m: die gleichen Bedeutungen wie vorstehend definiert haben,
   - L: für Br, I oder die Gruppe CF₃SO₂-O steht,
   mit Verbindungen der Formel (XII)

   M-Z (XII)

   worin
   - M: für einen Aryl oder Heteroarylrest, einen geradkettigen oder verzweigten Alkyl-, Alkenyl- oder Alkinylrest oder Cycloalkylrest oder für einen Arylalkyl, einen Arylalkenyl- oder einen Arylalkinylrest steht,
   - Z: für die Gruppierungen -B(OH)₂, -CH≡CH, -CH=CH₂ oder -Sn(nBu)₃ steht
   in Gegenwart einer Palladiumverbindung, gegebenenfalls zusätzlich in Gegenwart eines Reduktionsmittels und weiterer Zusatzstoffe und in Gegenwart einer Base umsetzt;
   oder
[G] Verbindungen der Formel (XIII) 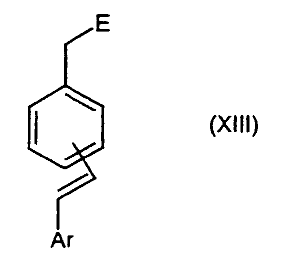 worin
   - Ar: für einen Aryl oder Heteroarylrest steht,
   - E: eine Abgangsgruppe bedeutet, die in Gegenwart einer Base substituiert wird.
   nach Verfahren D mit Verbindungen der Formel (VIII) umsetzt und die so erhaltenen Verbindungen der Formel (XIV) 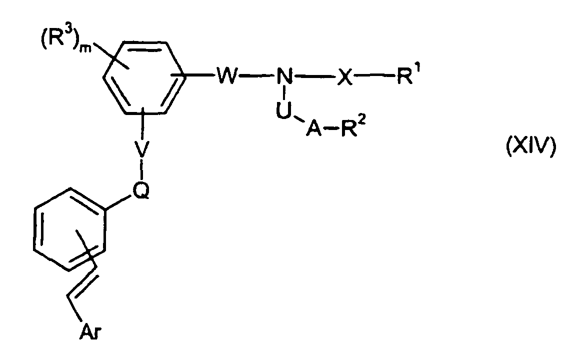 mit Wasserstoff in Gegenwart eines Katalysators hydriert.

Die erfindungsgemäßen Verfahren zur Herstellung von Verbindungen der Formel (I) werden nachstehend anhand beispielhafter, nicht einschränkender Ausführungsformen veranschaulicht:

### Beispiel für Reaktionssequenz nach Verfahren A /E:

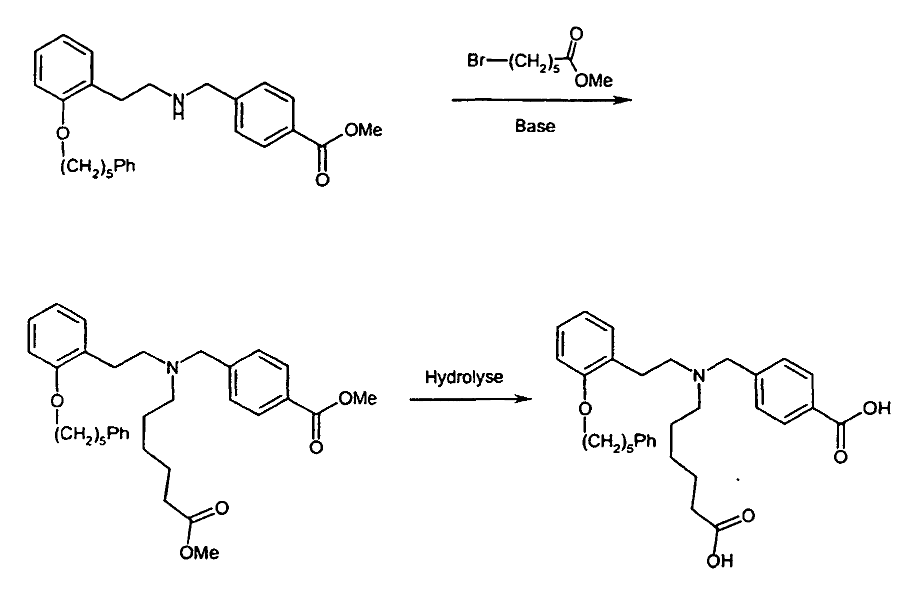

Steht (VIII) beispielsweise für Methyl 4-{[(2-methoxyphenethyl)amino]methyl}benzoat und (IX) für 2-Chlorphenylmethylchlorid, so lassen sich Verfahren D bzw. E wie im folgenden Schema gezeigt darstellen.

### Beispiel für Reaktionssequenz nach Verfahren D/E:

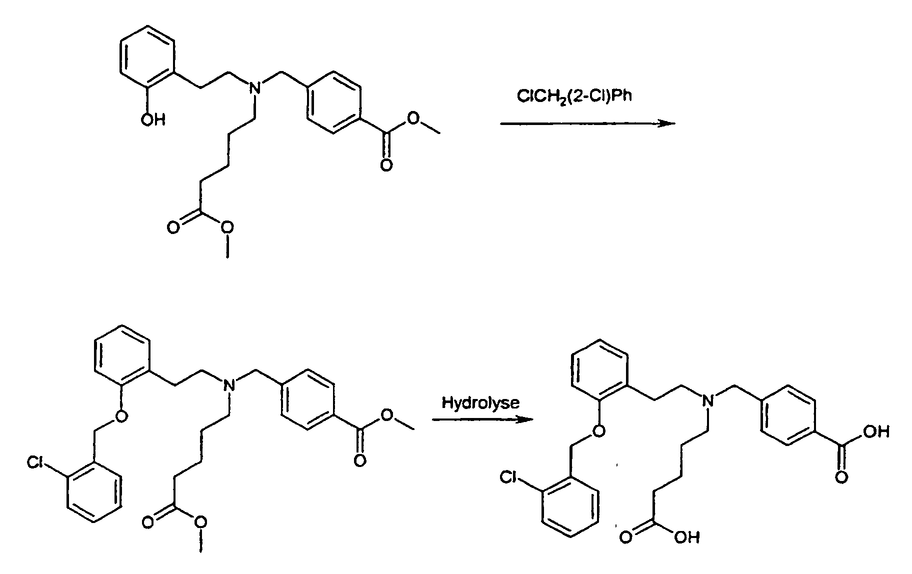

Steht (IV) beispielsweise für Methyl 4-{[(5-methoxy-5-oxopentyl)amino]methyl}benzoat und (V) für 1-[2-(benzyloxy)phenyl]-2-bromo-1-ethanon, so lassen sich Verfahren B bzw. E wie im folgenden Schema gezeigt darstellen:

### Beispiel für Reaktionssequenz nach Verfahren B/E:

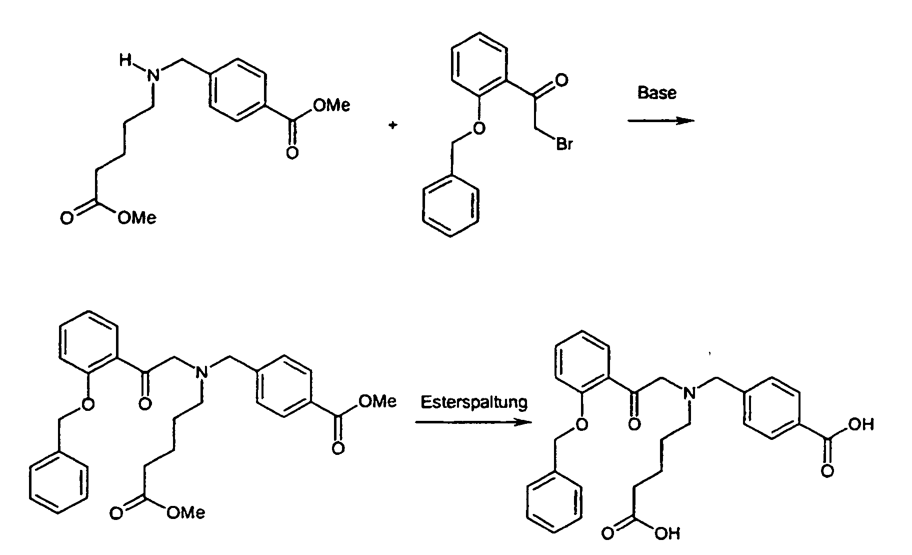

Steht (VI) beispielsweise für Methyl 5-{[2-(benzyloxy)phenethyl]amino}pentanoat und (VII) für Methyl 4-(bromomethyl)benzoat, so lassen sich Verfahren C bzw. E wie im folgenden Schema gezeigt darstellen:

### Beispiel für Reaktionssequenz nach Verfahren C/E:

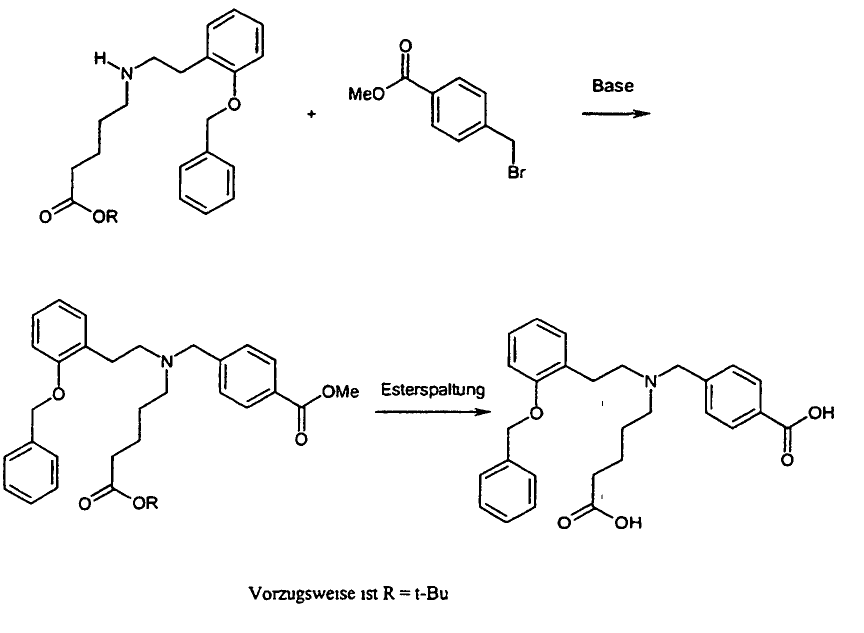

### Beispiel für Reaktionssequenz nach Verfahren D/F/E

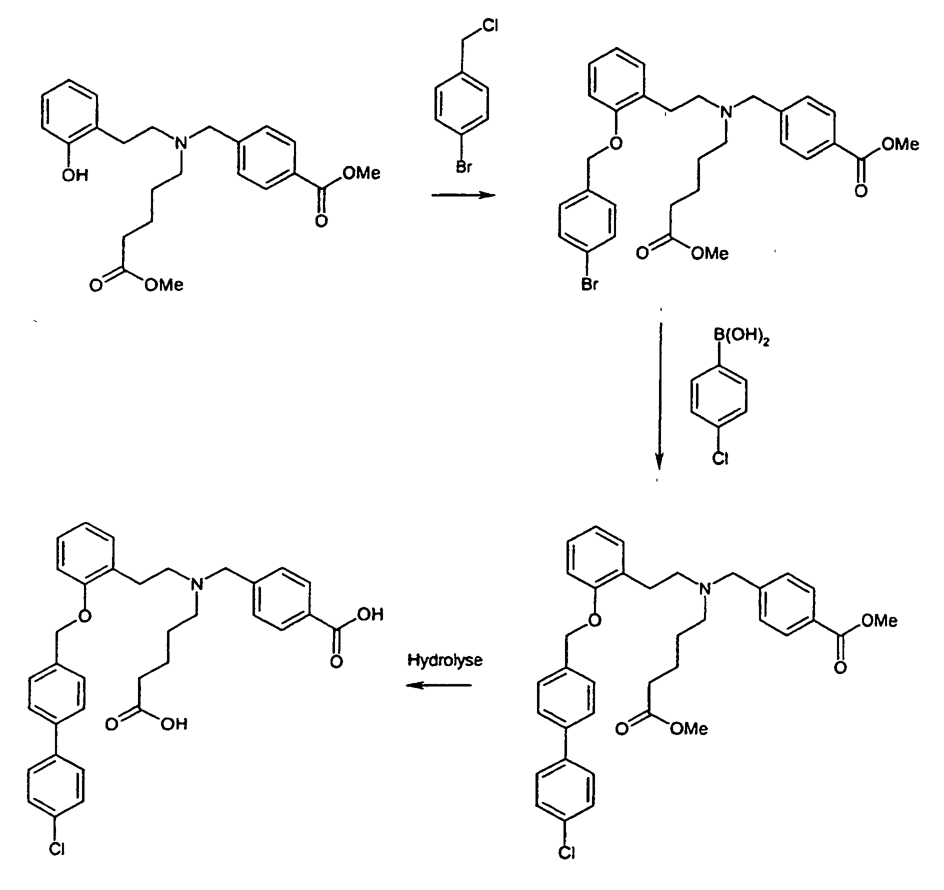

### Beispiel für Reaktionssequenz nach Verfahren D/G/E

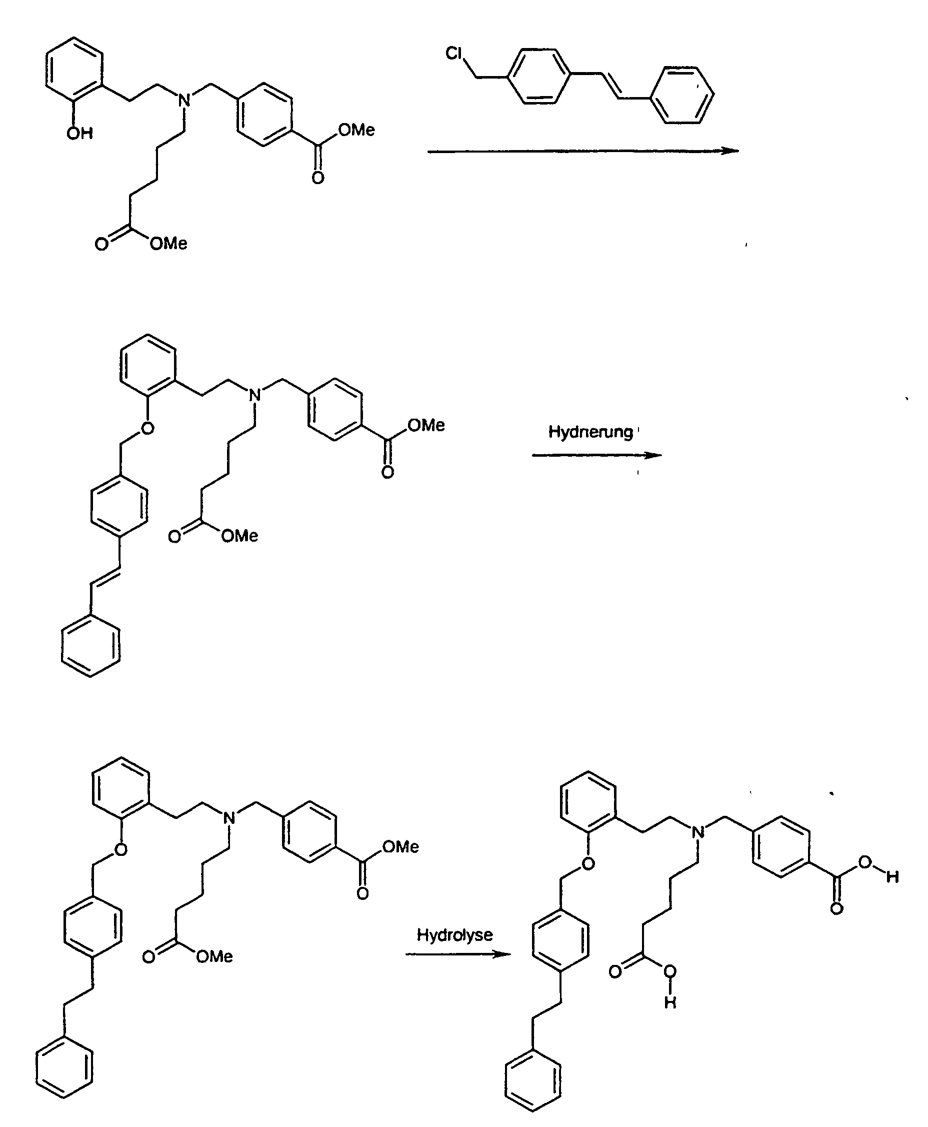

Für die erfindungsgemäßen Verfahren bevorzugte Lösungsmittel sind herkömmliche organische Lösungsmittel, welche sich unter den Reaktionsbedingungen nicht verändern, oder Wasser. Vorzugsweise können für das erfindungsgemäße Verfahren Ether wie Diethylether, Butylmethylether, Dioxan, Tetrahydrofuran, Glykoldimethylether oder Diethylenglykoldimethylether, oder Kohlenwasserstoffe wie Benzol, Toluol, Xylol oder Petrolether, oder Amide wie Dimethylformamid oder Hexamethylphosphortriamid, oder 1,3-Dimethyl-imidazolidin-2-on, 1,3-Dimethyl-tetrahydropyrimidin-2-on, Acetonitril, Essigsäureethylester oder Dimethylsulfoxid verwendet werden. Es ist selbstverständlich auch möglich, Gemische der vorstehend genannten Lösungsmittel zu verwenden.

Die für die erfindungsgemäßen Verfahren bevorzugten Basen umfassen herkömmlicherweise für basische Reaktionen eingesetzte basische Verbindungen. Vorzugsweise können Alkalimetallhydride wie beispielsweise Natriumhydrid oder Kaliumhydrid, oder Alkalimetallalkoholate wie Natriummethanolat, Natriumethanolat, Kaliummethanolat, Kaliumethanolat oder Kalium-t.-butylat, oder Carbonate wie Natriumcarbonat, Cäsiumcarbonat oder Kaliumcarbonat oder Amide wie Natriumamid oder Lithiumdiisopropylamid, oder Organolithium-Verbindungen wie Phenyllithium, Butyllithium oder Methyllithium oder Natriumhexamethyldisilazan verwendet werden.

Die erfindungsgemäßen Verfahren A bis C können vorzugsweise in Acetonitril jeweils durch Reaktion der Verbindungen (II) und (III), (IV) und (V) beziehungsweise (VI) und (VII) m Gegenwart einer Base wie Natriumcarbonat, Et₃N, DABCO, K₂CO₃, KOH, NaOH oder NaH durchgeführt werden. Die Reaktion kann im allgemeinen in einem Temperaturbereich von -20°C bis +90°C, vorzugsweise von 0°C bis +70°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Bei den erfindungsgemäßen Verfahren A bis C wird eine Verbindung der Formel (I) durch nukleophile Substitution einer Abgangsgruppe E in einer der Verbindungen der Formel (III), (V) oder (VII) durch die Aminfunktion einer der Verbindungen der Formel (II), (IV) oder (VI) dargestellt. Als Abgangsgruppen E kommen hierbei beispielsweise in Frage: Halogen, Tosylat, Mesylat, oder eine durch Reagenzien wie Diisopropylazodicarboxylat/PPh₃ aktivierte Hydroxyfunktion (Mitsonobu-Reaktion). Das erfindungsgemäße Verfahren D kann vorzugsweise in Acetonitril durch Reaktion der Verbindungen (VIII) und (IX) in Gegenwart einer Base wie Natriumcarbonat, Kaliumcarbonat, Et₃N, DABCO, K₂CO₃, KOH, NaOH oder NaH durchgeführt werden. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Beim erfindungsgemäßen Verfahren D wird eine Verbindung der Formel (I) durch nukleophile Substitution einer Abgangsgruppe E in der Verbindung der Formel (IX) durch die Hydroxy- oder Thiolfunktion der Verbindung der Formel (VIII) dargestellt. Als Abgangsgruppen E kommen hierbei beispielsweise in Frage: Halogen, Tosylat, Mesylat, oder eine durch Reagenzien wie Diisopropylazodicarboxylat/PPh₃ aktivierte Hydroxyfunktion (Mitsonobu-Reaktion).

Beim erfindungsgemäßen Verfahren E wird eine Verbindung der Formel (I), bei der R¹ und R² jeweils für eine freie Carboxylfunktion stehen, durch Überführung von Ester- und/oder Nitrilfunktionen der Verbindung (X) in die entsprechenden freien Carboxylfunktionen erhalten. Diese Reaktion kann beispielsweise durch Zugabe wässriger Lösungen starker Säuren wie z.B. HCl oder H₂SO₄, oder starker Basen wie z.B. NaOH, KOH oder LiOH erfolgen. Die Reaktion kann in einem der vorstehend genannten organischen Lösungsmitteln, in Wasser oder in Gemischen aus organischen Lösungsmitteln oder in Gemischen aus organischen Lösungsmitteln mit Wasser durchgeführt werden. Erfindungsgemäß bevorzugt ist beispielsweise die Durchführung der Reaktion in einem Gemisch aus Wasser und Methanol oder Dioxan. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Beim erfindungsgemäßen Verfahren F wird eine Verbindung der Formel (I) durch Umsetzung einer Verbindung der Formel (XI), welche eine substituierbare Gruppe L enthält, mit einer Verbindung der Gruppe (XII) in Gegenwart einer Palladiumverbindung sowie gegebenenfalls eines Reduktionsmittels und weiterer Zusatzstoffe im basischen Medium dargestellt. Die Reaktion stellt formal eine reduktive Kupplung der Verbindungen der Formeln (XI) und (XII) dar, wie sie z.B. in L.S. Hegedus, Organometallics in Synthesis, M. Schlosser, Ed., Wiley & Sons, 1994, beschrieben ist.

Als substituierbare Gruppe L bei den Verbindungen der Formel (XI) kann beispielsweise ein Halogenrest wie Br oder I oder eine herkömmliche Abgangsgruppe wie beispielsweise ein Triflatrest verwendet werden.

Die Verbindungen der Formel (XII) enthalten eine reaktive Gruppe Z, welche aus der Gruppe, bestehend aus -B(OH)₂, -CH≡CH, -CH=CH₂ oder -Sn(nBu)₃, ausgewählt werden kann.

Als Palladiumverbindung kann eine Palladium(II)-Verbindung wie z.B. Cl₂Pd(PPh₃)₂ oder Pd(OAc)₂ oder eine Palladium(0)-Verbindung wie z.B. Pd(PPh₃)₄ oder Pd₂(dba)₃ verwendet werden. Falls erforderlich, können dem Reaktionsgemisch noch zusätzlich ein Reduktionsmittel wie beispielsweise Triphenylphosphin oder andere Zusatzstoffe wie beispielsweise Cu(I)Br, NBu₄NCl, LiCl oder Ag₃PO₄ zugesetzt werden (vgl. hierzu T Jeffery, Tetrahedron lett. 1985, 26, 2667-2670; T. Jeffery, J. Chem. Soc., Chem. Commun. 1984, 1287-1289; S. Bräse, A. deMejiere in "Metalcatalyzied cross-coupling reactions", Ed. F. Diederich, P. J. Stang, Wiley-VCH, Weinheim 1998, 99-166).

Die Reaktion wird m Gegenwart einer herkömmlichen Base wie z.B. Na₂CO₃, NaOH oder Tnethylamin durchgeführt. Als Lösungsmittel kommen die vorstehend genannten organischen Lösungsmittel in Frage, wobei Ether wie beispielsweise Dimethoxyethan besonders bevorzugt sind. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Beim erfindungsgemäßen Verfahren G werden Verbindungen der Formel (I) dadurch erhalten, dass Verbindungen der Formel (XIII), welche eine Abgangsgruppe E enthalten, mit Verbindungen der Formel (VIII) gemäß dem erfindungsgemäßen Verfahren D umsetzt und die so erhaltenen Verbindungen der Formel (XIV) anschließend hydriert.

Der erste Schritt des Verfahrens G verläuft somit analog zum Verfahren D, wobei anstatt der Verbindungen der Formel (IX) hier Verbindungen der Formel (XIII) mit den Alkoholen oder Thiolen der Formel (XIII) umgesetzt werden. Man erhält so die ungesättigten Verbindungen der Formel (XIV), die durch herkömmliche Hydrierungsverfahren in die Verbindungen der Formel (I) überführt werden können.

Erfindungsgemäß bevorzugt ist die Hydrierung der Verbindungen der Formel (XIV) mit Wasserstoff in Gegenwart eines Katalysators wie beispielsweise Pd-Kohle oder PtO.

Das Verfahren G kann m einem der vorstehend genannten organischen Lösungsmittel durchgeführt werden. Bevorzugt ist hierbei Essigsäureethylester. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Die Amine der Formeln II, IV und VI sind neu und ebenfalls Gegenstand der Erfindung.

Die neuen Verbindungen der Formel II, IV und VI können in allgemein bekannter Weise nach folgenden Methoden erhalten werden:
a) durch Umsetzung von Aminen der Formeln (XV), (XVI) und (XVII) 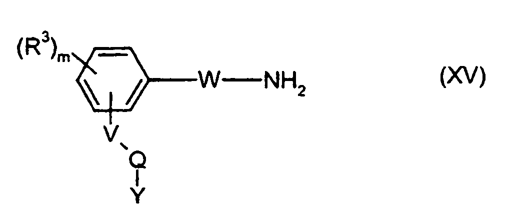 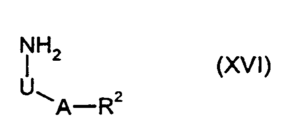 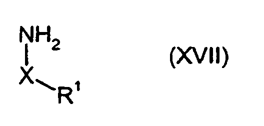 wobei die Reste R¹, R², R³, m, V, Q, U, W, X, Y und A die vorstehend angegebenen Bedeutungen haben;
   mit Carbonylverbindungen der Formeln (XVIII), (XIX), (XX) 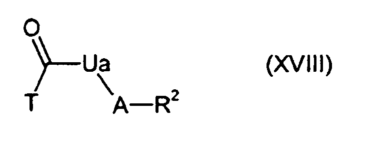 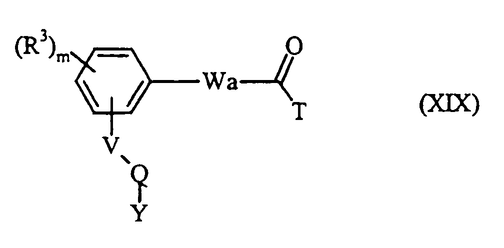 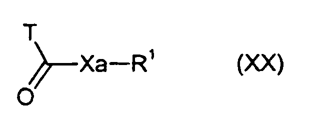 wobei
   - Ua, Wa und Xa: die Bedeutung von U, W und X haben, jedoch um eine Kohlenstoffeinheit verkürzt sind, und
   - T: Wasserstoff oder eine C₁-C₄-Alkylfunktion darstellt, die auch mit Ua oder Xa zu einem Cyclus verbunden sein kann,
   und die anderen Reste wie vorstehend definiert sind,
   zunächst zu einer Schiffschen Base umsetzt und diese dann mit gängigen Reduktionsmitteln, wie z.B. NaBH₄, H₂/Pd/C usw. reduziert oder direkt unter den Bedingungen einer reduktiven Alkylierung in Gegenwart eines Reduktionsmittels, wie z.B. H₂/Pd/C, NaCNBH₃, NaH(OAc)₃ umsetzt (vgl. Patai, Ed., The Chemistry of the Carbon-Nitrogen Double Bond, S. 276-293 und die dort zitierte Literatur);
b) durch Umsetzung von Aminen der Formeln (XV), (XVI) und (XVII) mit Verbindungen der Formeln (III), (V), (VII) (vgl. z.B. J. March, Advanced Organic Chemistry, fourth Edition, Wiley, 1992, Seite 411 bzw. die dort zitierte Literatur).

Amine der Formel (IIa) bzw. Verbindungen der Formel (VIII), 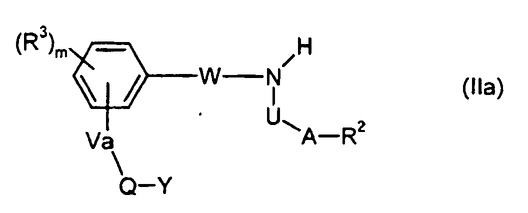 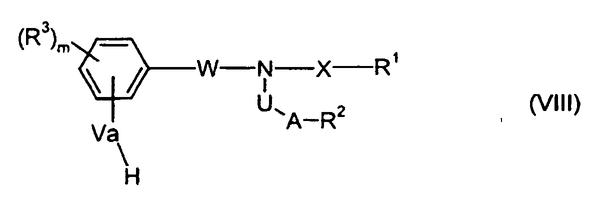 wobei Va für O oder S steht,
können in allgemein bekannter Weise nach folgendem Reaktionsschema erhalten werden:

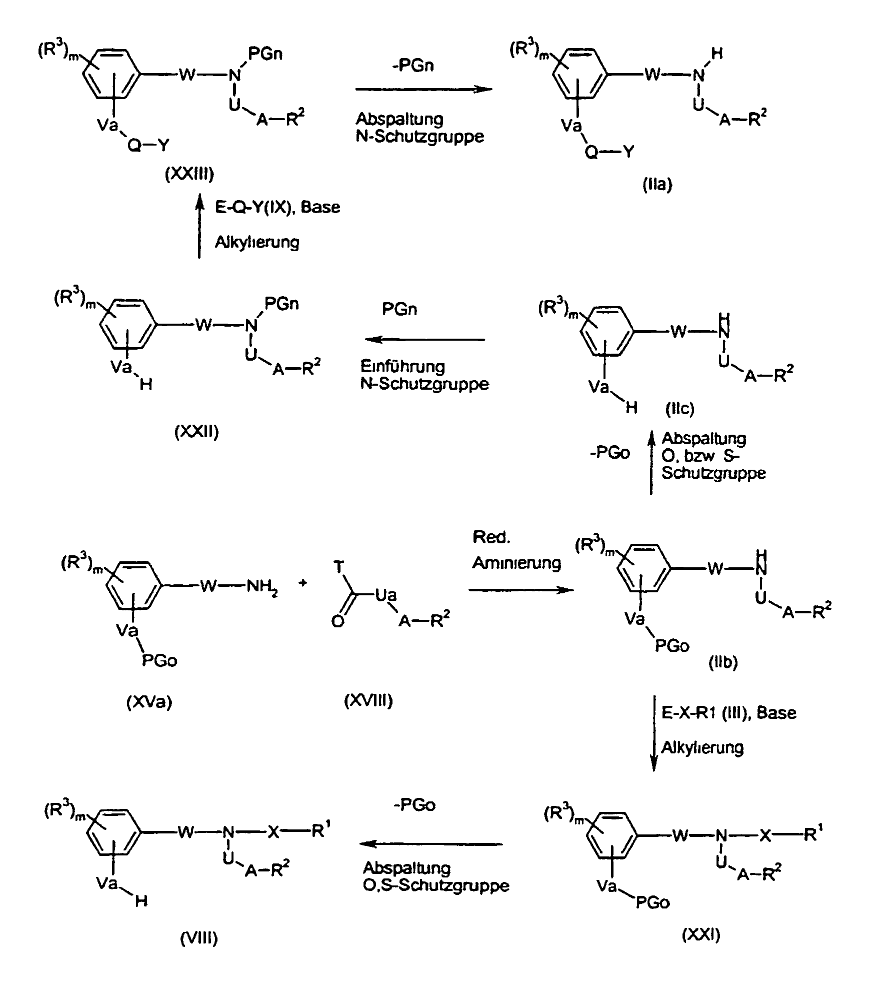

In obigem Schema steht PGo für eine gängige Phenol-, bzw. Thiophenolschutzgruppe, wie z.B CH₃, CH₂Ph, CH₂CH=CH₂, CH₂OCH₃, CH₂OCH₂SiMe₃, SiMe₃, PGn für eine Aminschutzgruppe, wie z.B. tBuOCO, T für Wasserstoff oder eine C₁-C₄-Alkylfunktion, die auch mit Ua zu einem Cyclus verbunden sein kann, und Ua hat die Bedeutung von U, ist jedoch um eine CH2-Gruppe verkürzt. Die anderen Reste sind wie vorstehend definiert.

(IIb) erhält man beispielsweise, indem man zunächst (XVa) mit (XVIII) zu einer Schiffschen Base umsetzt und diese dann mit gängigen Reduktionsmitteln, wie z.B. NaBH₄, H₂/Pd/C usw. reduziert oder direkt unter den Bedingungen emer reduktiven Alkylierung in Gegenwart eines Reduktionsmittels, wie z.B. H₂/Pd/C, NaCNBH₃ oder NaH(OAc)₃ umsetzt. Die Verbindung (IIb) kann durch Umsetzung mit einer Verbindung der Formel (III) in Gegenwart einer Base in eine Verbindung der Formel (XXI) überführt werden (vgl. Verfahren A).

Eine O- bzw. S-Schutzgruppe m (IIb) oder (XXI) kann mit einem geeigneten Reagenz abgespalten werden (vgl. hierzu T.W. Greene, P.G.M. Wuts, Protective Groups in Organic Synthesis, second edition, New York, 1991). Steht beispielsweise in Formel (IIb) oder (XXI)-Va-PGo für -O-CH₃, so lässt sich die Methylgruppe unter Bildung des Phenols durch Bortribromid in Methylenchlorid bei -70 bis 20°C, durch Trimethylsilyliodid in Chloroform bei 25-50°C oder durch Natnumethylthiolat in DMF bei 150°C abspalten.

Eine Verbindung der Formel (XXIII) lässt sich aus der so erhaltenen Verbindung der Formel (IIc) durch Schützen der Aminofunktion (vgl. hierzu T.W. Greene, P.G.M. Wuts, Protective Groups in Orgamc Synthesis, second edition, New York, 1991) und anschließende Umsetzung der so erhaltenen amingeschützten Verbindung der Formel (XXII) mit einer Verbindung der Formel (IX) erhalten (vgl. Verfahren D).

Eine N-Schutzgruppe wie in (XXII) kann nach gängigen Methoden eingeführt und wieder entfernt werden (vgl. hierzu T W. Greene, P.G.M. Wuts, Protective Groups in Organic Synthesis, second edition, New York, 1991). Steht in Formel (XXII) PGn beispielsweise für tBuOCO, so lässt sich die Schutzgruppe durch Reaktion des Amins mit Pyrrokohlensäure-tert. butylester in polaren oder unpolaren Lösungsmitteln bei 0°C bis 25 °C einführen. Die Abspaltung der Schutzgruppe zu (IIa) kann mit zahlreichen Säuren, wie z.B. HCl, H₂SO₄ oder CF₃COOH bei 0° bis 25 °C durchgeführt werden (vgl oben zitierte Literatur).

Substanzen der Formeln (III) sind komerziell erhältlich, literaturbekannt oder können nach literaturbekannten Verfahren synthetisiert werden (vgl. z.B. J. Chem. Soc 1958, 3065).

Substanzen der Formeln (V) sind literaturbekannt, oder können in Analogie zu literaturbekannten Verfahren synthetisiert werden (vgl. z.B. J. Med. Chem. 1989, 32, 1757; Indian J. Chem. Sect. B 1985, 24, 1015; Recl. Trav. Chim. Pays-Bas 1973, 92, 1281; Terahedron Lett. 1986, 37, 4327).

Substanzen der Formel (VII) sind komerziell erhältlich, literaturbekannt, oder können in Analogie zu literaturbekannten Verfahren synthetisiert werden (vgl. z.B. J. Org. Chem. 1959, 24, 1952; Collect Czech. Chem. Commun 1974, 39, 3527; Helv. Chim. Acta 1975, 58, 682; Liebigs Ann. Chem. 1981, 623).

Substanzen der Formel (IX) sind komerziell erhältlich, literaturbekannt, oder können m Analogie zu literaturbekannten Verfahren synthetisiert werden (vgl z.B. J. prakt. Chem. 1960, 341; Farmaco Ed. Sci. 1956, 378; Eur. J. Med. Chem. Chim. Ther. 1984, 19, 205; Bull. Soc. Chim. Fr. 1951, 97. Liebigs Ann. Chem. 1954, 586, 52; EP-A-0 334 137). Insbesondere können 4-Chlormethylbiphenylverbindungen, die einen weiteren Substituenten in 4'-Position tragen, durch Kupplung von 4-(B(OH)₂-Ph-CHO mit den entsprechenden in 4-Position substituierten Bromphenylverbindungen in Gegenwart von Palladium-Katalysatoren wie beispielsweise Pd(PPh₃)₄ oder PdCl₂(PPh₃)₂ und Natriumcarbonat zu den entsprechenden Biphenylverbindungen und anschließende Reduktion zum Alkohol mit NaBH₄ und Überführung in das entsprechende Chlorid mit z.B. SOCl₂ hergestellt werden.

Steht in den Formeln (III), (V), (VII) und (IX) E für Halogen, können die Verbindungen auch nach allgemein bekannten Verfahren, z.B. durch Umsetzung eines Alkohols mit einem Chlorierungsreagenz, wie z.B. Thionylchlorid oder Sulfurylchlorid hergestellt werden (vgl. z.B. J. March, Advanced Organic Chemistry, fourth Edition, Wiley, 1992, Seite 1274 bzw. die dort zitierte Literatur).

Amine der Formel (XV) sind kommerziell erhältlich, literaturbekannt, oder können in Analogie zu literaturbekannten Verfahren synthetisiert werden (vgl. z.B. Tetrahedron 1997, 53, 2075; J. Med. Chem. 1984, 27, 1321; WO97/29079; J. Org. Chem. 1982, 47, 5396). Beispielsweise können diese Verbindungen aus den entsprechenden Halogenidverbindungen und insbesondere Chloridverbindungen, bei denen anstatt der Reste W-NH₂ der Verbindungen der Formel (XV) eine Gruppe W'-Hal steht, wobei W' einen um ein C-Atom verkürzten Rest W darstellt, durch Substitution des Halogenidrestes durch eine Cyanogruppe unter Erhalt der entsprechenden Nitrilverbindungen und Reduktion der Nitrilgruppe oder durch Umsetzung entsprechender Aldehydverbindungen, bei denen anstatt der Reste W-NH₂ der Verbindungen der Formel (XV) eine Gruppe W'-CHO steht, wobei W' einen um ein C-Atom verkürzten Rest W darstellt, mit Nitromethan und anschließender Reduktion erhalten werden.

Amine der Formel (XVI) sind kommerziell erhältlich, literaturbekannt, oder können in Analogie zu hteraturbekannten Verfahren synthetisiert werden (vgl. z.B. J. Am. Chem. Soc. 1982, 104, 6801; Chem. Lett. 1984, 1733; J. Med. Chem. 1998, 41, 5219; DE-2059922).

Amine der Formel (XVII) sind kommerziell erhältlich, literaturbekannt, oder können in Analogie zu literaturbekannten Verfahren synthetisiert werden (vgl. z.B. J. Org. Chem. 1968, 33, 1581; Bull. Chem. Soc. Jpn. 1973, 46, 968; J. Am. Chem. Soc. 1958, 80, 1510; J. Org. Chem. 1961, 26, 2507; Synth. Commun. 1989, 19, 1787).
Amine der Formeln (XV), (XVI) und (XVII) können auch nach allgemein bekannten Verfahren, z.B. durch die Reduktion eines entsprechenden Nitrils, die Umsetzung eines entsprechenden Halogenids mit Phtalimid und nachfolgender Umsetzung mit Hydrazin oder die Umlagerung von Acylaziden in Gegenwart von Wasser hergestellt werden (vgl. z.B. J. March, Advanced Organic Chemistry, fourth Edition, Wiley, 1992, Seite 1276 bzw. die dort zitierte Literatur).

Carbonylverbindungen der Formel (XVIII) sind kommerziell erhältlich, literaturbekannt, oder können in Analogie zu literaturbekannten Verfahren synthetisiert werden (vgl. z.B. J. Med. Chem. 1989, 32, 1277; Chem. Ber. 1938, 71, 335; Bull. Soc. Chim. Fr. 1996, 123, 679).

Carbonylverbindungen der Formel (XIX) sind kommerziell erhältlich, literaturbekannt, oder können in Analogie zu literaturbekannten Verfahren synthetisiert werden, (vgl. z.B. WO96/11902; DE-2209128; Synthesis 1995, 1135; Bull. Chem. Soc. Jpn. 1985, 58, 2192).

Carbonylverbindungen der Formel (XX) sind kommerziell erhältlich, literaturbekannt, oder können in Analogie zu literaturbekannten Verfahren synthetisiert werden (vgl. z.B. Synthesis 1983, 942; J. Am Chem. Soc. 1992, 114, 8158).

Carbonylverbindungen der Formeln (XVIII), (XIX) und (XX) können auch nach allgemein bekannten Verfahren, z.B. durch Oxidation von Alkoholen, die Reduktion von Säurechloriden, oder die Reduktion von Nitrilen hergestellt werden (vgl. z.B. J. March, Advanced Organic Chemistry, fourth Edition, Wiley, 1992, Seite 1270 bzw. die dort zitierte Literatur).

Verbindungen der Formel (XII) sind kommerziell erhältlich, literaturbekannt, oder können in Analogie zu literaturbekannten Verfahren synthetisiert werden (vgl. z.B. für aromatische Boronsäuren: J. Chem. Soc. C 1966, 566. J. Org. Chem., 38, 1973, 4016; oder für Tributylzinnverbmdungen: Tetrahedron Lett. 31, 1990, 1347).

Verbindungen der Formel (XIII) sind kommerziell erhältlich, literaturbekannt, oder können in Analogie zu literaturbekannten Verfahren synthetisiert werden (vgl. z.B J. Chem. Soc. Chem. Commun., 17, 1994, 1919).

Die erfindungsgemäßen Verbindungen der allgemeinen Formel (I), zeigen ein nicht vorhersehbares, wertvolles pharmakologisches Wirkspektium.

Die erfindungsgemäßen Verbindungen der allgemeinen Formel (I), fuhren zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatcyclase und einem intrazellulären cGMP-Anstieg vermittelt.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, fibrotischen Erkrankungen wie Leberfibrose oder Lungenfibrose, asthmatischen Erkrankungen und Krankheiten des Urogenitalsystems wie beispielsweise Prostatahypertrophie, erektile Dysfunktion, weibliche sexuelle Dysfunktion und Inkontinenz sowie zur Behandlung von Glaucoma eingesetzt werden.

Die in der vorliegenden Erfindung beschriebenen Verbindungen der allgemeinen Formel (I), stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lem- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen, insbesondere die Verbindungen der allgemeinen Formel (I), zur Bekämpfung von Schmerzzuständen eingesetzt werden.

Zudem besitzen die erfindungsgemäßen Verbindungen anhmflammatonsche. Wirkung und können daher als entzündungshemmende Mittel eingesetzt werden.

### Gefäßrelaxierende Wirkung in vitro

Kaninchen werden durch intravenöse Injektion von Thiopental-Natnum narkotisiert bzw. getötet (ca. 50 mg/kg,) und entblutet. Die Arteria Saphena wird entnommen und in 3 mm breite Ringe geteilt. Die Ringe werden einzeln auf je einem triangelförmigen, am Ende offenen Häkchenpaar aus 0,3 mm starkem Spezialdraht (Remanium®) montiert. Jeder Ring wird unter Vorspannung in 5 ml Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O: 1,4; KH₂PO₄: 1,2; NaHCO₃: 25; Glucose 10; Rinderserumalbumin: 0,001%. Die Kontraktionskraft wird mit Statham UC2-Zellen erfasst, verstärkt und über A/D-Wandler (DAS-1802 HC, Keithley Instruments München) digitalisiert, sowie parallel auf Linienschreibem registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀). Das Standardapplikationsvolumen beträgt 5 µl. Der DMSO-Anteil in der Badlösung entspricht 0,1%.

Die Ergebnisse sind in Tabelle 1 gezeigt:

**Tabelle 1:**

| Gefäßrelaxierende Wirkung in vitro | |
|---|---|
| **Beispiel** | **IC**_{**50**} **(nM)** |
| 8 | 0,4 |
| 28 | 2,8 |
| 30 | 17 |
| 32 | 6,5 |
| 33 | 0,5 |
| 37 | 830 |
| 56 | 73 |
| 70 | 0,2 |
| 72 | 29 |
| 76 | 29 |
| 86 | 0,4 |
| 87 | 0,5 |
| 88 | 0,4 |
| 98 | 3,4 |
| 102 | 0,2 |
| 103 | 3,9 |
| 186 | 0,90 |

### Stimulation der rekombinanten löslichen Guanylatcyclase (sGC) in vitro

Die Untersuchungen zur Stimulation der rekombinanten löslichen Guanylatcyclase (sGC) und die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) wurden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch: Purified soluble guanylyl cyclase expressed in a baculovirus/Sf9 system: stimulation by YC-1, nitric oxide, and carbon oxide. J. Mol. Med. 77 (1999): 14-23.

Die Harn-freie Guanylatcyclase wurde durch Zugabe von Tween 20 zum Probenpuffer (0,5% in der Endkonzentration) erhalten.

Die Aktivierung der sGC durch eine Prüfsubstanz wird als n-fache Stimulation der Basalaktivität angegeben.

Die Ergebnisse sind in Tabelle 2 gezeigt:

**Tabelle 2:**

| Stimulation der rekombinanten löslichen Guanylatcyclase (sGC) in vitro | | | | | |
|---|---|---|---|---|---|
| Bsp. 87 Konzentration (µM) | Stimulation (n-fach) | | | | |
| | Häm-haltige sGC | | | Häm-freie sGC | |
| | Basal | + SNP (0.1 µM) | + ODQ (10 µM) | Basal | + ODQ (10 µM) |
| 0 | 1 | 15 | 1 | 1 | 1 |
| 0.1 | 15 | 41 | 132 | 353 | 361 |
| 1.0 | 18 | 47 | 115 | 491 | 457 |
| 10 | 24 | 60 | 181 | 529 | 477 |

Aus Tabelle 2 ist ersichtlich, dass eine Stimulation sowohl des Häm-haltigen als auch des Häm-freien Enzyms erreicht wird. Weiterhin zeigt eine Kombination aus sGC-Stimulator und Natriumnitroprussid (SNP), einem NO-Donor, keine synergistischen Effekt, d.h. die Wirkung von SNP wird nicht potenziert, wie dies bei über einem Häm-abhängigen Mechanismus wirkenden sGC-Stimulatoren zu erwarten wäre. Darüber hinaus wird die Wirkung des erfindungsgemäßen sGC-Stimulators durch den Häm-abhängigen Inhibitor der löslichen Guanylatcyclase ODQ nicht blockiert. Die Ergebnisse aus Tabelle 2 belegen somit den neuen Wirkmechanismus der erfindungsgemäßen Stimulatoren der löslichen Guanylatcyclase.

Zur vorliegenden Erfindung gehören pharmazeutische Zubereitungen, die neben nichttoxischen, inerten pharmazeutisch geeigneten Trägerstoffen die erfindungsgemäßen Verbindungen der allgemeinen Formel (I), enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen der allgemeinen Formel (I), sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen der allgemeinen Formel (I), auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- BABA:: n-Butylacetat/n-Butanol/Eisessig/Phosphatpuffer pH 6
(50:9:25.15; org. Phase)

### Laufmittel für die Dünnschichtchromatographie:

- T1 E1:: Toluol - Essigsäureethylester (1:1)
- T1 EtOH1:: Toluol - Methanol (1:1)
- C1 E1:: Cyclohexan - Essigsäureethylester (1:1)
- C1 E2:: Cyclohexan - Essigsäureethylester (1.2)

### Ausgangsverbindungen

### Beispiele I-IV) Verbindungen der Formel VIII:

### I.1. Methyl 4-{[(2-methoxyphenethyl)amino]methyl}benzoat

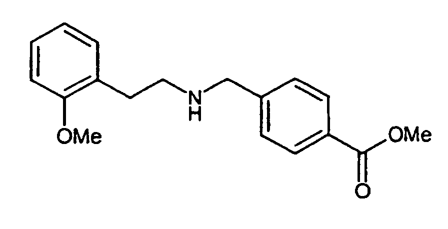

Eine Lösung von 9.23g (56.16 mmol) 2-Methoxyphenethylamin und 9.219 g (56.16 mmol) 4-Formylbenzoesäuremethylester in 35 ml Ethanol wird zwei Stunden unter Rückfluss erhitzt. Das Lösungsmittel wird unter vermindertem Druck abdestilliert, wobei 17.5 g des Imins erhalten werden, das ohne weitere Reinigung weiter umgesetzt wird.

17.5g (58.85 mmol) des Imins werden in 200 ml Methanol gelöst und portionsweise mit 4.45 g (117.7 mmol) Natriumborhydrid versetzt. Nach zwei Stunden Rühren bei Raumtemperatur wird das Reaktionsgemisch auf Wasser geschüttet, mit Ethylacetat extrahiert, die organischen Phasen mit gesättigter Natriumchloridlösung gewaschen und getrocknet. Nach Abdestillieren des Lösungsmittels im Vakuum verbleibt das Produkt als Öl.
Ausbeute: 16.04g (91 % der Theorie).
¹H-NMR (200 MHz, d⁶-DMSO): δ= 2.70 (m, 4H), 3.80 (s, 3H), 3.85 (s, 3H), 6.90 (m, 2H), 7.15 (m, 2H), 7.45 (d, 2H), 7.90 (s, 2H).

### I.2. Methyl 4-{[(5-ethoxy-5-oxopentyl)(2-methoxyphenethyl)amino]methyl}benzoat

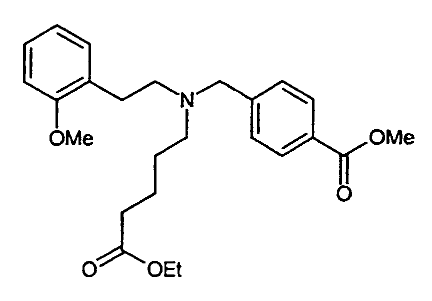

15.0 g (50.0 mmol) Methyl 4-{[(2-methoxyphenethyl)amino]methyl}benzoat aus Bsp. I.1., 11.52 g (55.0 mmol) 5-Bromvaleriansäureethylester, und 6.37 g (106.0 mmol) Natnumcarbonat werden in 30 ml Acetonitril gelöst und 18 Stunden unter Rückfluss erhitzt. Nach dem Abkühlen wird das Lösungsmittel im Vakuum zum größten Teil abdestilliert und der Rückstand mit Wasser versetzt. Man extrahiert mehrfach mit Essigester, wäscht die organischen Phasen mit gesättigter Natnumchloridlösung und entfernt nach Trocknen über Magnesiumsulfat das Lösungsmittel im Vakuum. Das Rohprodukt wird durch Blitzchromatographie an Kieselgel (0.04-0.063 nm) mit Cyclohexan/Essigester 4/1 als Laufmittel gereinigt.
Ausbeute: 17.77 g (80.4% der Theorie)
¹H-NMR (200 MHz, d⁶-DMSO): δ= 1.13 (t, 3H), 1.45 (m, 4H), 2.20 (t, 2H), 2.45 (t, 2H), 2.58 (m, 2H), 2.70 (m, 2H), 3.70 (s, 3H), 3.85 (s, 3H), 4.05 (q, 2H), 6.8-6.9 (m, 2H), 7.0-7.2 (m, 2H), 7.40(d, 2H), 7.86 (d, 2H).

### I. Methyl-4-{[(2-hydroxyphenethyl)(5-methoxy-5-oxopentyl)amino]methyl}benzoat

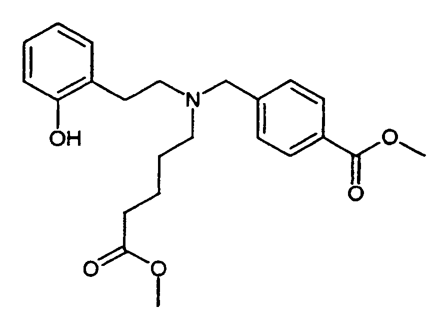

Eine Lösung aus 3.00 g (7.02 mmol) Methyl 4-{[(5-ethoxy-5-oxopentyl)(2-methoxyphenethyl)amino]methyl}benzoat aus Bsp. 1.2 in 60 ml Methylenchlorid wird auf 0°C gekühlt und 23.16 ml (23.16 mmol) einer 1N Bortribromid-Lösung in Methylenchlorid zugetropft. Es wird eine Stunde bei 0°C nachgerührt. Nach Zusatz von 30 ml trockenem Methanol wird der Ansatz 1 Stunde auf 60 °C erhitzt. Nach Abkühlen wird das Lösungsmittel im Vakuum entfernt, der Rückstand in einer Mischung aus 57 ml Ethylacetat und 3 ml Methanol aufgenommen und mit zehn prozentiger Natriumcarbonatlösung alkalisch gestellt. Die wässrige Phase wird mit Ethylacetat/Methanol 9/1 mehrfach extrahiert und die vereinigten organischen Phasen mit gesättigter Natriumchloridlösung gewaschen. Nach Trocknen über Magnesiumsulfat und Abdestillieren des Lösungsmittels im Vakuum wird das Rohprodukt durch Blitzchromatographie an Kieselgel (0.04-0.063 nm) mit Cyclohexan / Essigester 2/1 als Laufmittel gereinigt.
Ausbeute: 1.89 g (64.2 % der Theorie)
¹H-NMR (200 MHz, d⁶-DMSO): δ= 1.46 (m, 4H), 2.23 (t, 2H), 2.45 (t, 2H), 2.60 (m, 2H), 2.70 (m, 2H), 3.60 (s, 3H), 3.70 (s, 2H), 3.85 (s, 3H), 6.70 (m, 2H), 7.01(m, 2H), 7.45 (d, 2H), 7.90 (d, 2H), 9.50 (s, 1H)

### Auf gleiche Weise wurden erhalten:

### II. Methyl 4-{[(5-ethoxy-5-oxopentyl)(2-hydroxybenzyl)amino]methyl}benzoat

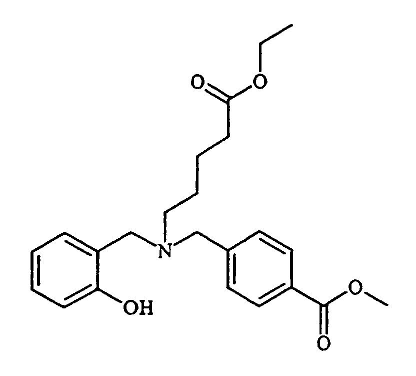

Diese Verbindung kann ausgehend von 2-Methoxybenzylamin statt 2-Methoxyphenethylamin analog zu Beispiel I erhalten werden.
¹H-NMR (200 MHz, d⁶-DMSO): δ= 1.15 (t, 3H), 1.50 (m, 4H), 2.15 (m, 2H), 2.40 (m, 2H), 3.65 (s, 4H), 3.85 (s, 3H), 4.01 (q, 2H), 6.75 (t, 2H), 7.0-7.2 (m, 2H), 7.45 (d, 2H), 7.94 (d, 2H), 10.0 (br. s, 1H)

### III. Methyl 4-{[(5-ethoxy-5-oxopentyl)(3-hydroxyphenethyl)amino]methyl}benzoat

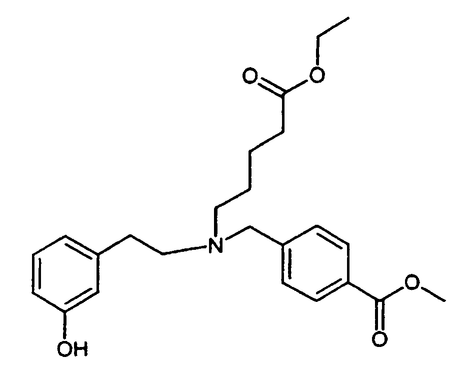

Diese Verbindung kann ausgehend von 3-Methoxyphenethylamin statt 2-Methoxyphenethylamin analog zu Beispiel I erhalten werden.
¹H-NMR (200 MHz, d⁶-DMSO): δ= 1.46 (m, 4H), 2.23 (t, 2H), 2.45 (t, 2H), 2.60 (m, 2H), 2.70 (m, 2H), 3.60 (s, 3H), 3.70 (s, 2H), 3.85 (s, 3H), 6.70 (m, 2H), 7.01(m, 2H), 7.45 (d, 2H), 7.90 (d, 2H), 9.50 (s, 1H).

### IV. Methyl 3-{[(5-ethoxy-5-oxopentyl)(2-hydroxyphenethyl)amino]methyl}benzoat

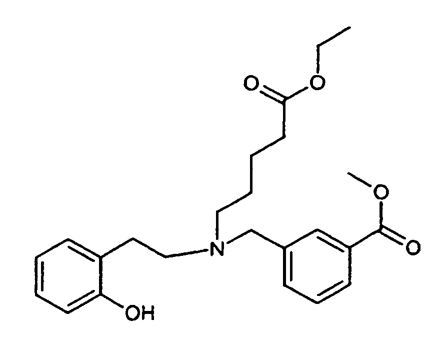

Diese Verbindung kann ausgehend von 3-Formylbenzoesäuremethylester statt 4-Formylbenzoesäuremethylester analog zu Beispiel I erhalten werden.
¹H-NMR (200 MHz, d⁶-DMSO): δ= 1.48 (m, 4H), 2.21(t, 2H), 2.47 (t, 2H), 2.64 (m, 2H), 2.71 (m, 2H), 3.60 (s, 3H), 3.70 (s, 2H), 3.85 (s, 3H), 6.70 (m, 2H), 7.0- 7.7 (d, 8H), 9.50 (s, 1H).

### Beispiel V - VIII) Verbindungen der Formel II:

### V.1. Methyl 4-{[(2-hydroxyphenethyl)amino]methyl}benzoat

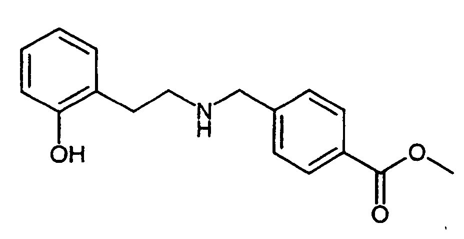

Zu 16.03 g (53.561 mmol) Methyl 4-{[(2-methoxyphenethyl)amino]methyl}benzoat aus Bsp. I.1 in 100 ml Methylenchlorid werden bei 0°C 176.8 ml (176.8 mmol) einer 1N Bortribromid-Lösung in Methylenchlorid getropft. Nach einer Stunde Rühren bei 0°C werden 150 ml Methanol zugesetzt und die Lösung 4 Stunden lang unter Rückfluss erhitzt. Das Lösungsmittel wird unter vermindertem Druck abdestilliert und der Rückstand m einem Gemisch von 190 ml Essigester und 10 ml Methanol aufgenommen. Man stellt mit 10 prozentiger Natriumcarbonat Lösung basisch und extrahiert mit Ethylacetat/Methanol 9/1. Die vereinigten organischen Phasen werden mit gesättigter Natriumchloridlösung gewaschen, über Magnesiumsulfat getrocknet und das Lösungsmittel im Vakuum abdestilliert das Rohprodukt wird durch Chromatograplue an Kieselgel ((0.04-0.063 nm) mit Methylenchlond/Methanol 100/2 als Laufmittel gereinigt.
Ausbeute: 6.80 g (42.9 % der Theorie.)
¹H-NMR (200 MHz, d⁶-DMSO): δ= 2.73 (s, 4H), 3.82 (s, 2H), 3.85 (s, 3H), 6.7 (m, 2H), 7.0 (d, 2H), 7.48 (d, 2H), 7.92 (d, 2H).

### V.2. Methyl 4-{[(tert-butoxycarbonyl)(2-hydroxyphenethyl)amino]methyl}benzoat

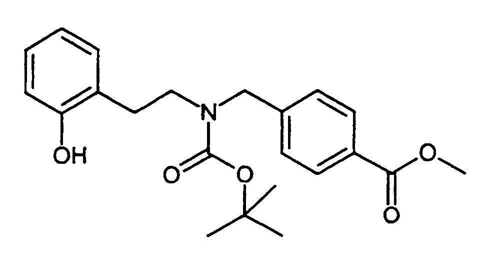

6.80 g (23.82 mmol) Methyl 4-{[(2-methoxyphenethyl)amino]methyl}benzoat aus Bsp. V.1. werden in 25 ml Methylenchlorid vorgelegt, und eine Lösung von 5.46 g (25.02 mmol) Pyrrokohlensäureester-tert.-butylester in 25 ml Methylenchlorid wird bei 0°C zugetropft. Nach 18 Stunden Rühren bei 22 °C wird das Lösungsmittel im Vakuum abdestilliert.
Ausbeute: 9.56 g (99 % der Theorie)
¹H-NMR (200 MHz, d⁶-DMSO): δ= 1.32 (s, 9H), 2.70 (t, 2H), 3.35 (m, 2H), 3.83 (s, 3H), 4.42 (s, 2H), 6.6-6.8 (m, 2H), 7.0 (m, 2H), 7.35 (d, 2H), 7.92 (d, 2H).

### V.3. Methyl-4-[((tert-butoxycarbonyl){2-[(5-phenylpentyl)oxy]phenethyl}amino)methyl]benzoat

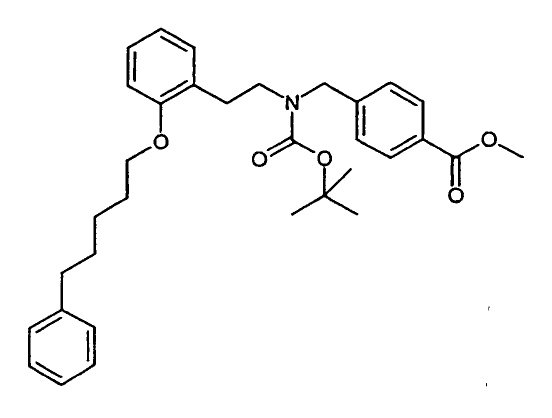

1.78 g (4.63 mmol) Methyl 4-{[(tert-butoxycarbonyl)(2-hydroxyphenethyl)amino]methyl}benzoat aus Bsp. V.2, 1.05 g (4.63 mmol) 5-Phenyl-1-brompentan und 0.77 g (5.55 mmol) Kaliumcarbonat werden in 15 ml Acetonitril 18 Stunden unter Rückfluss erhitzt. Das Reaktionsgemisch wird auf Wasser gegeben, mit Ethylacetat extrahiert, über Magnesiumsulfat getrocknet und das Lösungsmittel im Vakuum addestilliert. Man erhält einen Feststoff, der ohne Reinigung weiter umgesetzt wird.
Ausbeute: 2.42 g (88.8 % der Theorie.)
¹H-NMR (200 MHz, d⁶-DMSO): δ= 1.32 (s, 9H), 1.55 (m, 4H), 1.65(m, 2H), 2.70 (m, 2H), 3.36 (m, 2H), 3.79 (s, 3H), 3.90 (t, 2H), 4.40 (s, 2H), 6.8-6.9 (m, 2H), 7.1-7.3 (m, 9H), 7.94 (d, 2H)

### V. Methyl 4-[({2-[(5-phenylpentyl)oxy]phenethyl}amino)methyl]benzoat

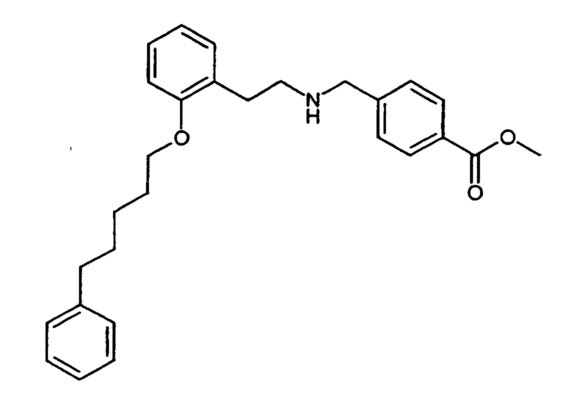

2.42 g (4.54 mmol) Methyl 4-[((tert-butoxycarbonyl){2-[(5-phenylpentyl)oxy]phenethyl}amino)methyl]benzoat aus Bsp. V.3 werden in ein Gemisch aus 4 ml Tnfluoressigsäure und 12 ml Methylenchlorid eingetragen und 18 Stunden bei 22 °C gerührt. Das Lösungsmittel wird am Rotationsverdampfer vollständig abdestilliert, der Rückstand in Wasser aufgenommen und das Produkt mit Ethylacetat mehrfach extrahiert. Die vereinigten organischen Phasen werden zweimal mit 2N Natronlauge gewaschen, über Magnesiumsulfat getrocknet und im Vakuum eingeengt.
Ausbeute: 8.25 g (77 % der Theorie.)
¹H-NMR (200 MHz, d⁶-DMSO): δ= 1.40 (m, 2H), 1.65 (m, 4H), 2.55 (t, 2H), 2.70 (m, 2H), 3.80 (s, 3H), 3.84 (s, 3H), 3.90 (t, 2H), 6.8-6.9 (m, 2H), 7.1-7.3 (m, 7H), 7.45 (d, 2H), 7.90 (d, 2H)

### Auf gleiche Weise wurden erhalten:

### VI. Methyl 4-({[2-(heptyloxy)phenethyl]amino}methyl)benzoat

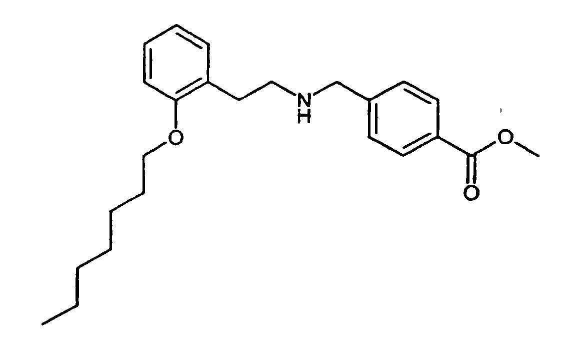

Diese Verbindung kann ausgehend von Heptylbromid statt 5-Phenyl-1-brompentan analog zu Beispiel V erhalten werden.
¹H-NMR (300 MHz, d⁶-DMSO) δ= 0.85 (t, 3H), 1.2-1.4 (m, 8H), 1.65 (m, 2H), 2.70 (s, 4H), 3.80 (s, 2H), 3.82 (s, 3H), 3.91 (t, 2H), 6.7-6.9 (m, 2H), 7.13 (d, 2H), 7.45 (d, 2H), 7.90 (d, 2H).

### VII. Methyl 4-({[2-([1,1'-biphenyl]-4-ylmethoxy)phenethyl]amino}methyl)benzoat

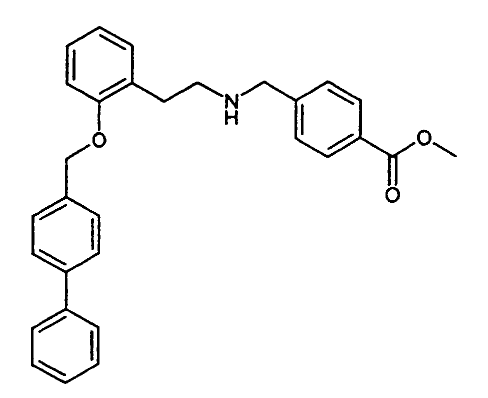

Diese Verbindung kann ausgehend von 4-Phenylbenzylbromid statt 5-Phenyl-1-brompentan analog zu Beispiel V erhalten werden.
¹H-NMR (200 MHz, d⁶-DMSO): δ= 2.75 (m, 4H), 3.80 (s, 3H), 3.82 (s, 2H), 5.13 (s, 2H), 6.7-7.6 (m, 15 H), 7.85 (d, 2H)

### VIII. Methyl 4-[({2-[(4-bromobenzyl)oxy]phenethyl}amino)methyl]benzoat

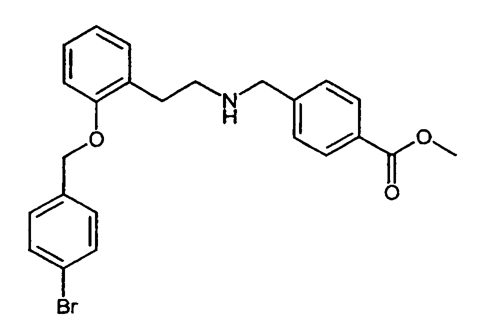

Diese Verbindung kann ausgehend von 4-Brombenzylbromid statt 5-Phenyl-1-brompentan analog zu Beispiel V erhalten werden.
¹H-NMR (200 MHz, d⁶-DMSO): δ= 2.75 (m, 4H), 3.80 (s, 3H), 3.82 (s, 2H), 5.13 (s, 2H), 6.7-7.6 (m, 10 H), 7.85 (d, 2H)

### IX. Methyl 4-{[{2-[4-(ethoxycarbonyl)phenoxy]ethyl}(2-hydroxyphenethyl)amino]methyl benzoat

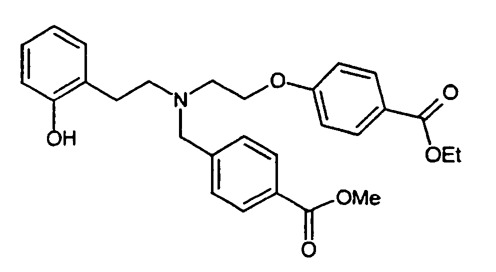

250 mg (0.88 mmol) Methyl-4-{[(2-hydroxyphenethyl)amino]methyl}benzoat aus Beispiel V.1., 311 mg (1.14 mmol) 4-(2-Bromoethoxy)benzoesäureethylester (Eastman Kodak CO, US-279082), und 250 mg (2.37 mmol) Natriumcarbonat werden in 3 ml Acetonitril gelöst und 18 Stunden unter Rückfluss erhitzt. Nach dem Abkühlen wird das Lösungsmittel im Vakuum abdestilliert und der Rückstand wird an Kieselgel (0.04-0.063 nm) mit Cyclohexan/Essigester 9/1 als Laufmittel gereinigt.
Ausbeute: 274 mg (65.5% der Theorie)
¹H-NMR (200 MHz, CDCl₃): δ= 1.13 (t, 3H), 2.80-3.05 (m, 6H), 3.80-4.35 (m, 9H), 6.70-8.00 (m, 12H), 11.40 (bs, 1H).

### X: Methyl-4-({(5-ethoxy-5-oxopentyl)[2-(2-hydroxyphenyl)ethyl]amino}methyl)benzoat

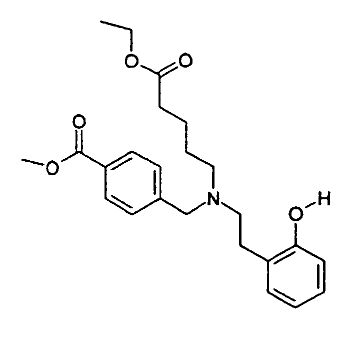

Diese Verbindung wurde analog zu Bsp. IX hergestellt mit der Ausnahme, dass Bromvaleriansäureethylester statt 4-(2-Bromoethoxy)benzoesäureethylester als Alkylierungsmittel verwendet wurde.
¹H-NMR (400 MHz, CDCl3): 1.20 (t, 3H), 1.60 (m, 4H), 2.20 (t, 2H), 2.50 (m, 2H), 2.80 (m, 4H), 3.80 (s, 2H), 3.90 (s, 3H), 4.10 (q, 2H), 6.70 (m, 1H), 6.90 (d, 1H), 6.95 (m, 1H), 7.10 (m, 1 H), 7.40 (d, 2H), 8.00 (d, 2H), 12.1 (bs, 1 H)

### XI Methyl-2-brom-4-({(5-ethoxy-5-oxopentyl)[2-(2-hydroxyphenyl)ethyl]amino} methyl)benzoat

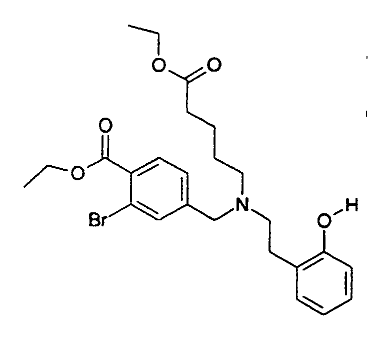

Diese Verbindung wurde analog zu Bsp. IX hergestellt mit der Ausnahme, dass Bromvaleriansäureethylester statt 4-(2-Bromoethoxy)benzoesäureethylester als Alkylierungsmittel verwendet und mit Methyl-2-brom-4-{[(2-hydroxyphenyl)ethyl]amino}methyl)benzoat (erhalten aus 2-Methoxyphenethylamin und 3-Brom-4-formylbenzoesäureethylester analog Bsp. V.1 [3-Brom-4-formylbenzoesäureethylester ist aus 2-Bromoterephthalsäurediethylester über Reduktion mit 1 eq. Lithiumaluminiumchlorid und Oxidierung des erhaltenen Alkohols mit Mangandioxid darstellbar] umgesetzt wurde.
¹H-NMR (200 MHz, CDCl₃): 1.20 (t, 3H), 1.40 (t, 3H), 1.60 (m, 4H), 2 20 (t, 2H), 2.50 (m, 2H), 2.80 (m, 4H), 3.80 (s, 2H), 4.10 (q, 2H), 4.40 (q, 2H), 6.70 (m, 1H), 6.90 (m, 2H), 7.10 (m, 1H), 7.40 (m, 1H), 7.60 (m, 1H), 7.70 (m, 1H), 11.70 (bs, 1H).

### XII: Methyl-4-({(5-methoxy-5-oxopentyl)[2-(5-fluor-2-hydroxyphenyl)ethyl]amino} methyl)benzoat

### XII.1. 5-Fluor-2-methoxybenzaldehyd

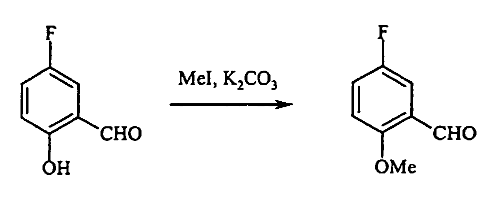

20,0 g (0,143 mol) 5-Fluor-2-hydroxybenzaldehyd werden in 250 ml Acetonitril gelöst. 81,04 g (0,57 mol) Iodmethan und 39,5 (285 mol) Kaliumcarbonat werden zugegeben, und die Suspension wird 3 Stunden unter Rückfluss erhitzt. Die Suspension wird filtriert und die Mutterlauge mit Essigsäureethylester verdünnt, zweimal mit Wasser gewaschen, über Magnesiumsulfat getrocknet, filtriert und die Lösungsmittel im Vakuum eingedampft.
Ausbeute: 20,0 g (90,9% d.Th.)
¹H-NMR: (200 MHz, CDCl₃): 3.90 (s, 3H), 6.90 (dd, J = 10 Hz, J = 5 Hz, 1H), 7.25 (m, 1H), 7.50 (dd, J = 10 Hz, J = 4 Hz, 1H), 10.40 (d, J = 4 Hz, 1H)

### XII.2. (5-Fluor-2-methoxyphenyl)methanol

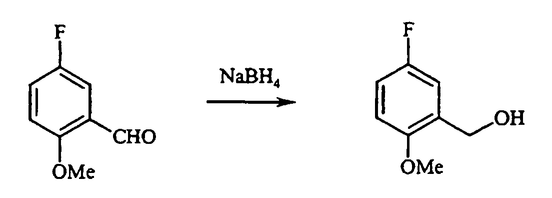

20,0 g (0,13 mol) 5-Fluor-2-methoxybenzaldehyd werden in 205 ml Methanol gelöst. Unter Argon werden 2,45 g (54,9 mol) Natriumborhydnd in kleinen Portionen zugegeben. Die Lösung wird 4 Stunden bei RT gerührt. Die Lösung wird eingeengt, der Rückstand in Wasser aufgenommen und 30 min. gerührt. Die wässrige Phase wird mit Essigester extrahiert und die organische Phase über Magnesiumsulfat getrocknet, filtriert und im Vakuum eingedampft.
Ausbeute: 19,0 g (93,8% d.Th.)
¹H-NMR: (300 MHz, CDCl₃): 3.80 (s, 3H), 4.60 (d, J = 7 Hz, 2H), 6.80 (dd, J = 14 Hz, J = 6 Hz, 1H), 6.95 (m, 1H), 7.05 (dd, J = 6 Hz, J = 4 Hz, 1H)

### XII.3. 2-(Chlormethyl)-4-fluor-1-methoxybenzol

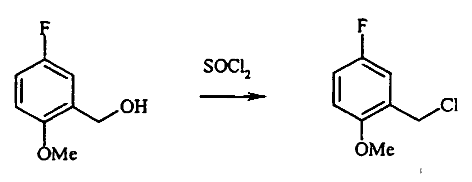

19,0 g (0,12 mol) (5-Fluor-2-methoxyphenyl)methanol werden in 105 ml Dichlormethan gelöst. Ein Tropfen DMF wird zugegeben, und anschliessend werden 26,6 ml (0,37 mol) Thionylchlorid langsam zugegeben. Die Lösung wird 2 Stunden bei RT gerührt und im Vakuum eingedampft. Der Rückstand wird in Essigsäureethylester aufgenommen, unter Kühlung mit Wasser versetzt, anschliessend mit gesättiger Natriumbicarbonatlösung und Wasser gewaschen, über Magnesiumsulfat getrocknet und im Vakuum eingedampft.
Ausbeute: 18,0 g (84,5% d.Th.)
¹H-NMR: (200 MHz, CDCl₃) 3.85 (s, 3H), 4.60 (s, 2H), 6.80 (dd, J = 14 Hz, J = 6 Hz, 1H), 7.00 (m, 1H), 7.10 (dd, J = 6 Hz, J = 4 Hz, 1H)

### XII.4. (5-Fluor-2-methoxyphenyl)acetonitril

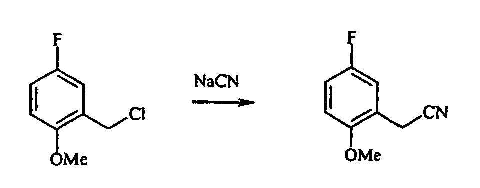

18,0 g (0,103 mol) 2-(Chloromethyl)-4-fluor-1-methoxybenzol werden in DMF:Wasser (5:1) gelöst, und 30,3 g (0,62 mol) Natriumcyanid und eine Spatelspitze Kaliumiodid werden zugegeben. Die Lösung wird über Nacht bei 120°C gerührt. Anschliessend wird die Lösung auf RT abkühlen gelassen, Wasser wird zugegeben und die Lösung mit Essigsäureethylester extrahiert, über Magnesiumsulfat getrocknet, filtriert und in Vakuum eingedampft. Der Rückstand wird über Silicagel mit Cyclohexan:Essigester (7:3) als Laufmittel chromatographiert.
Ausbeute: 14,5 g (85,2% d.Th.)
¹H-NMR: (200 MHz, CDCl₃): 3.70 (s, 2H), 3.85 (s, 3H), 6.80 (dd, J = 14 Hz, J = 6 Hz, 1H), 7.00 (m, 1H), 7.10 (dd, J = 6 Hz, J = 4 Hz, 1H)

### XII.5. 2-(5-Fluor-2-methoxyphenyl)ethylamin

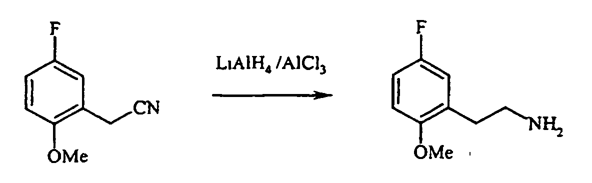

17,6 g (132 mmol) Aluminiumtrichlorid werden in THF unter Argon gelöst und auf 0°C abgekühlt. 87 ml Lithiumaluminiumhydridlösung (1M in THF) werden langsam zugetropft. Eine Lösung von 14,5 g (87,8 mmol) (5-Fluor-2-methoxyphenyl)acetonitril in 100 mL wird langsam zugegeben. Die Reaktionsmischung wird für 2 Stunden bei RT gerührt. Anschliessend wird bei 0°C Eis/Wasser zugegeben, mit Natriumhydroxydlösung alkalisch gestellt, mit Essigester extrahiert, getrocknet und einrotiert.
Ausbeute: 10,2 g (68,7% d.Th.)
¹H-NMR: (200 MHz, CDCl₃): 1.30 (bs, 2H), 2.70 (t, J = 6Hz, 2H), 2.90 (t, J = 6Hz, 2H), 3.80 (s, 3H), 6.70-6.90 (m, 3H)

### XII.6. 4-({[2-(5-Fluor-2-methoxyphenyl)ethyl]imino}methyl)benzoesäuremethylester

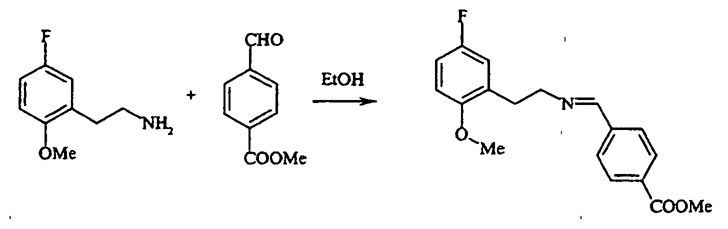

9,00 g (53 mmol) 2-(5-Fluor-2-methoxyphenyl)ethylamin und 8,73 g (53 mmol) 4-Formylbenzoesäuremethylester werden in 450 ml Ethanol gelöst, unter Rückfluss 2 Stunden erhitzt und anschliessend die Lösungsmittel im Vakuum eingedampft.
Ausbeute: 17,0 g (100% d.Th.)
¹H-NMR: (300 MHz, CDCl₃): 3.00 (t, J = 6Hz, 2H), 3.80 (s, 3H), 3.85 (t, 2H), 3.90 (s, 3H), 6.70-6.90 (m, 3H), 7.75 (d, 2H), 8.10 (d, 2H), 8.20 (s, 1H)

### XII.7. 4-({[2-(5-Fluor-2-methoxyphenyl)ethyl]amino}methyl)benzoesäuremethylester

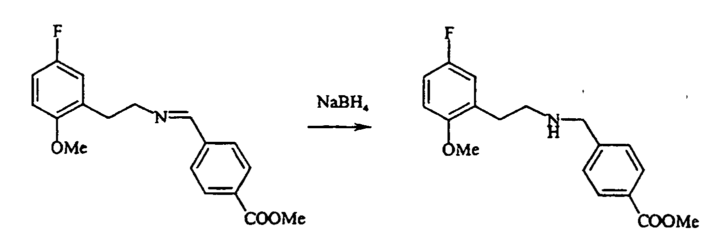

5,30 g (16,8 mmol) 4-({[2-(5-Fluor-2-methoxyphenyl)ethyl]imino}methyl)benzoesäuremethylester werden in 48,4 ml Methanol gelöst, und 1,27 g (33,6 mmol) Natriumborhydrid werden zugegeben. Die Lösung wird 2 Stunden bei RT gerührt, anschliessend wird Wasser zugegeben und mit Essigester extrahiert. Die organische Phase wird über Magnesiumsulfat getrocknet, filtriert und in Vakuum eingeengt. Der Rückstand wird m Essigester aufgenommen und mit verdünnter HCl extrahiert. Die wässrige Phase wird alkalisch gestellt und mit Essigester extrahiert, über Magnesiumsulfat getrocknet, filtriert und in Vakuum eingeengt.
Ausbeute: 4,79 g (89,8% d.Th.)
¹H-NMR: (200 MHz, CDCl₃): 3.00 (bs, 4H), 3.70 (s, 3H), 3.85 (s, 3H), 4.10 (bs, 2H), 6.70 (m, 1H), 6.90 (m, 2H), 7.70 (d, 2H), 8.00 (d, 2H), 10.20 (bs, 1H)

### XII.8. 4-({(5-Ethoxy-5-oxopentyl)[2-(5-fluor-2-methoxyphenyl)ethyl]amino}methyl)benzesäuremethylester

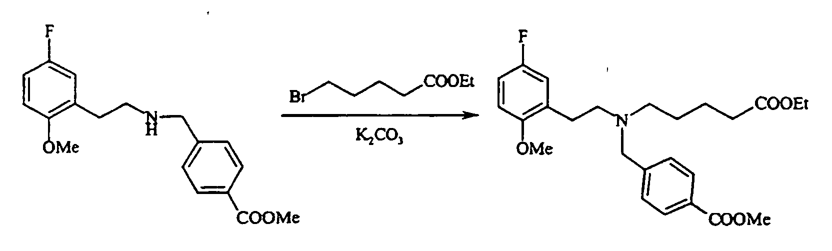

4,70 g (14,8 mmol) 4-({[2-(5-Fluor-2-methoxyphenyl)ethyl]amino}methyl)benzoesäuremethylester werden unter Argon in 25 ml Acetonitril gelöst. 3,25 g (15,6 mmol) Bromvaleriansäureethylester, 7,24 g (22,2 mmol) Caesiumcarbonat und eine Spatelspitze Kaliumiodid werden zugegeben und die Suspension wird über Nacht unter Rückfluss erhitzt. Das Feststoff wird abfiltriert, die Lösung eingeengt und der Rückstand wird über Silicagel (Cyclohexan.Essigester (4:1)) chromatographiert.
Ausbeute: 3,8 g (57,6% d.Th.)
¹H-NMR (300 MHz, CDCl₃): 1.20 (t, 3H), 1.50 (m, 4H), 2.30 (t, 2H), 2.50 (t, 2H), 2.60-2.80 (m, 4H), 3.65 (s, 2H), 3.70 (s, 3H), 3.90 (s, 3H), 4 10 (q, 2H), 6.70 (m, 1H), 6 80 (m, 2H), 7.35 (d, 2H), 7.90 (d, 2H)

### XII: 4-({(S-Methoxy-5-oxopentyl)[2-(5-fluor-2-hydroxyphenyl)ethyl]amino}methyl)benzoesäuremethylester

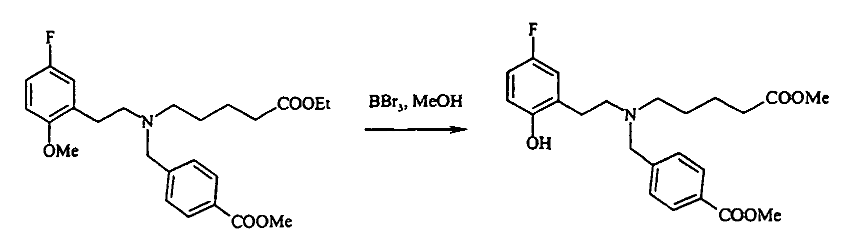

2,6 g (5,84 mmol) 4-({(5-Ethoxy-5-oxopentyl)[2-(5-fluor-2-methoxyphenyl)ethyl]amino}methyl)benzesäuremethylester werden in 50 ml Dichlormethan gelöst, auf 0°C abgekühlt, und 19,3 ml (19,3 mmol) einer 1N Lösung Bortribromid in Dichlormethan wird zugetropft. Die Lösung wird eine Stunde bei 0°C gerührt. 50 mL Methanol werden langsam bei 0°C zugetropft und die Reaktionmischung wird über Nacht unter Rückfluss erhitzt. Die Mischung wird abgekühlt und die Lösungsmittel werden unter Vakuum eingedampft. Der Rückstand wird in Essigester aufgenommen und mit Natriumcarbonat gewaschen, die wässrige Phase wird dreimal mit Essigester extrahiert und die vereigneten organischen Phasen werden mit gesättiger Natriumchloridlösung gewaschen, über Magnesiumsulfat getrocknet, filtriert und eingeengt. Der Rückstand wird über Silicagel (Cyclohexan:Essigester (5:1) bis Essigester:Methanol (9:1)) chromatographiert.
Ausbeute: 840 mg (34,5% d. Th.)
¹H-NMR (200 MHz, CDCl₃): 1.60 (m, 4H), 2.20 (m, 2H), 2.50 (m, 2H), 2.80 (m, 4H), 3.60 (s, 3H), 3.80 (s, 2H), 3.90 (s, 3H), 6.65 (m, 1H), 6.80 (m, 2H), 7.40 (d, 2H), 7.90 (d, 2H), 11.90 (bs, 1H)

### XIII: Tert-butyl-4-({[2-(2-{[4-(2-phenylethyl)benzyl]oxy}phenyl)ethyl]amino}methyl)benzoat

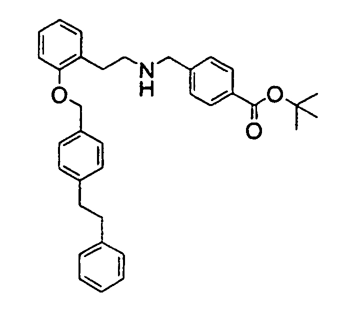

Diese Verbindung wurde analog zu Bsp. I.1 aus 2-(2-{[4-(2-Phenylethyl)benzyl]oxy}phenyl)ethylamin und 4-Formylbenzoesäuretertbutylester hergestellt.
¹H-NMR (400 MHz, DMSO): 1.50 (s, 9H), 2.60 (m, 4H), 2.80 (m, 4H), 3.80 (s, 2H), 5.00 (s, 2H), 6.80 (m, 1H), 6.90 (d, 1H), 7.10-7.40 (m, 13H), 7.80 (d, 2H)

### XIV: 4'-(Trifluoromethyl)-1,1'-biphenyl-4-Carbaldehyd

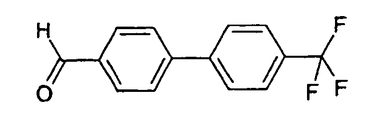

1 g (4.45 mmol) 1-Brom-4-(trifluoromethyl)benzol und 0.73 g (4.9 mmol) 4-Formylbenzoesäure werden in 30 mL Dimethoxyethan zusammengegeben und mit 15 ml 1M Natriumcarbonatlösung versetzt. Nach Zugabe von 110 mg Tetrakis(triphenylphosphin)palladium(II) wird 18 Stunden auf Rückflußtemperatur erhitzt. Die Reaktionslösung wird abgekühlt, Dichlormethan und Wasser wird zugegeben, die Mischung über Extrelut filtriert und das Lösungsmittel im Vakuum abdestilliert.
Ausbeute: 87%
1H-NMR (400 MHz, CDCl3): 7.70 (m, 6H), 8.00 (d, 2H), 10.00 (s, 1H).

### XV: [4'-(Trifluoromethyl)-1,1'-biphenyl-4-yl]methanol

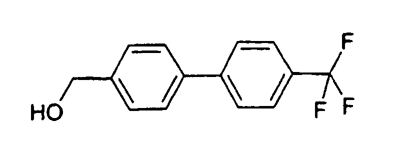

970 mg (3.88 mmol) des Aldehyds XIV werden in Methanol gelöst und 150 mg (3.88 mmol) Natriumhydrid werden zugegeben, 2 Stunden bei Raumtemperatur gerührt, eingeengt und Wasser zugegeben. Es wird 30 min gerührt und der Feststoff abfiltriert.
Ausbeute: 90%
1H-NMR (400 MHz, CDCl3) 1.75 (t, 1H), 4.80 (d, 2H), 7.40-7.90 (m, 8H).

### XVI: 4-(Chloromethyl)-4'-(trifluoromethyl)-1,1'-biphenyl

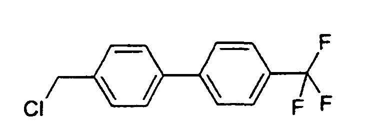

883 mg (3.49 mmol) des Alkohols XV werden in Dichlormethan gelöst, 2.5 mL (35 mmol) POCl₃ werden zugegeben und die Lösung wird 2 Stunden bei Raumtemperatur gerührt. Es wird mit Wasser gewaschen, getrocknet und eingeengt.
Ausbeute: 85%

### XVIIa: [2-(1,1'-biphenyl-4-ylmethoxy)phenyl]methanol

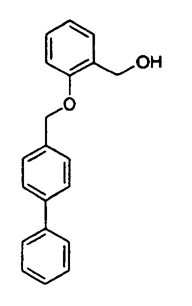

Eine Mischung aus 2.92 g (23.49 mmol) 2-Hydroxybenzylakohol, 5.00 g (24.67 mmol) 4-Phenylbenzylchlorid und 3.41 g (24.67 mmol) Kaliumcarbonat in 60 ml Aceton wurden über Nacht rückfließend erhitzt. Der gebildete Niederschlag wurde abfiltriert. Der Rückstand wurde in 1 N NaOH aufgenommen, und man extrahierte mit Ethylacetat. Die vereinten organischen Phasen wurden über Na₂SO₄ getrocknet und das Lösungsmittel entfernt. Das Produkt wurde chromatografisch gereinigt (Kieselgel, Cyclohexan/Ethylacetat 10: 1).
Ausbeute: 4.27 g (62.7%)
¹H NMR (300 MHz, CDCl₃): δ = 2.26 (t, J = 5.7 Hz, 1H), 4.75 (d, J = 5.7 Hz, 2H), 5.16 (s, 2H), 6.88 - 7.02 (m, 2H), 7.18 - 7.66 (m, 11H).

Analog wurden hergestellt:

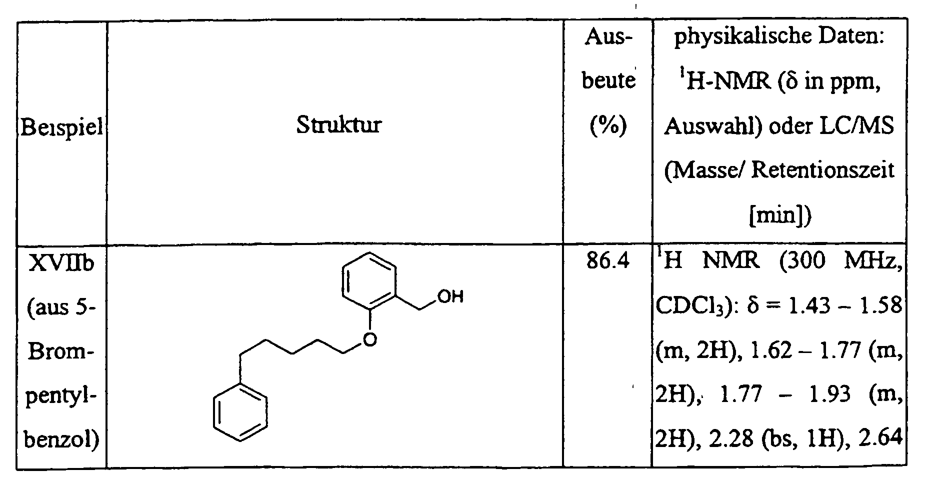

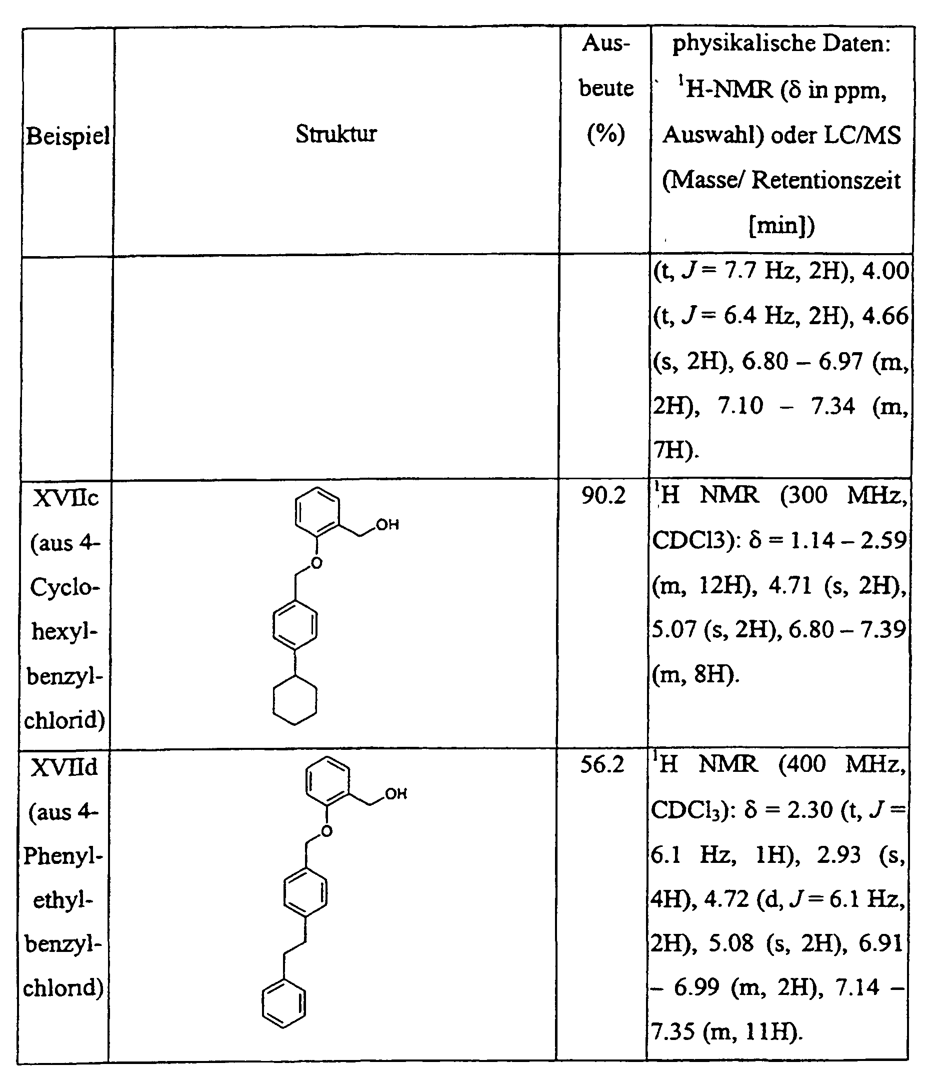

### XVIIIa: [2-(1,1'-Biphenyl-4-ylmethoxy)phenyl]acetonitril

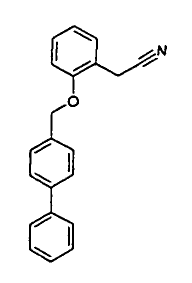

Zu einer Lösung von 6.49 ml (88.99 mmol) Thionylchlond in 150 ml Benzol wurde eine Lösung von 15.20 g (52.35 mmol) XVIIa in 300 ml Benzol zugetropft. Die Lösung wurde 2 h unter Rückfluss erhitzt. Man entfernte das Lösungsmittel und nahm den Rückstand in 350 ml DMF auf. Man gab 25.65 g (523.48 mmol) NaCN zu und erwärmte 16 h unter Rückfluss. Nachdem die Mischung sich auf Raumtemperatur abgekühlt hatte, versetzte man mit Wasser und saugte den Niederschlag ab.
Ausbeute: 13.6 g (81.5 %)
¹H NUR (400 MHz, CDCl₃): δ = 3.74 (s, 2H), 5.16 (s, 2H), 6.93 - 7.03 (m, 2H), 7.21 - 7.67 (m, 11H).

Analog wurden hergestellt:

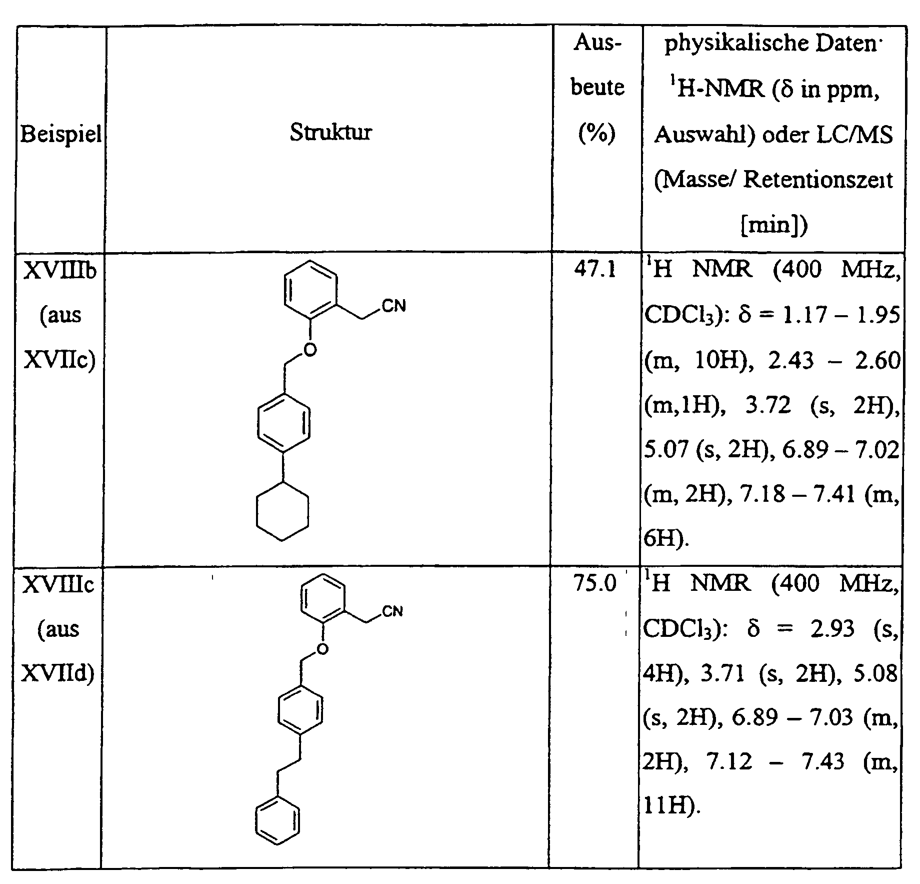

### XIXa: 2-[2-(1.1'-Biphenyl-4-ylmethoxy)phenyl]ethanamin hydrochlorid

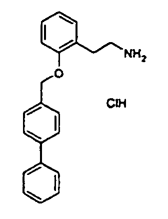

Zu einer Lösung von 52.93 ml (52.93 mmol) BH₃-THF (1 M in THF) wurde eine Lösung von 7.90 g (26.39 mmol) XVIIIa in 80 ml THF zugetroft. Die Lösung wurde 2 h unter Rückfluss erhitzt. Nachdem die Lösung sich auf Raumtemperatur abgekühlt hatte versetzte man sehr vorsichtig mit 150 ml 6 M Salzsäure und rührte die Mischung 16 h bei Raumtemperatur. Der gebildete Niederschlag wurde abfiltriert und im Hochvakuum getrocknet.
Ausbeute: 6.72 g (74.9 %)
¹H NMR (400 MHz, DMSO-d₆): δ = 2.89 - 3.01 (m, 4H), 5.20 (s, 2H), 6.85 - 7.78 (m, 13H), 7.99 (bs, 3H).

Analog wurden hergestellt:

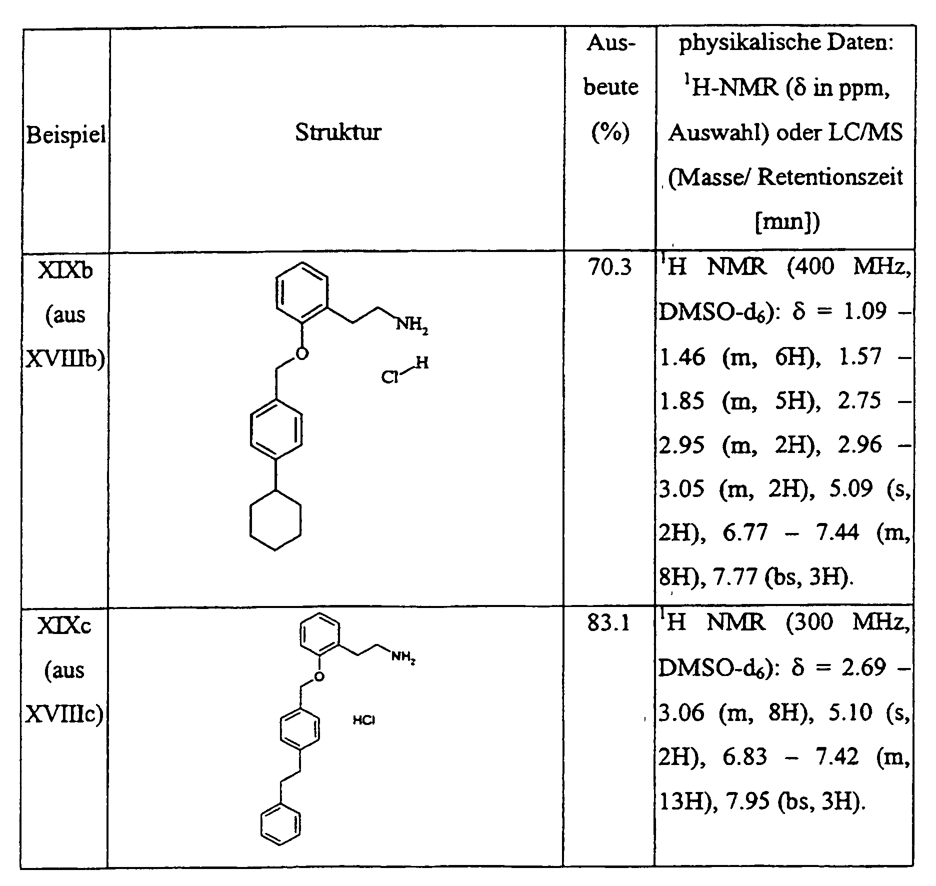

### XXa: tert-Butyl 5-({2-[2-(1,1'-biphenyl-4-ylmethoxy)phenyl]-ethyl}amino)pentanoat

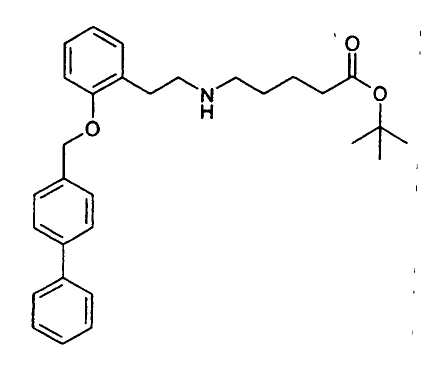

Zu einer Lösung von 3.00 g (8.83 mmol) XVIIIa in 50 ml DMF wurden 13.40 g (132.40 mmol) Triethylamin und 1.05 g (4.41 mmol) Bromvaleriansäure-tert.-butylester gegeben. Man rührte 16 h bei Raumtemperatur und kontrollierte die Reaktion per Dünnschichtchromatographie. Die Lösung wurde mit Wasser versetzt und man extrahierte mit Ethylacetat/Cyclohexan 1:1. Die vereinten organischen Phasen wurden über Na₂SO₄ getrocknet und das Lösungsmittel entfernt. Das Produkt wurde chromatografisch gereinigt (Kieselgel, CH₂Cl₂/MeOH 20:1).
Ausbeute: 0.85 g (41.9 %).
¹H NMR (300 MHz, DMSO-d₆): δ = 1.31 - 1.54 (m, 4H), 1.36 (s, 9H), 2.15 (t, *J* = 7.2 Hz, 2H), 2.56 (t, J= 6.8 Hz, 2H), 2.70 - 2.91 (m, 5H), 5.17 (s, 2H), 6.82 - 7.75 (m, 13H).

Analog wurden hergestellt:

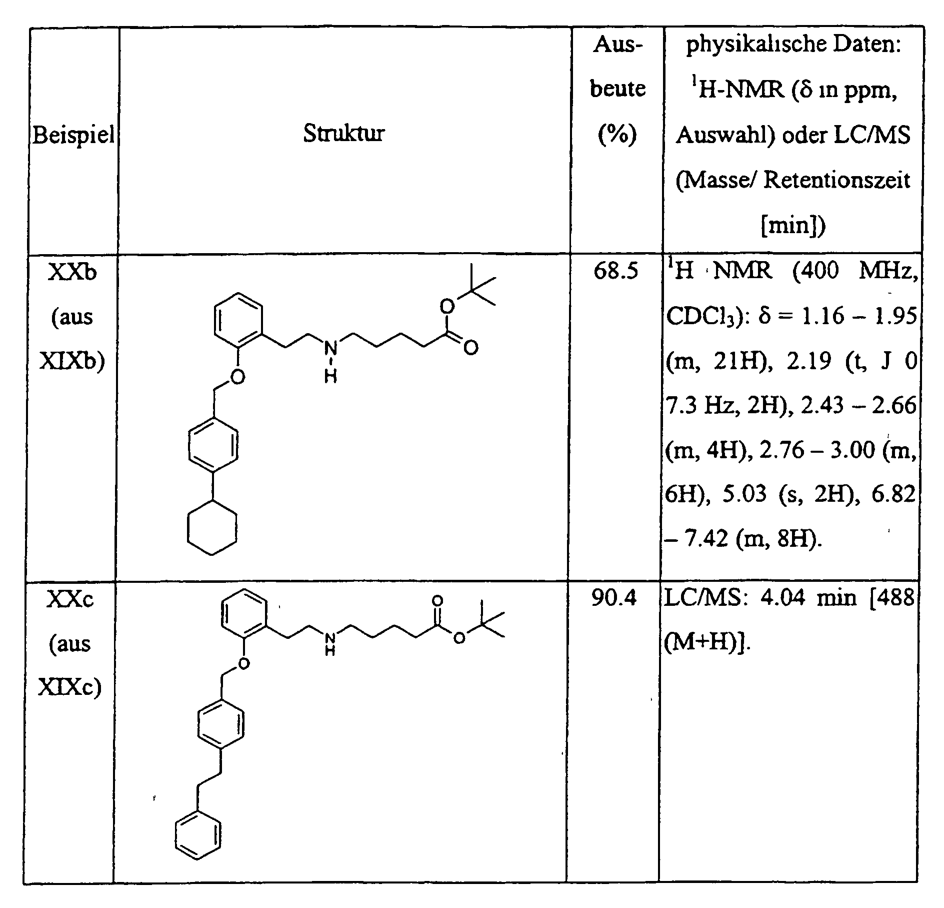

### XXI: 4-{[{2-[2-({4-[2-(4-{[Tert-butyl(dimethyl)silyl]oxy}phenyl)ethyl]benzyl}oxy)-phenyl]ethyl}(5-ethoxy-5-oxopentyl)amino]methyl}benzoesäuremethylester

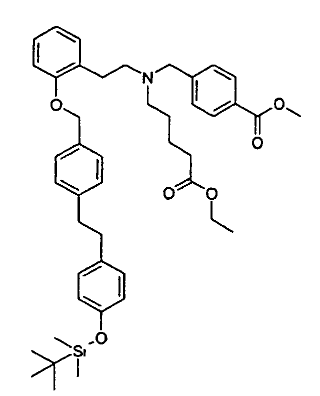

166 mg (0,403 mmol) 4-({(5-Ethoxy-5-oxopentyl)[2-(2-hydroxyphenyl)ethyl]amino} methyl)benzoesäuremethylester und 160 mg (0,443 mmol) t-Butyl(4-{2-[4-(chlormethyl)phenyl]ethyl}phenoxy)dimethylsilan (hergestellt aus 4-{[t-Butyl(dimethyl)silyl]oxy}benzaldehyd und [4-(Methoxycarbonyl)benzyl](triphenyl)phosphoniumchlorid über eine Wittigreaktion, anschliessende Hydrierung der Doppelbindung, Reduktion mit Lithiumaluminiumhydrid und Chlorierung analog XVI) werden in 6 ml Acetonitril gelöst. 263 mg (0,81mmol) Cäsiumcarbonat und eine Spatelspitze Kaliumiodid werden zugegeben, und die Mischung wird über Nacht zum Rückfluss erhitzt. Die Suspension wird filtriert, eingeengt und der Rückstand wird über Silicagel Cyclohexan : Essigester = 5:1 chromatographiert.
Ausbeute: 27 mg (9,1% d. Th.)
LC/MS: 738 (M+1), Rt=3.76
Bedingungen: Säule: Symmetry C18 2,1*150 mm; Eluent: Acetonitril + 0,6 g 30%ig HCl/1l H₂O; Gradient: 10 % Acetonitril bis 90 % Acetonitril; Fluss: 0,6 ml/min; Detektor: UV 210 nm

### Synthesebeispiele

### Beispiel 1: Methyl-4-{[{2-[(2-chlorobenzyl)oxy]phenethyl}(5-methoxy-5-oxopentyl)amino]methyl}benzoat (über Verfahren D)

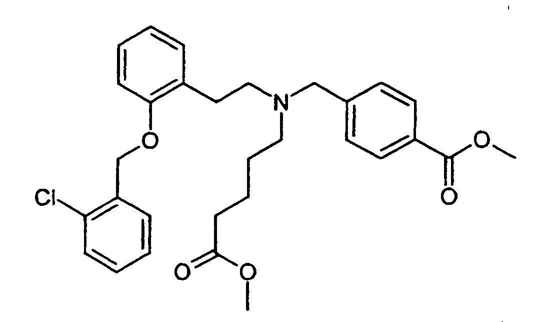

193.2 mg (0.484 mmol) Methyl 4-{[(2-hydroxyphenethyl)amino]methyl}benzoat aus Bsp. I, 77.9 mg (0.484 mmol) 2-Chlorbenzylchlorid und 80.2 mg (0.580 mmol) Kaliumcarbonat werden in 2.0 ml Acetonitril 18 Stunden unter Rückfluss erhitzt. Der Ansatz wird auf Wasser geschüttet und mit Ethylacetat extrahiert. Nach Trocknen über Magnesiumsulfat und Abdestillieren des Lösungsmittels im Vakuum wird das Rohprodukt durch Blitzchromatographie an Kieselgel (0.04-0.063 um) mit Cyclohexan/Essigester 2/1 als Laufmittel gereinigt.
Ausbeute: 245.2 mg (83.5 % der Theorie)
¹H-NMR (200 MHz, d⁶-DMSO): δ= 1.40 (m, 4H), 2.15 (t, 2H), 2.40 (dd, 2H), 2.57 (m, 2H), 2.72 (m, 2H), 3.53 (s, 3H), 3.82 (s, 3H), 5.08 (s, 2H), 6.9-7.5 (m, 10H), 7.82 (d, 2H).

### Beispiel 2: 4-[((4-carboxybutyl){2-[(2-chlorobenzyl)oxy]phenethyl}amino)methyl]benzoesäure (über Verfahren E)

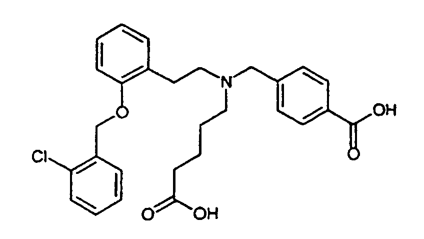

124.8 mg (0.238 mmol) Methyl-4-{[{2-[(2-chlorobenzyl)oxy]phenethyl}(5-methoxy-5- oxopentyl)amino]methyl}benzoat aus Bsp. 1 werden in 0.3 ml Methanol und 0.17 ml Wasser vorgelegt und mit 0.2 ml einer 40 prozentigen Natriumhydroxidlösung versetzt. Es wird eine Stunde bei 60°C gerührt, abgekühlt und das Methanol im Vakuum abdestilliert. Die wässrige Phase wird durch Zugabe eines Citronensäure-/Natronlaugepuffers auf pH 4 gestellt und der entstandene Niederschlag abgetrennt. Durch Verrühren in kochendem Cyclohexan erhält man feinkristallines Produkt.
Ausbeute: 65.70 mg (54.4 % der Theorie)
¹H NMR (200 MHz, d⁶-DMSO): δ= 1.35 (br.m 4H), 1.98 (br. m, 2H), 2.37 (m (2H), 2.58 (m, 2H), 2.70 (m, 2H), 5.12 (s, 2H), 6.8-7.6 (m, 10H), 7.75 (d, 2H), 13.5 (br.s, 1H).

### Beispiel 3: Methyl-4-[((5-ethoxy-3,3-dimethyl-2,5-dioxopentyl){2-[(5-phenylpentyl)oxy]phenethyl}amino)methyl]benzoat (über Verfahren A)

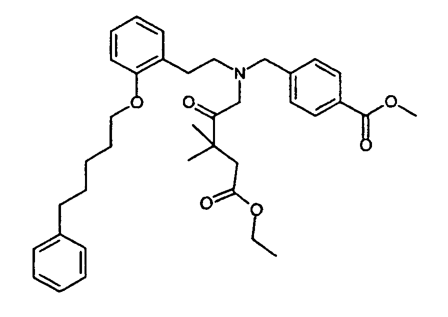

200.0 mg (0.463 mmol) Methyl 4-[({2-[(5-phenylpentyl)oxy]phenethyl}amino)methyl]benzoat aus Bsp. V, 116.4 mg (0.463 mmol) 5-Brom-3,3-dimethyllaevulinsäureethylester und 58.9 mg (0.56 mmol) Natriumcarbonat werden in 1 ml Acetonitril 18 Stunden auf 60 °C erhitzt. Das Lösungsmittel wird am Rotationsverdampfer abdestilliert, der Rückstand auf Wasser gegeben und mit Essigester extrahiert. Die organische Phase wird mit gesättigter Natriumchloridlösung gewaschen, über Magnesiumsulfat getrocknet und eingeengt. Das Rohprodukt wird durch Chromatographie an Kieselgel (0.04-0.063 nm) mit Cycolhexan/Ethylacetat 10/1 gereinigt.
Ausbeute:163.1 mg (58.5 % der Theorie)
¹H-NMR (200 MHz, d⁶-DMSO): δ= 1.09 (s, 6H), 1.10 (t, 3H), 1.35 (m, 2H), 1.60 (m, 4H), 2.55 (m, 2H), 2.70 (s, 2H), 3.75 (s, 3H), 3.96 (q, 2H), 6.7-6.9 (m, 2H), 7.0-7.3 (m, 7H), 7.40 (d, 2H), 7.85 (d, 2H).

### Beispiel 4: Methyl 4-{[{2-[(4-bromobenzyl)oxy]phenethyl}(5-ethoxy-5-oxopentyl) amino]methyl}benzoat (über Verfahren D)

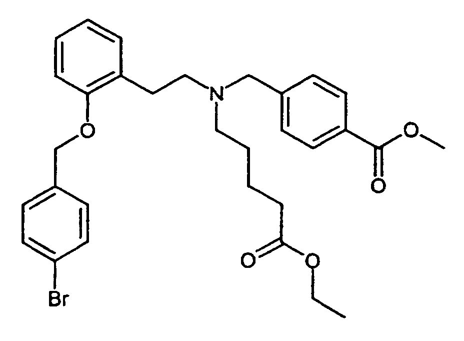

5.00 g (11.0 mmol) Methyl 4-[({2-[(4-bromobenzyl)oxy]phenethyl}amino)methyl]benzoat aus Bsp. VIII, 2.30 g (11.0 mmol) 5-Bromvaleriansäureethylester und 1.109 g (13.21 mmol) Natriumhydrogencarbonat werden in 30 ml Acetonitril 18 Stunden unter Rückfluss erhitzt. Das Reaktionsgemisch wird mit Wasser versetzt und mit Methylenchlorid extrahiert. Die organische Phase wird mit gesättigter Natriumchloridlösung gewaschen, über Magnesiumsulfat getrocknet und das Lösungsmittel im Vakuum abdestilliert. Der Rückstand wird durch Chromatographie an Kieselgel mit Methylenchlorid/Methanol 100/1 als Laufmittel gereimgt.
Ausbeute: 5.69 g (88.1 % der Theorie)
¹H-NMR (200 MHz, d⁶-DMSO): δ= 1.1 (m, 2H), 1.4 (m, 2H), 2.15 (t, 3H), 24 (t, 2H), 2.6 (m, 2H), 2.8 (m, 2H), 3.63 (s, 2H), 3.80 (s, 2H), 4.0(q, 2H), 5.10 (s, 2H), 6.85 (t, 2H), 7.0-7.2 (m, 8H), 7.4-7.8 (m), 7.9 (d, 2H)

### Beispiel 5: Methyl4-{[{2-[(4'-chloro[1,1'-biphenyl]-4-yl)methoxy]phenethyl}(5-ethoxy-5-oxopentyl)amino]methyl}benzoat (über Verfahren F)

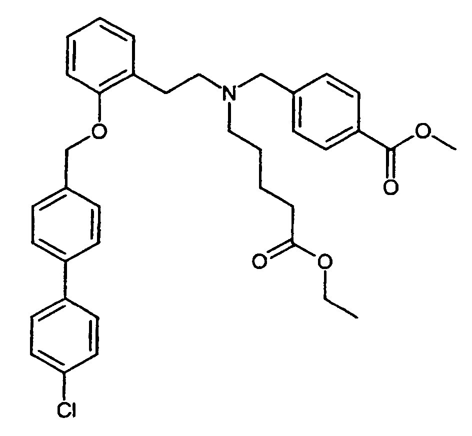

300.0 mg (0.51 mmol) Methyl-4-{[{2-[(4-bromobenzyl)oxy]phenethyl}(5-ethoxy-5-oxopentyl) amino]methyl}benzoat aus Bsp. 4 werden in 3 ml Dimethoxyethan vorgelegt und nacheinander mit 101.7 mg (0.62 mmol) 4-Chlorphenylboronsäure und 0.57 ml 2M Natriumcarbonatlösung versetzt. Nach Zugabe von 10.0 mg Dichlorobis(triphenylphosphine)palladium(II) wird 18 Stunden auf Rückflusstemperatur erhitzt. Die Reaktionslösung wird abgekühlt, mit 20 ml Ethylacetat versetzt und nacheinander mit 5 prozentiger Natriumhydrogenphosphatlösung, Wasser und gesättigter Natriumchloridlösung gewaschen. Die Organische Phase wird über Magnesiumsulfat getrocknet und das Lösungsmittel im Vakuum abdestilliert. Das Rohprodukt wird über Kieselgel mit Cyclohexan/Ethylacetat=10:1 als Laufmittel chromatographiert.
Ausbeute: 240.5 mg (74.3 % der Theorie)
¹H-NMR (200 MHz, d⁶-DMSO): δ= 1.10 (t, 3H), 1.43 (m, 4H), 2.15 (t, 2H), 2.45 (t, 2H), 2.62 (m, 2H), 2.75 (m, 2H), 3.63 (s, 2H), 3.80 (s, 3H), 3.97 (q, 2H), 5.09 (s, 2H), 6.85 (t, 1H), 7.01 (d, 1H), 7.13 (dd, 2H), 7.36 (d, 2H), 7.5-7.7 (m, 8H), 7.83 (d, 2H).

### Beispiel 6: Methyl-4-({(5-methoxy-5-oxopentyl)[2-({4-[(E)-2-phenylethenyl]benzyl}oxy}phenethyl] amino}methyl)benzoat (über Verfahren D)

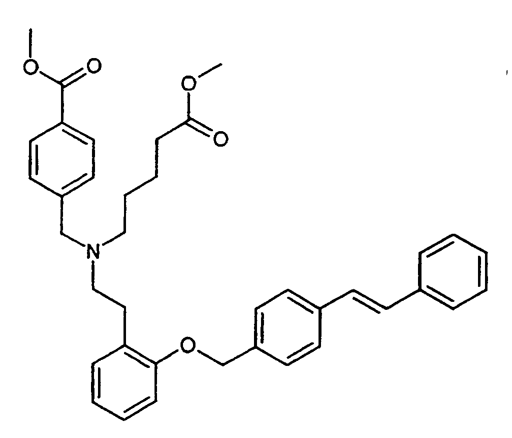

1.0 g (2.50 mmol) Methyl-4-{[(2-hydroxyphenethyl)-(5-methoxy-5-oxopentyl)amino]methyl}benzoat aus Bsp. I, 0.687 g (3.00 mmol) 4-(Chlormethyl)stilben und 0.520 g (3.75 mmol) Kaliumcarbonat werden in 10.0 ml Acetonitril 18 Stunden unter Rückfluss erhitzt. Die Lösung wird filtriert und das Lösungsmittel im Vakuum addestilliert. Das Rohprodukt wird durch Chromatographie an Kieselgel mit Cyclohexan/Ethylacetat 4/1 als Laufmittel gereinigt.
Ausbeute: 1.32 g (79.9 % der Theorie)
¹H-NMR (300 MHz, d⁶-DMSO): δ= 1.4-1.6 (m, 4H), 2.17 (t, 2H), 2.43 (t, 2H), 2.6 (m, 2H), 2.75 (m, 2H, 3.55 (s, 3H), 3.64 (s, 2H), 3.70 (s, 3H), 5.05 (s, 2H), 6.7-7.4 (m, 11H), 7.55 (t, 4H), 7.85 (d, 2H).

### Beispiel 7: Methyl 4-[((5-methoxy-5-oxopentyl){2-[(4-phenethylbenzyl)oxy]phenethyl}amino)methyl]benzoat (über Verfahren G)

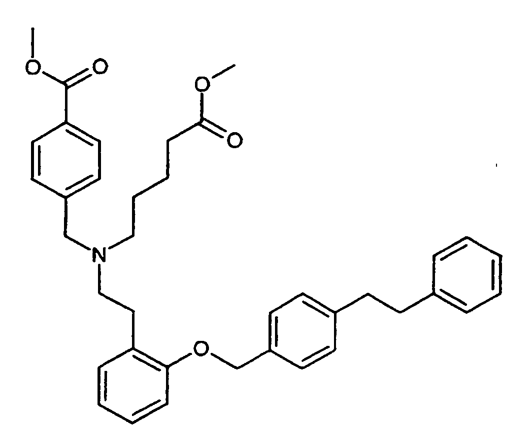

781.8 mg (1.34 mmol) Methyl-4-({(5-methoxy-5-oxopentyl)[2-({4-[(E)-2-phenylethenyl] benzyl}oxy)phenethyl]amino}methyl)benzoat aus Bsp. 6 und 80.0 mg 10% Palladium auf Aktivkohle werden in 10 ml Ethylacetat unter Atmosphärendruck hydriert. Nach 1 Stunde ist die berechnete Menge Wasserstoff aufgenommen. Die Lösung wird filtriert und das Lösungsmittel unter vermindertem Druck abdestilliert. Das Rohprodukt wird durch Chromatographie an Kieselgel mit Cyclohexan/ Ethylacetat=10:1 als Laufmittel gereinigt.
Ausbeute: 309 mg (38.9 % der Theorie)
¹H -NMR (300 MHz, d⁶-DMSO): δ= 1.42 (m, 4H), 2.15 (t, 2H), 2.41 (t, 2H), 2.57 (m, 2H), 2.72 (m, 2H), 2.85 (s, 4H), 3.55 (s, 3H), 3.60 (s, 2H), 3.82 (s, 2H), 4.98 (s, 2H), 6.8-7.4 (m, 15H), 7.85 (d, 2H).

### Beispiel 8: 4-[((4Carboxybutyl)-{2-[(4-phenethylbenzyl)oxy]phenethyl}amino)methyl]benzoesäure Hydrochlorid (über Verfahren E)

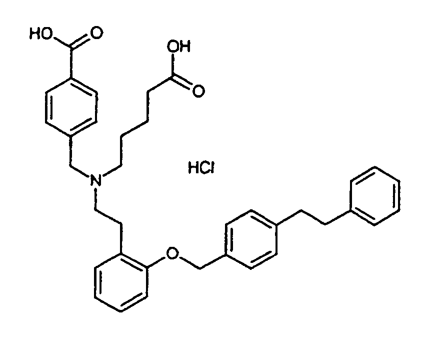

262.60 mg (0.442 mmol) Methyl 4-[((5-methoxy-5-oxopentyl){2-[(4-phenethylbenzyl)oxy]phenethyl}amino)methyl]benzoat aus Bsp. 7 werden in 2 ml Dioxan vorgelegt, mit 0.2 ml 45 prozentiger NaOH versetzt und 18 Stunden auf 60°C erhitzt. Das Dioxan wird unter vermindertem Druck abdestilliert, der Rückstand in Wasser aufgenommen und mit 2N HCl auf pH 4 gestellt. Der ausgefallene Niederschlag wird abfiltriert und getrocknet. 50 mg des Produkts werden in 2ml Methylenchlorid und 1ml Methanol gelöst, mit 1ml einer 4N Lösung von HCl in Dioxan vesetzt und 1h bei Raumtemperatur nachgerührt. Das Lösungsmittel wird unter vermindertem Druck abdestilliert und der Rückstand mit Ether/Petrolether verührt.
Ausbeute: 34.0 mg (56.2 % der Theorie) weiße Kristalle
¹H -NMR (300 MHz, d⁴-Methanol): δ= 1.52 (m, 2H), 1.72 (m, 2H), 2.25 (t, 2H), 2.90 (m, 4H), 3.15 (m, 2H), 3.30 (m, 4H), 4.38 (s, 2H), 5.08 (s, 2H), 6.8-7.3 (m, 13H), 7.55 (d, 2H), 8.05 (d, 2H).

### Beispiel 8a: 4-[((4Carboxybutyl)-{2-[(4-phenethylbenzyl)oxy]phenethyl}amino)methyl]benzoesäure

Die freie Carbonsäure wurde auf gleichem Weg, aber ohne den letzten Schntt der Umsetzung mit HCl hergestellt:
¹H -NMR (300 MHz, d⁶-DMSO): δ= 1.45 (m, 4H), 2.10 (m, 2H), 2.30-3.60 (m), 5.08 (s, 2H), 6.80 (m, 1H), 6.90 (m, 1H), 7.00-7.50 (m, 13H), 12.5 (bs).

Auf analoge Weise können folgende Verbindungen hergestellt werden:

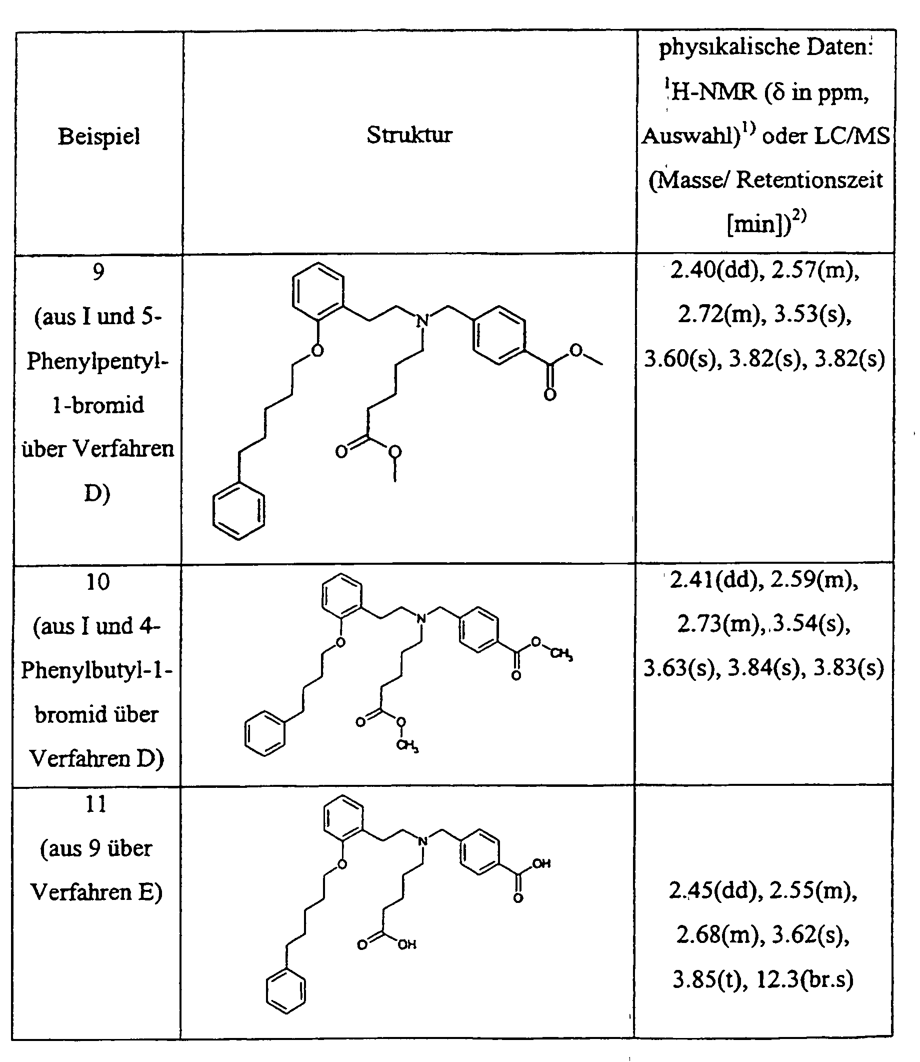

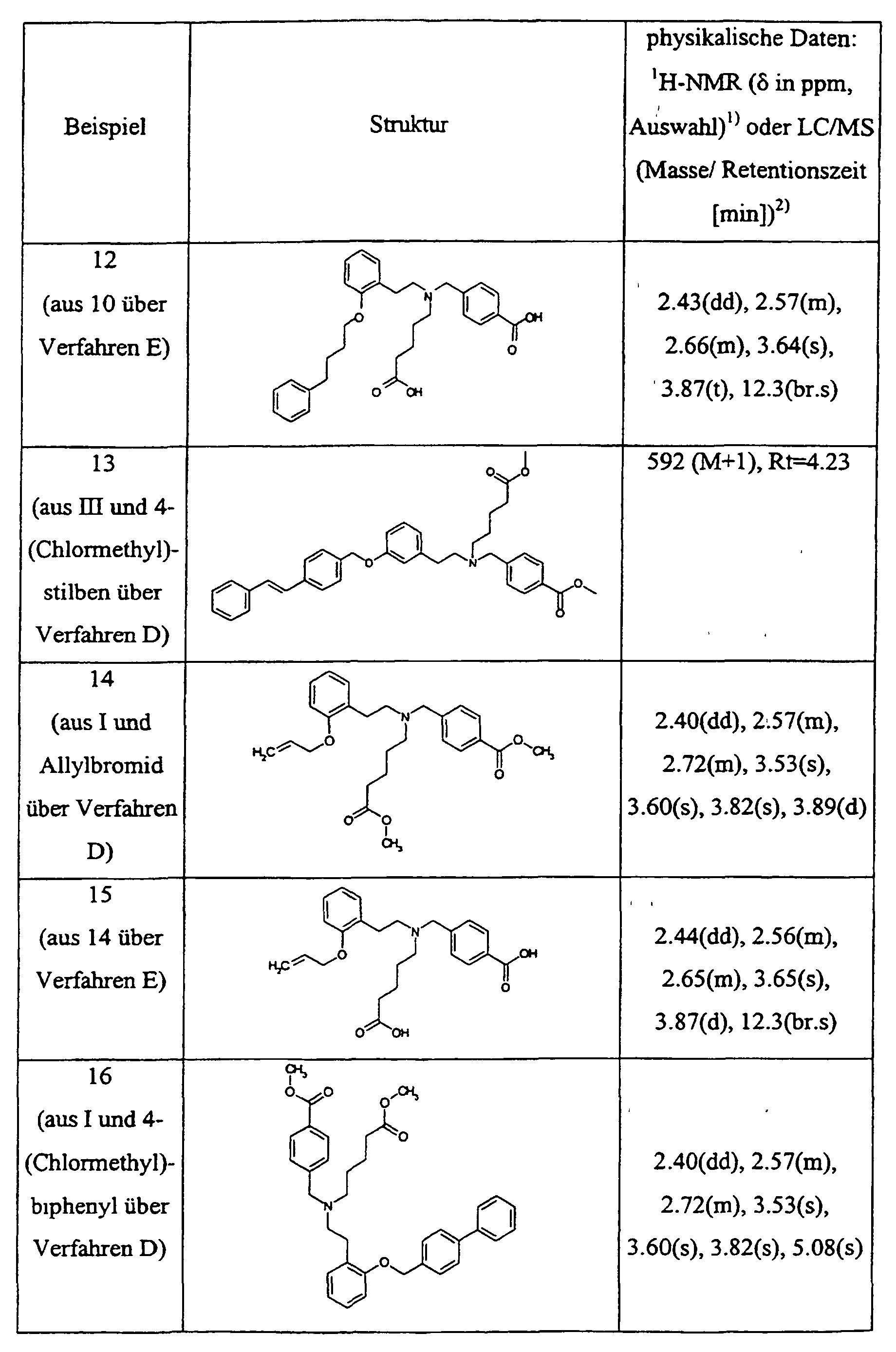

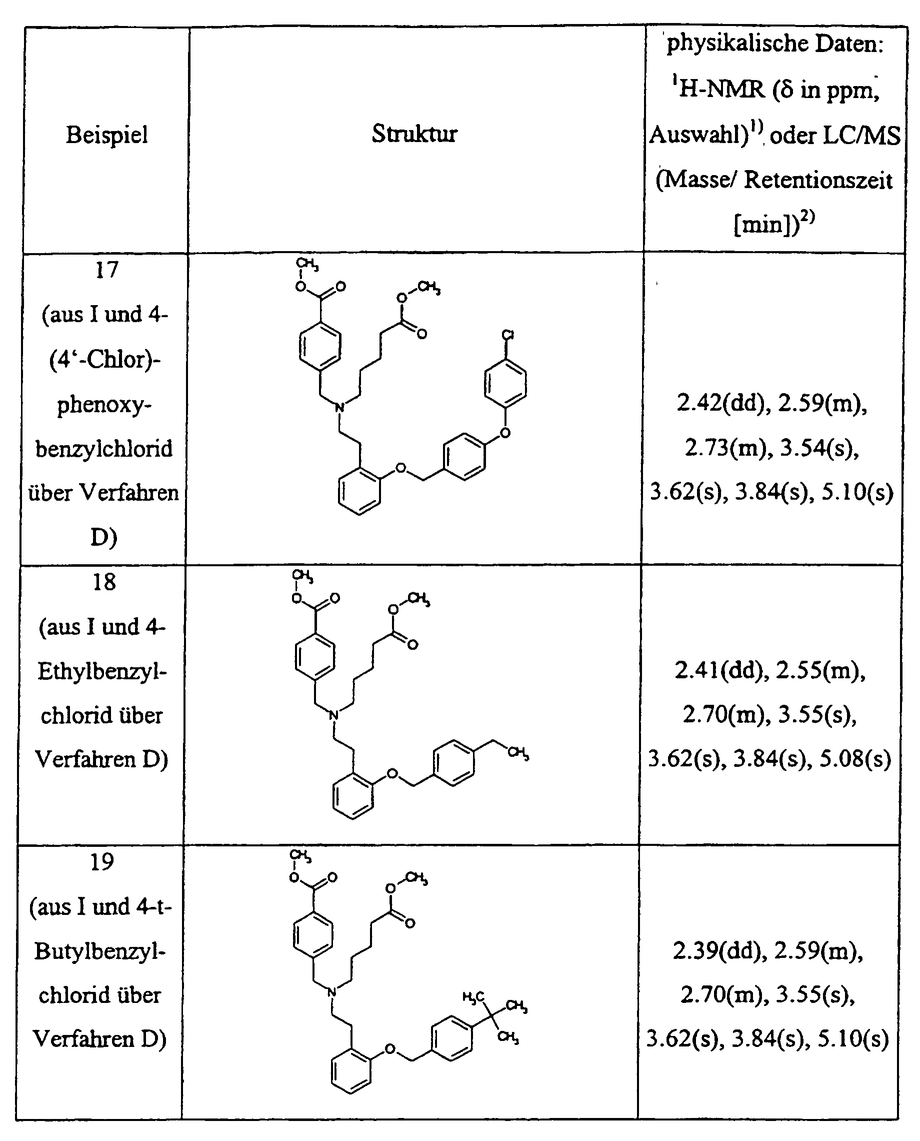

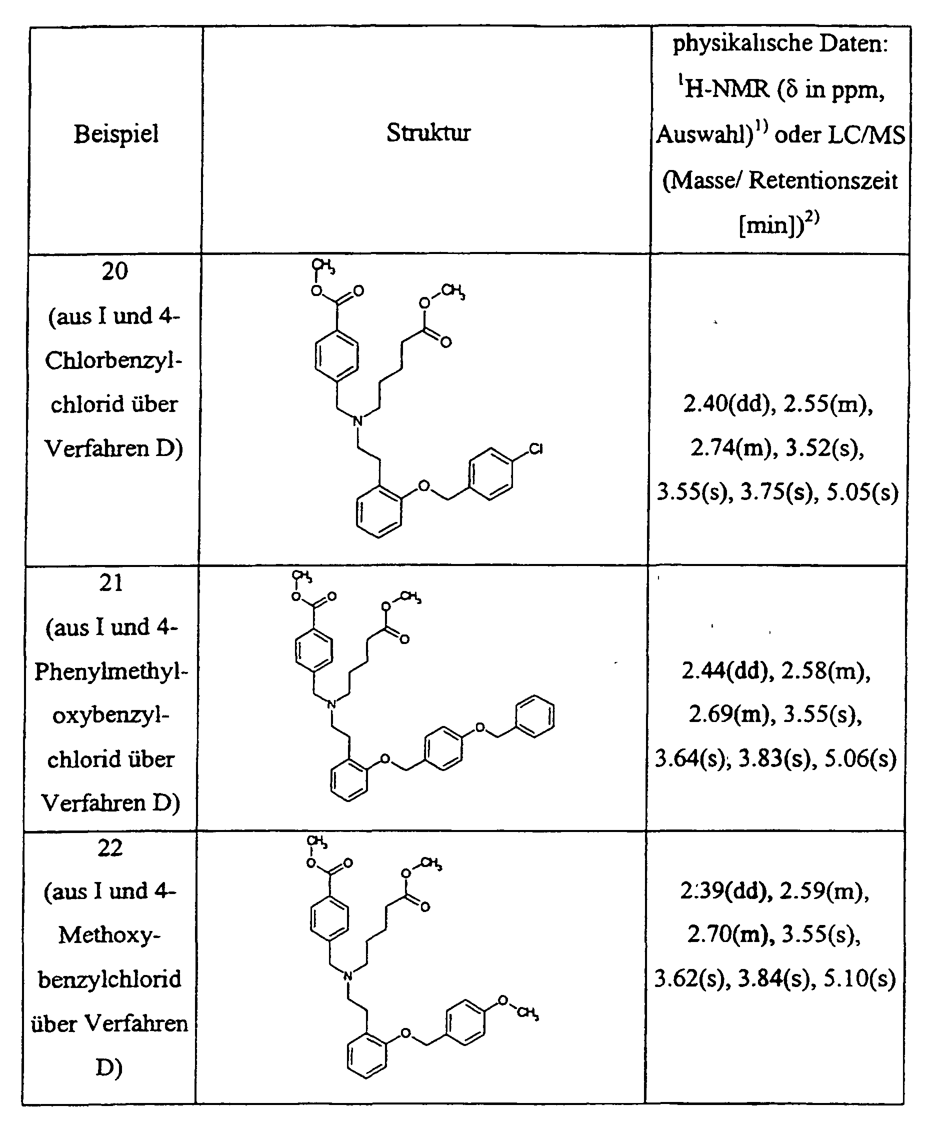

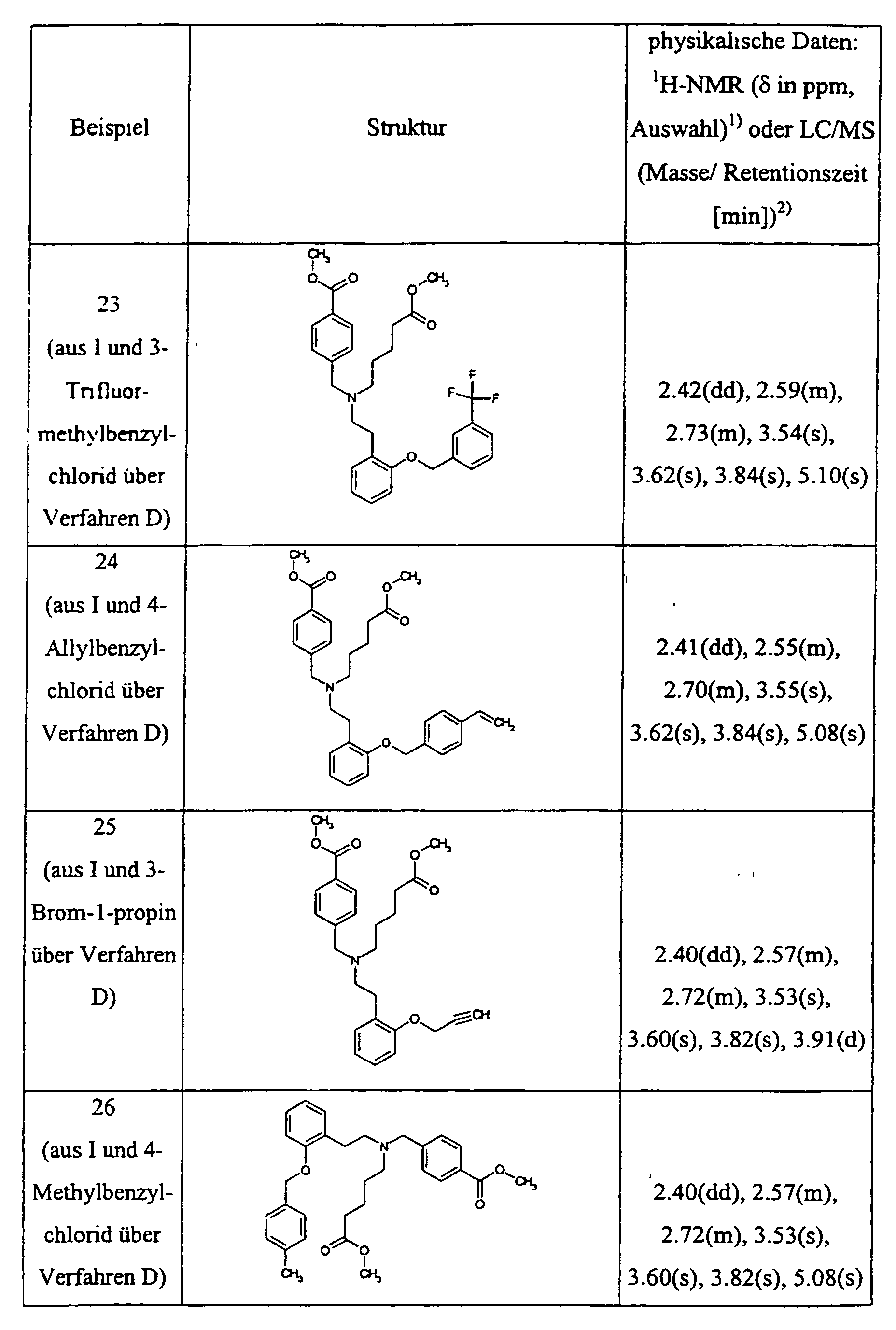

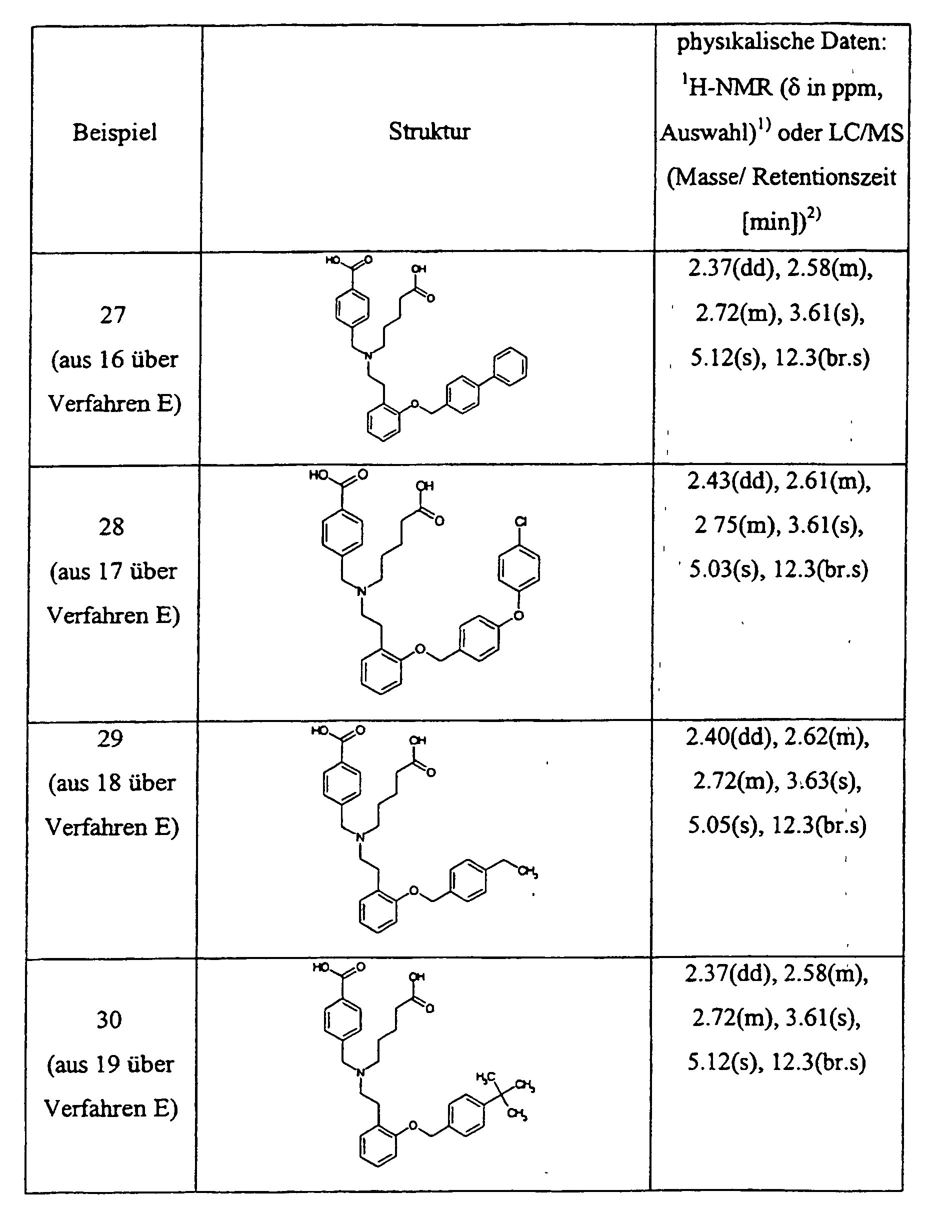

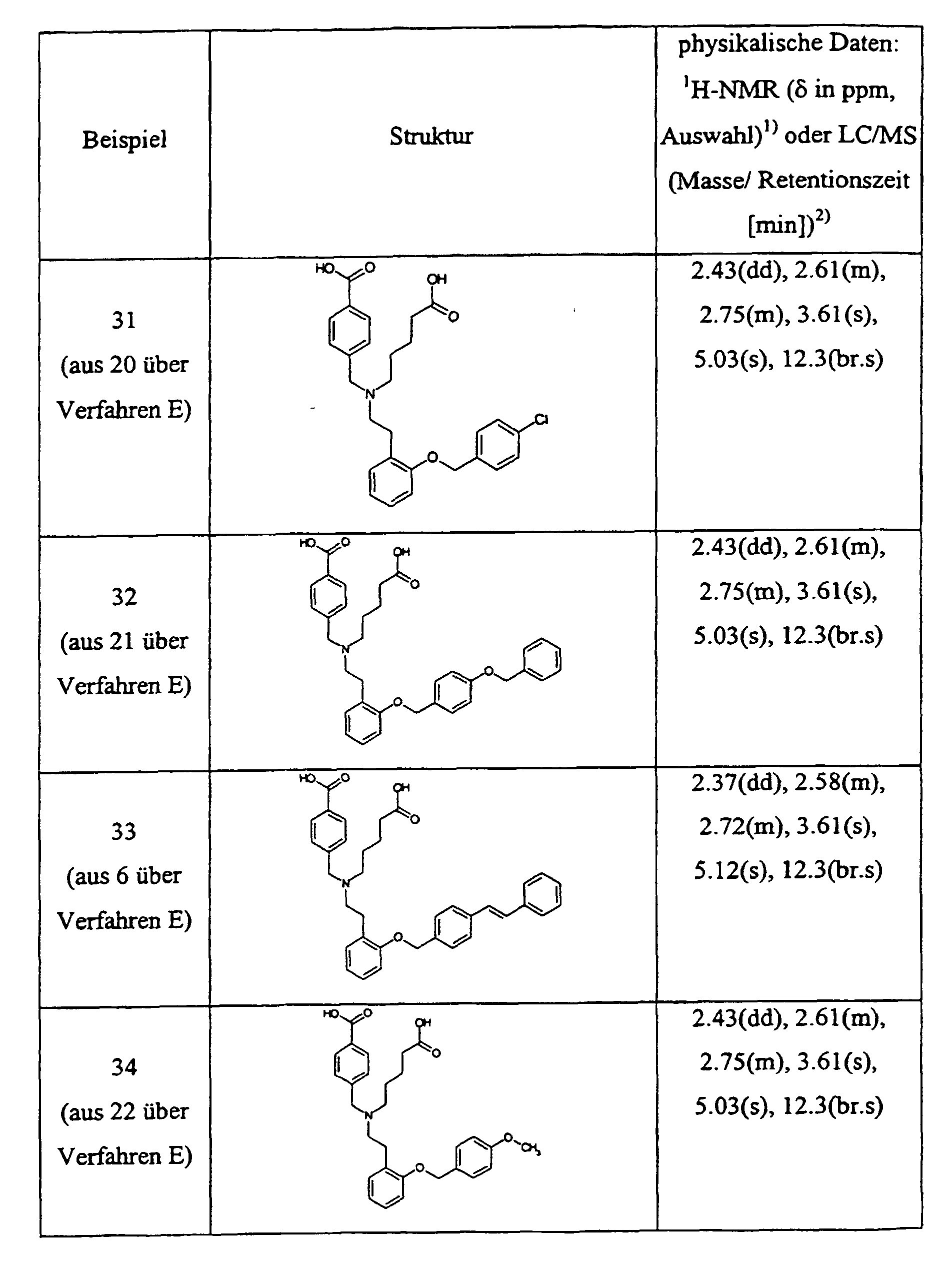

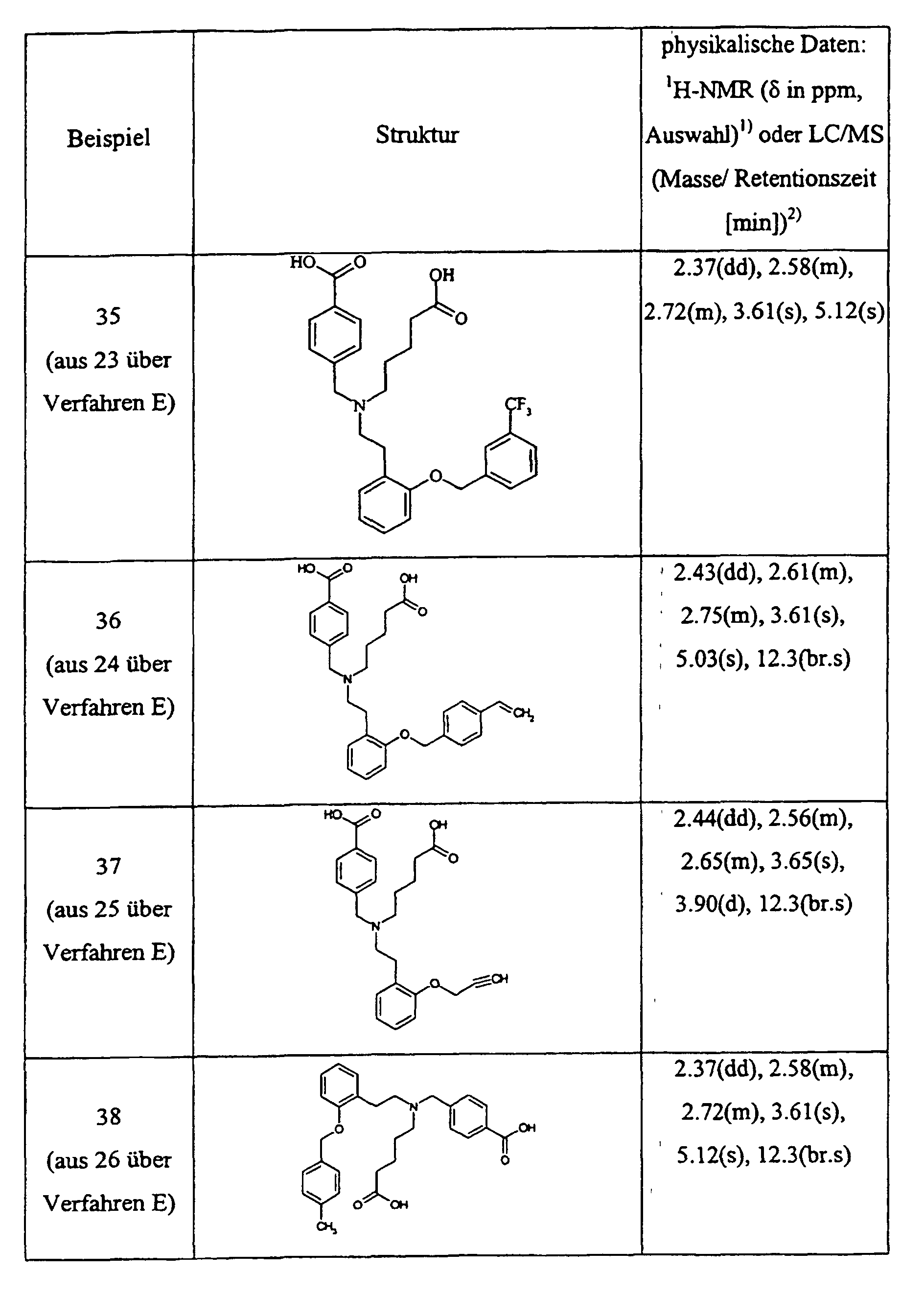

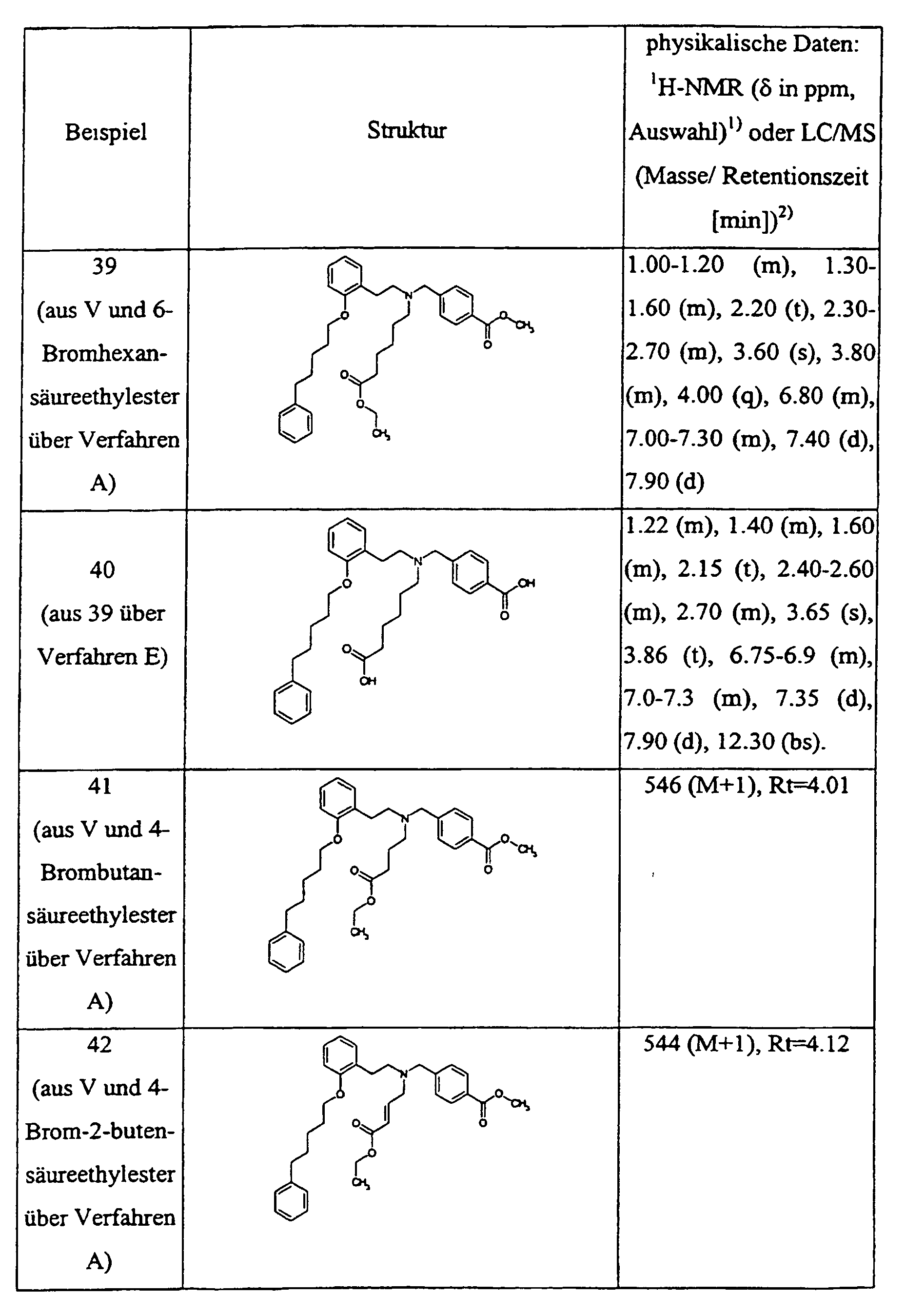

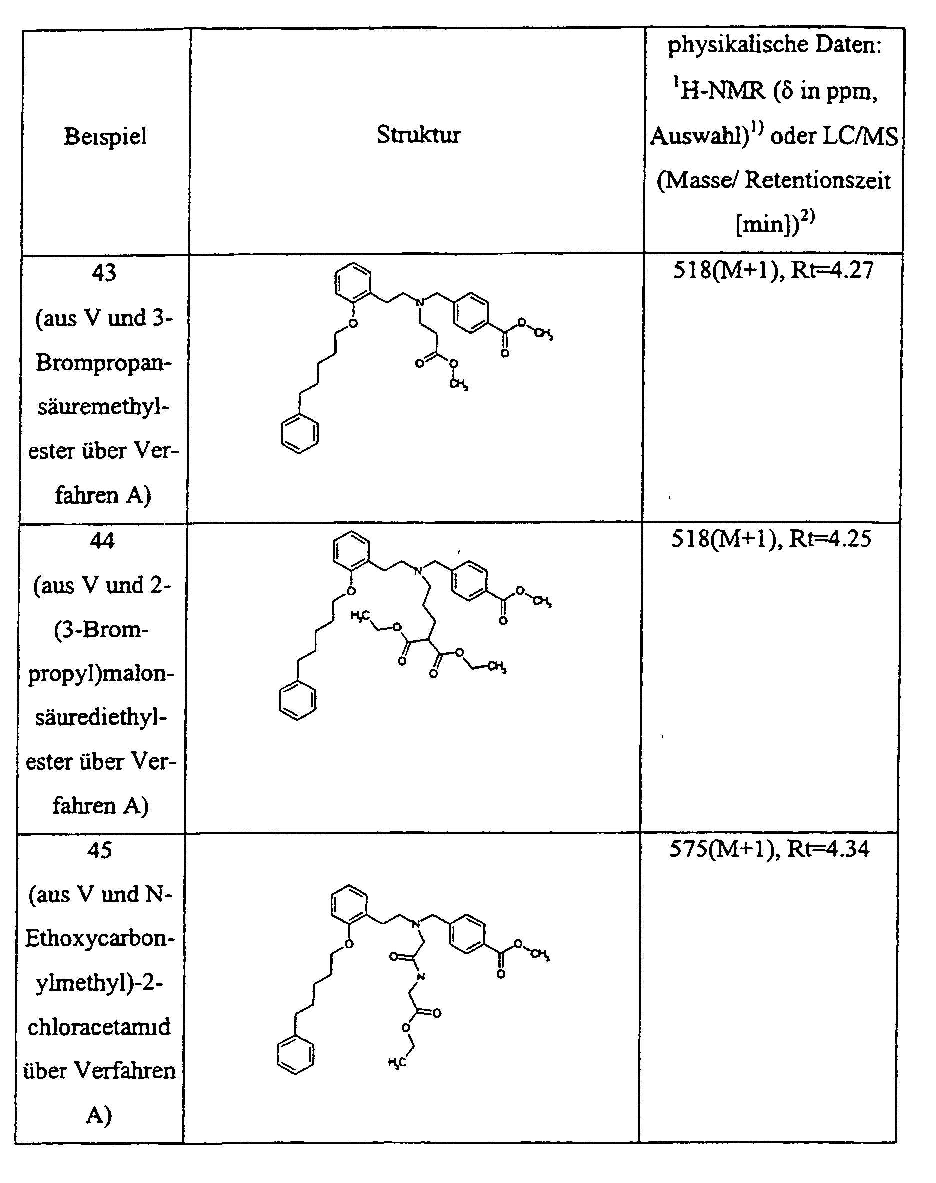

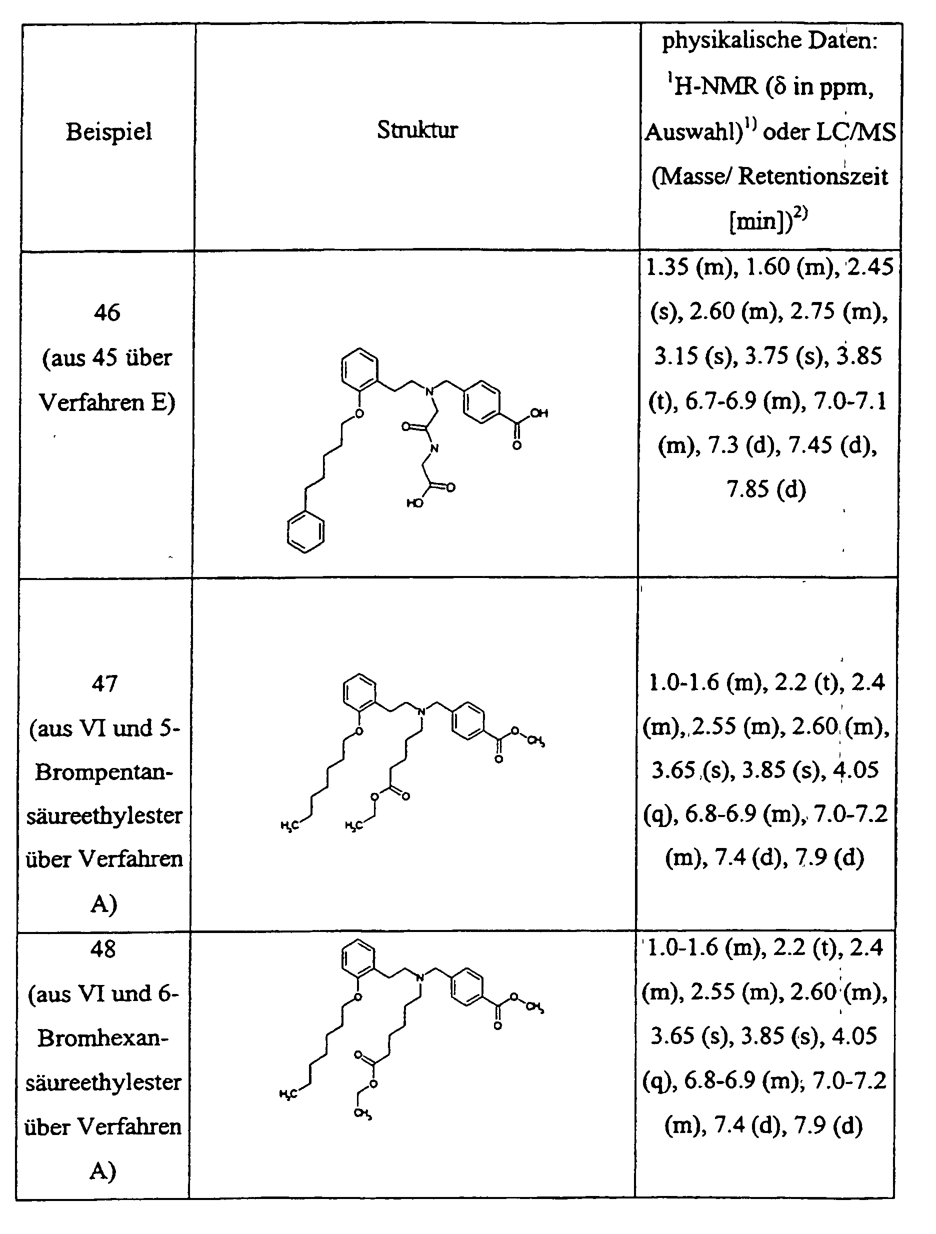

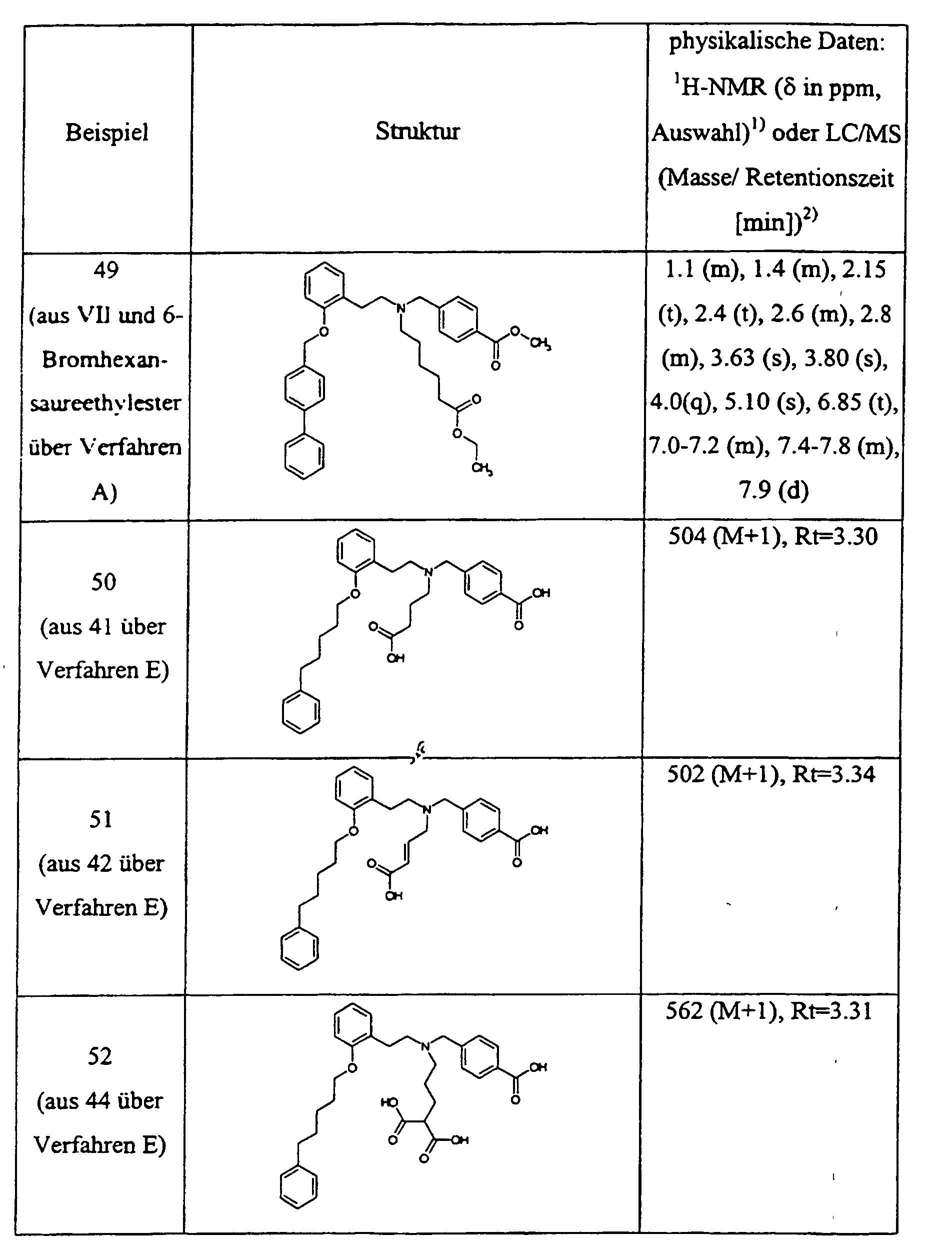

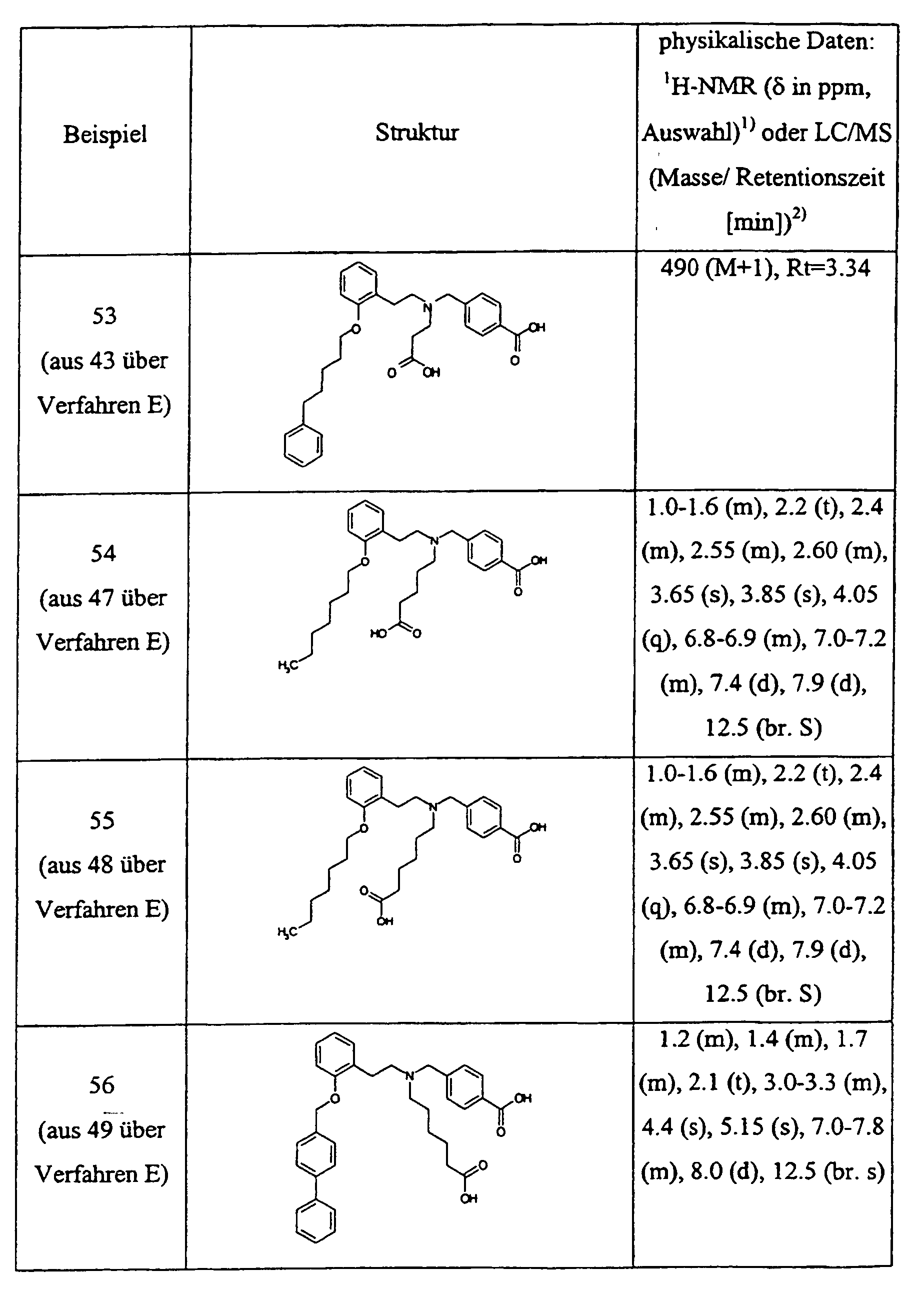

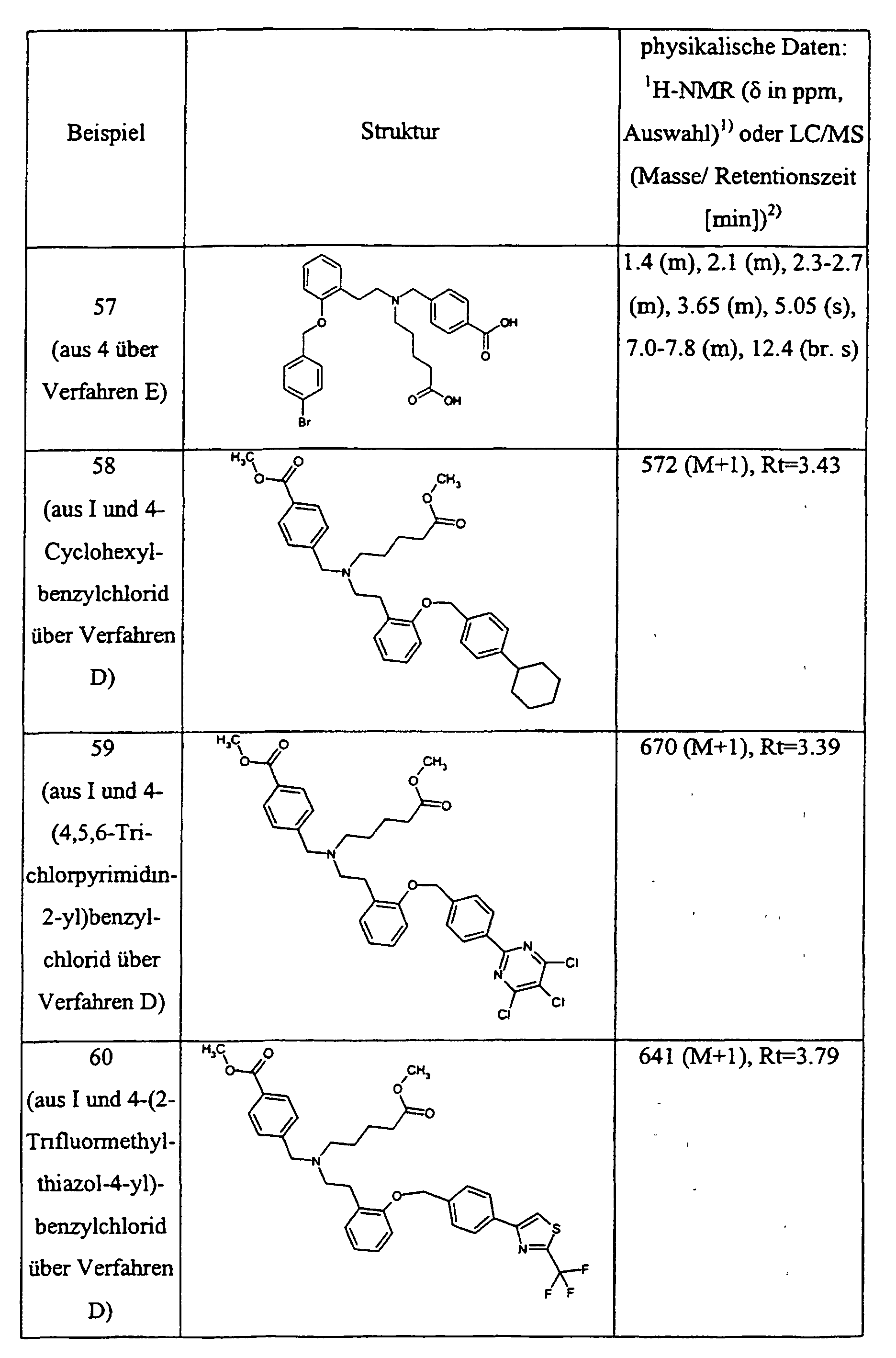

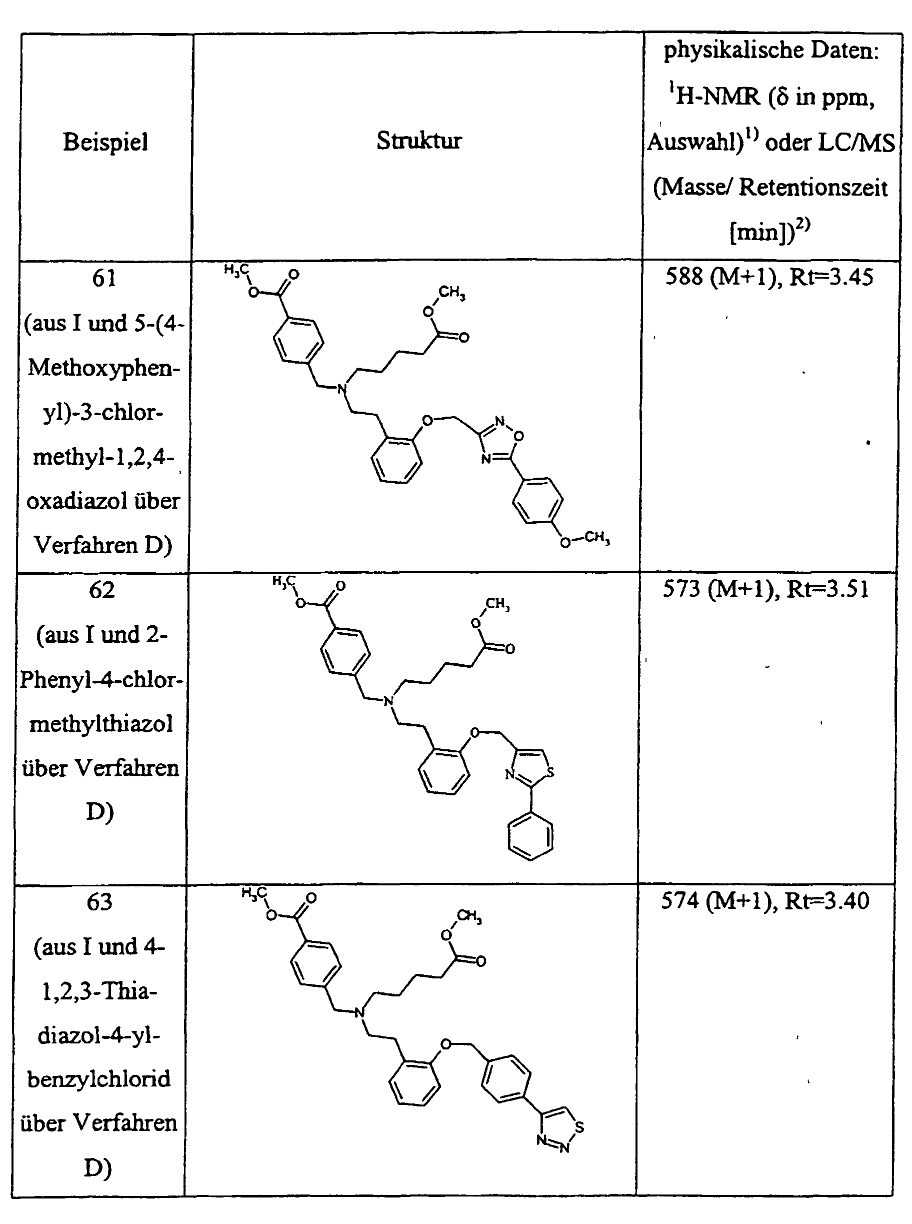

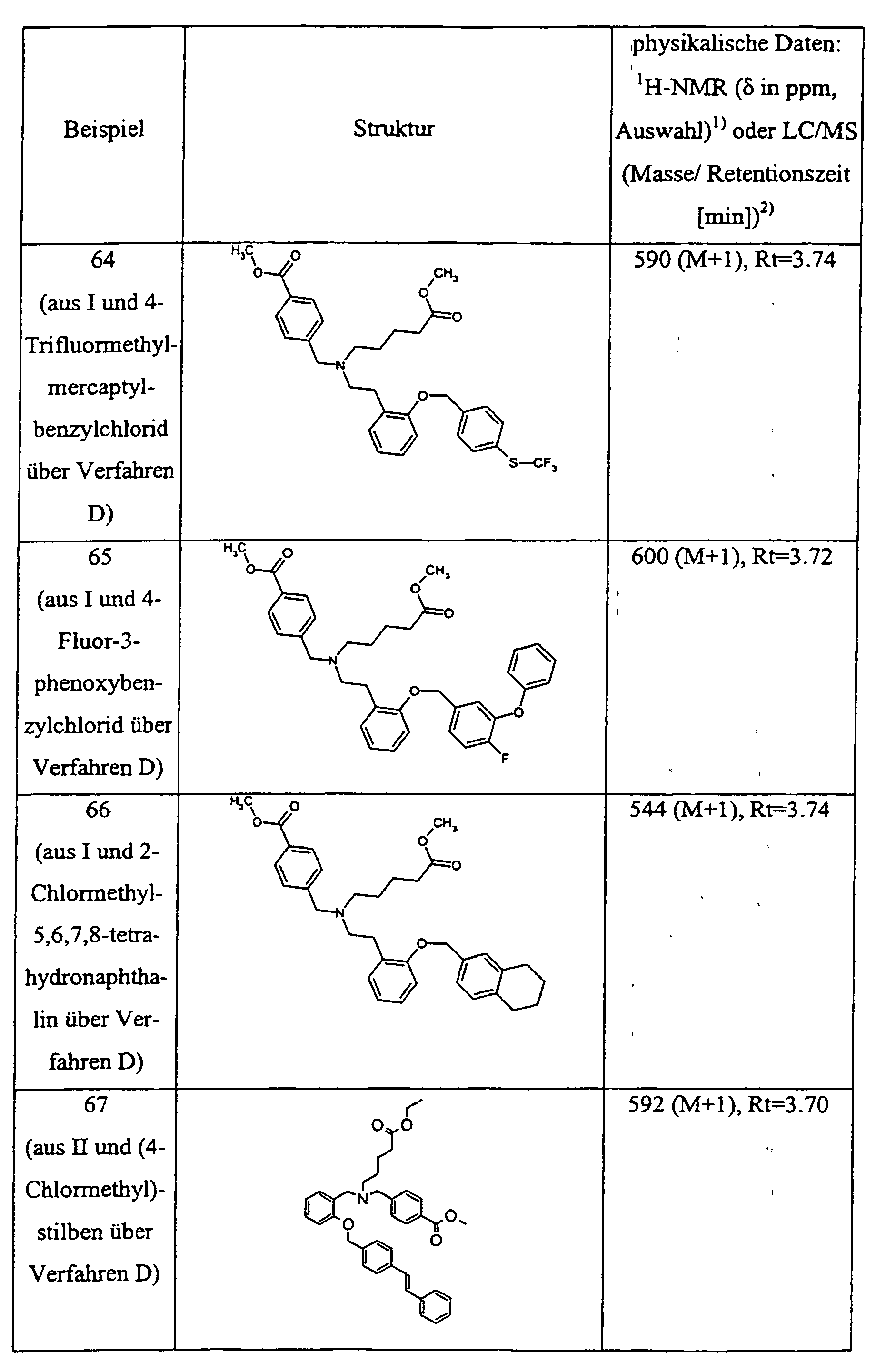

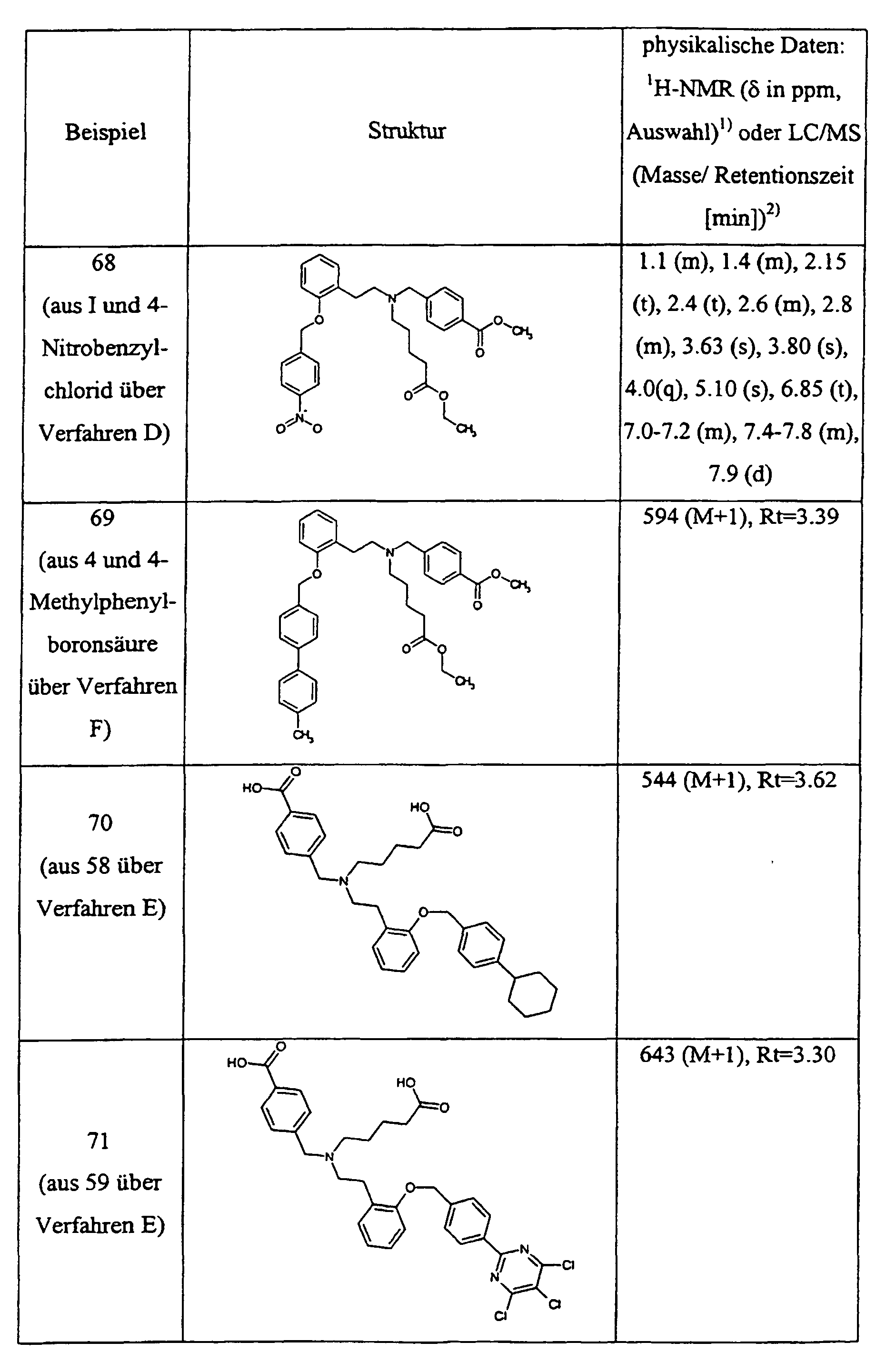

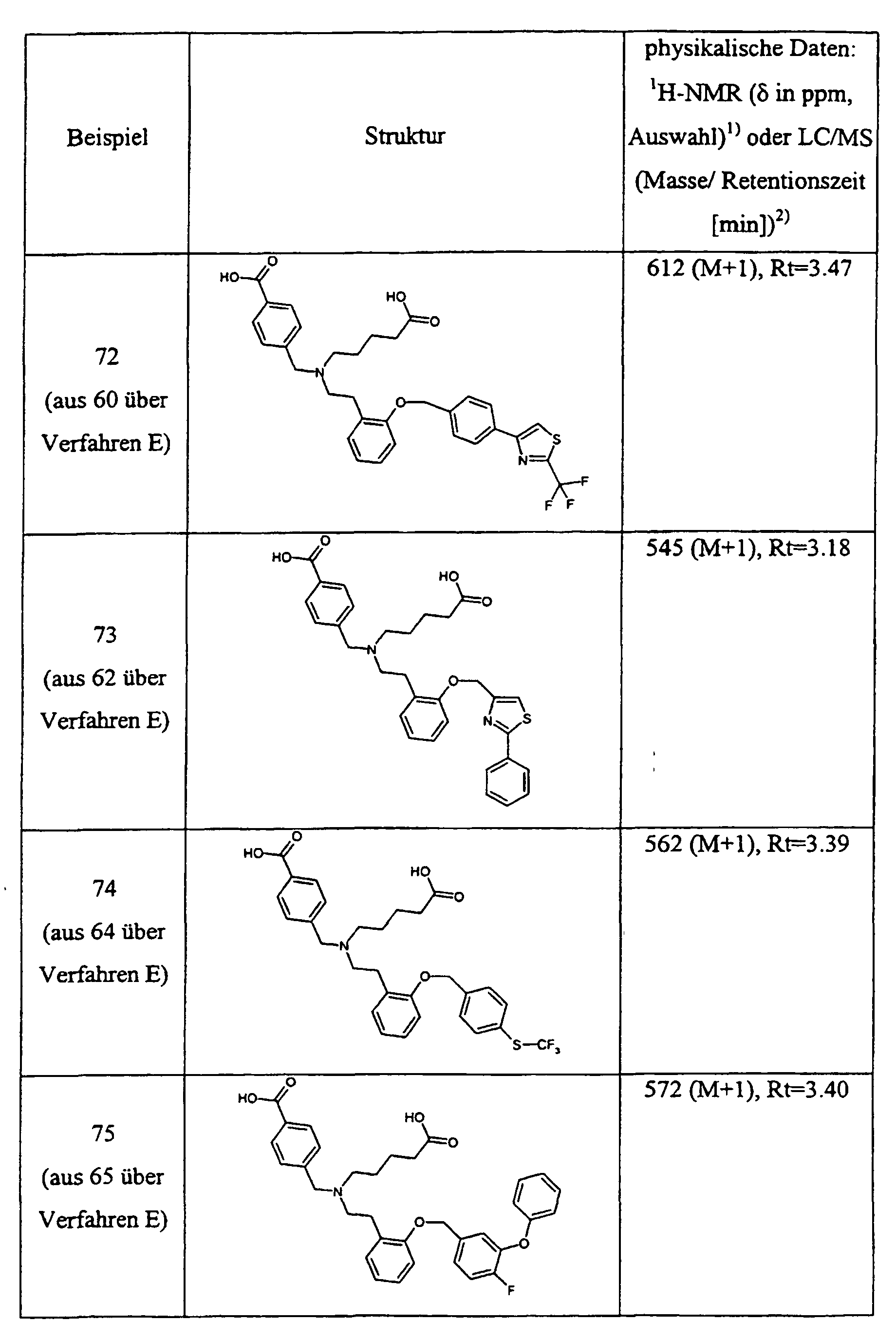

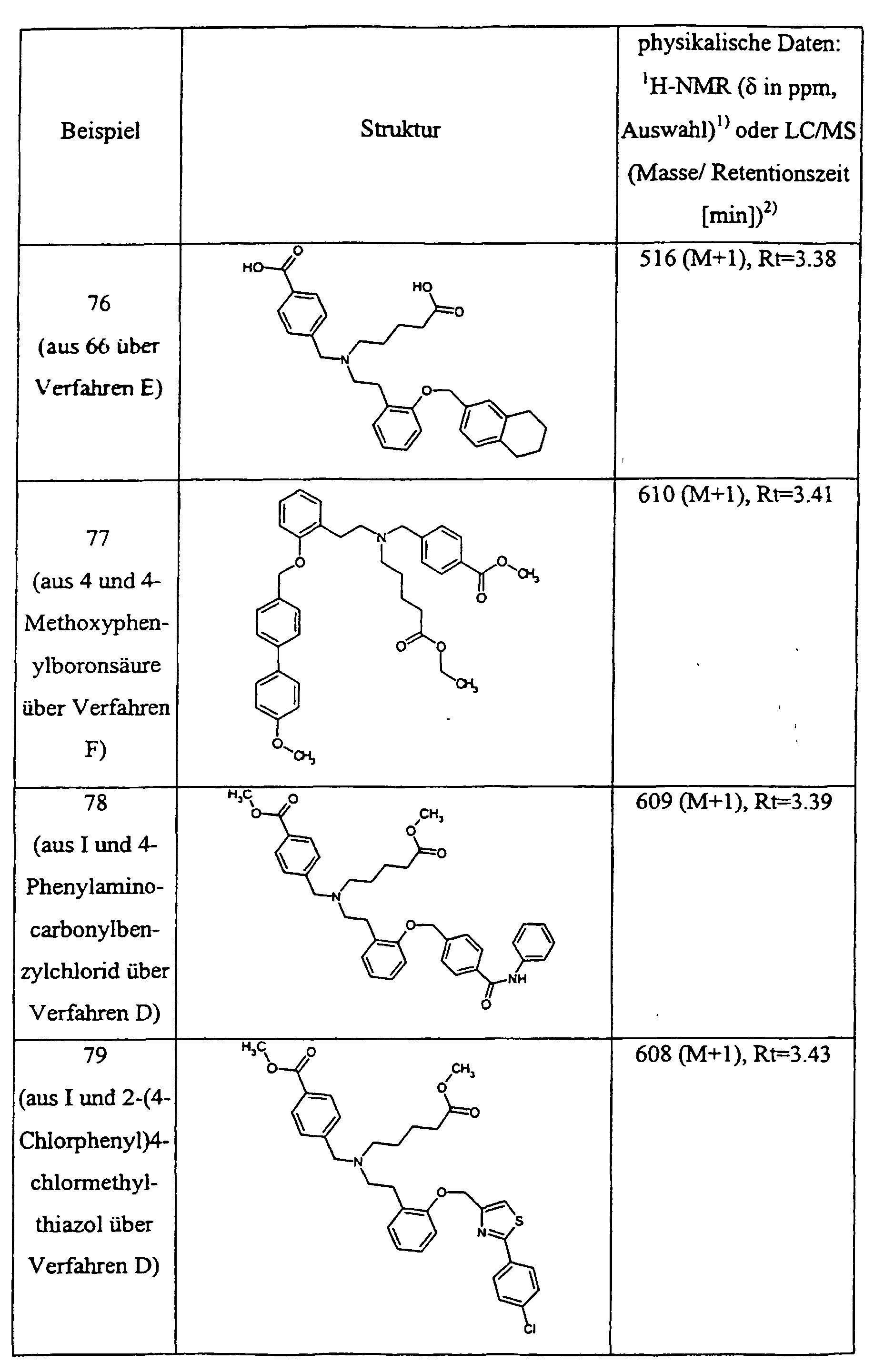

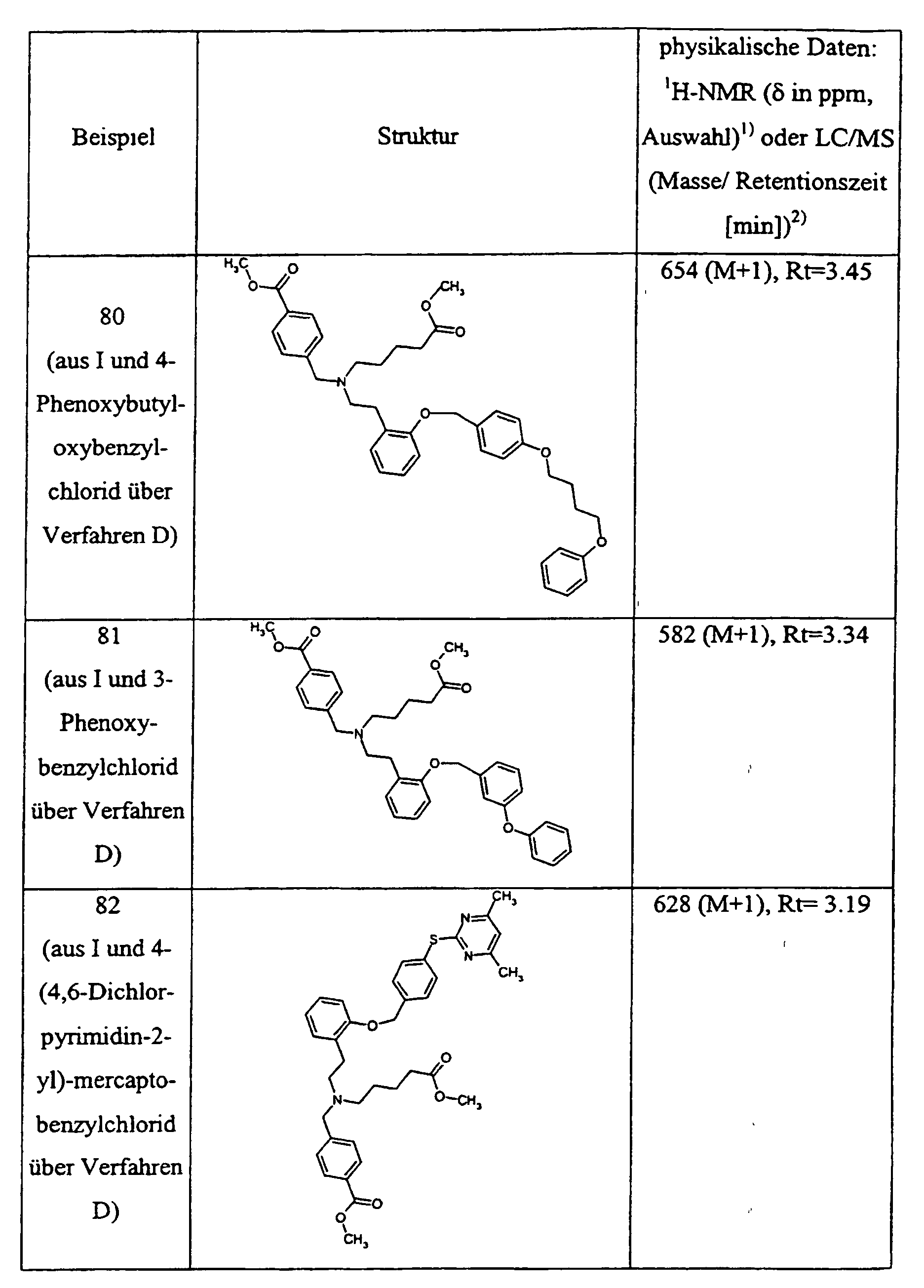

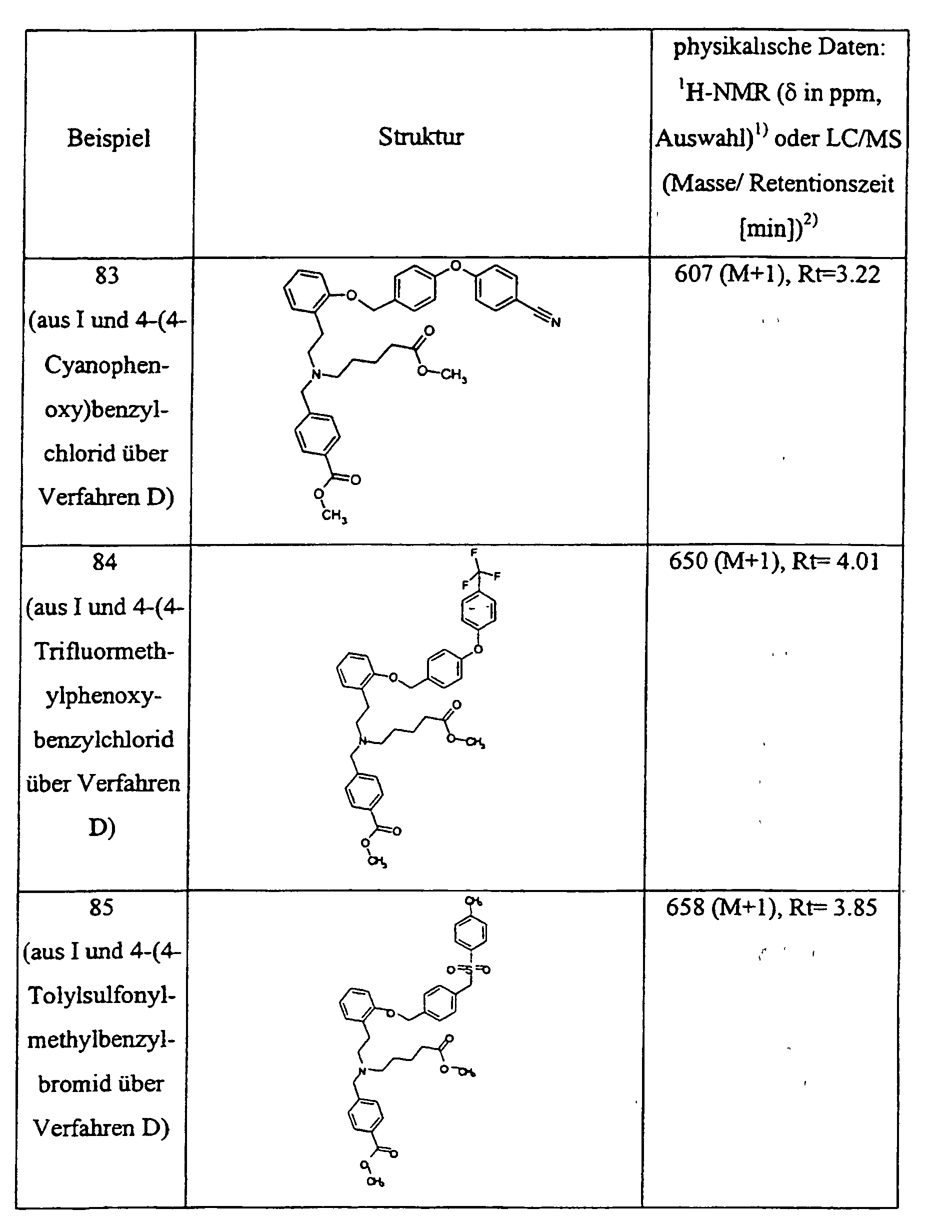

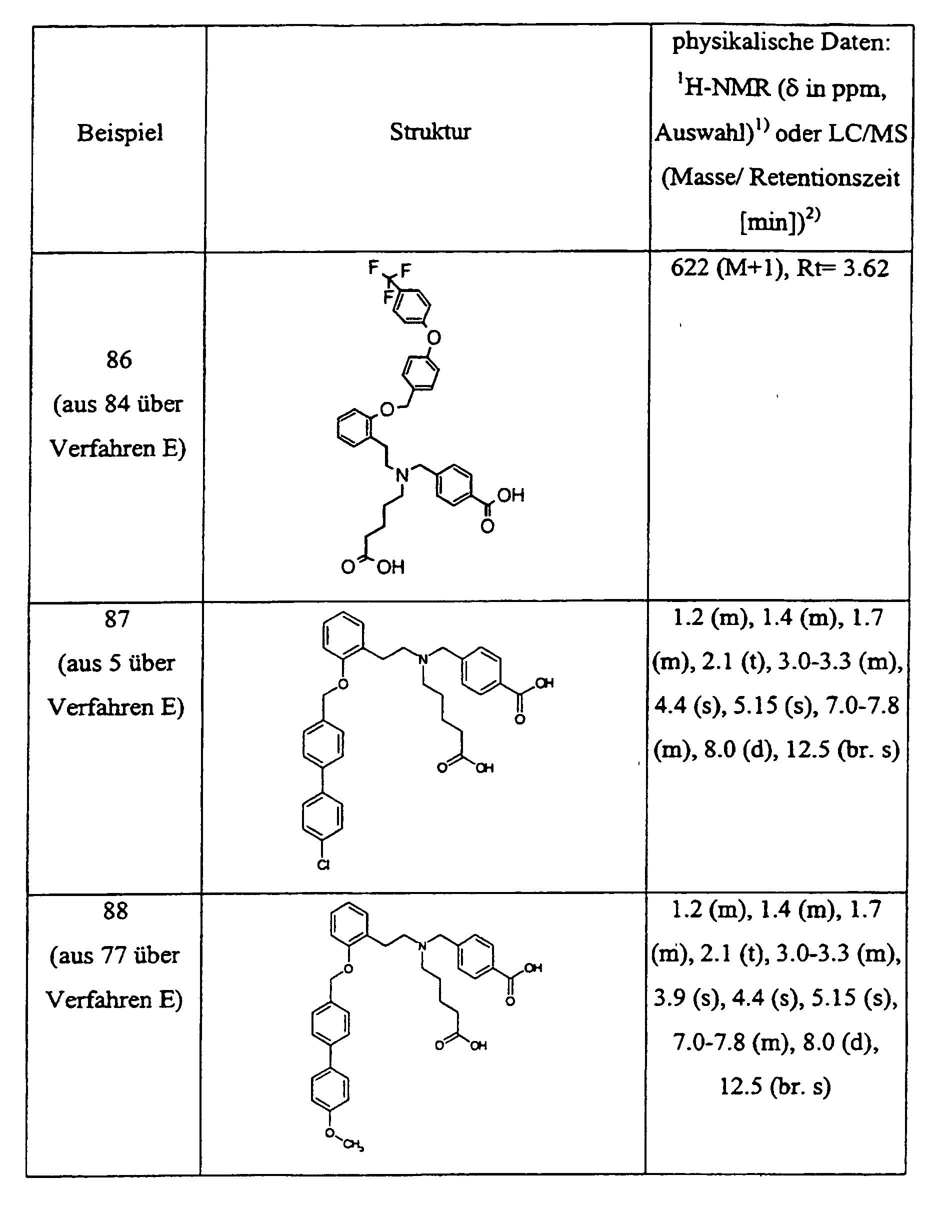

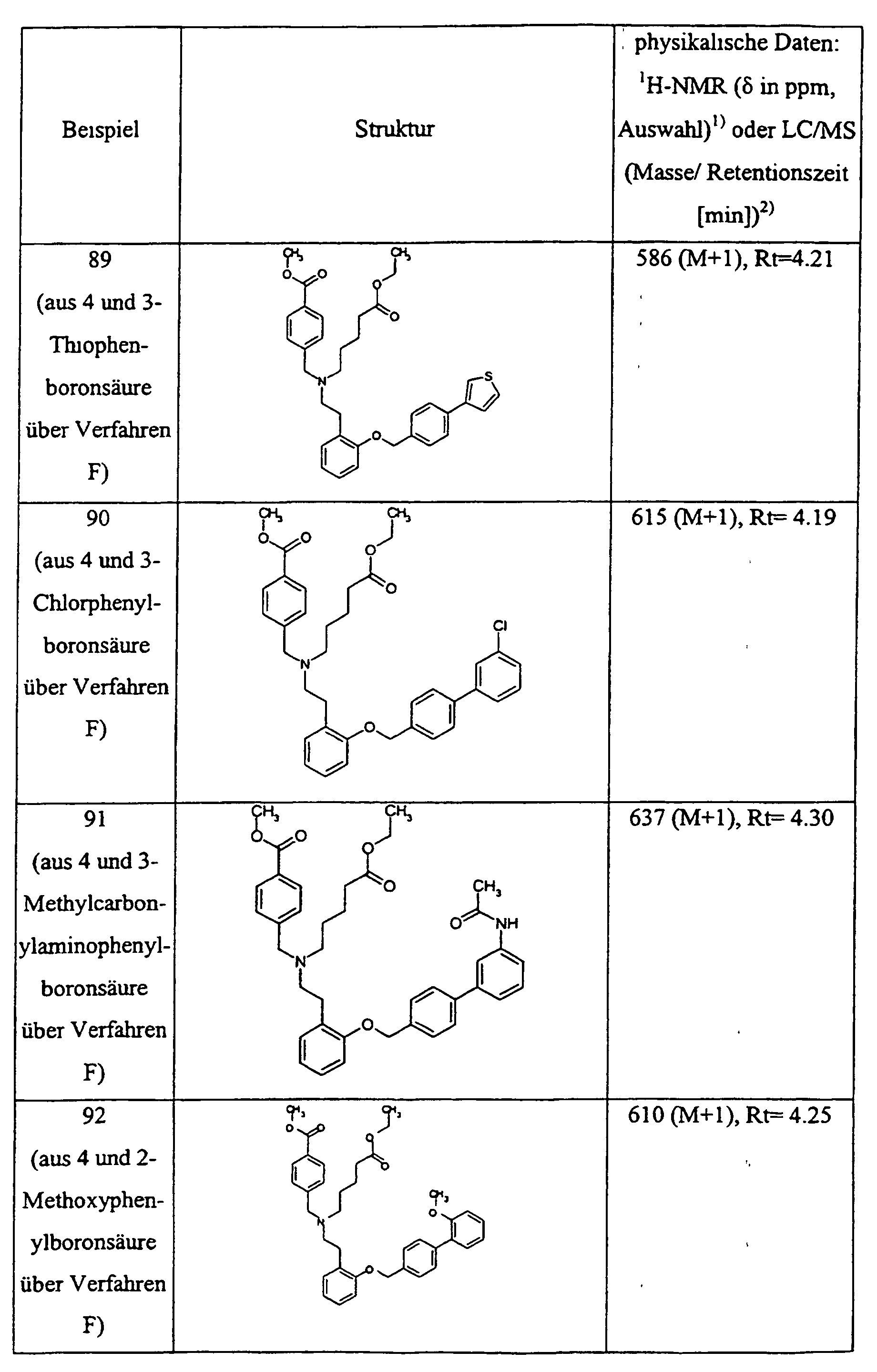

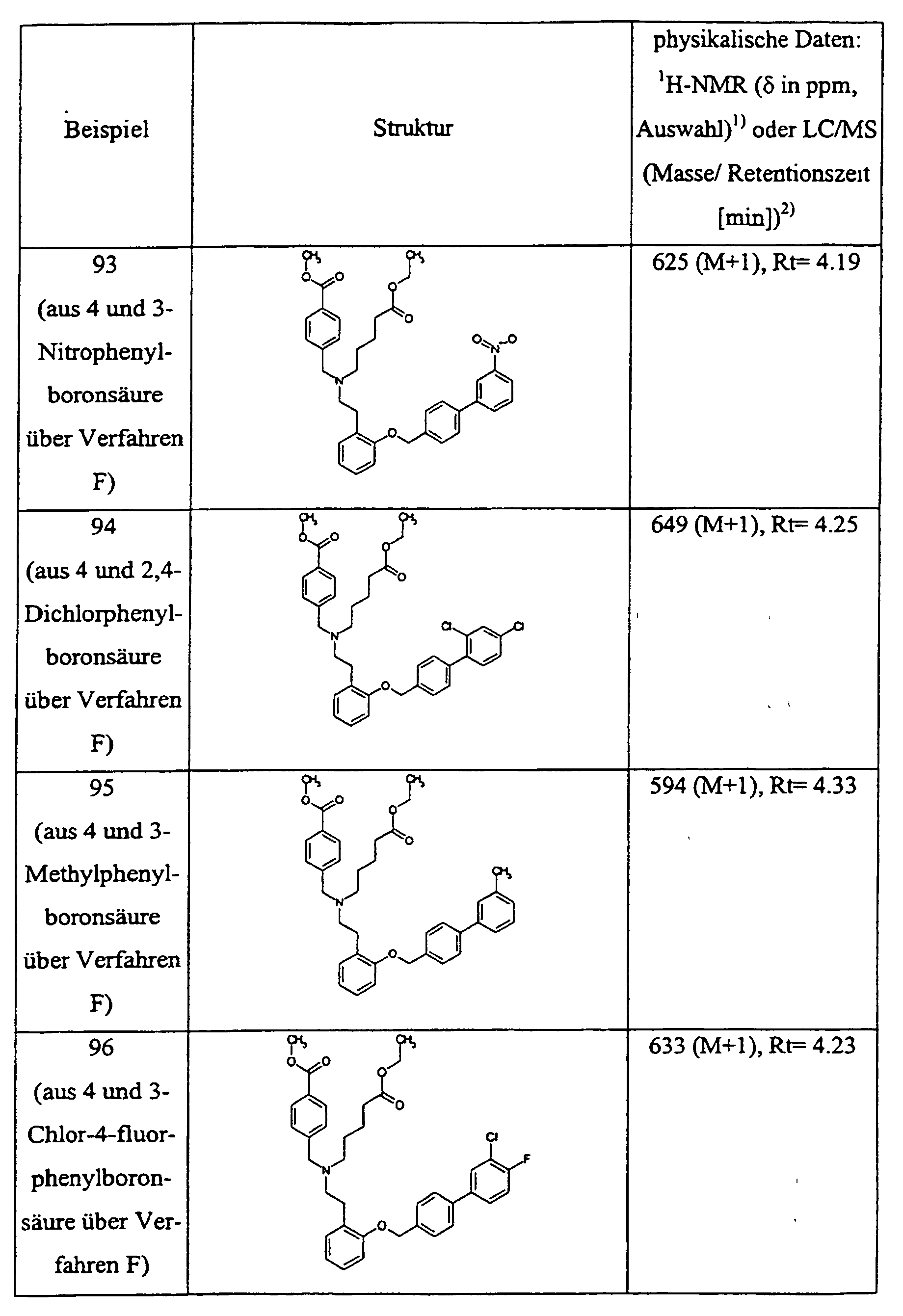

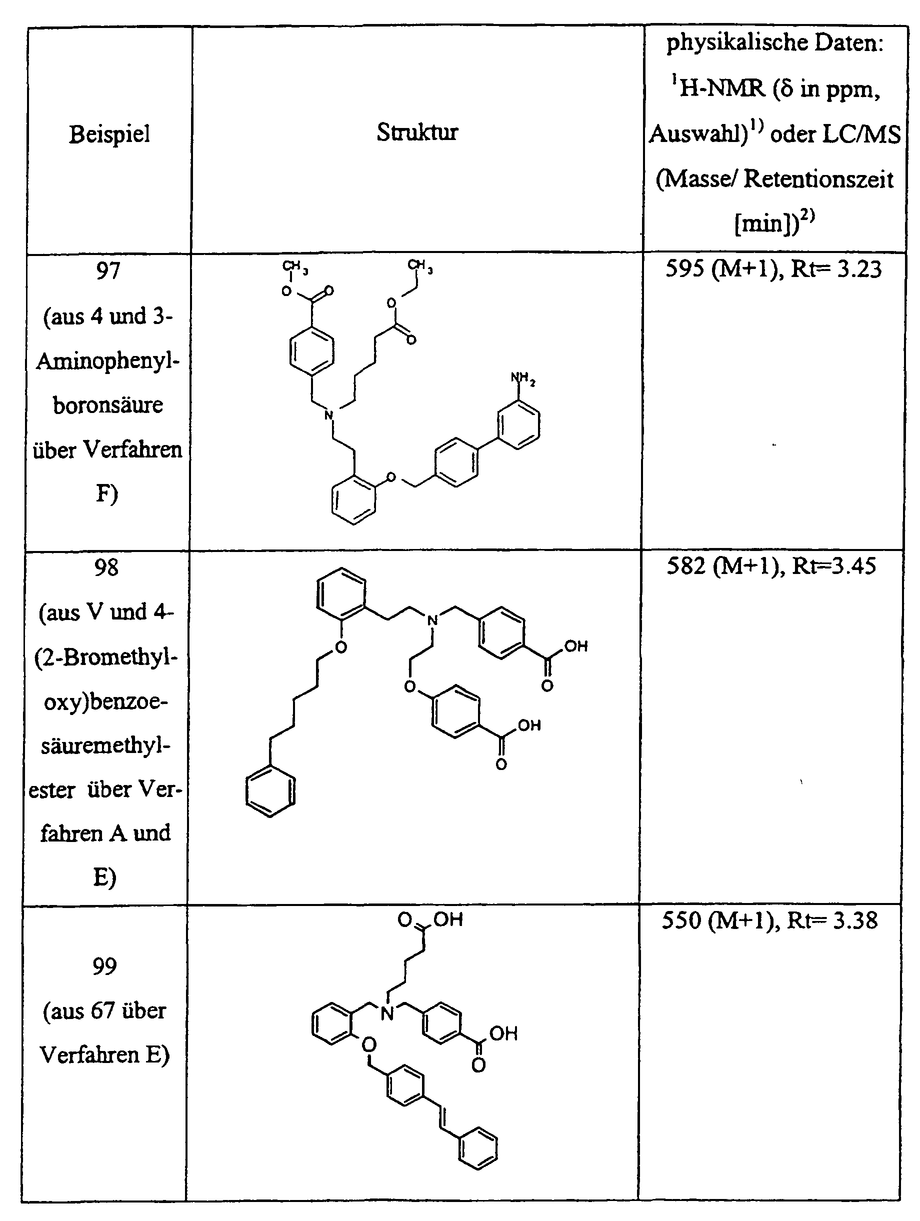

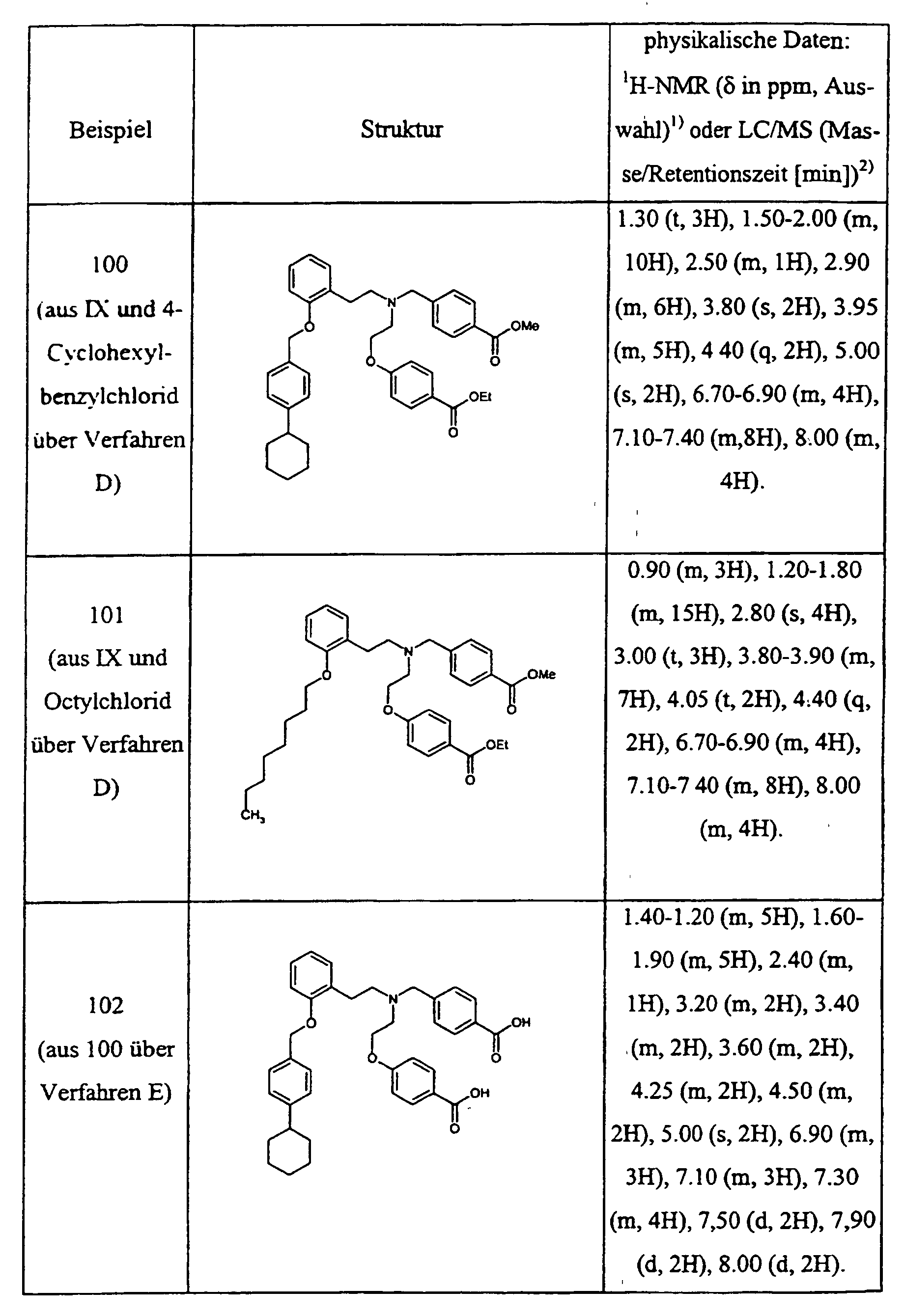

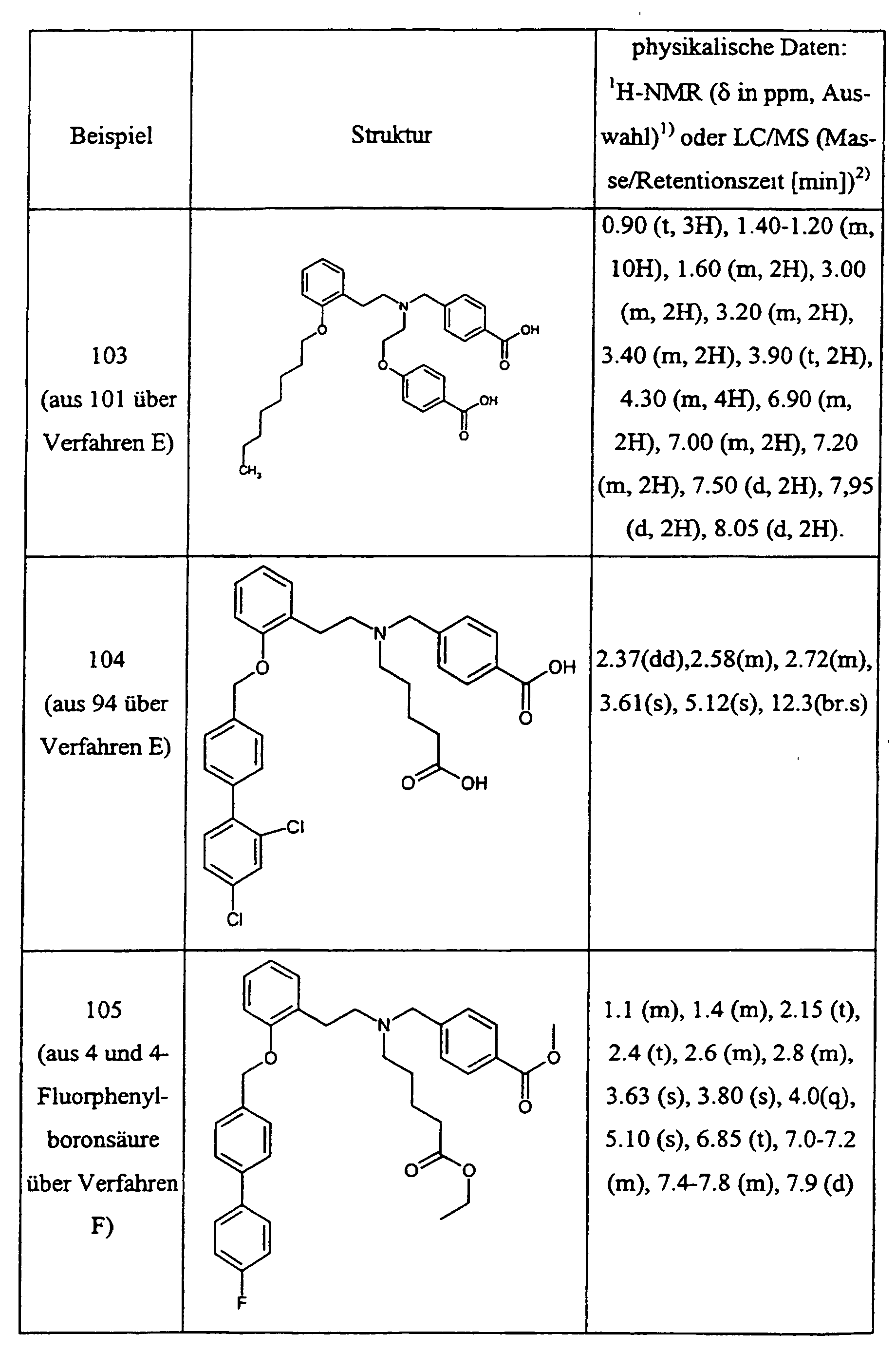

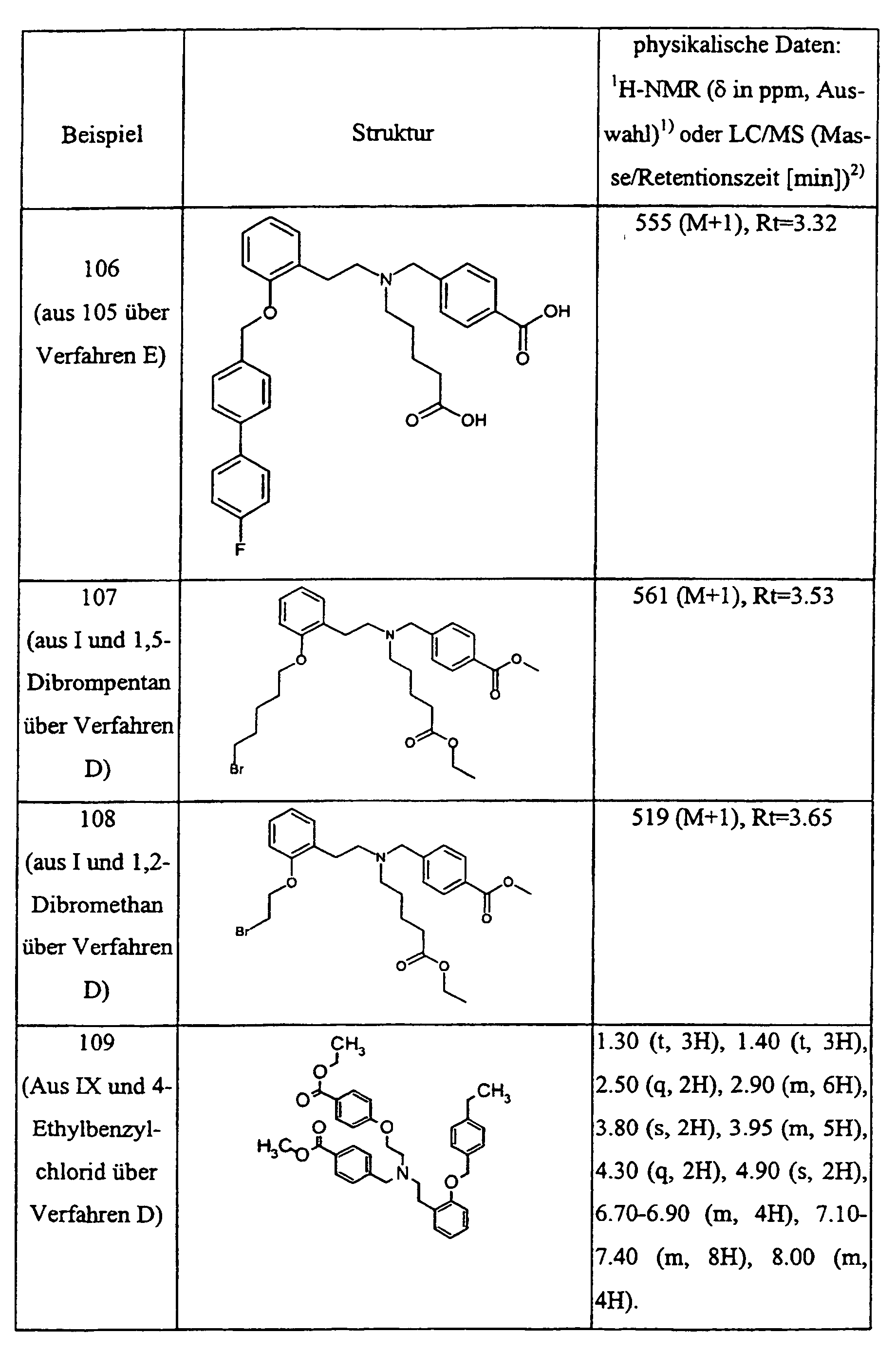

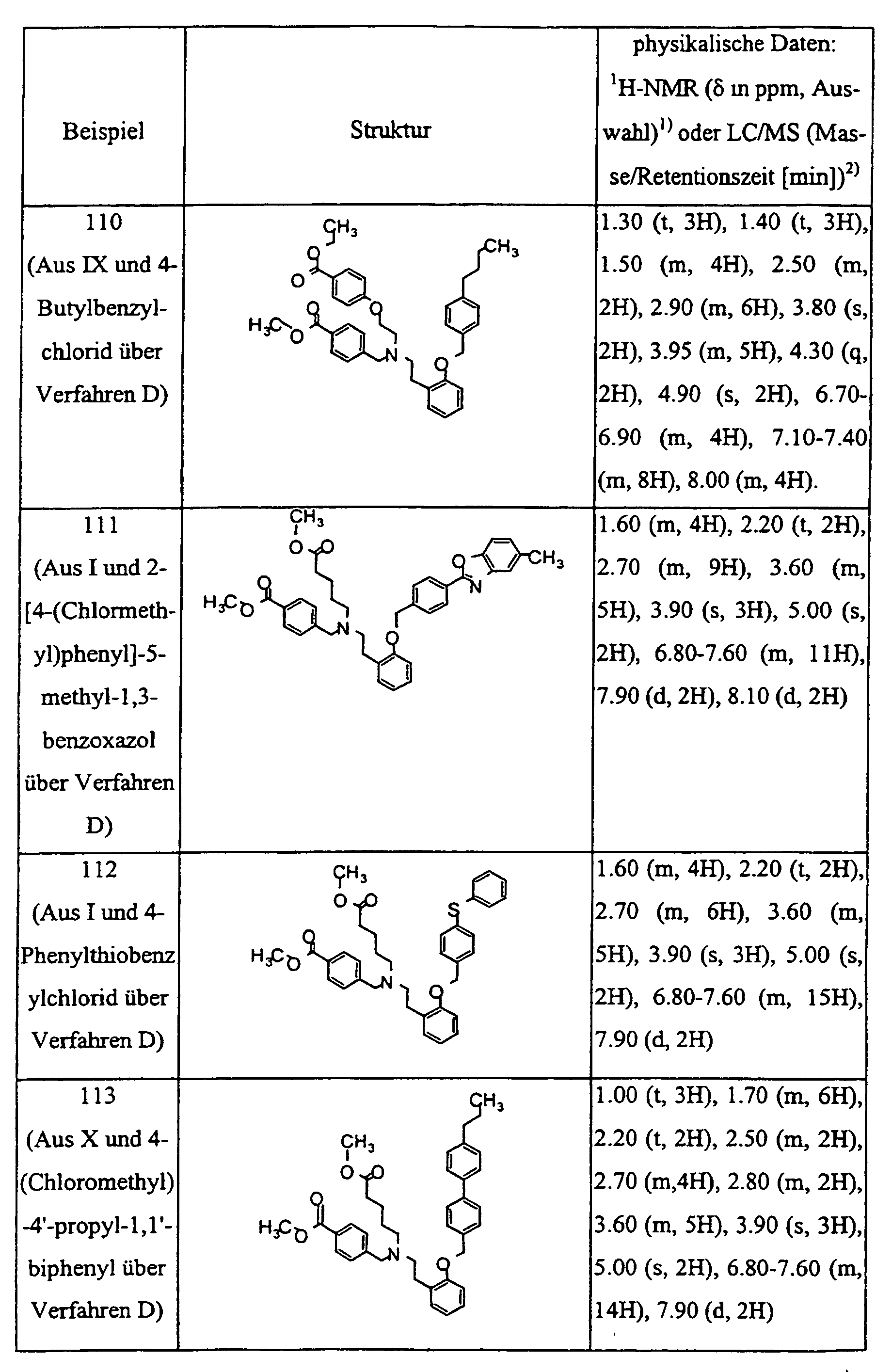

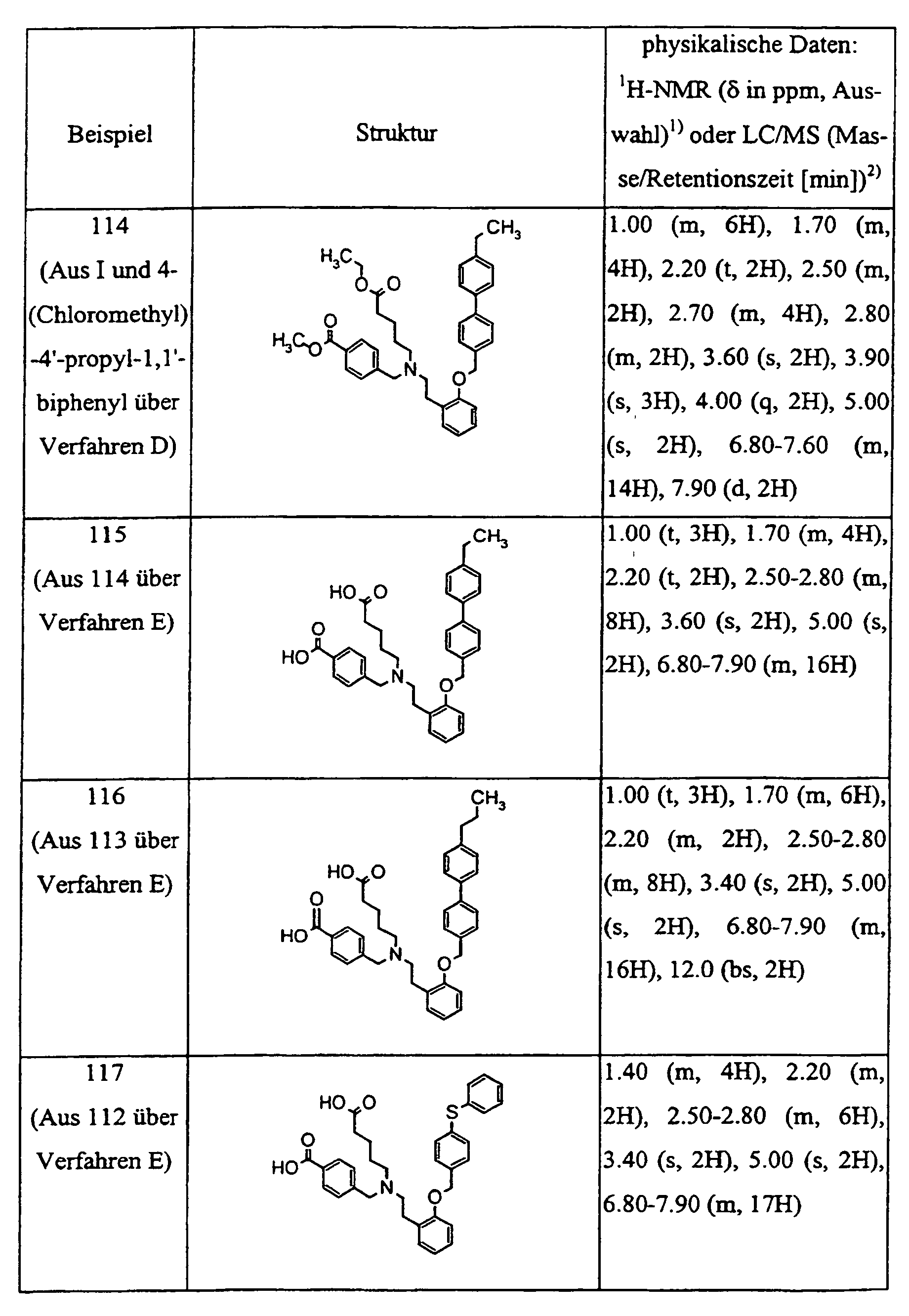

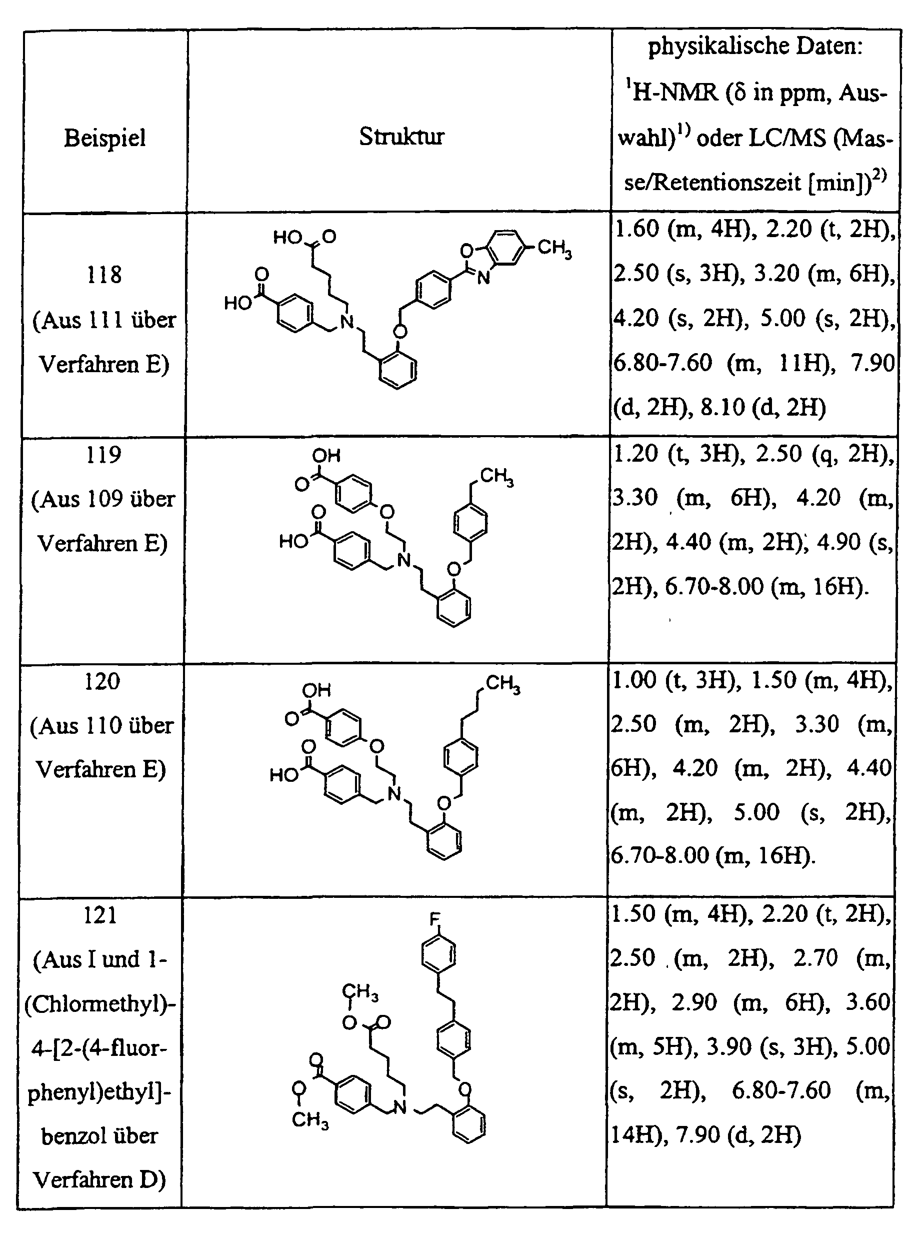

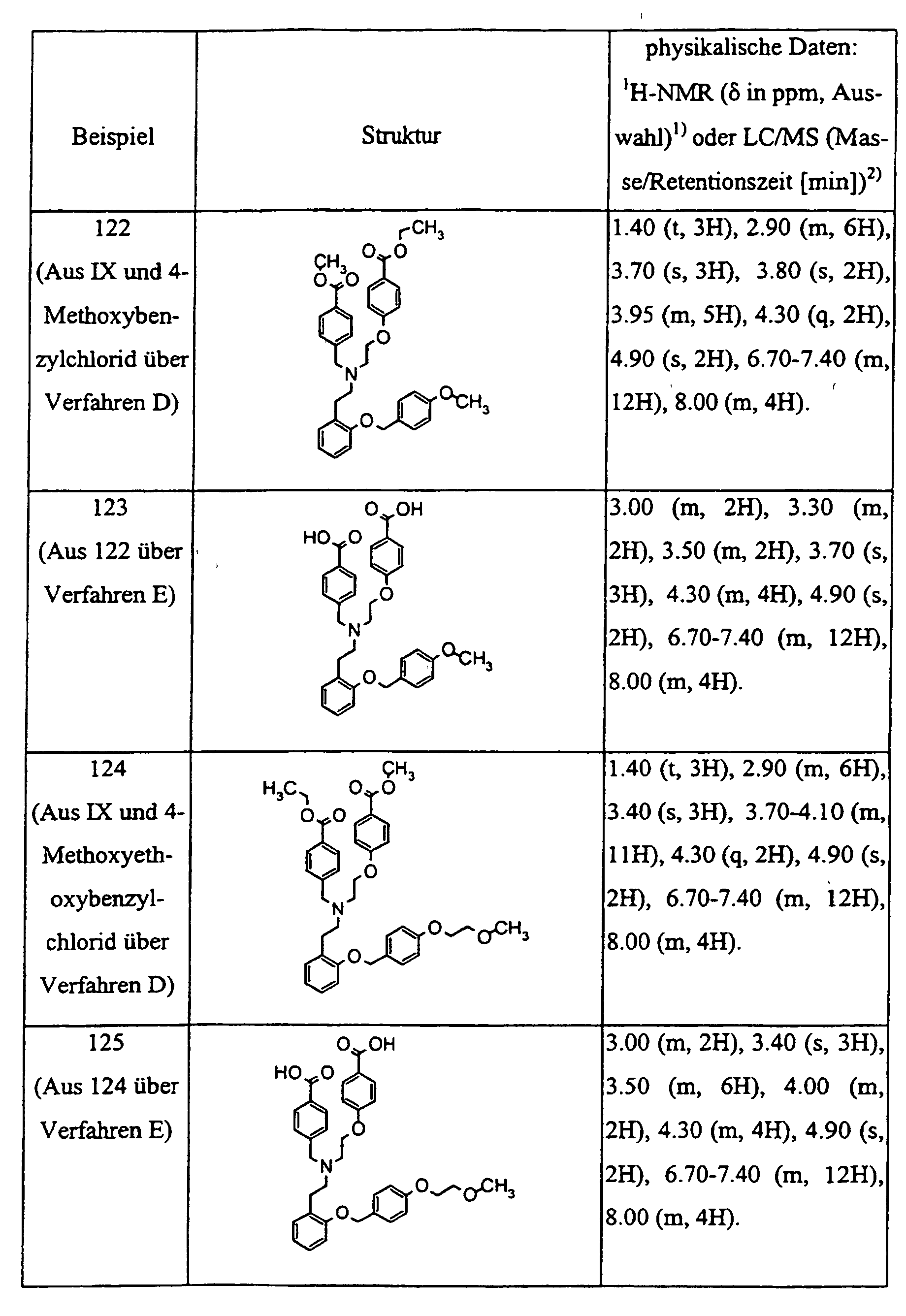

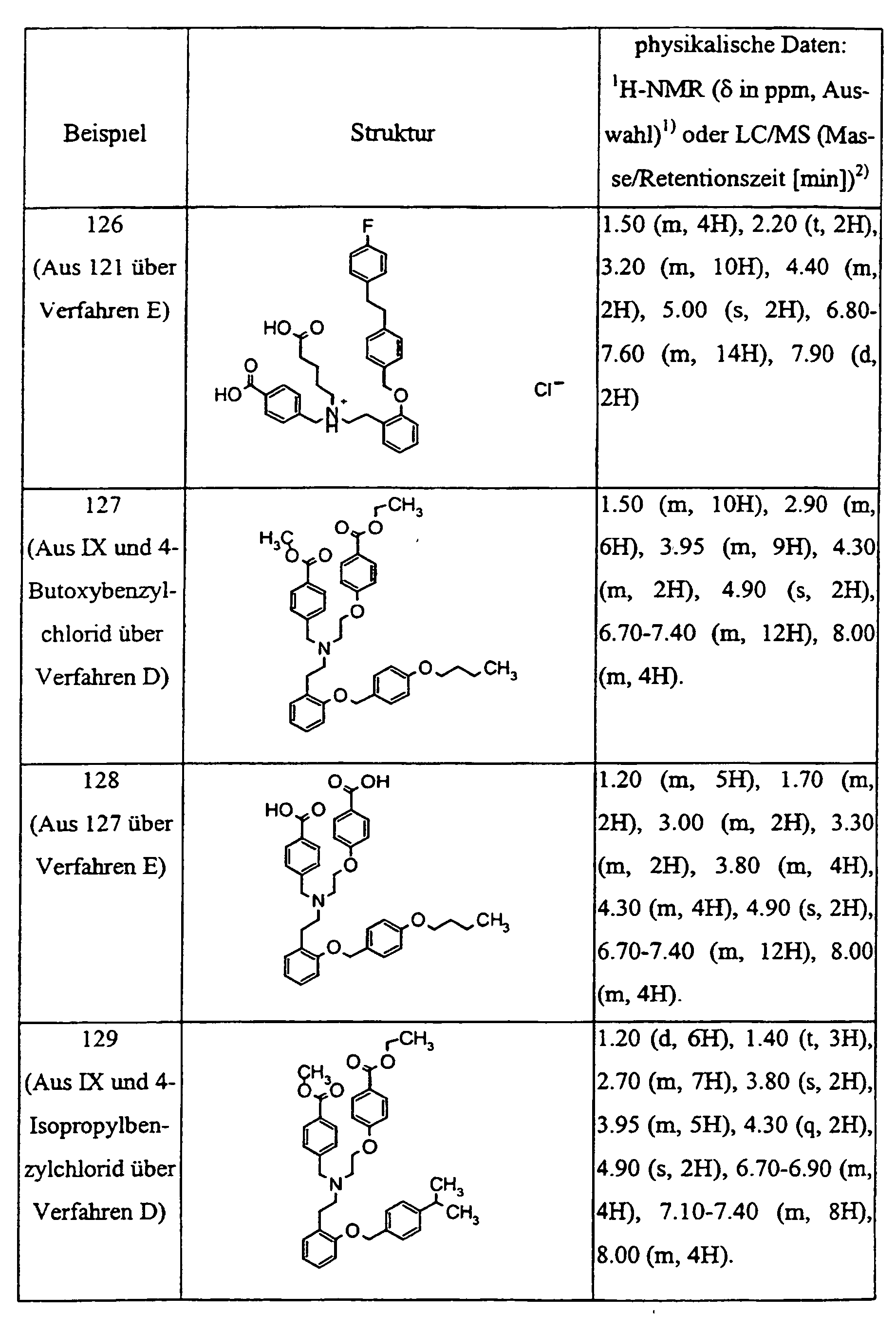

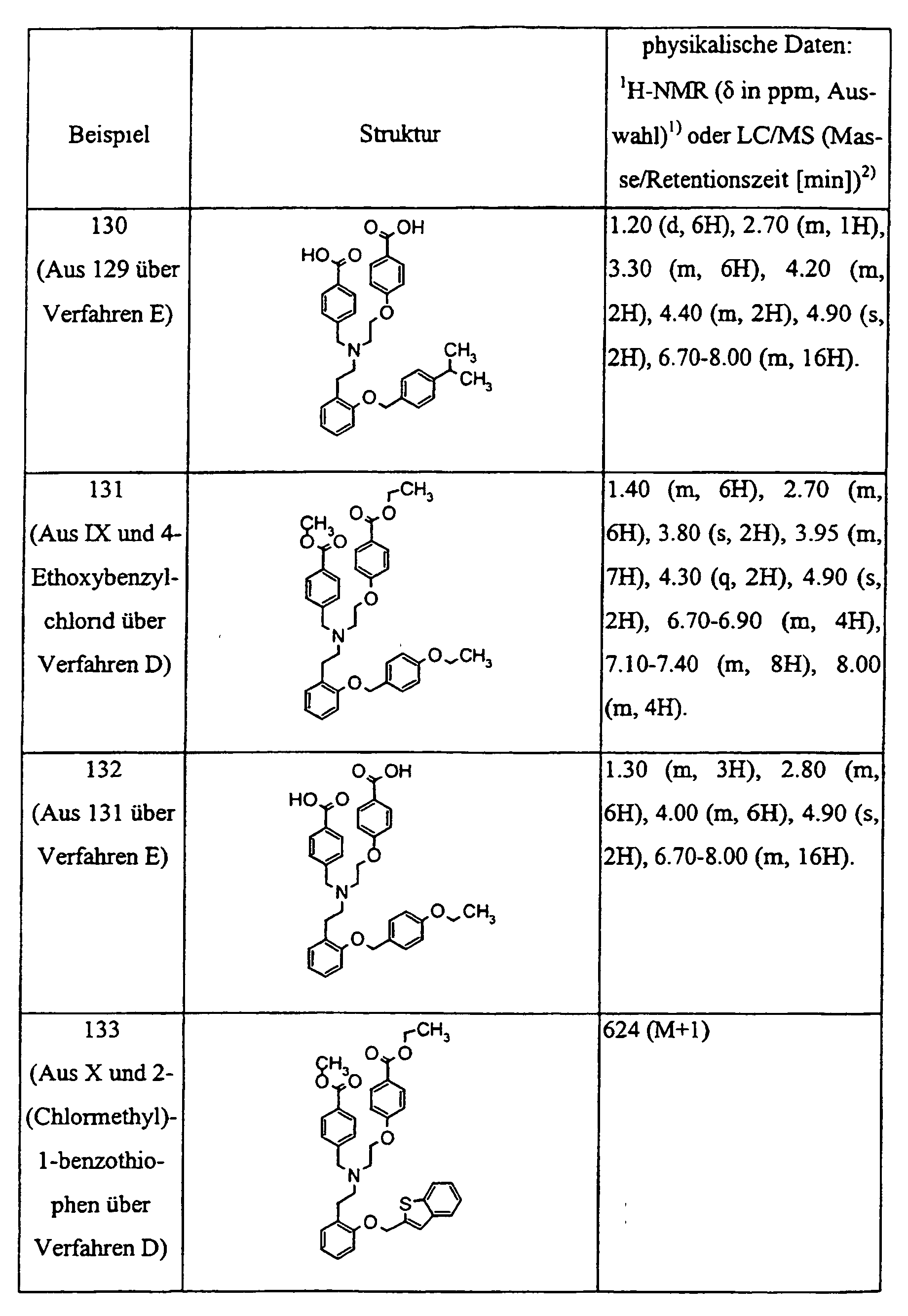

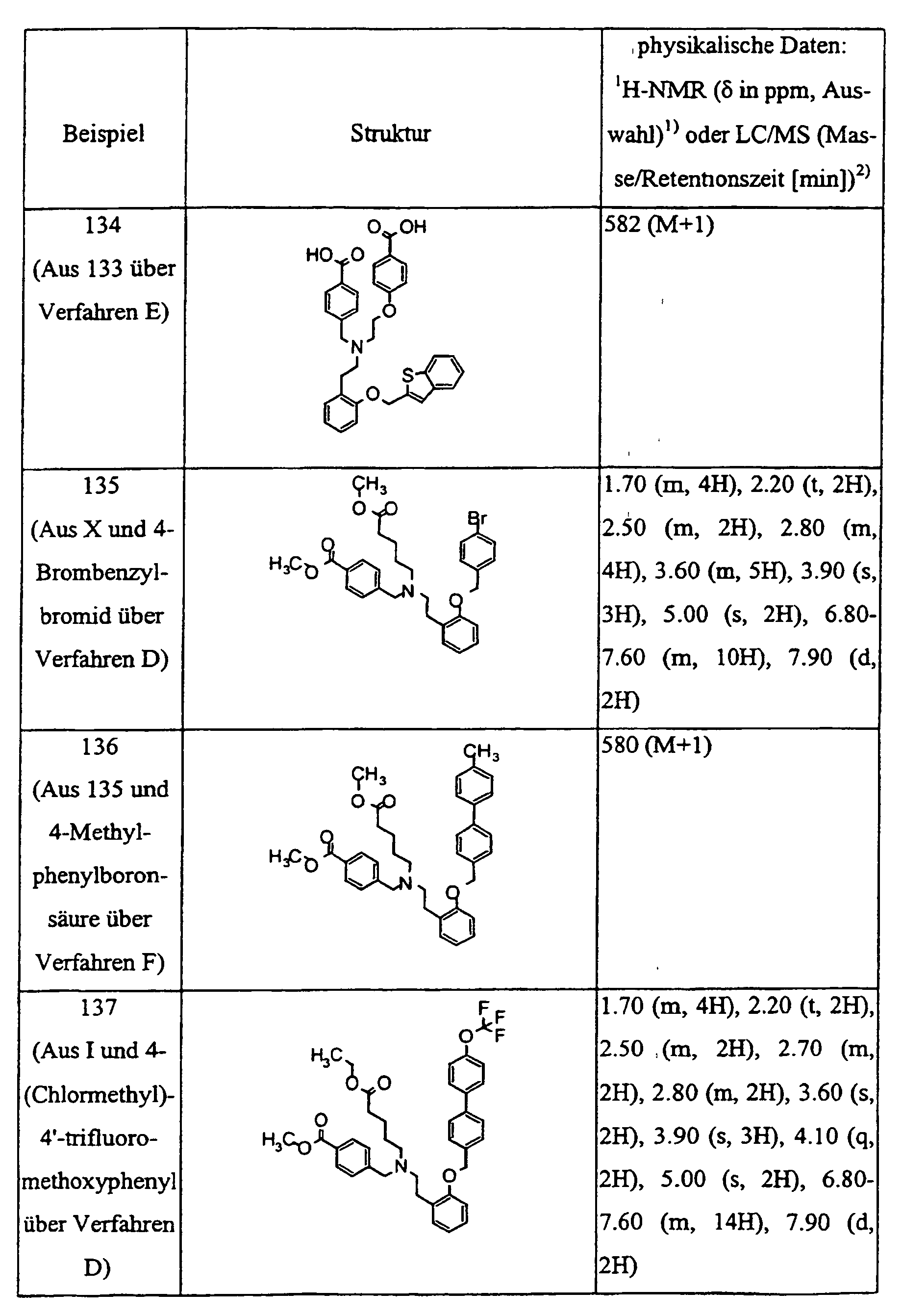

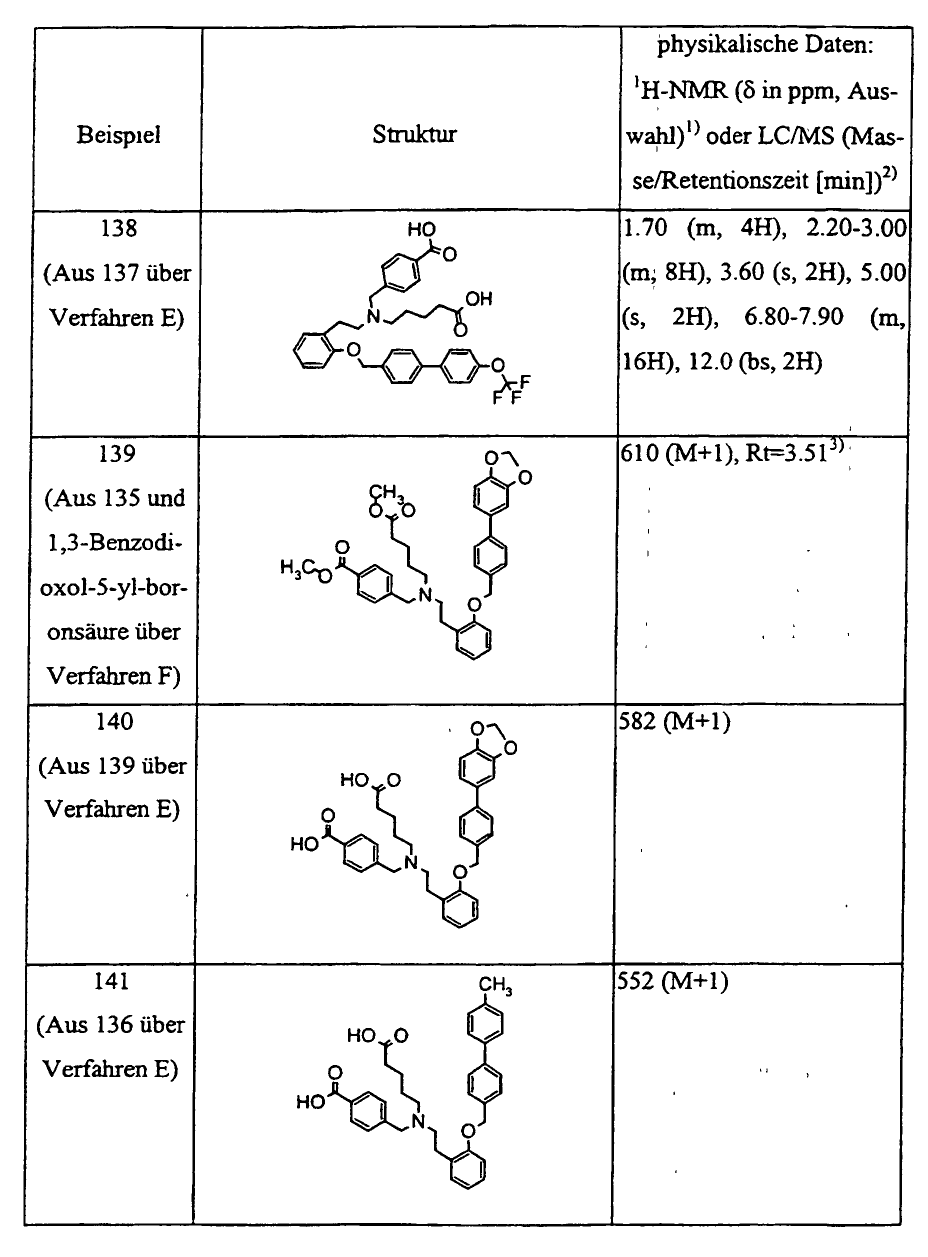

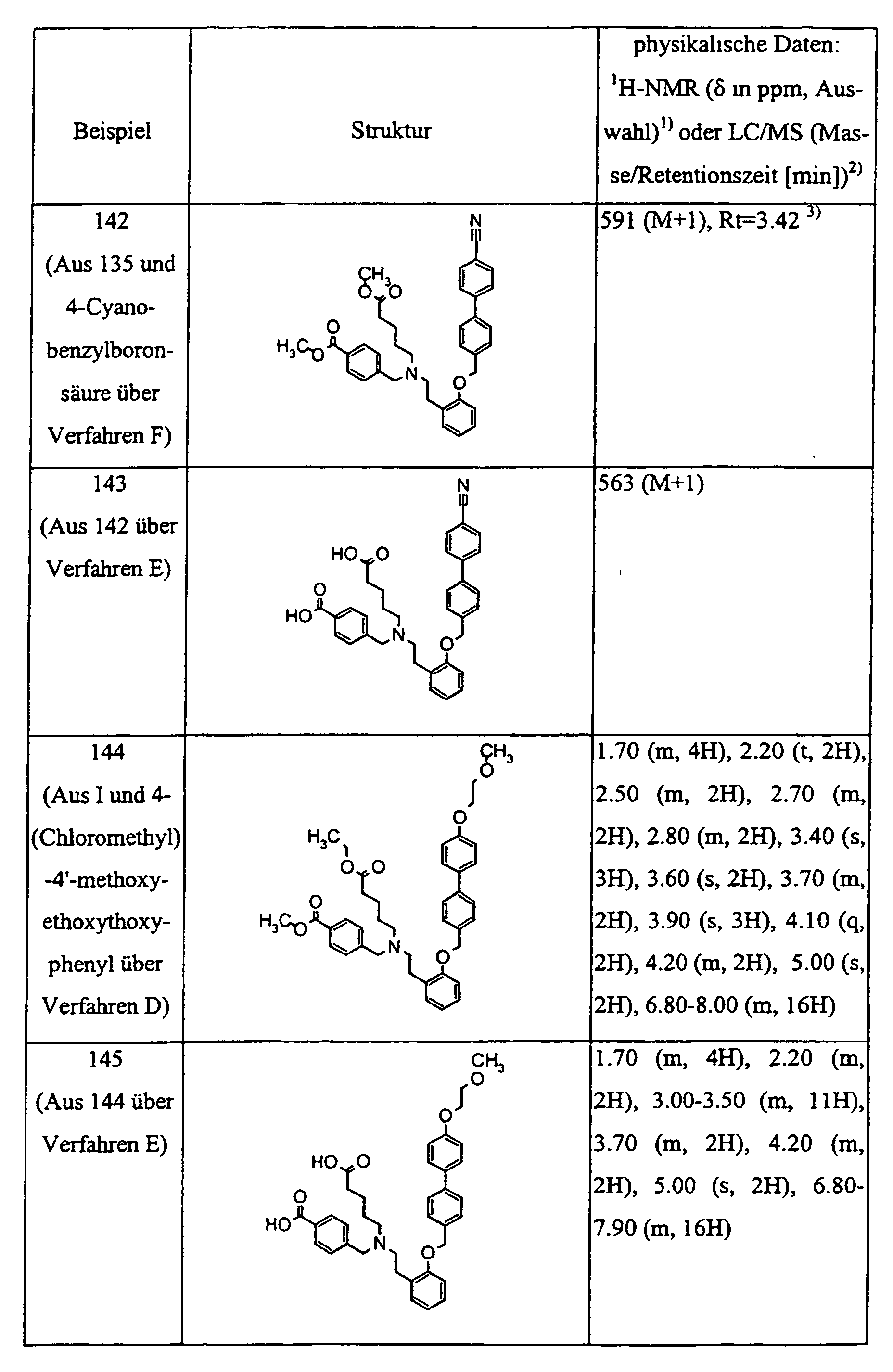

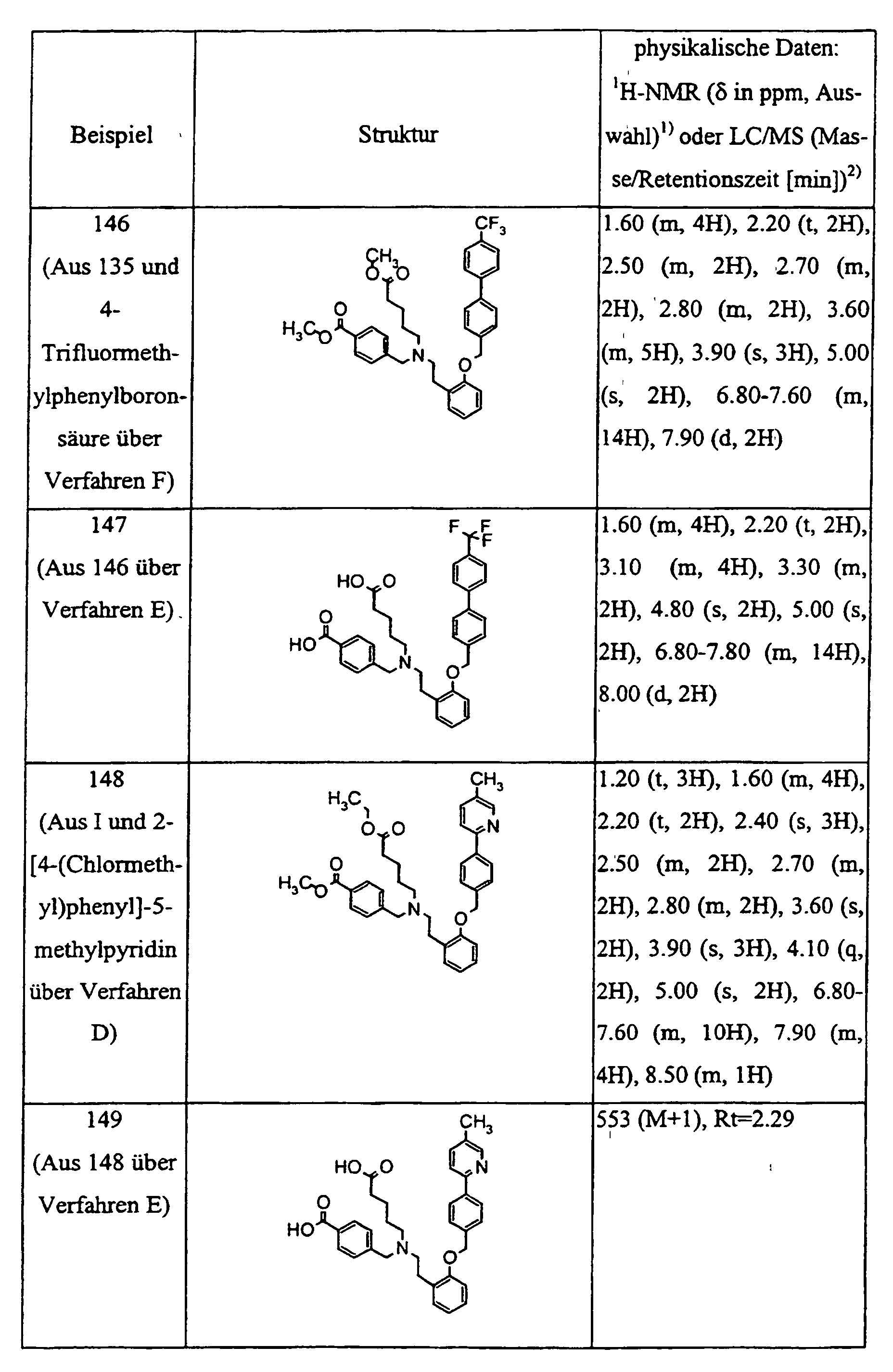

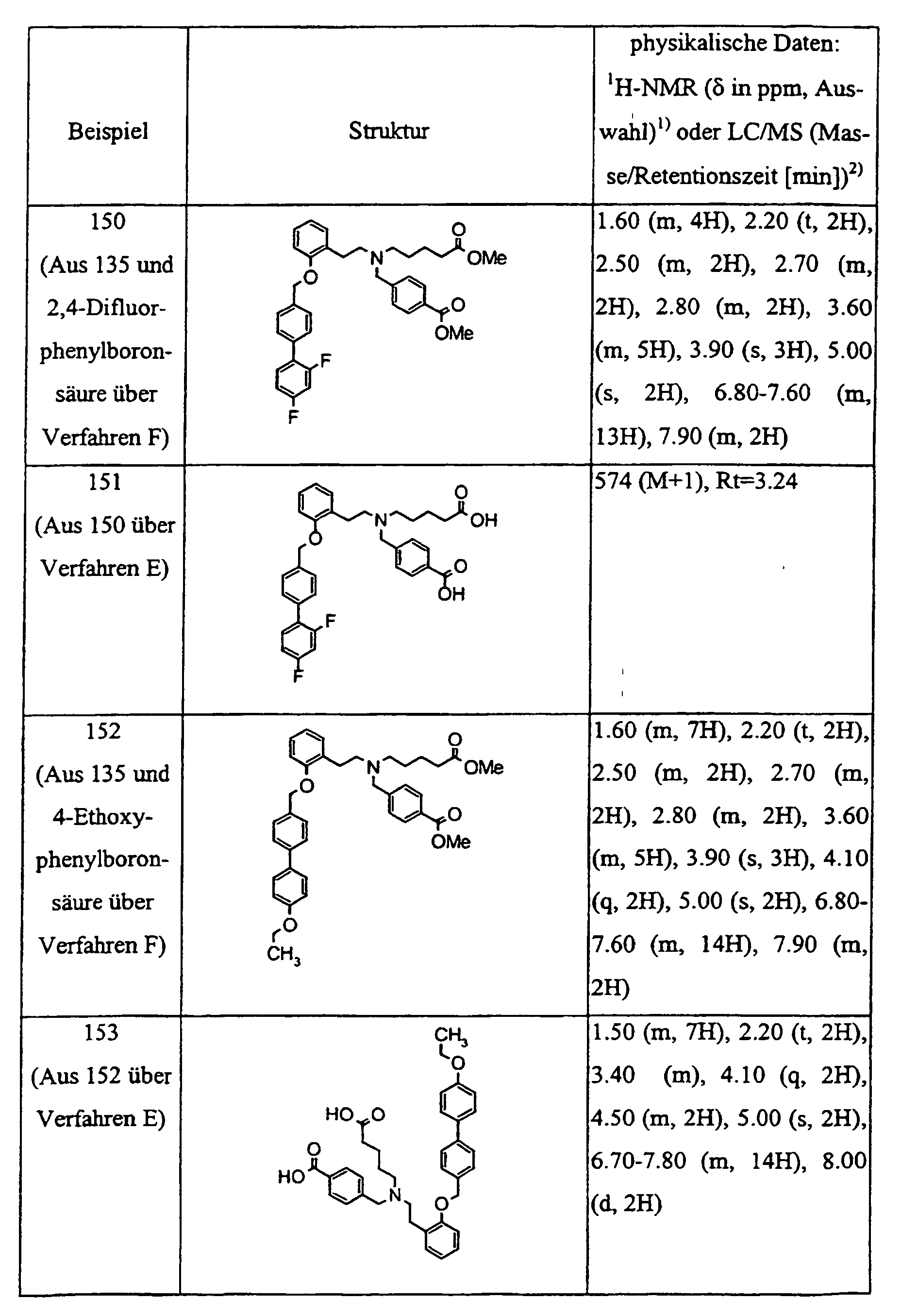

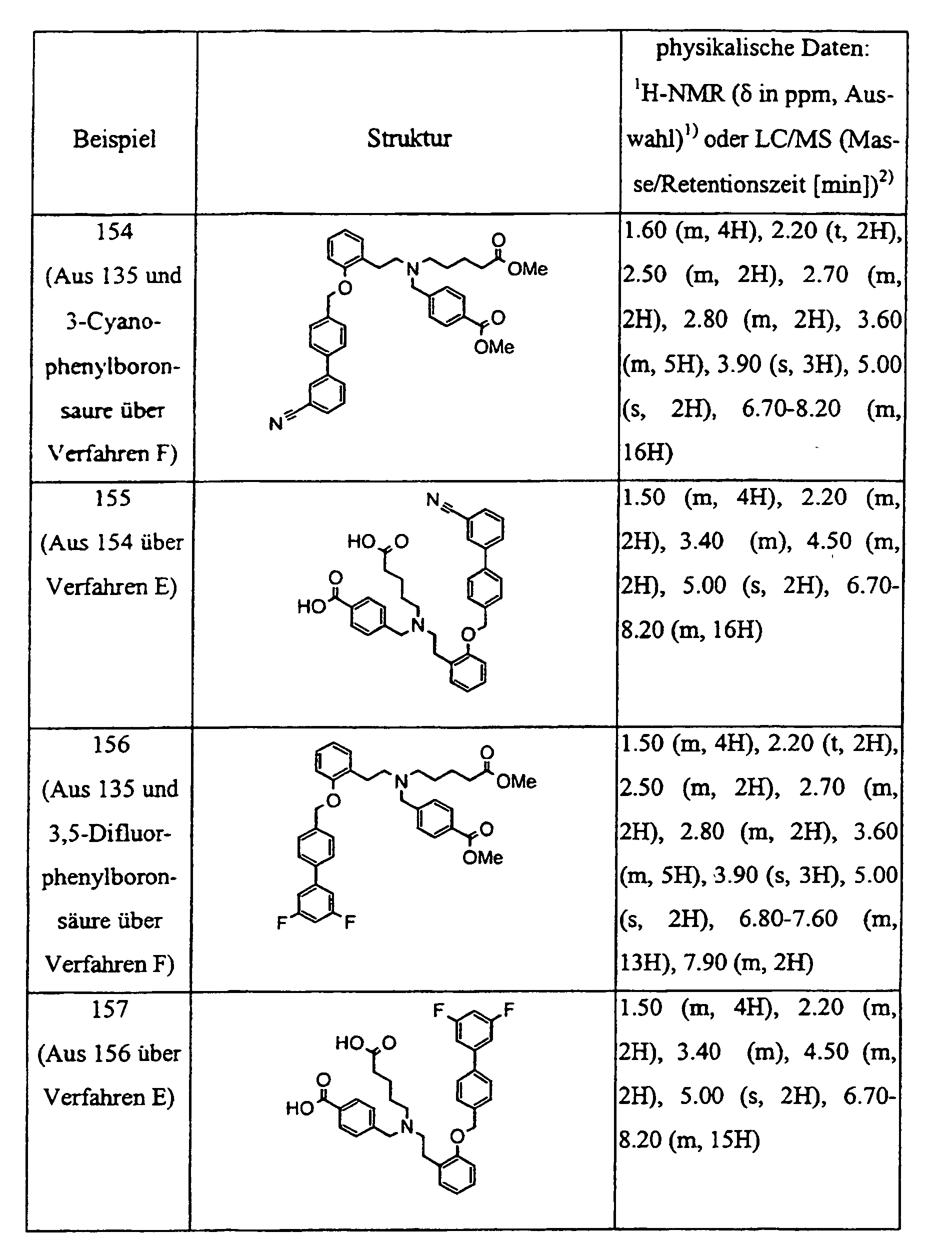

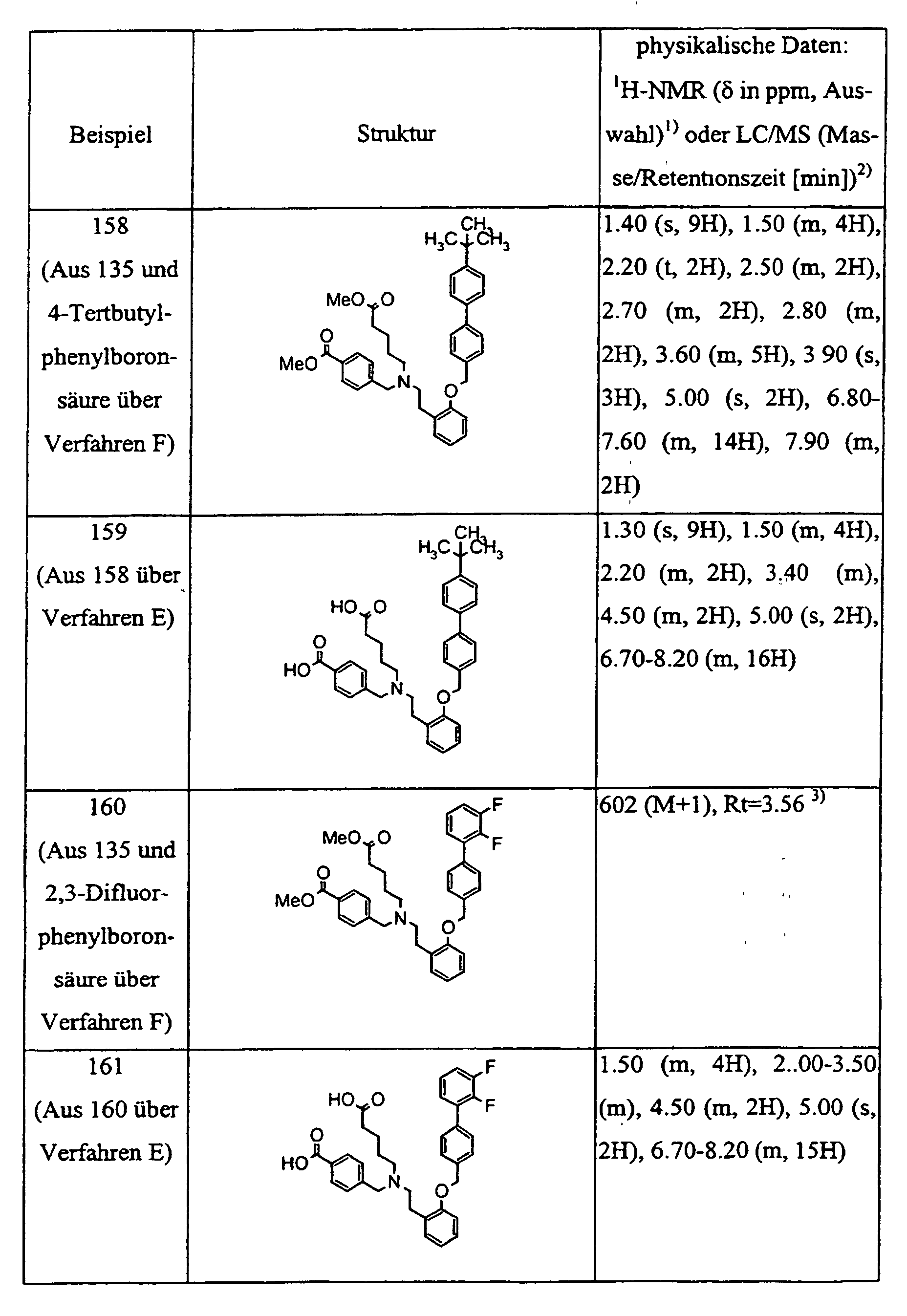

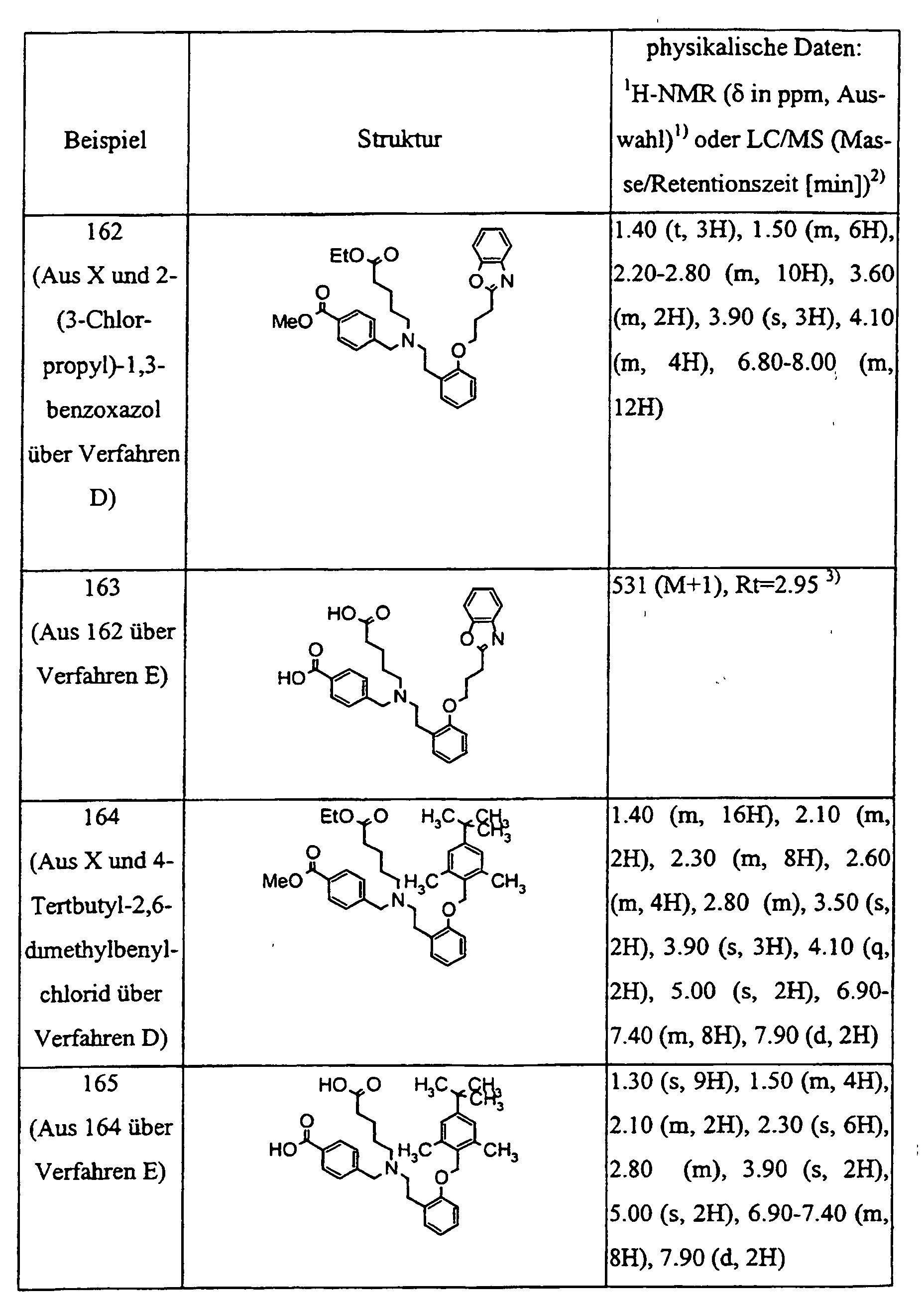

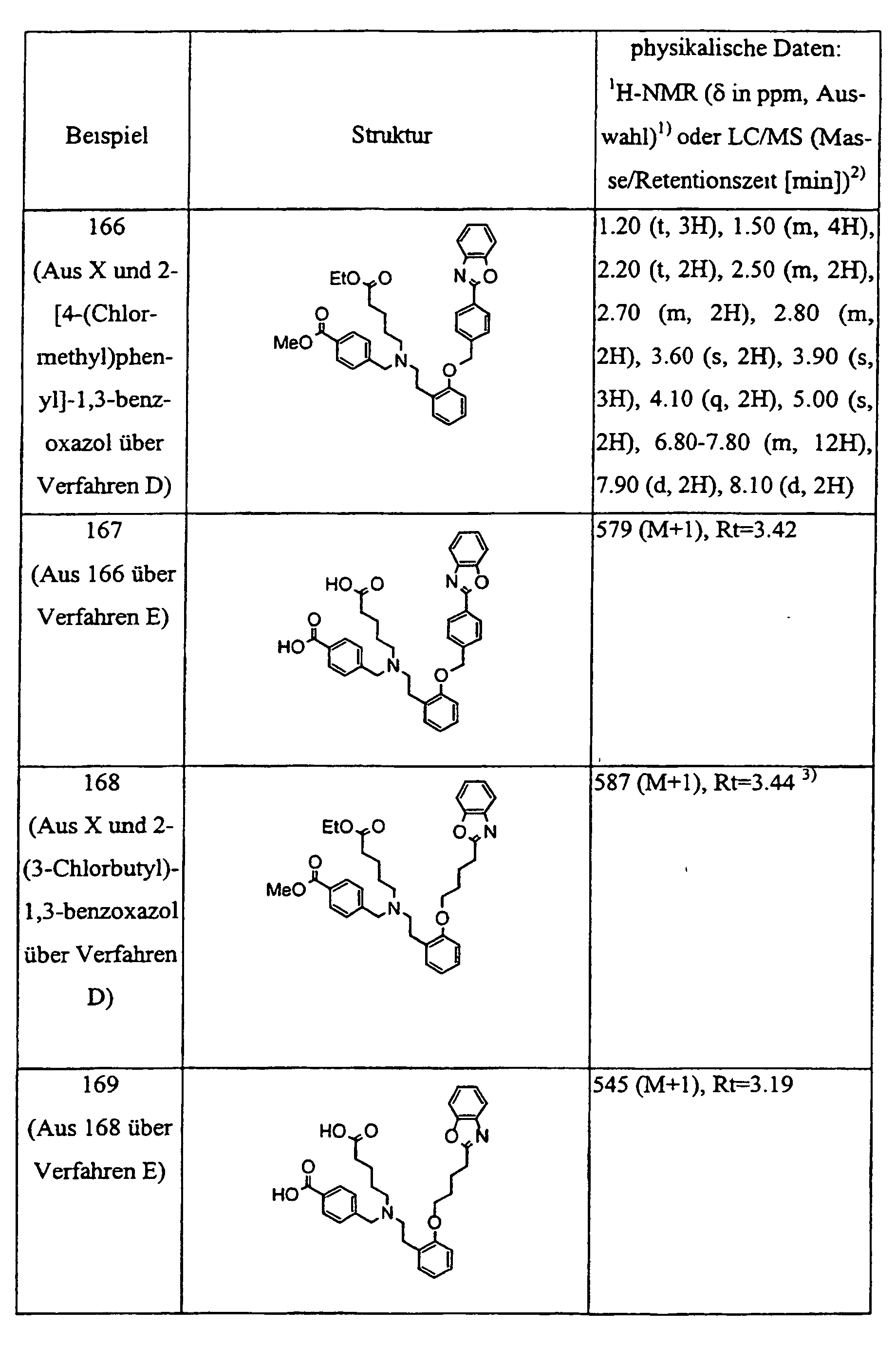

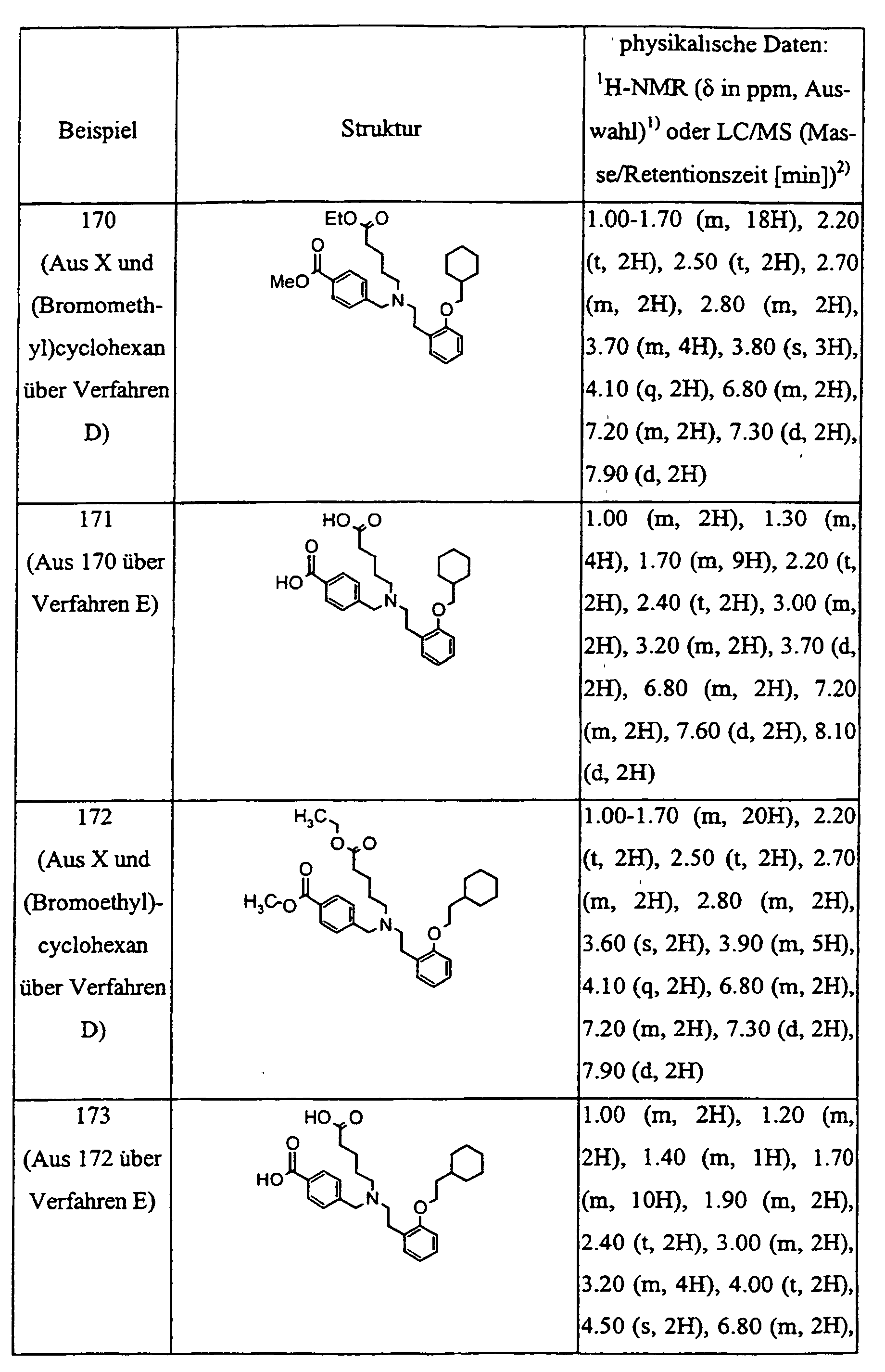

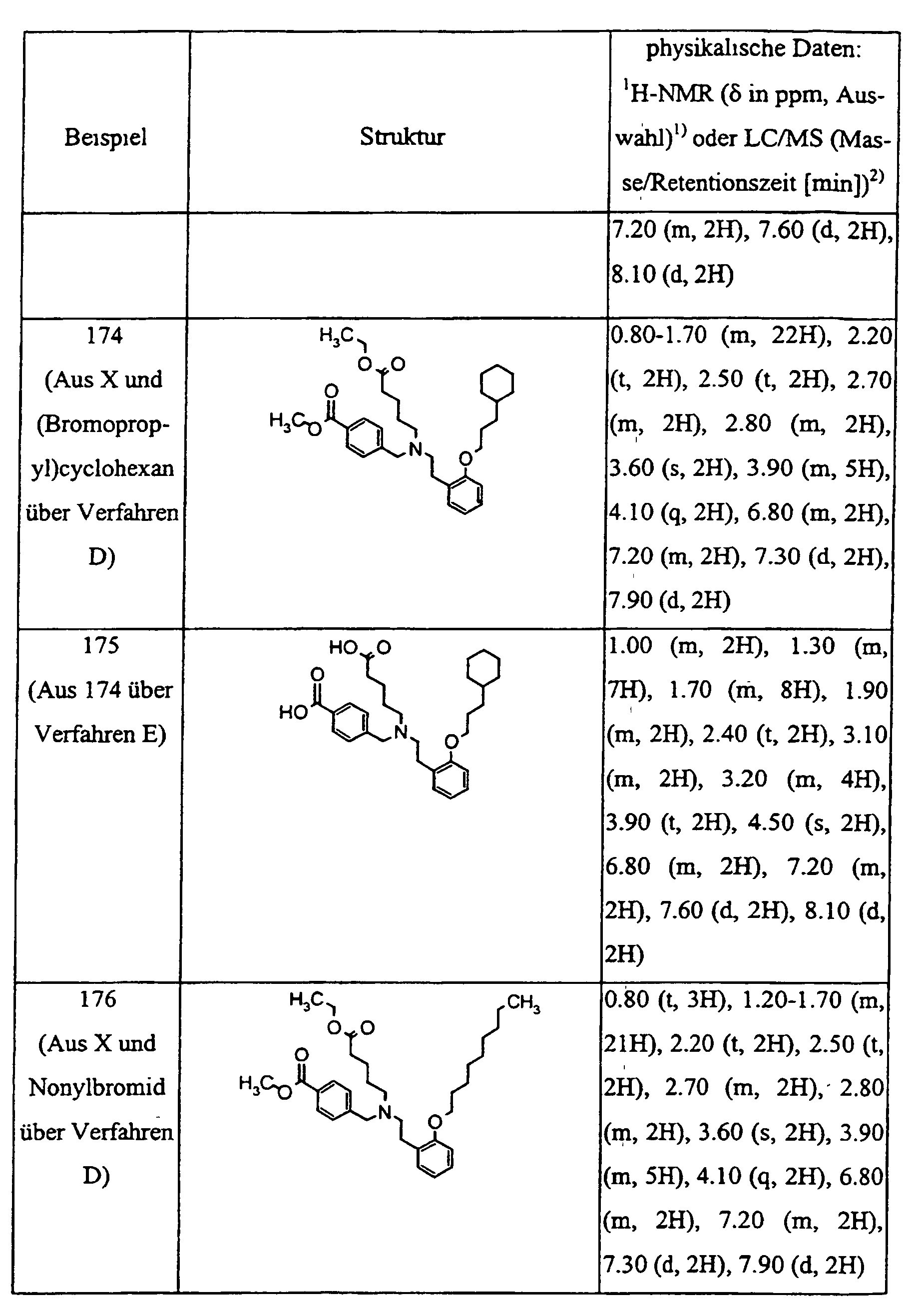

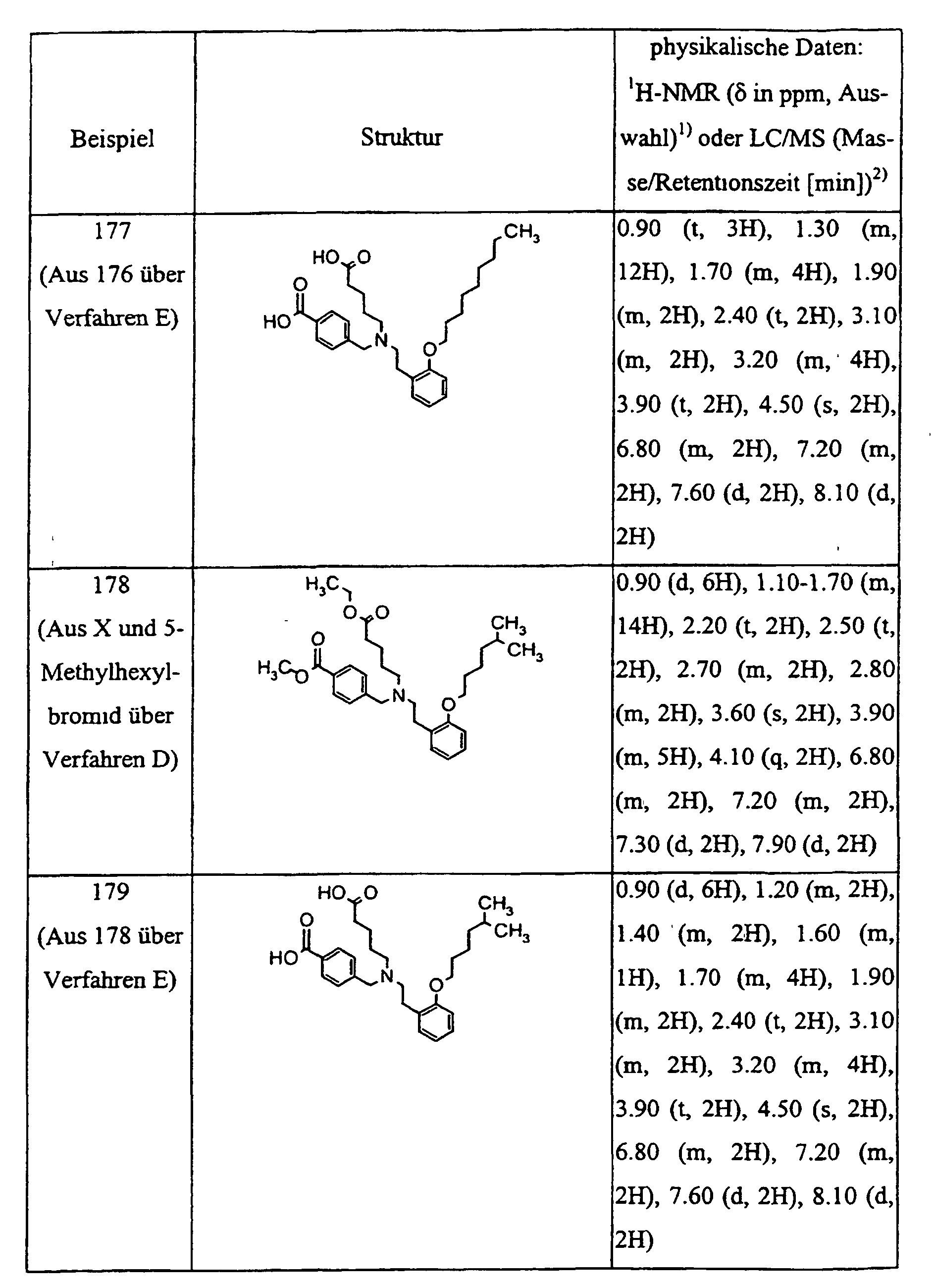

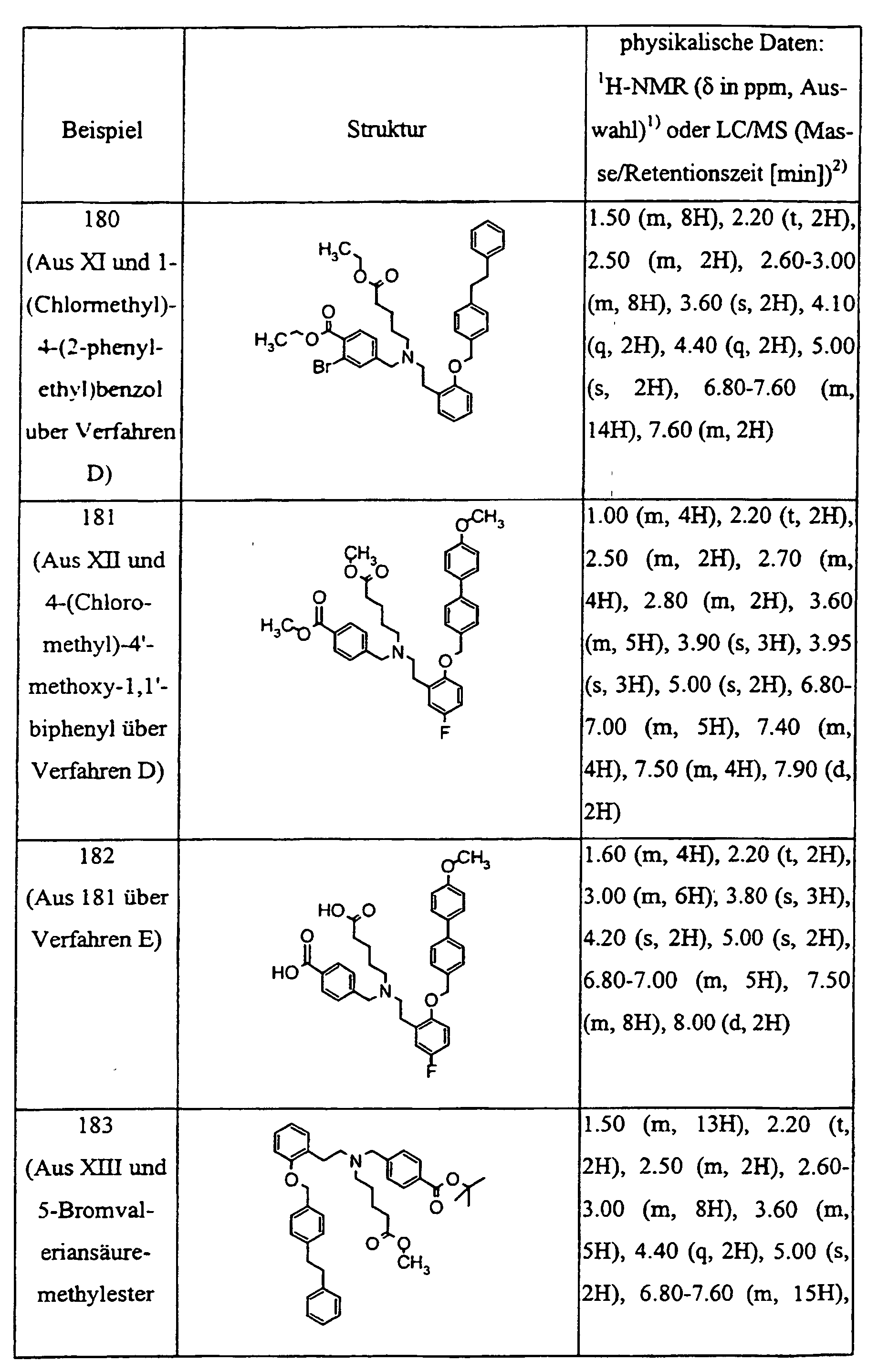

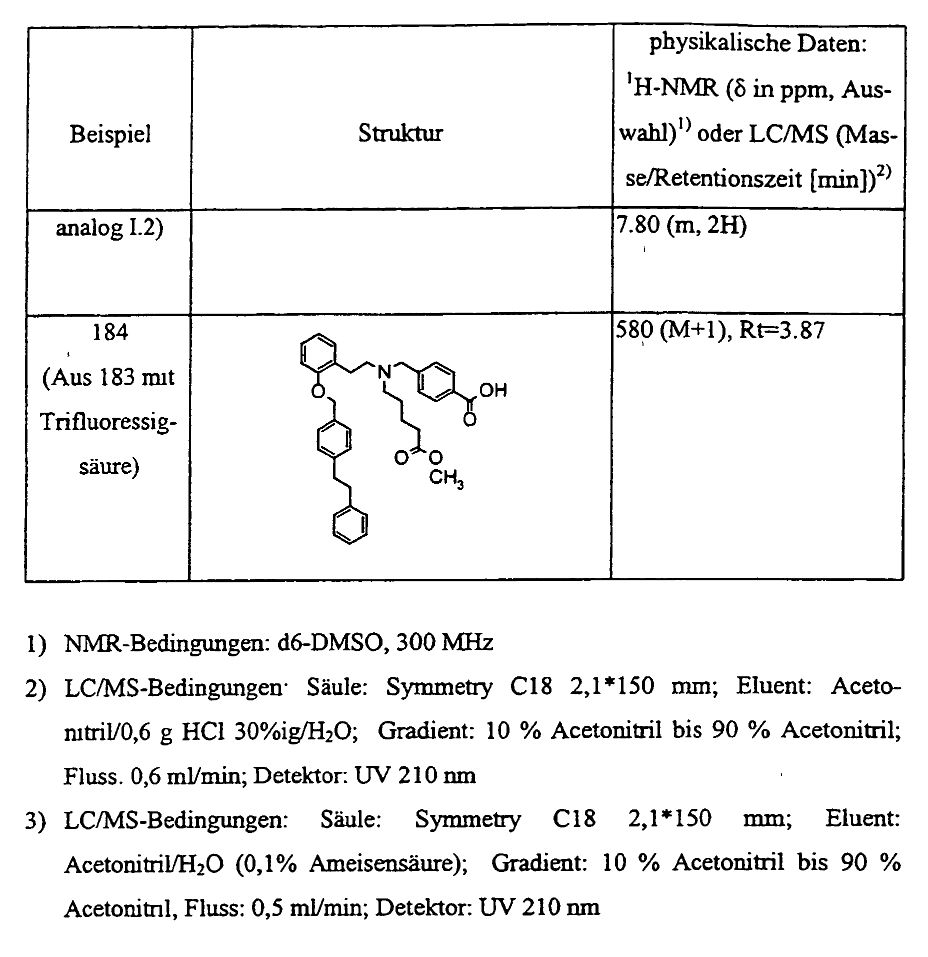

### Beispiel 185: 4-{[(2-{5-Fluor-2-[(4'-methyl-1,1'-biphenyl-4-yl)methoxy]phenyl}ethyl)(5-methoxy-5-oxopentyl)amino]methyl}benzoesäuremethylester

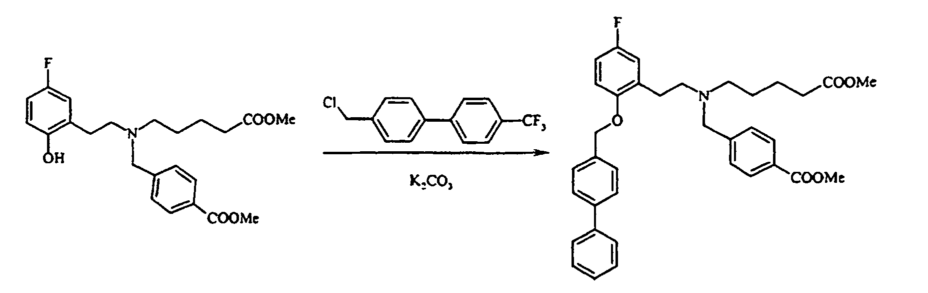

447 mg (0,93 mmol)4-({(5-Methoxy-5-oxopentyl)[2-(5-fluor-2-hydroxyphenyl)ethyl]amino}methyl)benzoesäuremethylester aus Bsp. XII und 277 mg (1,02 mmol) 4-(Chlormethyl)-4'-(trifluormethyl)-1,1'-biphenyl werden in 10 ml Acetonitril gelöst. 455 mg (1,40 mmol) Cäsiumcarbonat und eine Spatelspitze Kaliumiodid werden zugegeben und die Mischung wird 48 Stunden unter Rückfluss erhitzt. Die Suspension wird filtriert, eingeengt, und der Rückstand wird über Silicagel Cayclohexan:Essigester (5:1) chromatographiert.
Ausbeute: 447 mg (73,6% d. Th.)
¹H-NMR (d6-DMSO, 300 MHz): 1.00 (m, 4H), 2.20 (t, 2H), 2.50 (m, 2H), 2.70 (m, 4H), 2.80 (m, 2H), 3.60 (m, 5H), 3.90 (s, 3H), 5.00 (s, 2H), 6.80-7.00 (m, 3H), 7.30 (d, 4H), 7.40 (d, 2H), 7.50 (d, 2H), 7.70 (m, 4H), 7.90 (d, 2H).

### Beispiel 186: 4-{[(4-Carboxybutyl)(2-{5-fluoro-2-[(4'-methyl-1,1'-biphenyl-4-yl)methoxy]phenyl}ethyl)amino]methyl}benzoesäure

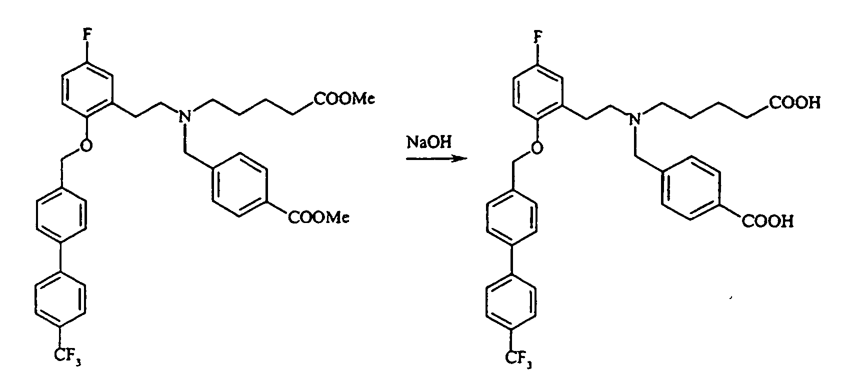

0,45 g (0,69 mmol) 4-{[(2-{5-Fluor-2-[(4'-methyl-1,1'-biphenyl-4-yl)methoxy]phenyl}ethyl)(5-methoxy-5-oxopentyl)amino]methyl}benzoesäuremethylester aus Bsp. 185 werden in 8 ml Methanol gelöst. 0,5 ml Natronlauge (45%) und 1,5 ml Dichlormethan werden zugegeben, und die Lösung wird 8 Stunden bei RT gerührt. Die Reaktion wird mit Ethylether extrahiert, die wässrige Phase wird mit Schwefelsäure sauer gestellt, mit Essigester extrahiert, über Extrelut filtriert und eingeengt.
Ausbeute: 245 mg (57,3% d.Th.)
¹H-NMR: (300 MHz, MeOD): 1.60 (m, 4H), 2.20 (t, 2H), 3.00 (m, 4H), 3.20 (m, 2H), 4.20 (s, 2H), 5.10 (s, 2H), 7.00 (m, 3H), 7.50 (m, 4H), 7.70 (m, 6H), 7.90 (d, 2H).

### Beispiel 187: Methyl 4-{[(5-ethoxy-5-oxopentyl)(2-{[5-(4-phenylpiperazino)-pentyl]oxy}phenethyl)amino]methyl}benzoat

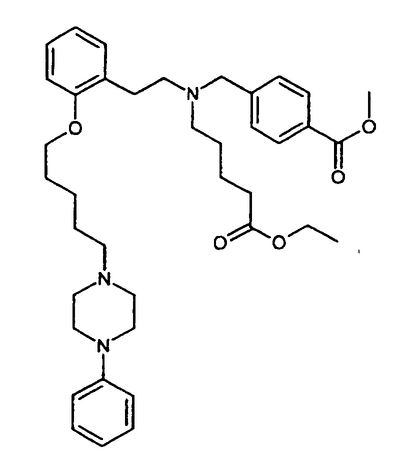

200.0 mg (0.355 mmol) Methyl-4-{[{2-[(5-bromopentyl)oxy]phenethyl}(5-ethoxy-5-oxopentyl)amino] methyl}benzoat aus Bsp. 107 , 69.21 mg N-Phenylpiperazin und 71.95 mg (0.711 mmol) Triethylamin werden in 2 ml Tetrahydrofuran 18 Stunden unter Rückfluss erhitzt. Die Reaktionslösung wird mit Wasser gewaschen, eingeengt und über Kieselgel mit Essigester/Methanol 10/1 als Laufmittel chromatographiert.
Ausbeute: 66.0 mg (28.83 % der Theorie)
¹H -NMR (300 MHz, d⁶-DMSO): δ= 1.12 (t, 3H), 1.44 (m, 8H), 1.65 (m, 2H), 2.35 (m, 4H), 2.45 (m, 4H), 2.55 (m, 2H), 2.72 (m, 2H), 3.10 (m, 4H), 3.65 (s, 2H), 3.85 (s, 3H), 3.88(t, 2H), 4.05 (m, 2H), 6.70-6.90 (m, 5H), 7.0-7.2 (m, 4H), 7.4 (d, 2H), 7.8 (d, 2H).

Auf analoge Weise können erhalten werden:

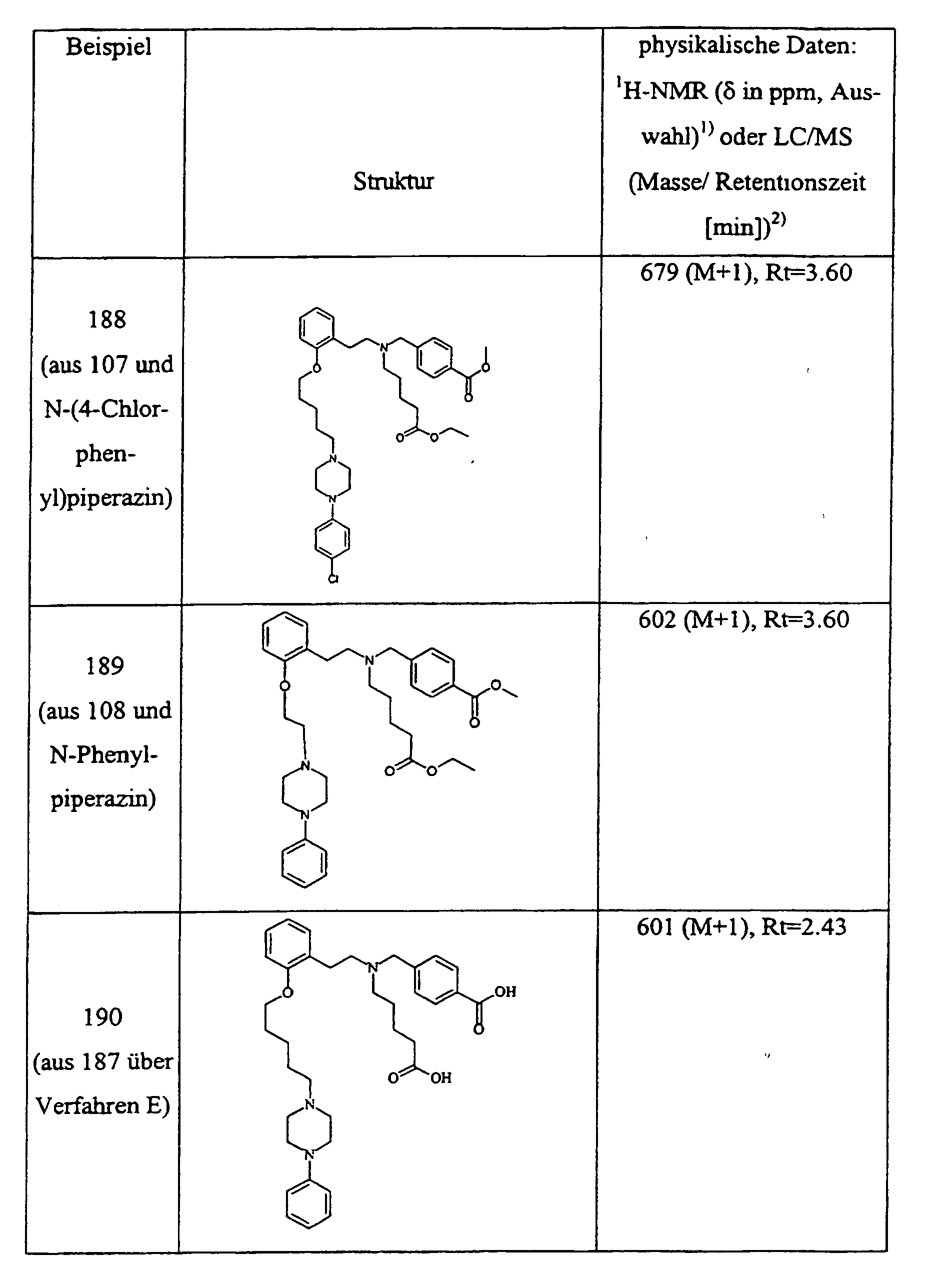

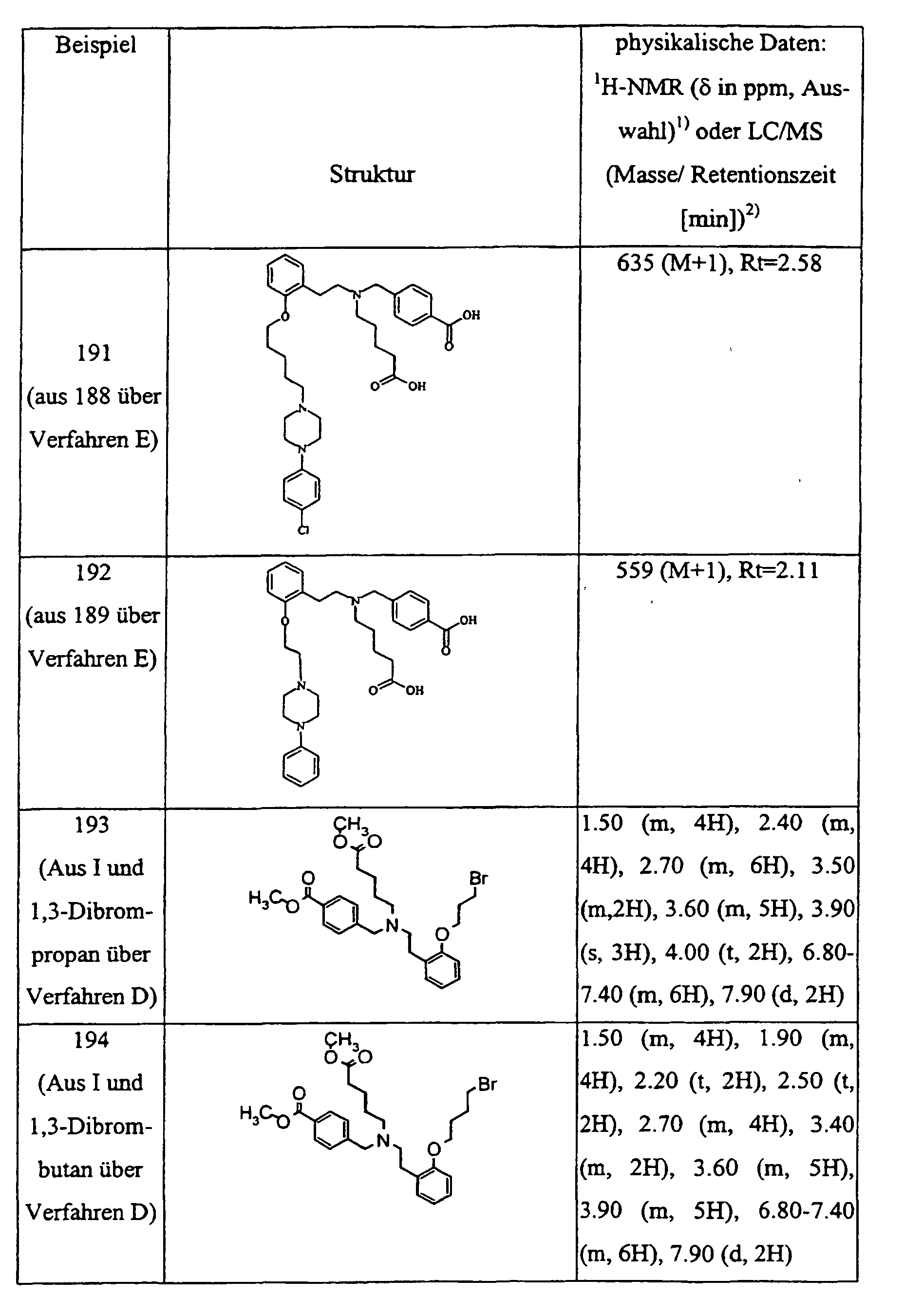

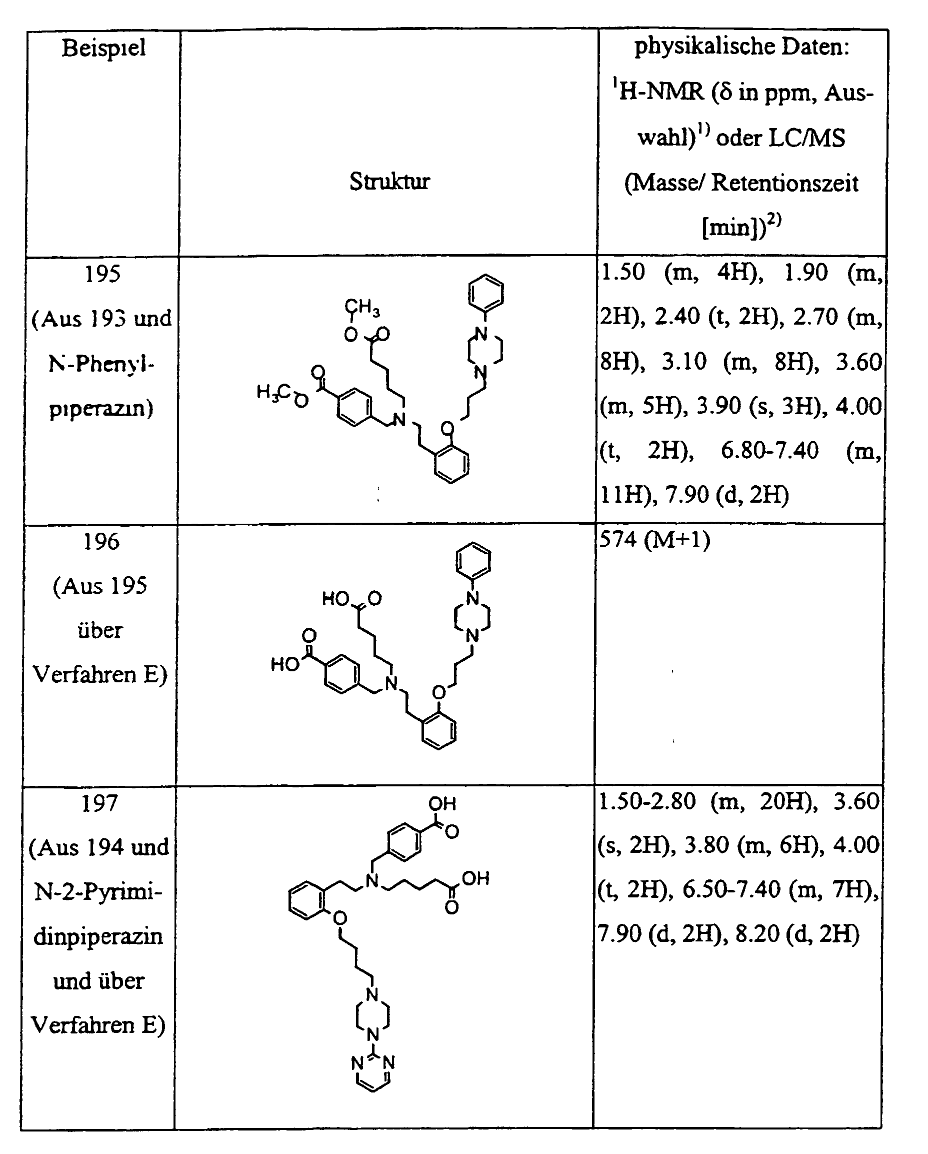

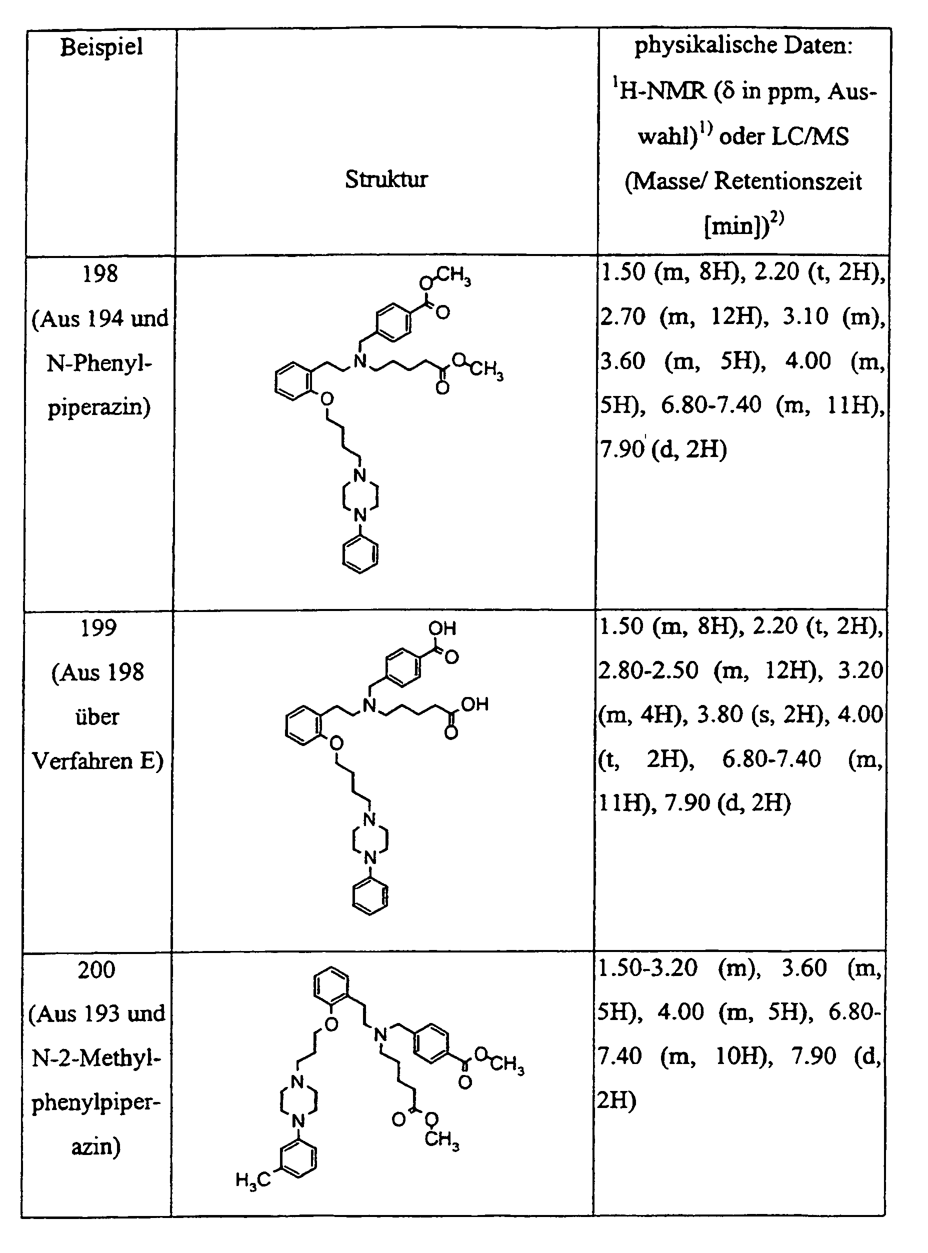

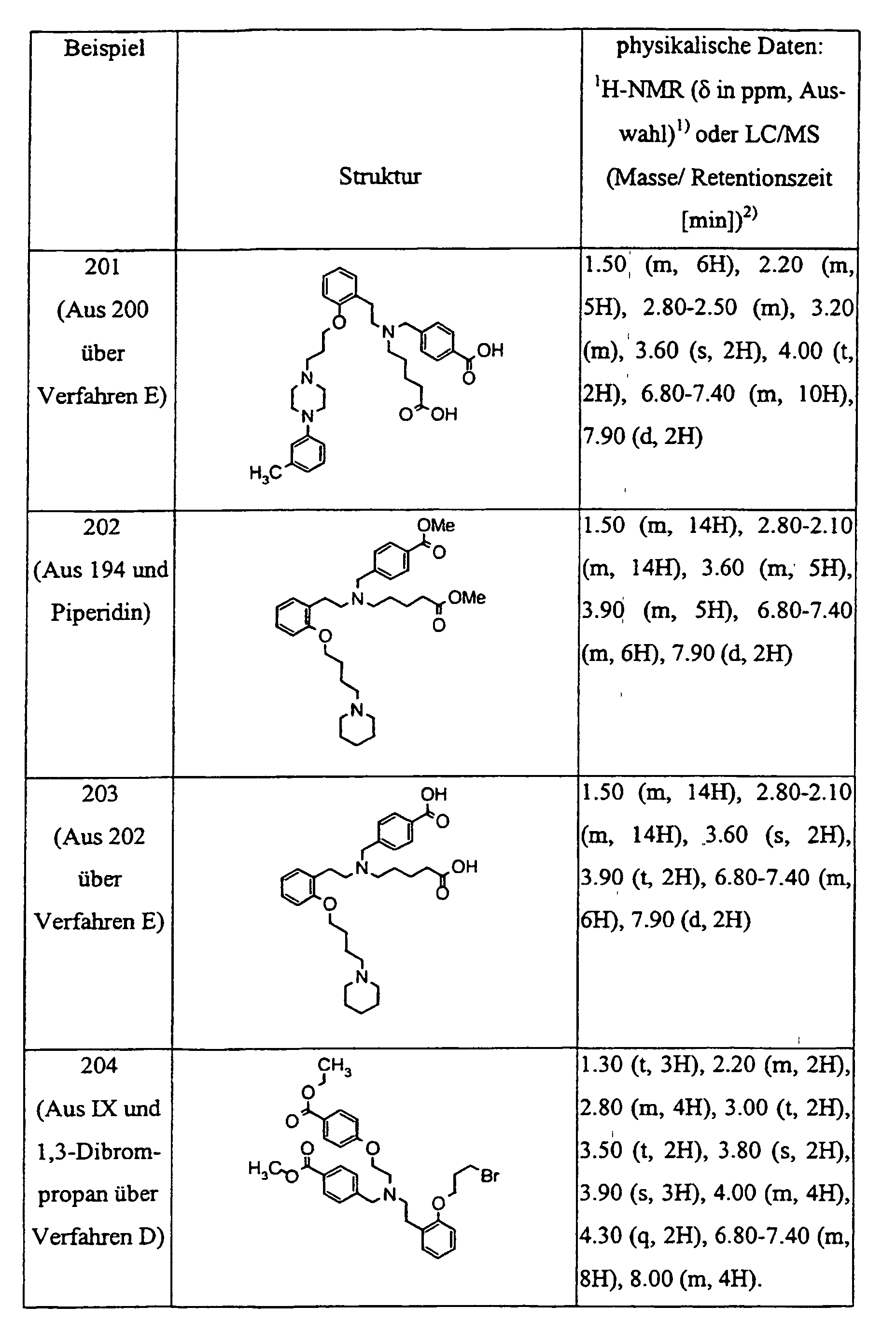

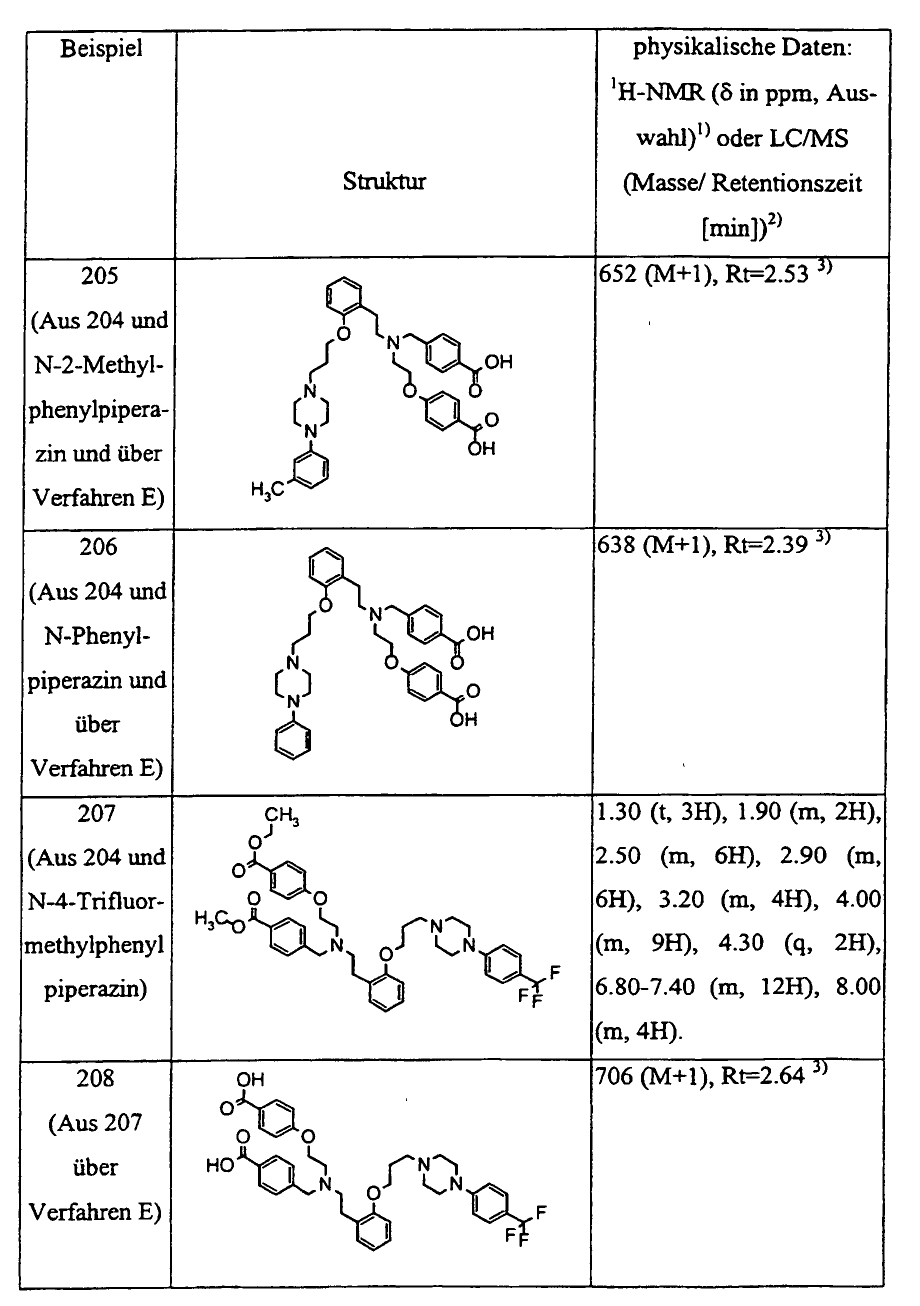

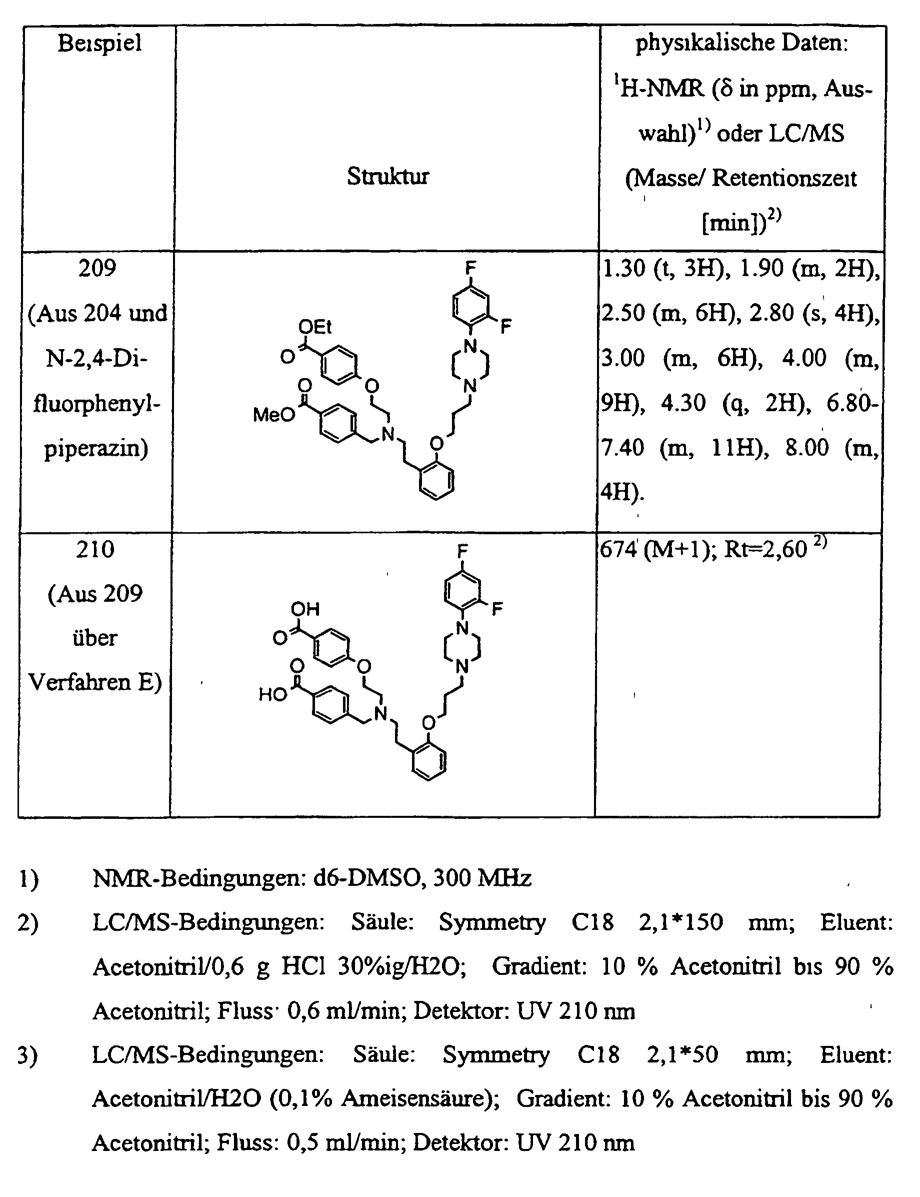

### 211: Methyl-6-{[{2-[2-(1,1'-biphenyl-4-ylmethoxy)phenyl]ethyl}(5-tert-butoxy-5-oxopentyl)-amino]methyl}nicotinat

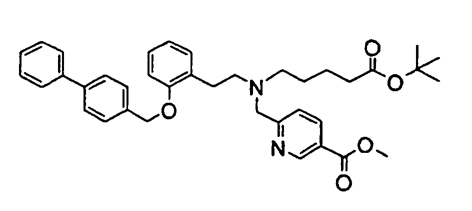

Zu einer Lösung von 132.0 mg (0.29 mmol) XXa in 3 ml DMF wurden 198.5 mg (1.44 mmol) Kaliumcarbonat, 121.1 mg (0.32 mmol) Methyl-6-(bromomethyl)nicotinat sowie eine katalytische Menge KI zugegeben. Man rührte 16 h bei Raumtemperatur und kontrollierte die Reaktion per Dünnschichtchromatographie. Die Lösung wurde mit Wasser versetzt und man extrahierte mit Ethylacetat/Cyclohexan 1:1. Die vereinten organischen Phasen wurden über Na₂SO₄ getrocknet und das Lösungsmittel entfernt. Das Produkt wurde chromatografisch gereinigt (Kieselgel, Cyclohexan/Ethylacetat 10: 1).
Ausbeute: 55.8%
¹H NMR (300 MHz, CDCl₃). δ = 1.16 - 1.58 (m, 4H), 1.40 (s, 9H), 2.11 (t, J= 7.2 Hz, 2H), 2.54 (t, J = 6.4 Hz, 2H), 2.70 - 2.81 (m, 2H), 2.82 - 2.92 (m, 2H), 3.81 (s, 2H), 3.89 (s, 3H), 5.04 (s, 2H), 6.82 - 7.62 (m, 14H), 8.04 - 8.17 (m, 1H), 9.02 - 9.08 (m, 1H).

Auf analoge Weise wurden hergestellt:

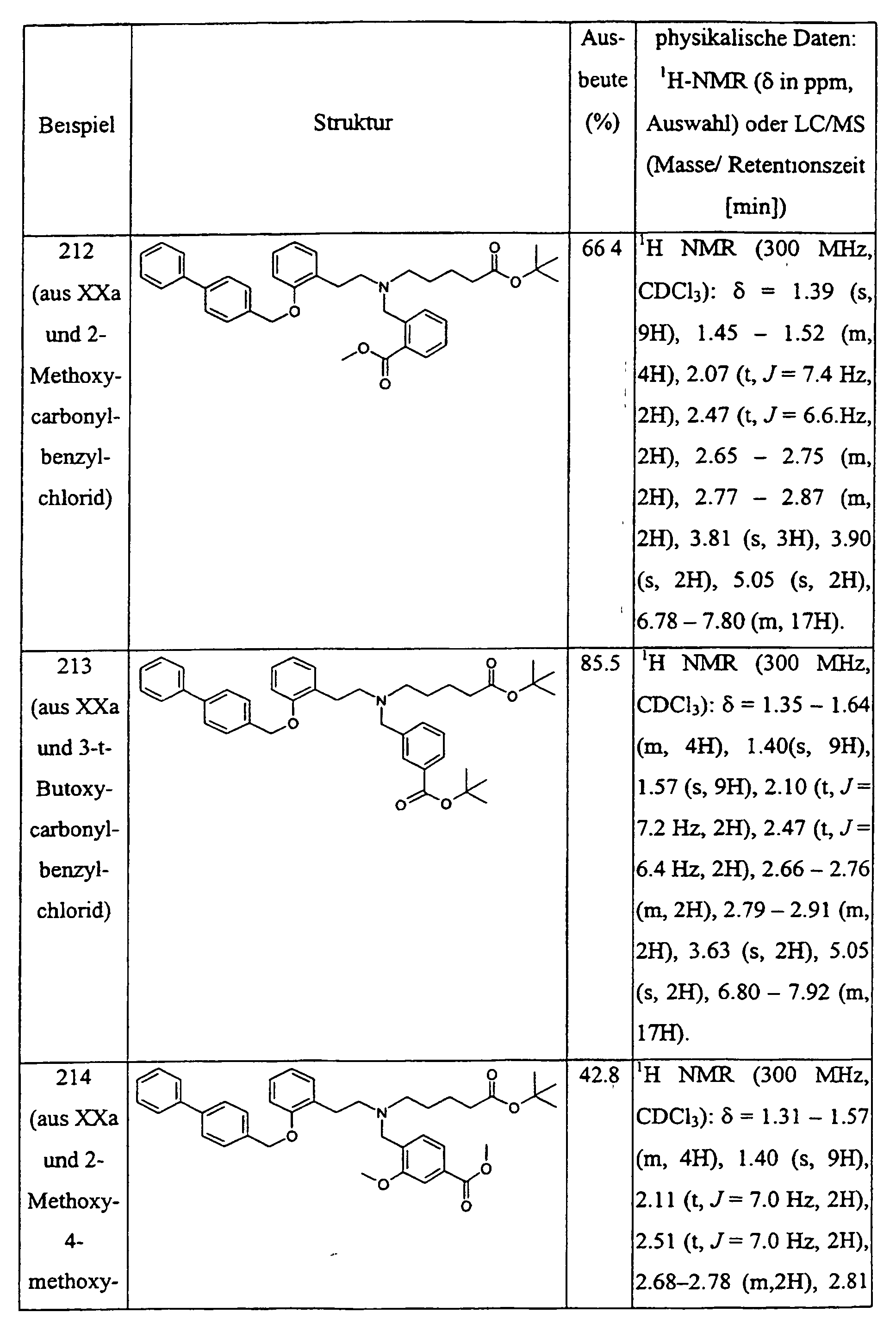

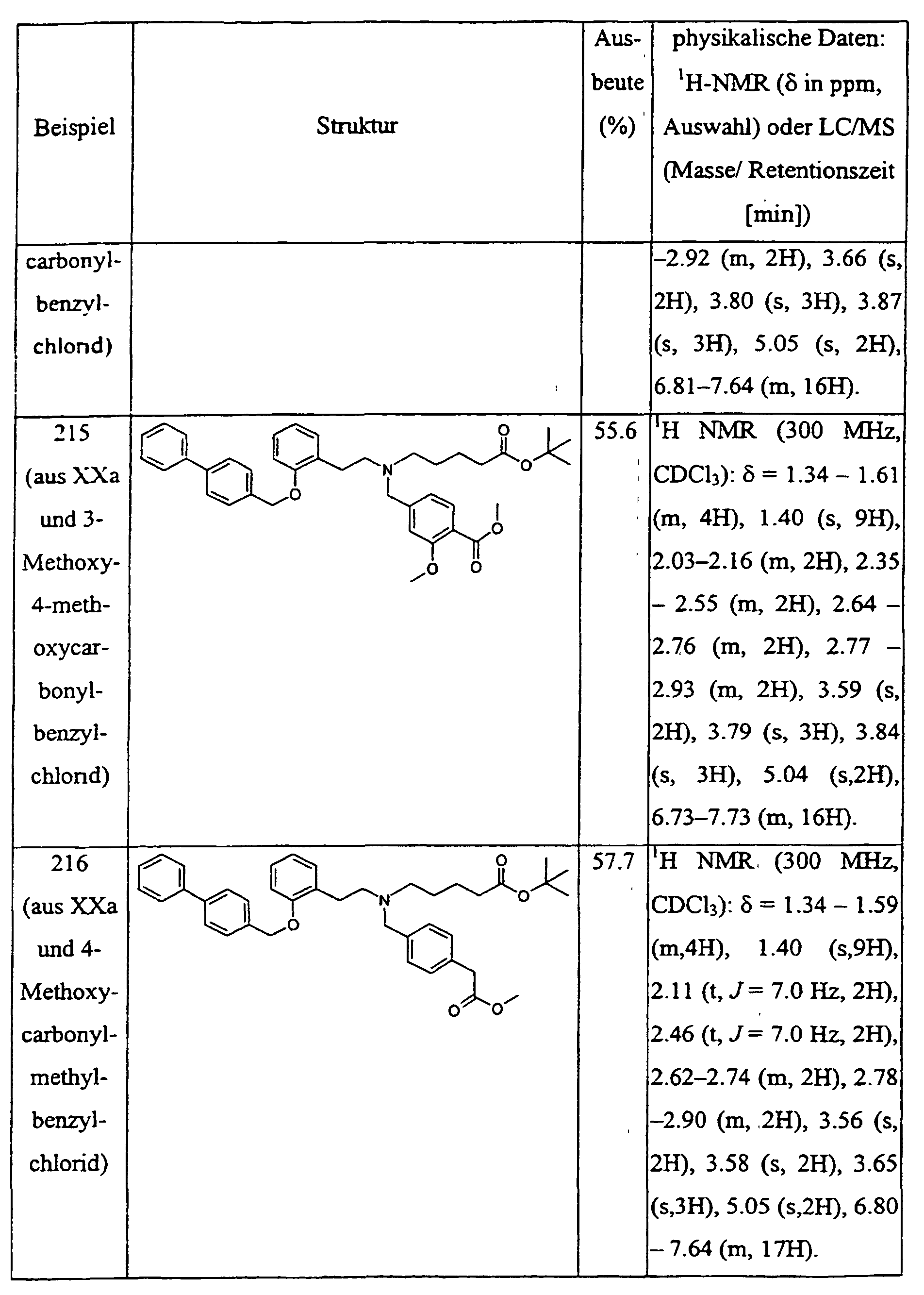

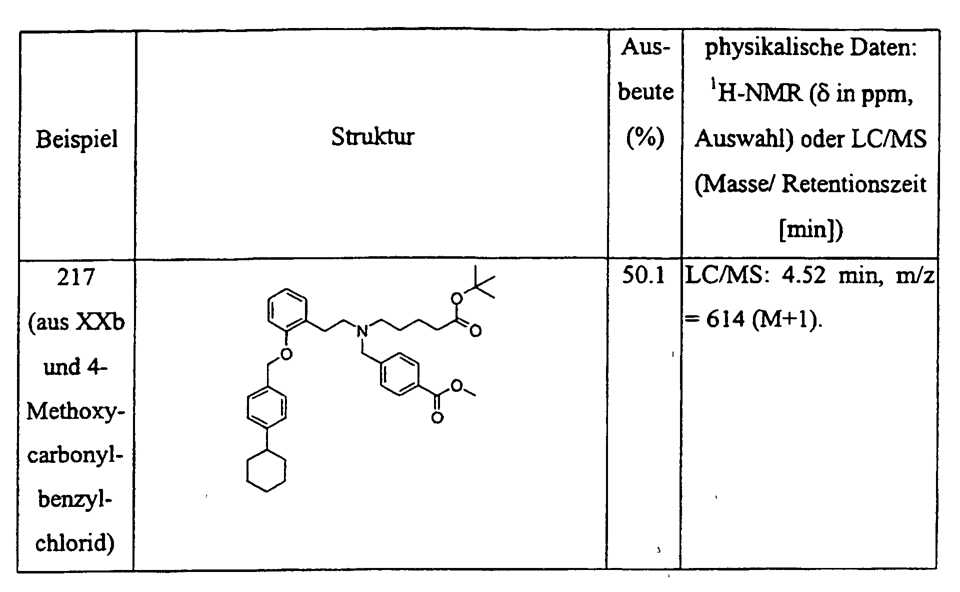

### 218: 5-{{2-[2-(1,1'-Biphenyl-4-ylmethoxy)phenyl]ethyl}[2-methoxy-4-(methoxycarbonyl)-benzyl]-amino}pentansaure Hydrochlorid

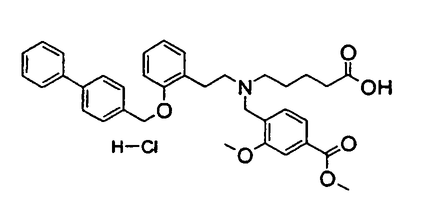

Zu einer Lösung von 96.7 mg (0.15 mmol) der Verbindung aus Bsp. 214 in 3 ml Dioxan wurden 5 ml 1 M HCl in Dioxan zugegeben. Man rührte bei Raumtemperatur und kontrollierte die Reaktion per Dünnschichtchromatographie. Nachdem die Reaktion beendet war, entfernte man das Lösungsmittel und reinigte das Produkt chromatografisch (Kieselgel, CH₂Cl₂/MeOH 10:1).
Ausbeute: 51.8 mg (55.2 %)
¹H NMR (400 MHz, DMSO-d₆): δ = 1.37 - 1.49 (m, 2H), 1.59 - 1.80 (m, 2H), 2.03 - 2.26 (m, 2H), 2.95 - 3.37 (m, 6H), 3.83 (s, 3H), 3.87 (s, 3H), 4.34 (s, 2H), 5.15 (s, 2H), 6.82 - 7.77 (m, 16H), 9.45 (bs, 1H), 12.08 (bs, 1H).

Auf analoge Weise wurden die folgenden Verbindungen hergestellt, wobei eine weitergehende Verseifung des Monoesters auf folgende Weise erreicht wurde:
Eine Mischung aus 0.078 mmol Monoester, 1 ml Wasser, 200 µl 45%ige NaOH und 2 ml Dioxan wurden 16 h bei Raumtemperatur gerührt. Man säuerte mit 1 N HCl an und entfernt das Lösungsmittel. Der Rückstand wird in Ethanol aufgenommen und man filtriert das gebildete Natriumchlond ab. Das Produkt wurde chromatographisch (präparative Dünnschichtchromatografie, EtOH) gereinigt.

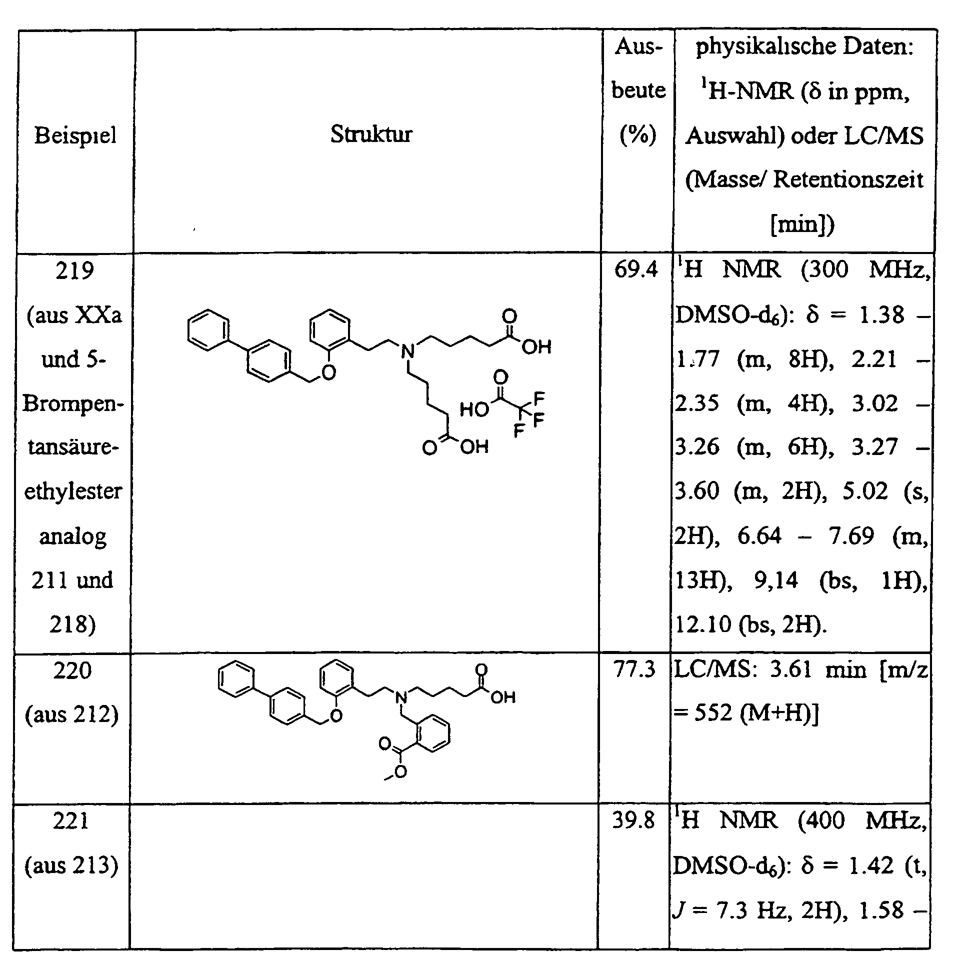

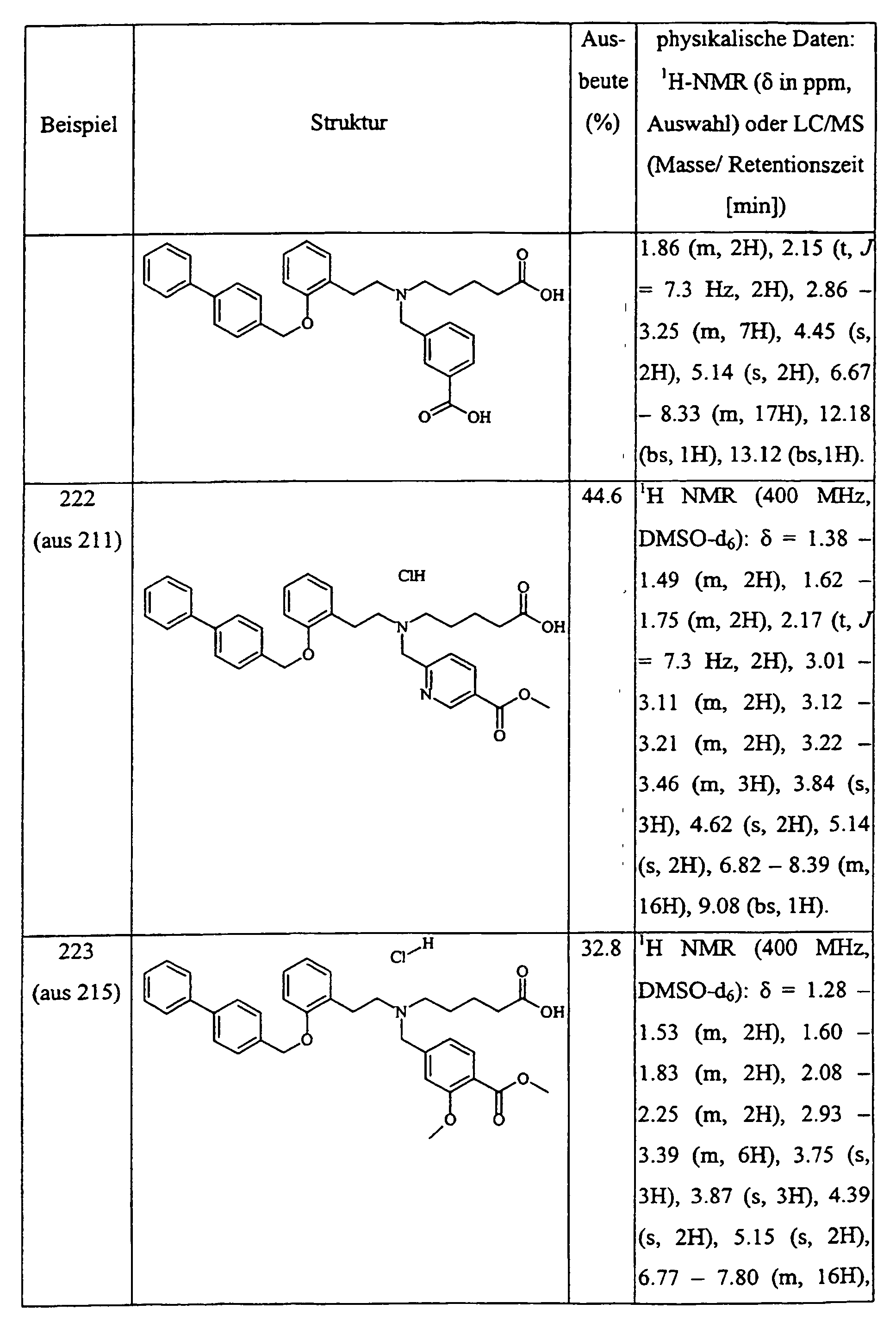

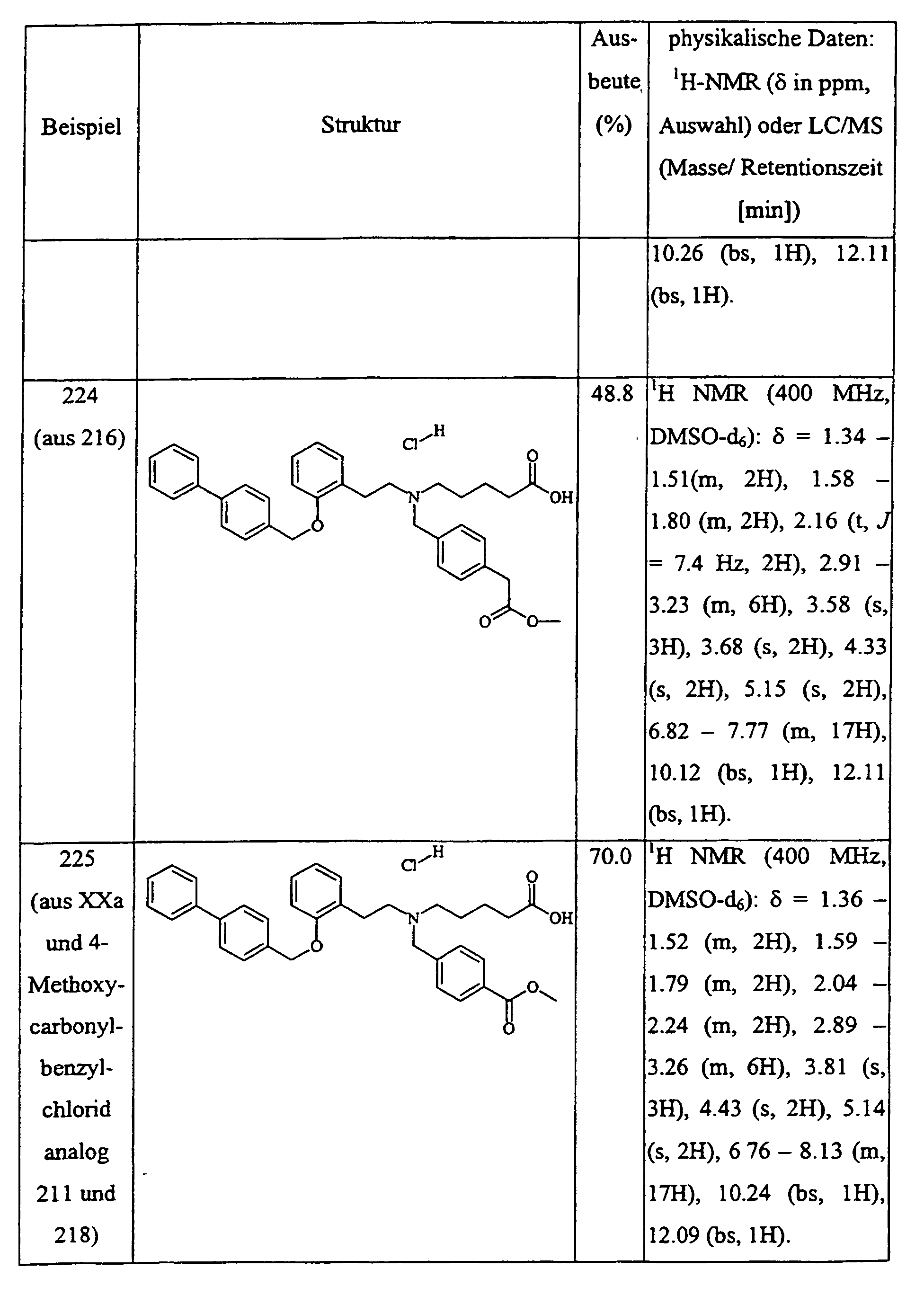

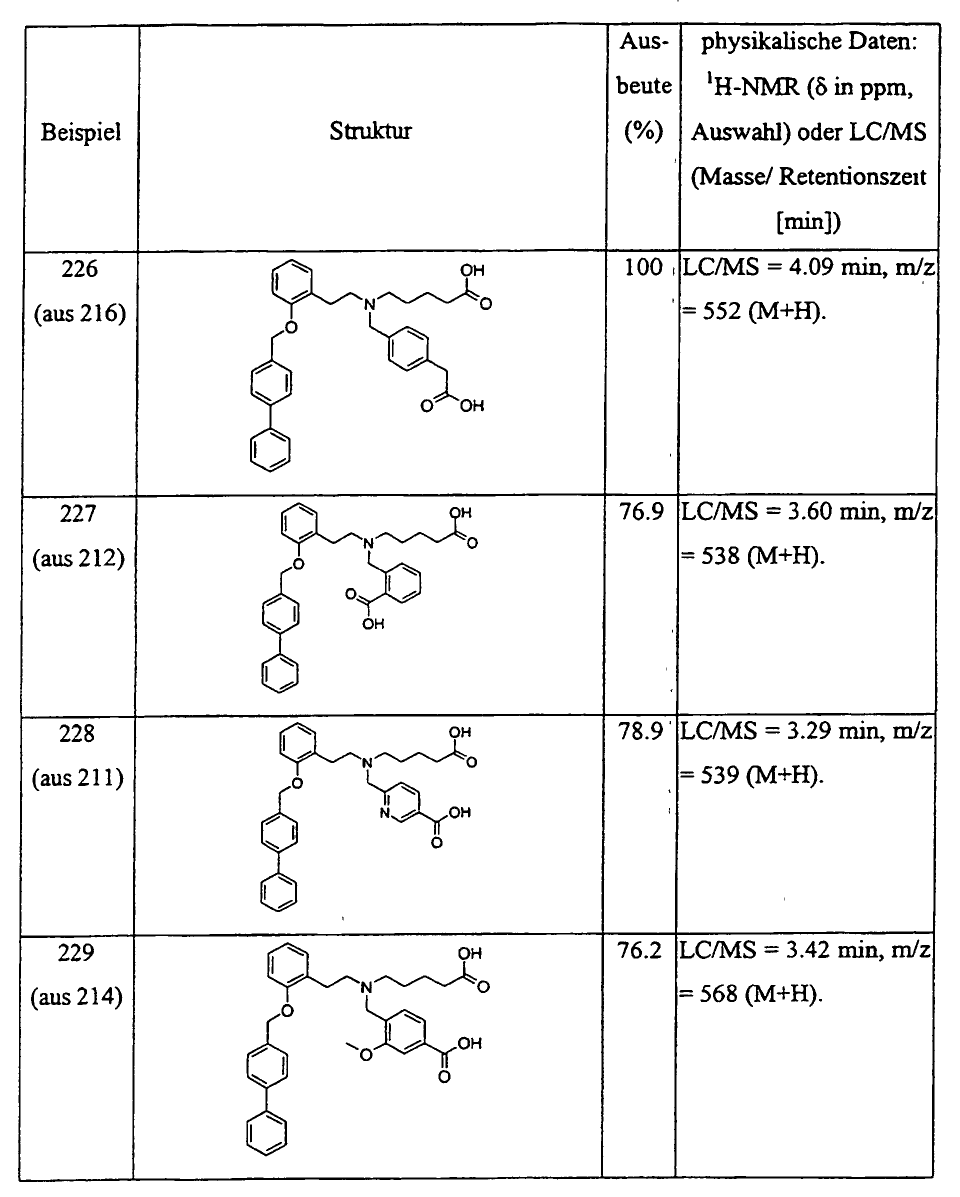

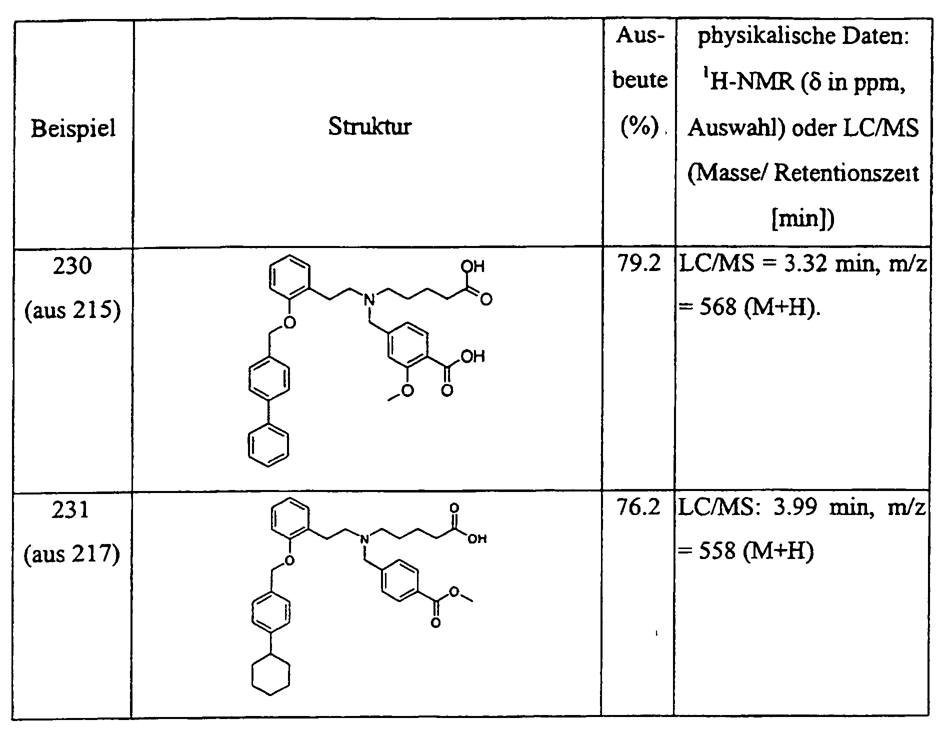

### 232: 4-[((4-carboxybutyl){2-[2-({4-[2-(4-hydroxyphenyl)ethyl]benzyl}oxy)phenyl]ethyl}amino)methyl]benzoesäure

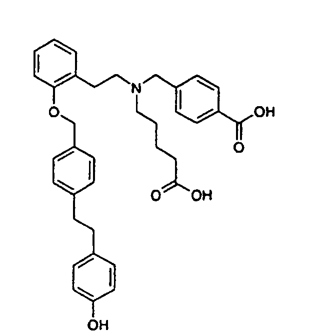

27 mg (0,037 mmol) 4-{[{2-[2-({4-[2-(4-{[Tert-butyl(dimethyl)silyl]oxy}phenyl)ethyl]benzyl}oxy)phenyl]ethyl}(5-ethoxy-5-oxopentyl)amino]methyl}benzoesäuremethylester aus XXI werden in 10 ml THF gelöst. 0,03 ml Tetrabutylammoniumfluorid (1M Lösung in THF) werden zugegeben und die Lösung wird 1 Stunde bei RT gerührt. Die Lösungsmittel werden im Vakuum eingedampft. Der Rückstand wird in 2 ml Methanol gelöst. 0,05 ml Natronlauge 45% und 0,2 mL Dichlormethan werden zugegeben und die Lösung wird 8 Stunden bei RT gerührt. Die Mischung wird eingeengt, Wasser wird zugegeben und die Lösung wird mit Schwefelsäure sauer gestellt. Das Feststoff wird abfiltriert und getrocknet
Ausbeute: 20 mg (93% d.Th.)
¹H -NMR (300 MHz, MeOD): δ= 1.45 (m, 4H), 2.30 (t, 2H), 2.80 (m, 4H), 3.00-3.40 (m), 4.80 (s, 2H), 5.00 (s, 2H), 6.60 (m, 2H), 6.90 -7.30 (10H), 7.50 (d, 2H), 8.00 (d, 2H).

## Patentansprüche

1. Verbindungen der allgemeinen Formel (I) 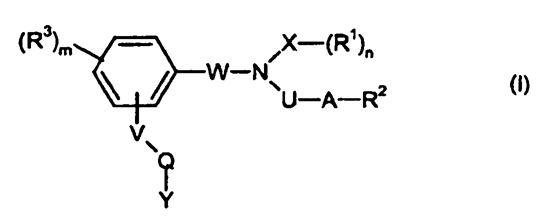 worin
V fehlt, O, S oder NR⁴ bedeutet,
worin
R⁴ Wasserstoff oder Methyl bedeutet,
Q fehlt, geradkettiges oder verzweigtes Alkylen mit bis zu 9 Kohlenstoffatomen oder geradkettiges oder verzweigtes Alkendiyl oder geradkettiges oder verzweigtes Alkindiyl mit bis zu 4 Kohlenstoffatomen bedeutet, die einfach durch Halogen substituiert sein können,
Y H, NR⁸R⁹, Cyclohexyl, Phenyl, Naphthyl oder einen Heterocyclus aus der Gruppe 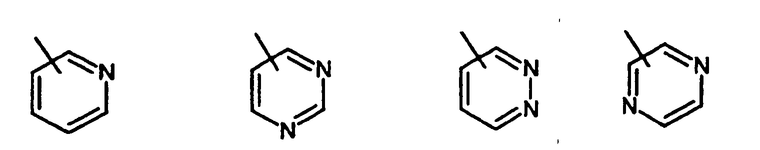 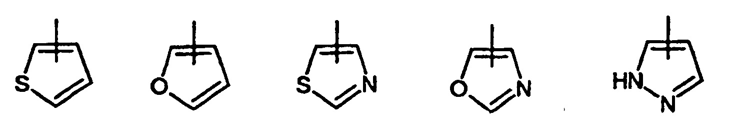 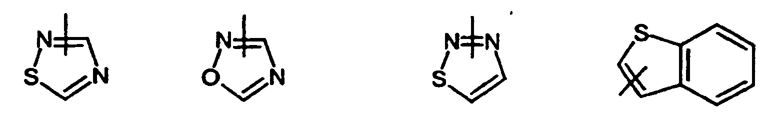 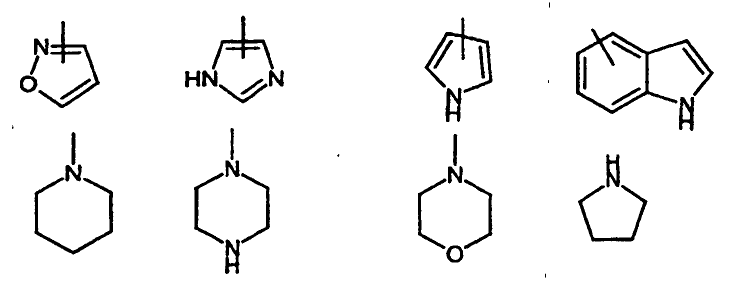 bedeutet, die auch über N gebunden sein können,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Alkoxyalkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 6 Kohlenstoffatomen, F, Cl, Br, I, NO₂, SR⁶, NR⁸R⁹, NR⁷COR¹⁰ oder CONR¹¹R¹² substituiert sein können,
worin
R⁶ Wasserstoff, geradkettiges oder verzweigtes Alkyl mit bis zu 8 Kohlenstoffatomen, oder geradkettiges oder verzweigtes Halogenalkyl mit bis zu 4 Kohlenstoffatomen bedeutet,
R⁷ Wasserstoff, oder geradkettiges oder verzweigtes Alkyl mit bis zu 4 Kohlenstoffatomen bedeutet,
R⁸, R⁹, R¹¹ und R¹² unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann, oder zwei Substituenten aus R⁸ und R⁹ oder R¹¹ und R¹² miteinander unter Bildung eines fünf- oder sechsgliedrigen Ring verbunden sein können, der durch O oder N unterbrochen sein kann,
R¹⁰ Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 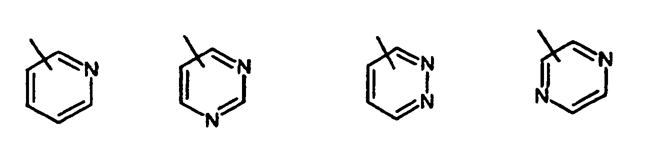 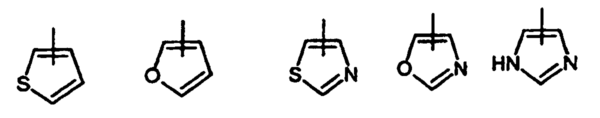 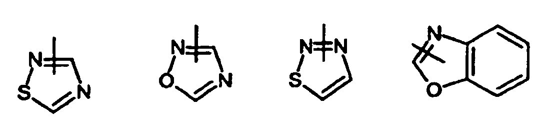 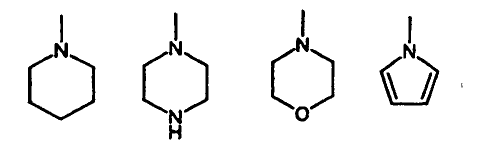 substituiert sein können,
welche direkt oder über eine Gruppe aus O, S, SO, SO₂, NR⁴, SO₂NR⁷, CONR⁷, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden sein können und ein- bis dreifach durch geradkettiges oder verzweigtes Alkyl, geradkettiges oder verzweigtes Alkoxy, geradkettiges oder verzweigtes Alkoxyalkoxy, geradkettiges oder verzweigtes Halogenalkyl oder geradkettiges oder verzweigtes Alkenyl mit jeweils bis zu 4 Kohlenstoffatomen, F, Cl, Br, I, CN, SCH₃, OCF₃, NO₂, NR⁸R⁹ oder NR¹⁴COR¹⁷ substituiert sein können,
worin
R¹⁴ Wasserstoff: geradkettiges oder verzweigtes Alkyl mit bis zu 8 Kohlenstoffatomen oder Cycloalkyl mit 3 bis 8 Kohlenstoffatomen bedeutet,
und
R¹⁷ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff oder Fluor bedeutet,
m eine ganze Zahl von 1 bis 2 bedeutet,
W CH₂, -CH₂CH₂-, CH₂CH₂CH₂, CH=CHCH₂ bedeutet,
U -CH₂- bedeutet,
A Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
R² COOR²⁴ bedeutet,
worin
R²⁴ Wasserstoff oder geradkettiges oder verzweigtes Alkyl mit bis zu 4 Kohlenstoffatomen bedeutet,
X geradkettiges oder verzweigtes Alkylen mit bis zu 8 Kohlenstoffatomen oder geradkettiges oder verzweigtes Alkendiyl mit bis zu 8 Kohlenstoffatomen bedeutet, die jeweils eine bis drei Gruppen aus Phenyl, Phenyloxy, O, CO oder CONR³⁰ enthalten können,
worin
R³⁰ Wasserstoff, geradkettiges oder verzweigtes Alkyl mit bis zu 6 Kohlenstoffatomen oder Cycloalkyl mit 3 bis 6 Kohlenstoffatomen bedeutet,
n 1 oder 2 bedeutet;
R¹ COOR³⁵ bedeutet,
worin
R³⁵ Wasserstoff oder geradkettiges oder verzweigtes Alkyl mit bis zu 6 Kohlenstoffatomen bedeutet.

2. Verbindungen nach Anspruch 1,
worin
V O bedeutet,
Q geradkettiges oder verzweigtes Alkylen mit bis zu 9 Kohlenstoffatomen oder geradkettiges oder verzweigtes Alkendiyl oder geradkettiges oder verzweigtes Alkindiyl mit bis zu 4 Kohlenstoffatomen bedeutet, die einfach durch Halogen substituiert sein können,
Y H, Cyclohexyl, Phenyl oder einen Heterocyclus aus der Gruppe 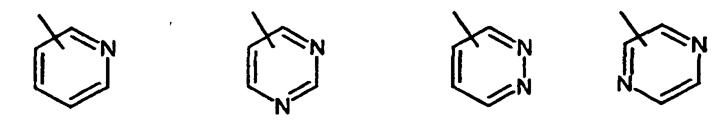 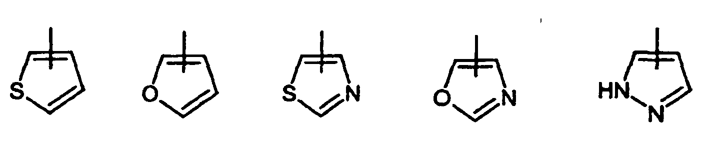 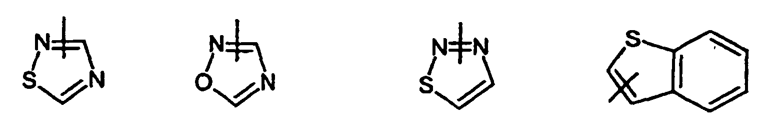 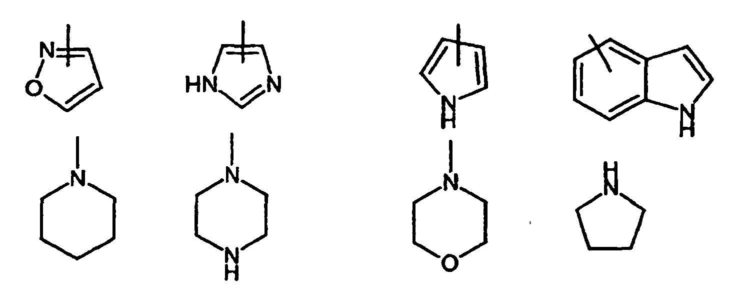 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Alkoxyalkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 6 Kohlenstoffatomen, F, Cl, Br, I, NO₂, SR⁶, NR⁸R⁹, NR⁷COR¹⁰ oder CONR¹¹R¹² substituiert sein können,
worin
R⁶ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁷ Wasserstoff, oder geradkettiges oder verzweigtes Alkyl mit bis zu 4 Kohlenstoffatomen bedeutet,
R⁸, R⁹, R¹¹ und R¹² unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann,
oder zwei Substituenten aus R⁸ und R⁹ oder R¹¹ und R¹² miteinander unter Bildung eines fünf- oder sechsgliedrigen Ring verbunden sein können, der durch O oder N unterbrochen sein kann,
R¹⁰ wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 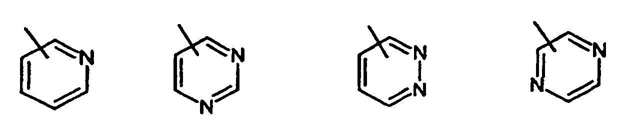 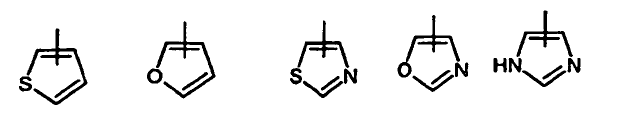 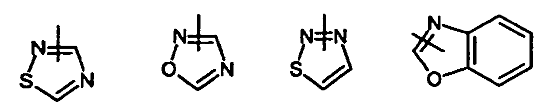 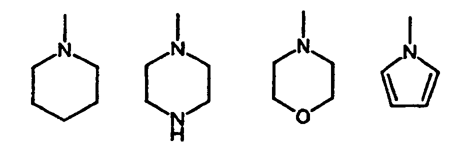 substituiert sein können,
welche direkt oder über eine Gruppe aus O, S, SO, SO₂, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden sein können und ein- bis dreifach durch geradkettiges oder verzweigtes Alkyl, geradkettiges oder verzweigtes Alkoxy, geradkettiges oder verzweigtes Alkoxyalkoxy, geradkettiges oder verzweigtes Halogenalkyl oder geradkettiges oder verzweigtes Alkenyl mit jeweils bis zu 4 Kohlenstoffatomen, F, Cl, Br, I, CN, SCH₃, OCF₃, NO₂, NR⁸R⁹ oder NR¹⁴COR¹⁷ substituiert sein können,
worin
R¹⁴ Wasserstoff geradkettiges oder verzweigtes Alkyl mit bis zu 6 Kohlenstoffatomen oder Cycloalkyl mit 3 bis 6 Kohlenstoffatomen bedeutet,
und
R¹⁷ Wasserstoff, geradkettiges oder verzweigtes Alkyl mit bis zu 6 Kohlenstoffatomen, geradkettiges oder verzweigtes Alkenyl mit bis zu 6 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 6 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff oder Fluor bedeutet,
m eine ganze Zahl von 1 bis 2 bedeutet,
W -CH₂- oder -CH₂CH₂- bedeutet,
U -CH₂- bedeutet,
A Phenyl bedeutet, das gegebenenfalls ein- bis dreifach durch Methyl, Ethyl, n-Propyl, i-Pmpyl, n-Butyl, i-Butyl, s-Butyl, t-Butyl, CF₃, Methoxy, Ethoxy, F, Cl, Br substituiert sein kann,
R² COOR²⁴ bedeutet,
worin
R²⁴ Wasserstoff oder geradkettiges oder verzweigtes Alkyl mit bis zu 4 Kohlenstoffatomen bedeutet,
X geradkettiges oder verzweigtes Alkylen mit bis zu 6 Kohlenstoffatomen oder geradkettiges oder verzweigtes Alkendiyl mit bis zu 6 Kohlenstoffatomen bedeutet, die jeweils eine bis drei Gruppen aus Phenyloxy, O, CO oder CONR³⁰ enthalten können,
worin
R³⁰ Wasserstoff, geradkettiges oder verzweigtes Alkyl mit bis zu 6 Kohlenstoffatomen oder Cycloalkyl mit 3 bis 6 Kohlenstoffatomen bedeutet,
n 1 oder 2 bedeutet;
R¹ COOR³⁵ bedeutet,
worin
R³⁵ Wasserstoff oder geradkettiges oder verzweigtes Alkyl mit bis zu 4 Kohlenstoffatomen bedeutet.

3. Verbindungen nach nsprüch 1,
worin
V O bedeutet,
Q geradkettiges oder verzweigtes Alkylen mit bis zu 9 Kohlenstoffatomen oder geradkettiges oder verzweigtes Alkendiyl oder geradkettiges oder verzweigtes Alkindiyl mit bis zu 4 Kohlenstoffatomen bedeutet, die einfach durch Halogen substituiert sein können,
Y H, Cyclohexyl, Phenyl oder einen Heterocyclus aus der Gruppe 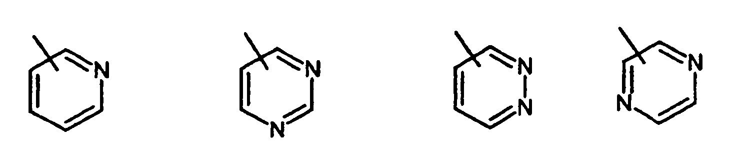 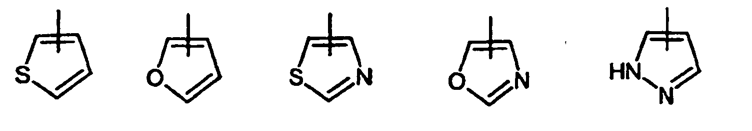 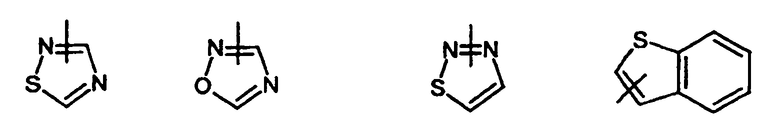 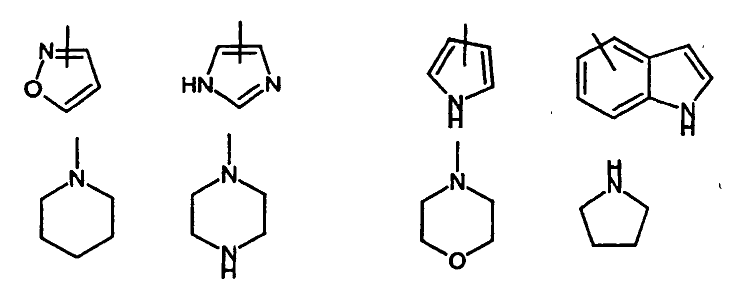 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Alkoxyalkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 6 Kohlenstoffatomen, F, Cl, Br, I, NO₂, SR⁶, NR⁸R⁹, NR⁷COR¹⁰ oder CONR¹¹R¹² substituiert sein können,
worin
R⁶ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁷ Wasserstoff, oder geradkettiges oder verzweigtes Alkyl mit bis zu 4 Kohlenstoffatomen bedeutet,
R⁸, R⁹, R¹¹ und R¹² unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann,
oder zwei Substituenten aus R⁸ und R⁹ oder R¹¹ und R¹² miteinander unter Bildung eines fünf- oder sechsgliedrigen Ring verbunden sein können, der durch O oder N unterbrochen sein kann,
R¹⁰ Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 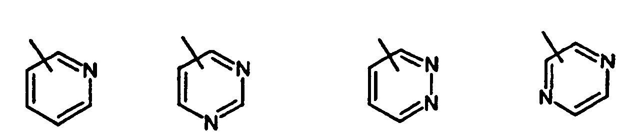 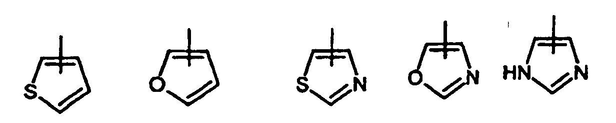 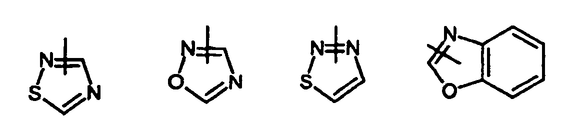 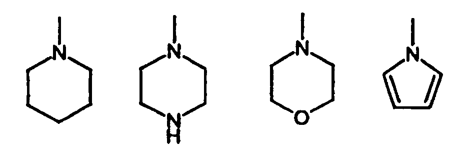 substituiert sein können,
welche direkt oder über eine Gruppe aus O, S, SO, SO₂, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden sein können und ein- bis dreifach durch geradkettiges oder verzweigtes Alkyl, geradkettiges oder verzweigtes Alkoxy, geradkettiges oder verzweigtes Alkoxyalkoxy, geradkettiges oder verzweigtes Halogenalkyl oder geradkettiges oder verzweigtes Alkenyl mit jeweils bis zu 4 Kohlenstoffatomen, F, Cl, Br, I, CN, SCH₃, OCF₃, NO₂, NR⁸R⁹ oder NR¹⁴COR¹⁷ substituiert sein können,
worin
R¹⁴ Wasserstoff. geradkettiges oder verzweigtes Alkyl mit bis zu 6 Kohlenstoffatomen oder Cycloalkyl mit 3 bis 6 Kohlenstoffatomen bedeutet,
und
R¹⁷ Wasserstoff, geradkettiges oder verzweigtes Alkyl mit bis zu 6 Kohlenstoffatomen, geradkettiges oder verzweigtes Alkenyl mit bis zu 6 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 6 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff oder Fluor bedeutet,
m eine ganze Zahl von 1 bis 2 bedeutet,
W -CH₂- oder -CH₂CH₂- bedeutet,
U -CH₂- bedeutet,
A Phenyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
R² COOH bedeutet,
X geradkettiges oder verzweigtes Alkylen mit bis zu 6 Kohlenstoffatomen oder geradkettiges oder verzweigtes Alkendiyl mit bis zu 6 Kohlenstoffatomen bedeutet, die jeweils eine bis drei Gruppen aus Phenyloxy, O, CO oder CONR³⁰ enthalten können,
worin
R³⁰ Wasserstoff, geradkettiges oder verzweigtes Alkyl mit bis zu 6 Kohlenstoffatomen oder Cycloalkyl mit 3 bis 6 Kohlenstoffatomen bedeutet,
n 1 oder 2 bedeutet;
R¹ COOH bedeutet.

4. Verbindungen nach Anspruch 1,
worin
V O bedeutet,
Q CH₂ bedeutet,
Y Phenyl bedeutet, das mit einem Rest substituiert ist, der aus der Gruppe, bestehend aus 2-Phenylethyl, Cyclohexyl, 4-Chlorphenyl, 4-Methoxyphenyl, 4-Trifluormethylphenyl, 4-Cyanophenyl, 4-Chlorphenoxy, 4-Methoxyphenoxy, 4-Trifluormethylphenoxy, 4-Cyanophenoxy, 4-Methylphenyl ausgewählt ist,
R³ Wasserstoff oder Fluor bedeutet,
m eine ganze Zahl von 1 bis 2 bedeutet,
W -CH₂CH₂- bedeutet,
U -CH₂- bedeutet,
A Phenyl bedeutet,
R² COOH bedeutet, wobei _{R2} in 4-Position zum Rest U angeordnet ist,
X (CH₂)₄ bedeutet,
R¹ COOH bedeutet.

5. Verbindung nach Anspruch 1 mit der folgenden Struktur

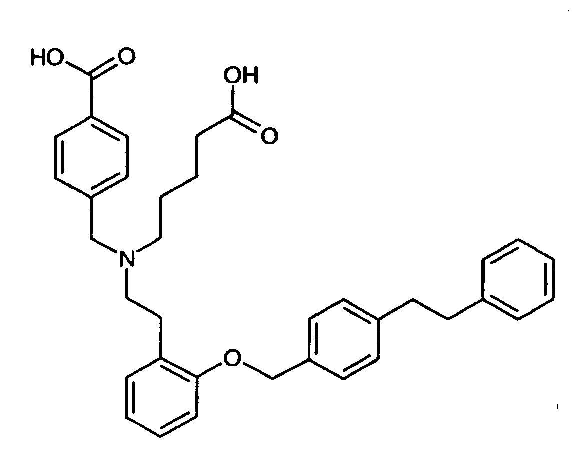

6. Verfahren zur Herstellung von Verbindungen der allgemeinen Formel (I), **dadurch gekennzeichnet, dass** man
[A] Verbindungen der Formel (II) 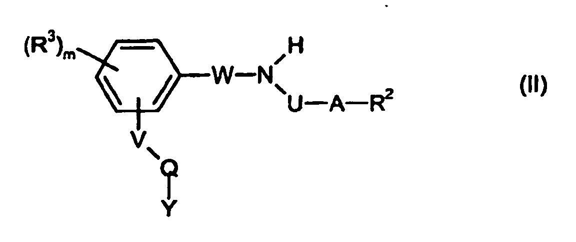 mit Verbindungen der Formel (III)
E-X-R¹ (III)
umsetzt,
worin
R¹, R², R³, V, Q, Y, W, X, U, A und m die gleichen Bedeutungen wie in Anspruch 1 haben,
E entweder eine Abgangsgruppe bedeutet, die in Gegenwart einer Base substituiert wird, oder eine gegebenenfalls aktivierte Hydroxyfunktion ist;
oder
[B] Verbindungen der Formel (IV) 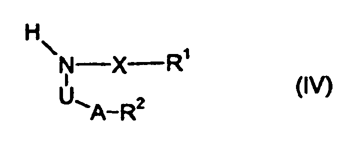 mit Verbindungen der Formel (V) 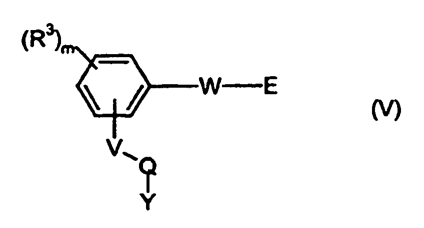 umsetzt,
worin
R¹, R², R³, V, Q, Y, W, X, U, A und m die gleichen Bedeutungen wie in Anspruch 1 haben,
E entweder eine Abgangsgruppe bedeutet, die in Gegenwart einer Base substituiert wird, oder eine gegebenenfalls aktivierte Hydroxyfunktion ist;
oder
[C] Verbindungen der Formel (VI) 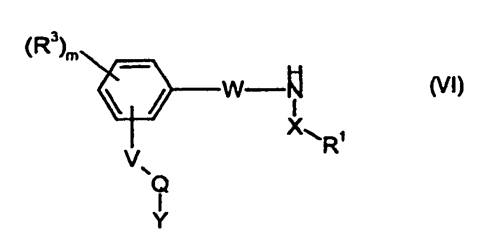 mit Verbindungen der Formel (VII)
E-U-A-R² (VII)
umsetzt,
worin
R¹, R², R³, V, Q, Y, W, X, U, A und m die gleichen Bedeutungen wie in Anspruch 1 haben,
E entweder eine Abgangsgruppe bedeutet, die in Gegenwart einer Base substituiert wird, oder eine gegebenenfalls aktivierte Hydroxyfunktion ist;
oder
[D] Verbindungen der Formel (VIII), 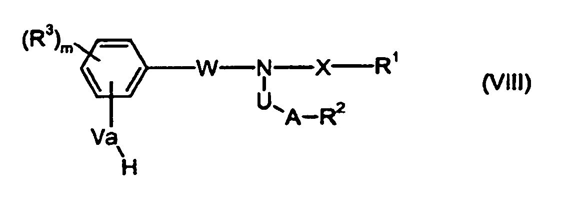 worin
Va für O oder S steht und
W, A, X, U, R¹, R², R³ und m die in Anspruch 1 angegebene Bedeutung haben
mit Verbindungen der Formel (IX) 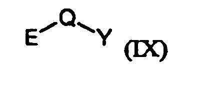 umsetzt,
worin
Q, Y die gleichen Bedeutungen wie in Anspruch 1 haben,
E entweder eine Abgangsgruppe bedeutet, die in Gegenwart einer Base substituiert wird, oder eine gegebenenfalls aktivierte Hydroxyfunktion ist;
oder
[E] Verbindungen der Formel (X), 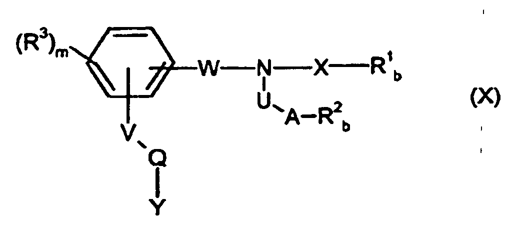 worin
R³, V, Q, Y, W, X, U, A und m die gleichen Bedeutungen wie in Anspruch 1 haben,
R¹ _{b} und R² _{b} jeweils unabhängig für CN oder COOAlk stehen, wobei Alk für einen geradkettigen oder verzweigten Alkylrest mit bis zu 6 Kohlenstoffatomen steht,
mit wässrigen Lösungen starker Säuren oder starker Basen in die entsprechenden freien Carbonsäuren überführt.
oder
[F] Verbindungen der Formel (XI) 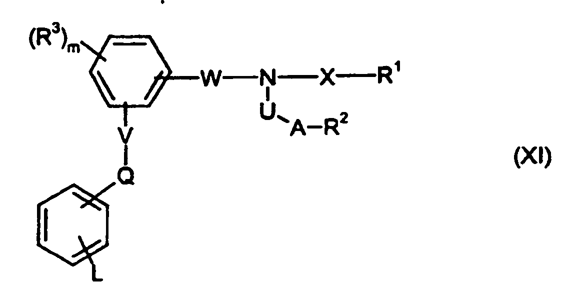 worin
R¹, R², R³, V, Q, Y, W, X, U, A und m die gleichen Bedeutungen wie in Anspruch 1 haben,
L für Br, I oder die Gruppe CF₃SO₂-O steht,
mit Verbindungen der Formel (XII)
M-Z (XII)
worin
M für einen Aryl oder Heteroarylrest, einen geradkettigen oder verzweigten Alkyl-, Alkenyl- oder Alkinylrest oder Cycloalkylrest oder für einen Arylalkyl, einen Arylalkenyl- oder einen Arylalkinylrest steht,
Z für die Gruppierungen -B(OH)₂, -CH≡CH, -CH=CH₂ oder -Sn(nBu)₃ steht
in Gegenwart einer Palladiumverbindung, gegebenenfalls zusätzlich in Gegenwart eines Reduktionsmittels und weiterer Zusatzstoffe und in Gegenwart einer Base umsetzt;
oder
[G] Verbindungen der Formel (XIII) 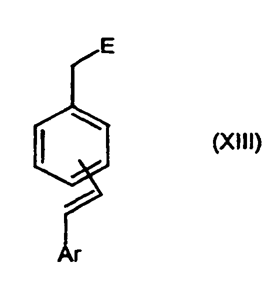 worin
Ar für einen Aryl oder Heteroarylrest steht,
E eine Abgangsgruppe bedeutet, die in Gegenwart einer Base substituiert wird.
nach Verfahren D mit Verbindungen der Formel (VIII) umsetzt und die so erhaltenen Verbindungen der Formel (XIV) 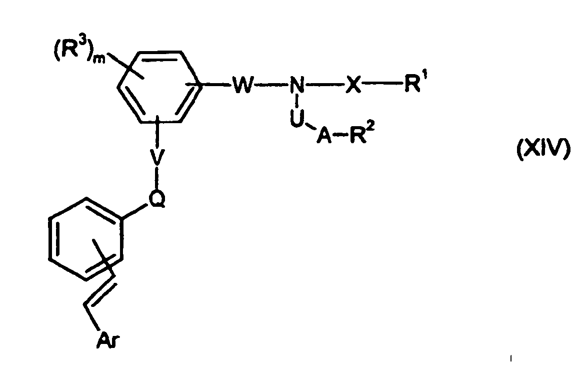 mit Wasserstoff in Gegenwart eines Katalysators hydriert.

7. Verbindungen der Formel (II) 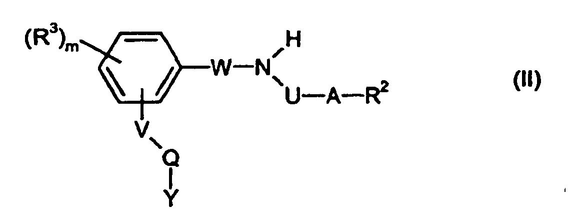 worin
V, Q, Y, R³, m, W, N, U, A und R² die in Anspruch 1 angegebene Bedeutung haben.

8. Verbindungen der Formel (IV) 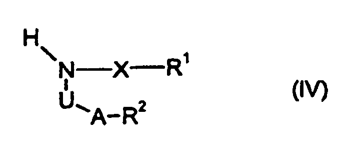 worin
U, A, X, R¹ und R² die in Anspruch 1 angegebene Bedeutung haben.

9. Verbindungen der Formel (VI) 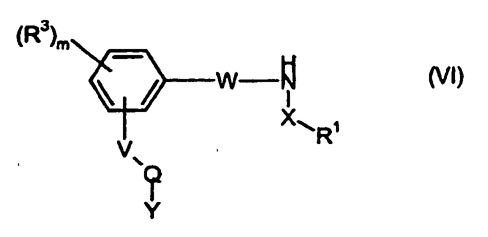 worin
V, Q, Y, R³, m, W, X und R¹ die in Anspruch 1 angegebene Bedeutung haben.

10. Arzneimittel enthaltend mindestens eine Verbindung der allgemeinen Formel (I) gemäß einem der Ansprüche 1 bis 5.

11. Verwendung von Verbindungen der Formel (I) gemäß einem der Ansprüche 1 bis 5 zur Herstellung eines Arzneimittels zur Behandlung von Herz-Kreislauf-Erkrankungen.

12. Verwendung von Verbindungen der allgemeinen Formel (I) gemäß einem der Ansprüche 1 bis 5 zur Herstellung von Arzneimitteln zur Behandlung von Angina pectoris, Ischämien und Herzinsuffizienz.

13. Verwendung von Verbindungen der allgemeinen Formel (I) gemäß einem der Ansprüche 1 bis 5 zur Herstellung von Arzneimitteln zur Behandlung von Hypertonie, thromboembolischen Erkrankungen, Arteriosklerose und venösen Erkrankungen.

14. Verwendung von Verbindungen der allgemeinen Formel (I) gemäß einem der Ansprüche 1 bis 5 zur Herstellung von Arzneimitteln zur Behandlung von fibrotischen Erkrankungen.

15. Verwendung nach Anspruch 14, **dadurch gekennzeichnet, dass** die fibrotische Erkrankung Leberfibrose ist.

## Claims

1. Compounds of the general formula (I) 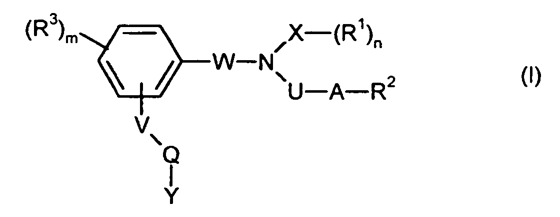 in which
V is absent, O, S or NR⁴,
in which
R⁴ is hydrogen or methyl,
Q is absent, straight-chain or branched alkylene having up to 9 carbon atoms or straight-chain or branched alkenediyl or straight-chain or branched alkinediyl having up to 4 carbon atoms which may be monosubstituted by halogen,
Y is H, NR⁸R⁹, cyclohexyl, phenyl, naphthyl or a heterocycle from the group consisting of 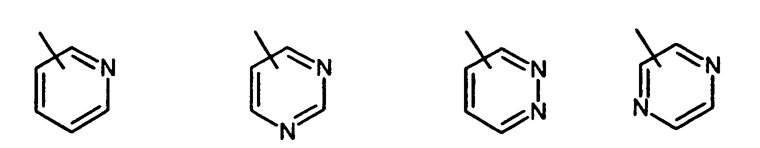 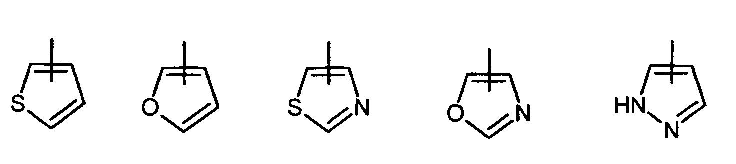 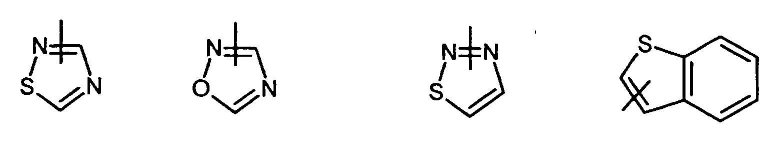 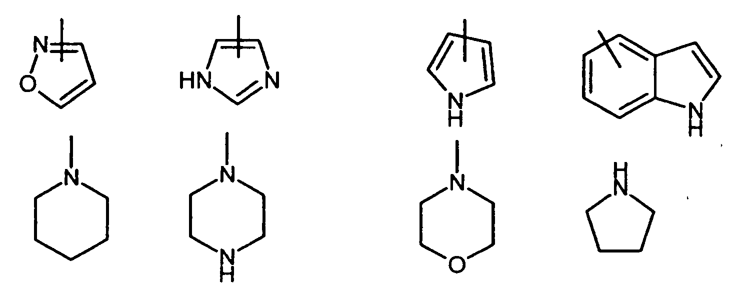 which may also be attached via N,
where the cyclic radicals may in each case be mono- to trisubstituted by straight-chain or branched alkyl, straight-chain or branched alkenyl, straight-chain or branched alkinyl, straight-chain or branched alkoxy, straight-chain or branched alkoxyalkoxy, straight-chain or branched halogenoalkyl, straight-chain or branched halogenoalkoxy having in each case up to 4 carbon atoms, straight-chain or branched cycloalkyl having 3 to 6 carbon atoms, F, Cl, Br, I, NO₂, SR⁶, NR⁸R⁹, NR⁷COR¹⁰ or CONR¹¹R¹²,
in which
R⁶ is hydrogen, straight-chain or branched alkyl having up to 8 carbon atoms, or straight-chain or branched halogenoalkyl having up to 4 carbon atoms,
R⁷ is hydrogen, or straight-chain or branched alkyl having up to 4 carbon atoms,
R⁸, R⁹, R¹¹ and R¹² independently of one another are hydrogen, straight-chain or branched alkyl having up to 4 carbon atoms or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN,
or two substituents R⁸ and R⁹ or R¹¹ and R¹² may be attached to one another forming a five- or six-membered ring which may be interrupted by O or N,
R¹⁰ is hydrogen, straight-chain or branched alkyl having up to 4 carbon atoms or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 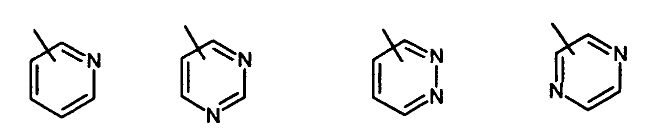 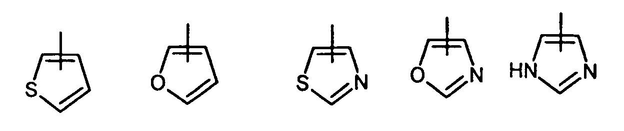 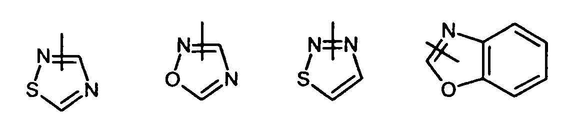 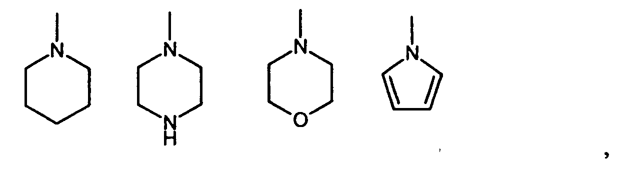 which may be attached directly or via a group O, S, SO, SO₂, NR⁴, SO₂NR⁷, CONR⁷, straight-chain or branched alkylene, straight-chain or branched alkenediyl, straight-chain or branched alkyloxy, straight-chain or branched oxyalkyloxy, straight-chain or branched sulphonylalkyl, straight-chain or branched thioalkyl having in each case 4 carbon atoms and which may be mono- to trisubstituted by straight-chain or branched alkyl, straight-chain or branched alkoxy, straight-chain or branched alkoxyalkoxy, straight-chain or branched halogenoalkyl or straight-chain or branched alkenyl having in each case up to 4 carbon atoms, F, Cl, Br, I, CN, SCH₃, OCF₃, NO₂, NR⁸R⁹ or NR¹⁴COR¹⁷,
in which
R¹⁴ is hydrogen, straight-chain or branched alkyl having up to 8 carbon atoms, or cycloalkyl having 3 to 8 carbon atoms,
and
R¹⁷ is hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may furthermore optionally be substituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ is hydrogen or fluorine,
m is an integer from 1 to 2,
W is CH₂, -CH₂CH₂-, CH₂CH₂CH₂, CH=CHCH₂,
U is -CH₂-,
A is phenyl, pyridyl, thienyl or thiazolyl which may optionally be mono- to trisubstituted by methyl, ethyl, n-propyl, i-propyl, n-butyl, i-butyl, s-butyl, t-butyl, CF₃, methoxy, ethoxy, F, Cl, Br,
R² is COOR²⁴,
in which
R²⁴ is hydrogen or straight-chain or branched alkyl having up to 4 carbon atoms,
X is straight-chain or branched alkylene having up to 8 carbon atoms or straight-chain or branched alkenediyl having up to 8 carbon atoms which may in each case contain one to three groups from the group consisting of phenyl, phenyloxy, O,CO and CONR³⁰,
in which
R³⁰ is hydrogen, straight-chain or branched alkyl having up to 6 carbon atoms or cycloalkyl having 3 to 6 carbon atoms,
n is 1 or 2,
R¹ is COOR³⁵,
in which
R³⁵ is hydrogen or straight-chain or branched alkyl having up to 6 carbon atoms.

2. Compounds according to Claim 1,
in which
V is O,
Q is straight-chain or branched alkylene having up to 9 carbon atoms or straight-chain or branched alkenediyl or straight-chain or branched alkinediyl having up to 4 carbon atoms which may be monosubstituted by halogen,
Y is H, cyclohexyl, phenyl or a heterocycle from the group consisting of 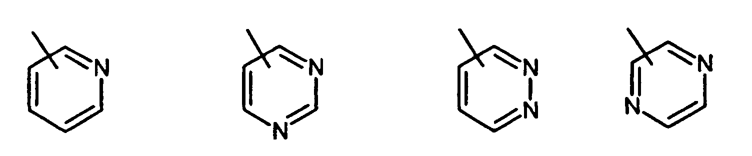 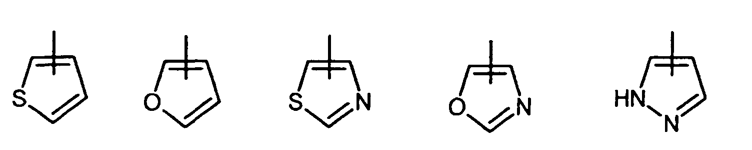 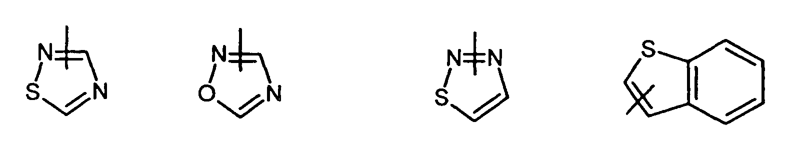 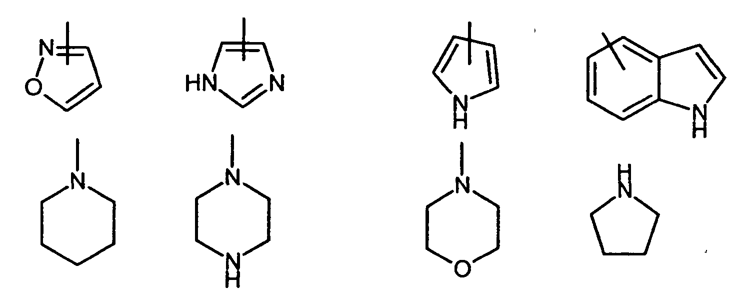 where the cyclic radicals may in each case be mono- to trisubstituted by straight-chain or branched alkyl, straight-chain or branched alkenyl, straight-chain or branched alkinyl, straight-chain or branched alkoxy, straight-chain or branched alkoxyalkoxy, straight-chain or branched halogenoalkyl, straight-chain or branched halogenoalkoxy having in each case up to 4 carbon atoms, straight-chain or branched cycloalkyl having 3 to 6 carbon atoms, F, Cl, Br, I, NO₂, SR⁶, NR⁸R⁹, NR⁷COR¹⁰ or CONR¹¹R¹²,
in which
R⁶ is hydrogen, straight-chain or branched alkyl having up to 4 carbon atoms or straight-chain or branched halogenoalkyl having up to 4 carbon atoms,
R⁷ is hydrogen, or straight-chain or branched alkyl having up to 4 carbon atoms,
R⁸, R⁹, R¹¹ and R¹² independently of one another are hydrogen, straight-chain or branched alkyl having up to 4 carbon atoms or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN,
or two substituents R⁸ and R⁹ or R¹¹ and R¹² may be attached to one another forming a five- or six-membered ring which may be interrupted by O or N,
R¹⁰ is hydrogen, straight-chain or branched alkyl having up to 4 carbon atoms or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 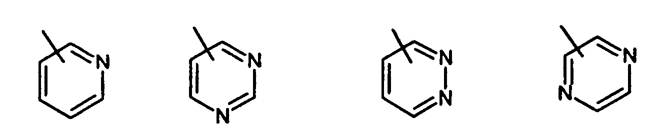 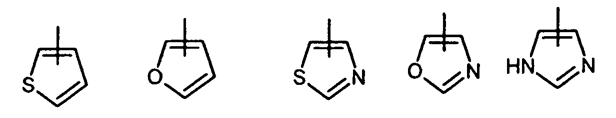 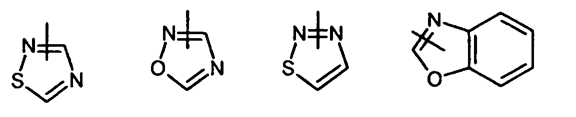 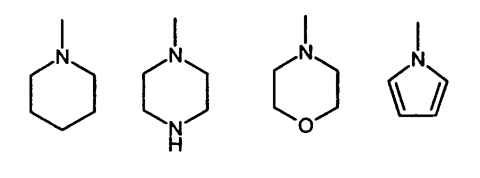 which may be attached directly or via a group O, S, SO, SO₂ straight-chain or branched alkylene, straight-chain or branched alkenediyl, straight-chain or branched alkyloxy, straight-chain or branched oxyalkyloxy, straight-chain or branched sulphonylalkyl, straight-chain or branched thioalkyl having in each case up to 4 carbon atoms and which may be mono- to trisubstituted by straight-chain or branched alkyl, straight-chain or branched alkoxy, straight-chain or branched alkoxyalkoxy, straight-chain or branched halogenoalkyl or straight-chain or branched alkenyl having in each case up to 4 carbon atoms, F, Cl, Br, I, CN, SCH₃, OCF₃, NO₂, NR⁸R⁹ or NR¹⁴COR¹⁷,
in which
R¹⁴ is hydrogen, straight-chain or branched alkyl having up to 6 carbon atoms or cycloalkyl having 3 to 6 carbon atoms,
and
R¹⁷ is hydrogen, straight-chain or branched alkyl having up to 6 carbon atoms, straight-chain or branched alkenyl having up to 6 carbon atoms, aryl having 6 to 10 carbon atoms, an aromatic heterocycle having 1 to 9 carbon atoms and up to 3 heteroatoms from the group consisting of S, N and O or cycloalkyl having 3 to 6 carbon atoms, which may furthermore optionally be substituted by F, Cl, Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ is hydrogen or fluorine,
m is an integer from 1 to 2,
W is -CH₂- or -CH₂CH₂-,
U is -CH₂-,
A is phenyl which may optionally be mono- to trisubstituted by methyl, ethyl, n-propyl, i-propyl, n-butyl, i-butyl, s-butyl, t-butyl, CF₃, methoxy, ethoxy, F, Cl, Br,
R² is COOR²⁴,
in which
R²⁴ is hydrogen or straight-chain or branched alkyl having up to 4 carbon atoms,
X is straight-chain or branched alkylene having up to 6 carbon atoms or straight-chain or branched alkenediyl having up to 6 carbon atoms, which may each contain one to three groups from the group consisting of phenyloxy, O, CO and CONR³⁰,
in which
R³⁰ is hydrogen, straight-chain or branched alkyl having up to 6 carbon atoms or cycloalkyl having 3 to 6 carbon atoms,
n is 1 or 2,
R¹ is COOR³⁵,
in which
R³⁵ is hydrogen or straight-chain or branched alkyl having up to 4 carbon atoms.

3. Compounds according to Claim 1,
in which
V is O,
Q is straight-chain or branched alkylene having up to 9 carbon atoms or straight-chain or branched alkenediyl or straight-chain or branched alkinediyl having up to 4 carbon atoms which may be monosubstituted by halogen,
Y is H, cyclohexyl, phenyl or a heterocycle from the group consisting of 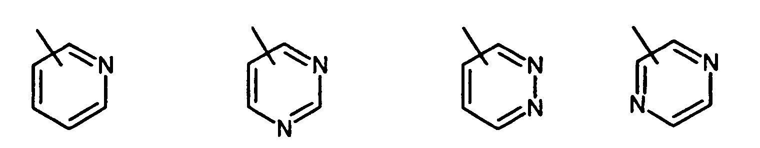 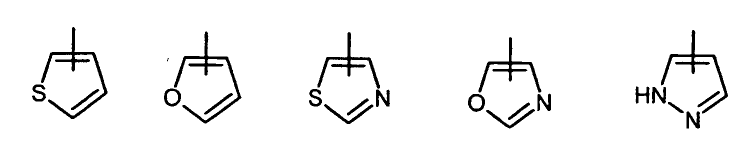 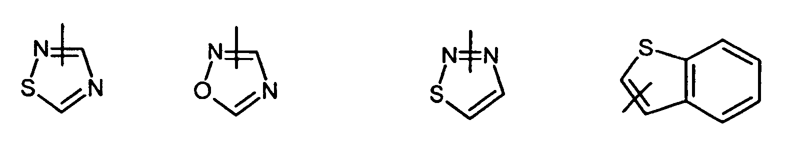 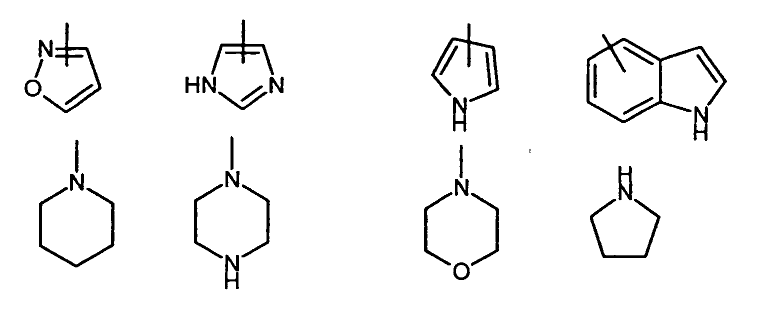 where the cyclic radicals may in each case be mono- to trisubstituted by straight-chain or branched alkyl, straight-chain or branched alkenyl, straight-chain or branched alkinyl, straight-chain or branched alkoxy, straight-chain or branched alkoxyalkoxy, straight-chain or branched halogenoalkyl, straight-chain or branched halogenoalkoxy, having in each case up to 4 carbon atoms, straight-chain or branched cycloalkyl having 3 to 6 carbon atoms, F, Cl, Br, I, NO₂, SR⁶, NR⁸R⁹, NR⁷COR¹⁰ or CONR¹¹R¹²,
in which
R⁶ is hydrogen, straight-chain or branched alkyl having up to 4 carbon atoms or straight-chain or branched halogenoalkyl having up to 4 carbon atoms,
R⁷ is hydrogen or straight-chain or branched alkyl having up to 4 carbon atoms,
R⁸, R⁹, R¹¹ and R¹² independently of one another are hydrogen, straight-chain or branched alkyl having up to 4 carbon atoms or phenyl,
where the phenyl radical may be mono- to trisubstituted by F, Cl Br, hydroxyl, methyl, ethyl, n- propyl, i-propyl, n-butyl, s-butyl, i-butyl, t-butyl, methoxy, ethoxy, amino, acetylamino, NO₂, CF₃, OCF₃ or CN,
or two substituents R⁸ and R⁹ or R¹¹ and R¹² may be attached to one another forming a five- or six-membered ring which may be interrupted by O or N,
R¹⁰ is hydrogen, straight-chain or branched alkyl having up to 4 carbon atoms or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 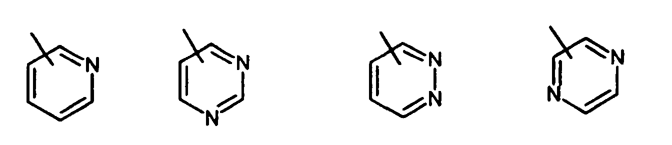 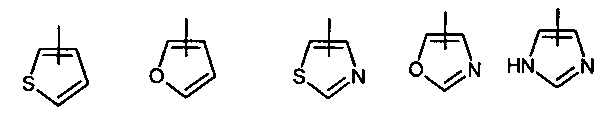 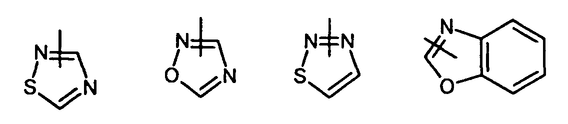 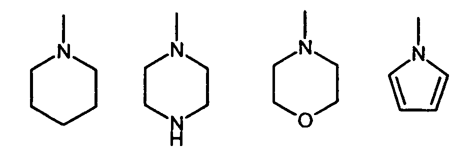 which may be attached directly or via a group O, S, SO, SO₂, straight-chain or branched alkylene, straight-chain or branched alkenediyl, straight-chain or branched alkyloxy, straight-chain or branched oxyalkyloxy, straight-chain or branched sulphonylalkyl, straight-chain or branched thioalkyl having in each case up to 4 carbon atoms and which may be mono- to trisubstituted by straight-chain or branched alkyl, straight-chain or branched alkoxy, straight-chain or branched alkoxyalkoxy, straight-chain or branched halogenoalkyl or straight-chain or branched alkenyl having in each case up to 4 carbon atoms, F, Cl, Br, I, CN, SCH₃, OCF₃, NO₂, NR⁸R⁹ or NR¹⁴COR¹⁷,
in which
R¹⁴ is hydrogen, straight-chain or branched alkyl having up to 6 carbon atoms or cycloalkyl having 3 to 6 carbon atoms,
and
R¹⁷ is hydrogen, straight-chain or branched alkyl having up to 6 carbon atoms, straight-chain or branched alkenyl having up to 6 carbon atoms, aryl having 6 to 10 carbon atoms, an aromatic heterocycle having 1 to 9 carbon atoms and up to 3 heteroatoms from the group consisting of S, N and O or cycloalkyl having 3 to 6 carbon atoms, which may furthermore optionally be substituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ is hydrogen or fluorine,
m is an integer from 1 to 2,
W is -CH₂- or -CH₂CH₂-,
U is -CH₂-,
A is phenyl which may optionally be mono- to trisubstituted by methyl, ethyl, n-propyl, i-propyl, n-butyl, i-butyl, s-butyl, t-butyl, CF₃, methoxy, ethoxy, F, Cl, Br,
R² is COOH,
X is straight-chain or branched alkylene having up to 6 carbon atoms or straight-chain or branched alkenediyl having up to 6 carbon atoms which may in each case contain one to three groups from the group consisting of phenyloxy, O, CO and CONR³⁰,
in which
R³⁰ is hydrogen, straight-chain or branched alkyl having up to 6 carbon atoms or cycloalkyl having 3 to 6 carbon atoms,
n is 1 or 2,
R¹ is COOH.

4. Compounds according to Claim 1,
in which
V is O,
Q is CH₂,
Y is phenyl which is substituted by a radical selected from the group consisting of 2-phenylethyl, cyclohexyl, 4-chlorophenyl, 4-methoxyphenyl, 4-trifluoromethylphenyl, 4-cyanophenyl, 4-chlorophenoxy, 4-methoxyphenoxy, 4-trifluoromethylphenoxy, 4-cyanophenoxy, 4-methylphenyl,
R³ is hydrogen or fluorine,
m is an integer from 1 to 2,
W -is CH₂CH₂-,
U is -CH₂-,
A is phenyl,
R² is COOH, where _{R2} is located in the 4-position to the radical U,
X is (CH₂)₄,
R¹ is COOH.

5. Compound according to Claim 1, having the following structure

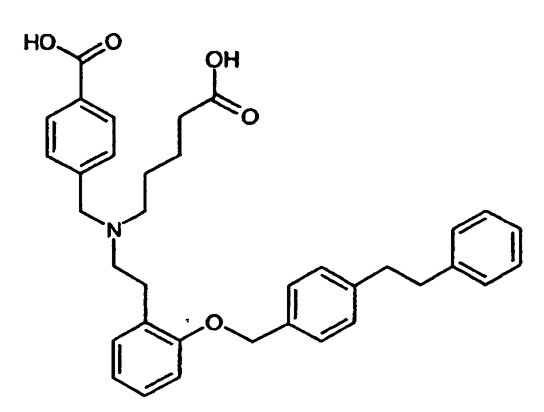

6. Process for preparing compounds of the general formula (I),
**characterized in that**
[A] compounds of the formula (II) 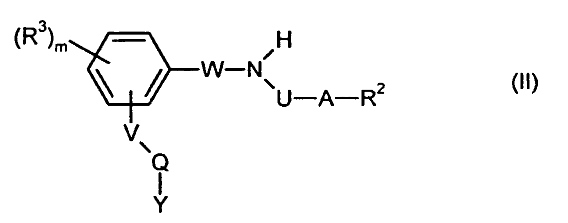 are reacted with compounds of the formula (III)
E-X-R¹ (III)
in which
R¹, R², R³, V, Q, Y, W, X, U, A and m are as defined in Claim 1,
E is either a leaving group which is substituted in the presence of a base or is an optionally activated hydroxyl function;
or
[B] compounds of the formula (IV) 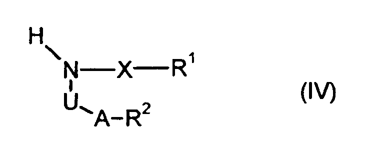 are reacted with compounds of the formula (V) 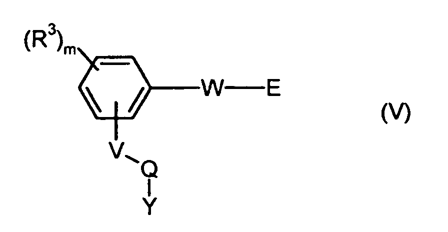 in which
R¹, R², R³, V, Q, Y, W, X, U, A and m are as defined in Claim 1,
E is either a leaving group which is substituted in the presence of a base or is an optionally activated hydroxyl function;
or
[C] compounds of the formula (VI) 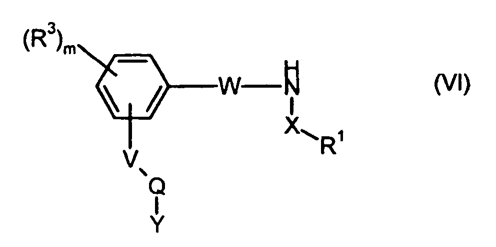 are reacted with compounds of the formula (VII)
E-U-A-R² (VII)
in which
R¹, R², R³, V, Q, Y, W, X, U, A and m are as defined in Claim 1,
E is either a leaving group which is substituted in the presence of a base or is an optionally activated hydroxyl function;
or
[D] compounds of the formula (VIII), 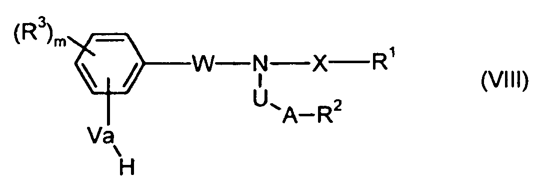 in which
Va is O or S and
W, A, X, U, R¹, R², R³ and m are as defined in Claim 1,
are reacted with compounds of the formula (IX) 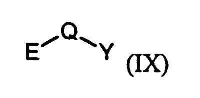 in which
Q, Y are as defined in Claim 1,
E is either a leaving group which is substituted in the presence of a base or is an optionally activated hydroxyl function;
or
[E] compounds of the formula (X) 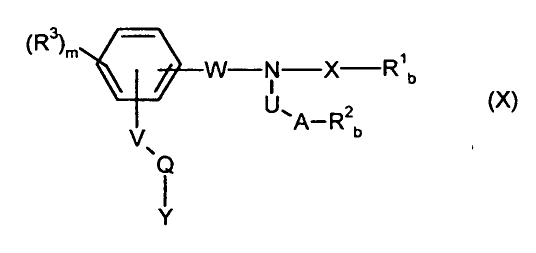 in which
R³, V, Q, Y, W, X, U, A and m are as defined in Claim 1,
R¹ _{b} and R² _{b} independently each represent CN or COOAlk, where Alk represents a straight-chain or branched alkyl radical having up to 6 carbon atoms,
are converted with aqueous solutions of strong acids or strong bases into the corresponding free carboxylic acids;
or
[F] compounds of the formula (XI) 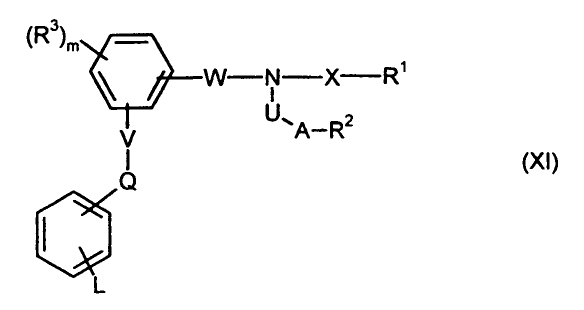 in which
R¹, R², R³, V, Q, Y, W, X, U, A and m are as defined in Claim 1,
L represents Br, I or the group CF₃SO₂-O,
are reacted with compounds of the formula (XII)
M-Z (XII)
in which
M represents an aryl or heteroaryl radical, a straight-chain or branched alkyl, alkenyl or alkinyl radical or cycloalkyl radical or represents an arylalkyl, an arylalkenyl or an arylalkmyl radical,
Z represents the groupings -B(OH)₂, -CH≡CH, -CH=CH₂ or -Sn(nBu)₃,
in the presence of a palladium compound, if appropriate additionally in the presence of a reducing agent and further additives and in the presence of a base;
or
[G] compounds of the formula (XIII) 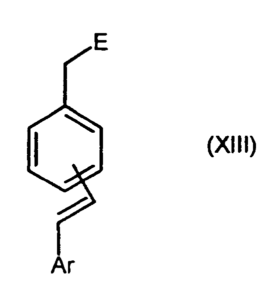 in which
Ar represents an aryl or heteroaryl radical,
E is a leaving group which is substituted in the presence of a base,
are reacted according to process D with compounds of the formula (VIII) and the resulting compounds of the formula (XIV) 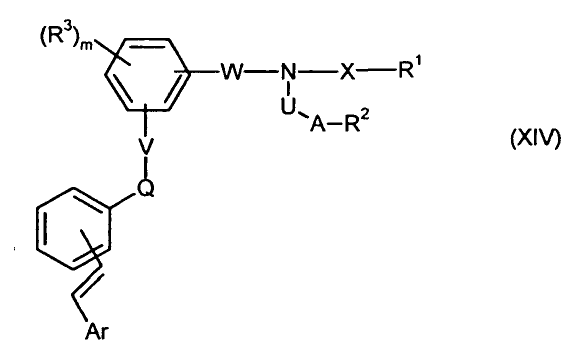 are hydrogenated with hydrogen in the presence of a catalyst.

7. Compounds of the formula (II) 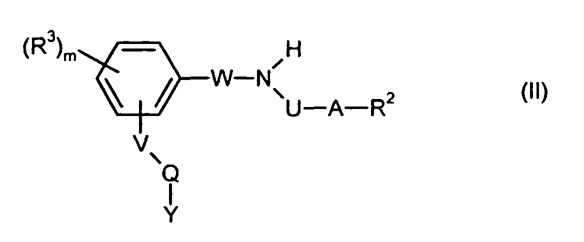 in which
V, Q, Y, R³, m, W, N, U, A and R² are as defined in Claim 1.

8. Compounds of the formula (IV) 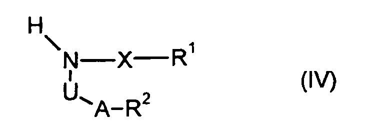 in which
U, A, X, R¹ and R² are as defined in Claim 1.

9. Compounds of the formula (VI) 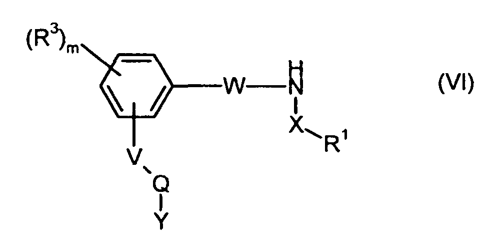 in which
V, Q, Y, R³, m, W, X and R¹ are as defined in Claim 1.

10. Medicaments, comprising at least one compound of the general formula (I) according to any of Claims 1 to 5.

11. Use of compounds of the formula (I) according to any of Claims 1 to 5 for preparing a medicament for the treatment of cardiovascular disorders.

12. Use of compounds of the general formula (I) according to any of Claims 1 to 5 for preparing medicaments for the treatment of angina pectoris, ischaemias and cardiac insufficiency.

13. Use of compounds of the general formula (I) according to any of Claims 1 to 5 for preparing medicaments for the treatment of hypertension, thromboembolic disorders, arteriosclerosis and venous disorders.

14. Use of compounds of the general formula (I) according to any of Claims 1 to 5 for preparing medicaments for the treatment of fibrotic disorders.

15. Use according to Claim 14, **characterized in that** the fibrotic disorder is hepatic fibrosis.

## Revendications

1. Composés de formule générale (I) 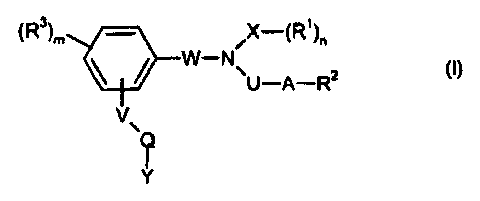 dans laquelle
V est manquant, représente O, S ou NR⁴
où
R⁴ représente l'hydrogène ou le groupe méthyle,
Q est manquant, est un reste alkylène linéaire ou ramifié ayant jusqu'à 9 atomes de carbone ou un reste alcènediyle linéaire ou ramifié ou un reste alcynediyle linéaire ou ramifié ayant jusqu'à 4 atomes de carbone, qui peuvent être substitués une fois par un halogène,
Y représente H, un reste NR⁸R⁹, cyclohexyle, phényle, naphtyle ou l'un des hétérocycles du groupe 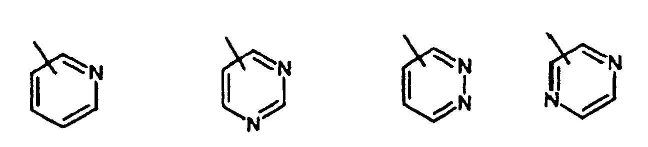 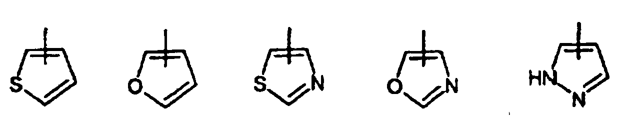 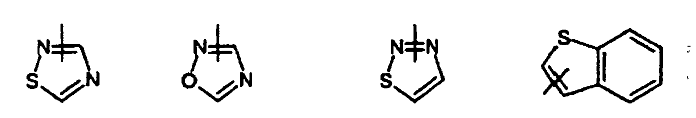 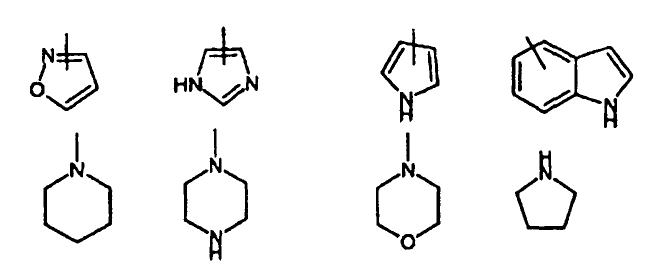 qui peuvent aussi être liés par l'intermédiaire de N, les restes cycliques pouvant être substitués chacun une à trois fois par un radical alkyle linéaire ou ramifié, alcényle linéaire ou ramifié, alcynyle linéaire ou ramifié, alkoxy linéaire ou ramifié, alkoxyalkoxy linéaire ou ramifié, halogénalkyle linéaire ou ramifié, halogénalkoxy linéaire ou ramifié ayant chacun jusqu'à 4 atomes de carbone, cycloalkyle linéaire ou ramifié ayant 3 à 6 atomes de carbone, F, Cl, Br, I, NO₂, SR⁶, NR⁸R⁹, NR⁷COR¹⁰ ou CONR¹¹R¹²,
où
R⁶ représente l'hydrogène, un reste alkyle linéaire ou ramifié ayant jusqu'à 8 atomes de carbone ou un reste halogénalkyle linéaire ou ramifié ayant jusqu'à 4 atomes de carbone,
R⁷ représente l'hydrogène ou un reste alkyle linéaire ou ramifié ayant jusqu'à 4 atomes de carbone,
R⁸, R⁹, R¹¹ et R¹² représentent, indépendamment les uns des autres, l'hydrogène, un reste alkyle linéaire ou ramifié ayant jusqu'à 4 atomes de carbone, ou un reste phényle,
le reste phényle pouvant porter un à trois substituants F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants constitués de R⁸ et R⁹ ou de R¹¹ et R¹² peuvent être liés ensemble en formant un noyau pentagonal ou hexagonal qui peut être interrompu par O ou N,
R¹⁰ représente l'hydrogène, un reste alkyle linéaire ou ramifié ayant jusqu'à 4 atomes de carbone, ou un reste phényle,
le reste phényle pouvant porter un à trois substituants F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être substitués chacun une à trois fois par un radical phényle ou un hétérocycle du groupe des hétérocycles , 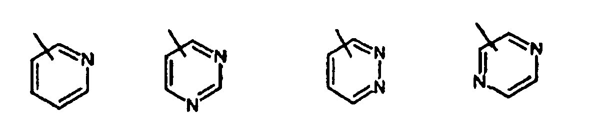 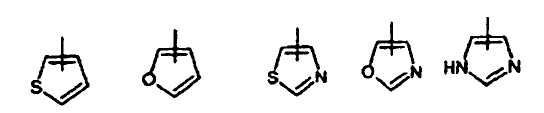 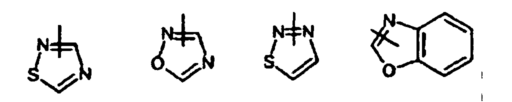 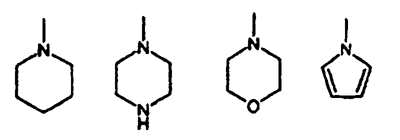 qui peuvent être liés directement ou par l'intermédiaire d'un groupe formé de O, S, SO, SO₂, NR⁴, SO₂NR⁷, CONR⁷, alkylène linéaire ou ramifié, alcènediyle linéaire ou ramifié, alkyloxy linéaire ou ramifié, oxyalkyloxy linéaire ou ramifié, sulfonylalkyle linéaire ou ramifié, thioalkyle linéaire ou ramifié ayant dans chaque cas jusqu'à 4 atomes de carbone, et qui peuvent porter un à trois substituants alkyle linéaire ou ramifié, alkoxy linéaire ou ramifié, alkoxyalkoxy linéaire ou ramifié, halogénalkyle linéaire ou ramifié ou alcényle linéaire ou ramifié ayant chacun jusqu'à 4 atomes de carbone, F, Cl, Br, I, CN, SCH₃, OCF₃, NO₂, NR⁸R⁹ ou NR¹⁴COR¹⁷,
où
R¹⁴ représente l'hydrogène, un reste alkyle linéaire ou ramifié ayant jusqu'à 8 atomes de carbone ou un groupe cycloalkyle ayant 3 à 8 atomes de carbone
et
R¹⁷ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O ou cycloalkyle ayant 3 à 8 atomes de carbone, ces restes pouvant en outre porter éventuellement un substituant F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou O,
R³ représente l'hydrogène ou le fluor,
m est un nombre entier égal à 1 ou 2,
W est un groupe CH₂, -CH₂CH₂-, CH₂CH₂CH₂, CH=CHCH₂,
U est un groupe -CH₂-,
A est un groupe phényle, pyridyle, thiényle ou thiazolyle qui peut éventuellement porter un à trois substituants méthyle, éthyle, n-propyle, isopropyle, n-butyle, isobutyle, sec.-butyle, tertiobutyle, CF₃, méthoxy, éthoxy, F, Cl, Br,
R² est un groupe COOR²⁴,
dans lequel
R²⁴ représente l'hydrogène ou un reste alkyle linéaire ou ramifié ayant jusqu'à 4 atomes de carbone,
X est un reste alkylène linéaire ou ramifié ayant jusqu'à 8 atomes de carbone ou un reste alcènediyle linéaire ou ramifié ayant jusqu'à 8 atomes de carbone, ces restes pouvant contenir dans chaque cas un à trois groupes de la série phényle, phényloxy, O, CO ou CONR³⁰,
où
R³⁰ représente l'hydrogène, un reste alkyle linéaire ou ramifié ayant jusqu'à 6 atomes de carbone ou un reste cycloalkyle ayant 3 à 6 atomes de carbone,
n a la valeur 1 ou 2 ;
R¹ est un groupe COOR³⁵,
dans lequel
R³⁵ représente l'hydrogène ou un reste alkyle linéaire ou ramifié ayant jusqu'à 6 atomes de carbone.

2. Composés suivant la revendication 1,
dans lesquels
V représente O,
Q est un reste alkylène linéaire ou ramifié ayant jusqu'à 9 atomes de carbone ou un reste alcènediyle linéaire ou ramifié ou alcynediyle linéaire ou ramifié ayant jusqu'à 4 atomes de carbone, ces restes pouvant être substitués une fois par un halogène,
Y représente H, un reste cyclohexyle, phényle ou un hétérocycle choisi dans le groupe 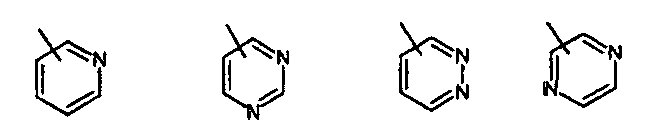 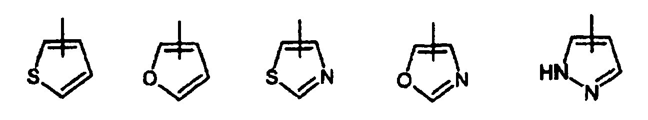 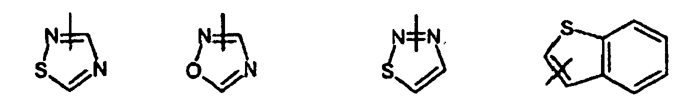 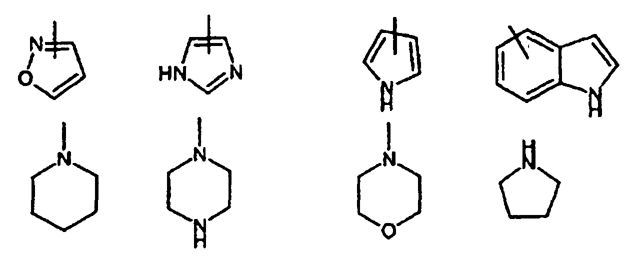 les restes cycliques pouvant être substitués chacun une à trois fois par un radical alkyle linéaire ou ramifié, alcényle linéaire ou ramifié, alcynyle linéaire ou ramifié, alkoxy linéaire ou ramifié, alkoxyalkoxy linéaire ou ramifié, halogénalkyle linéaire ou ramifié, halogénalkoxy linéaire ou ramifié ayant dans chaque cas jusqu'à 4 atomes de carbone, cycloalkyle linéaire ou ramifié ayant 3 à 6 atomes de carbone, F, Cl, Br, I, NO₂, SR⁶, NR⁸R⁹, NR⁷COR¹⁰ ou CONR¹¹R¹²,
où
R⁶ représente l'hydrogène, un reste alkyle linéaire ou ramifié ayant jusqu'à 4 atomes de carbone ou un reste halogénalkyle linéaire ou ramifié ayant jusqu'à 4 atomes de carbone,
R⁷ représente l'hydrogène ou un reste alkyle linéaire ou ramifié ayant jusqu'à 4 atomes de carbone,
R⁸, R⁹, R¹¹ et R¹² représentent, indépendamment les uns des autres, l'hydrogène, un reste alkyle linéaire ou ramifié ayant jusqu'à 4 atomes de carbone, ou un reste phényle,
le reste phényle pouvant porter un à trois substituants F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants constitués de R⁸ et R⁹ ou de R¹¹ et R¹² peuvent être liés ensemble en formant un noyau pentagonal ou hexagonal qui peut être interrompu par O ou N,
R¹⁰ représente l'hydrogène, un reste alkyle linéaire ou ramifié ayant jusqu'à 4 atomes de carbone, ou un reste phényle,
le reste phényle pouvant porter un à trois substituants F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être substitués chacun une à trois fois par un radical phényle ou un hétérocycle du groupe 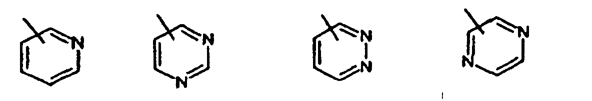 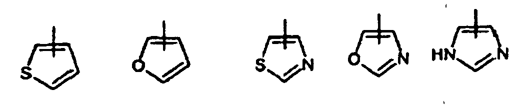 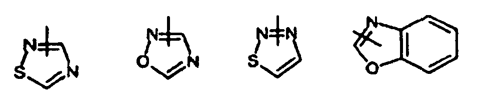 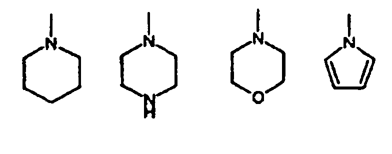 qui peuvent être liés directement ou par l'intermédiaire d'un groupe constitué de O, S, SO, SO₂, alkylène linéaire ou ramifié, alcènedilyle linéaire ou ramifié, alkyloxy linéaire ou ramifié, oxyalkyloxy linéaire ou ramifié, sulfonylalkyle linéaire ou ramifié, thioalkyle linéaire ou ramifié ayant dans chaque cas jusqu'à 4 atomes de carbone et qui peuvent être substitués une à trois fois par un radical alkyle linéaire ou ramifié, alkoxy linéaire ou ramifié, alkoxyalkoxy linéaire ou ramifié, halogénalkyle linéaire ou ramifié ou alcényle linéaire ou ramifié ayant dans chaque cas jusqu'à 4 atomes de carbone, F, Cl, Br, I, CN, SCH₃, OCF₃, NO₂, NR⁸R⁹ ou NR¹⁴COR¹⁷,
où
R¹⁴ représente l'hydrogène, un reste alkyle linéaire ou ramifié ayant jusqu'à 6 atomes de carbone ou un reste cycloalkyle ayant 3 à 6 atomes de carbone,
et
R¹⁷ représente l'hydrogène, un reste alkyle linéaire ou ramifié ayant jusqu'à 6 atomes de carbone, un reste alcényle linéaire ou ramifié ayant jusqu'à 6 atomes de carbone, un reste aryle ayant 6 à 10 atomes de carbone, un hétérocycle aromatique ayant 1 à 9 atomes de carbone et jusqu'à 3 hétéroatomes de la série S, N et/ou O ou un reste cycloalkyle ayant 3 à 6 atomes de carbone, qui peuvent éventuellement porter en outre un substituant F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou O,
R³ représente l'hydrogène ou le fluor,
m est le nombre entier 1 ou 2,
W est un groupe -CH₂- ou -CH₂CH₂-,
U est un groupe -CH₂-,
A désigne un reste phényle qui peut être substitué une à trois fois par un radical méthyle, éthyle, n-propyle, isopropyle, n-butyle, isobutyle, sec.-butyle, tertiobutyle, CF₃, méthoxy, éthoxy, F, Cl, Br,
R² est un groupe COOR²⁴,
dans lequel
R²⁴ représente l'hydrogène ou un reste alkyle linéaire ou ramifié ayant jusqu'à 4 atomes de carbone,
X est un reste alkylène linéaire ou ramifié ayant jusqu'à 6 atomes de carbone ou un reste alcènediyle linéaire ou ramifié ayant jusqu'à 6 atomes de carbone, ces restes pouvant contenir dans chaque cas un à trois groupes de la série phényloxy, O, CO ou CONR³⁰,
où
R³⁰ représente l'hydrogène, un reste alkyle linéaire ou ramifié ayant jusqu'à 6 atomes de carbone ou un reste cycloalkyle ayant 3 à 6 atomes de carbone,
n a la valeur 1 ou 2 ;
R¹ est un groupe COOR³⁵,
dans lequel
R³⁵ représente l'hydrogène ou un reste alkyle linéaire ou ramifié ayant jusqu'à 4 atomes de carbone.

3. Composés suivant la revendication 1,
dans lesquels
V représente 0,
Q représente un reste alkylène linéaire ou ramifié ayant jusqu'à 9 atomes de carbone ou un reste alcènediyle linéaire ou ramifié ou alcynediyle linéaire ou ramifié ayant jusqu'à 4 atomes de carbone, ces restes pouvant être substitués une fois par un halogène,
Y représente H, un reste cyclohexyle, phényle ou un hétérocycle du groupe des hétérocycles 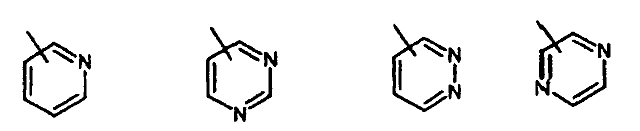 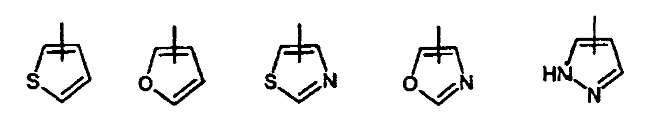 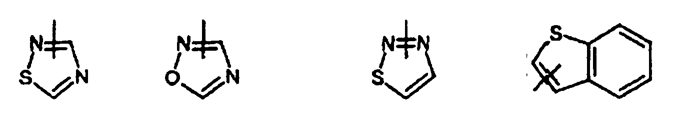 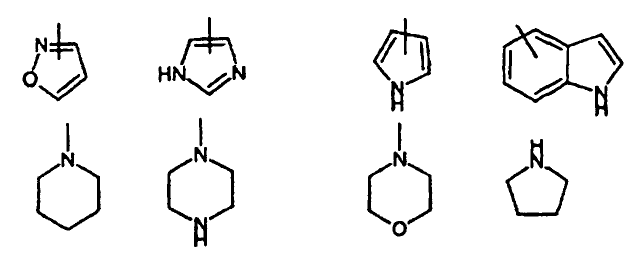 les restes cycliques pouvant être substitués dans chaque cas une à trois fois par un radical alkyle linéaire ou ramifié, alcényle linéaire ou ramifié, alcynyle linéaire ou ramifié, alkoxy linéaire ou ramifié, alkoxyalkoxy linéaire ou ramifié, halogénalkyle linéaire ou ramifié, halogénalkoxy linéaire ou ramifié, ayant dans chaque cas jusqu'à 4 atomes de carbone, cycloalkyle linéaire ou ramifié ayant 3 à 6 atomes de carbone, F, Cl, Br, I, NO₂, SR⁶, NR⁸R⁹, NR⁷COR¹⁰ ou CONR¹¹R¹²,
où
R⁶ représente l'hydrogène, un reste alkyle linéaire ou ramifié ayant jusqu'à 4 atomes de carbone ou un reste halogénalkyle linéaire ou ramifié ayant jusqu'à 4 atomes de carbone,
R⁷ représente l'hydrogène ou un reste alkyle linéaire ou ramifié ayant jusqu'à 4 atomes de carbone,
R⁸, R⁹, R¹¹ et R¹² représentent, indépendamment les uns des autres, l'hydrogène, un reste alkyle linéaire ou ramifié ayant jusqu'à 4 atomes de carbone, ou un reste phényle,
le reste phényle pouvant porter un à trois substituants F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants constitués de R⁸ et R⁹ ou de R¹¹ et R¹² peuvent être liés ensemble en formant un noyau pentagonal ou hexagonal qui peut être interrompu par O ou N,
R¹⁰ représente l'hydrogène, un reste alkyle linéaire ou ramifié ayant jusqu'à 4 atomes de carbone, ou un reste phényle,
le reste phényle pouvant porter un à trois substituants F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être substitués chacun une à trois fois par un radical phényle ou un hétérocycle du groupe des hétérocycles 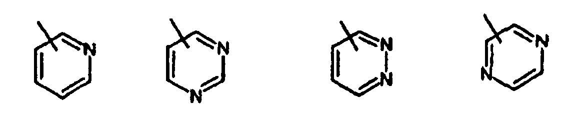 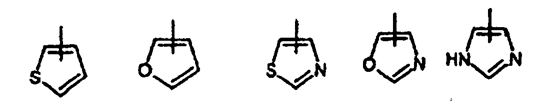 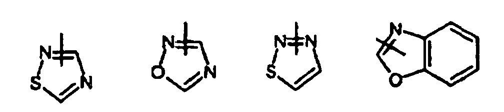 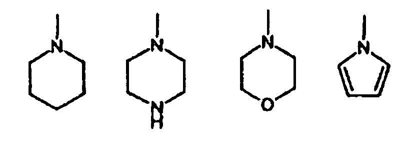 qui peuvent être liés directement ou par l'intermédiaire d'un groupe O, S, SO, SO₂, alkylène linéaire ou ramifié, alcènediyle linéaire ou ramifié, alkyloxy linéaire ou ramifié, oxyalkyloxy linéaire ou ramifié, sulfonylalkyle linéaire ou ramifié, thioalkyle linéaire ou ramifié ayant dans chaque cas jusqu'à 4 atomes de carbone et qui peuvent être substitués une à trois fois par un radical alkyle linéaire ou ramifié, alkoxy linéaire ou ramifié, alkoxyalkoxy linéaire ou ramifié, halogénalkyle linéaire ou ramifié ou alcényle linéaire ou ramifié ayant chacun jusqu'à 4 atomes de carbone, F, Cl, Br, I, CN, SCH₃, OCF₃, NO₂, NR⁸R⁹ ou NR¹⁴COR¹⁷,
où
R¹⁴ représente l'hydrogène, un reste alkyle linéaire ou ramifié ayant jusqu'à 6 atomes de carbone ou cycloalkyle ayant 3 à 6 atomes de carbone,
et
R¹⁷ représente l'hydrogène, un reste alkyle linéaire ou ramifié ayant jusqu'à 6 atomes de carbone, un reste alcényle linéaire ou ramifié ayant jusqu'à 6 atomes de carbone, un reste aryle ayant 6 à 10 atomes de carbone, un hétérocycle aromatique ayant 1 à 9 atomes de carbone et jusqu'à 3 hétéroatomes de la série S, N et/ou O ou un reste cycloalkyle ayant 3 à 6 atomes de carbone, ces restes pouvant éventuellement porter en outre un substituant F, Cl, Br, hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou O,
R³ représente l'hydrogène ou le fluor,
m représente le nombre entier 1 ou 2,
W est un groupe -CH₂- ou -CH₂CH₂-,
U est un groupe -CH₂-,
A est un reste phényle qui peut porter éventuellement un à trois substituants méthyle, éthyle, n-propyle, isopropyle, n-butyle, isobutyle, sec.-butyle, tertiobutyle, CF₃, méthoxy, éthoxy, F, Cl, Br,
R² est un groupe COOH,
X est un reste alkylène linéaire ou ramifié ayant jusqu'à 6 atomes de carbone ou un reste alcènediyle linéaire ou ramifié ayant jusqu'à 6 atomes de carbone, ces restes pouvant contenir chacun un à trois groupes de la série phényloxy, 0, CO ou CONR³⁰,
où
R³⁰ représente l'hydrogène, un reste alkyle linéaire ou ramifié ayant jusqu'à 6 atomes de carbone ou un reste cycloalkyle ayant 3 à 6 atomes de carbone,
n a la valeur 1 ou 2 ;
R¹ est un groupe COOH.

4. Composés suivant la revendication 1,
dans lesquels
V représente O,
Q est un groupe CH₂,
Y représente un groupe phényle qui est substitué avec un reste choisi dans le groupe constitué des restes 2-phényléthyle, cyclohexyle, 4-chlorophényle, 4-méthoxyphényle, 4-trifluorométhylphényle, 4-cyanophényle, 4-chlorophénoxy, 4-méthoxyphénoxy, 4-trifluorométhylphénoxy, 4-cyanophénoxy, 4-méthylphényle,
R³ représente l'hydrogène ou le fluor,
m est le nombre entier 1 ou 2,
W est un groupe -CH₂CH₂-,
U est un groupe -CH₂-,
A est un groupe phényle,
R² est un groupe COOH, R² étant en position 4 par rapport au reste U,
X est un groupe (CH₂)₄,
R¹ est un groupe COOH.

5. Composé suivant la revendication 1, de formule

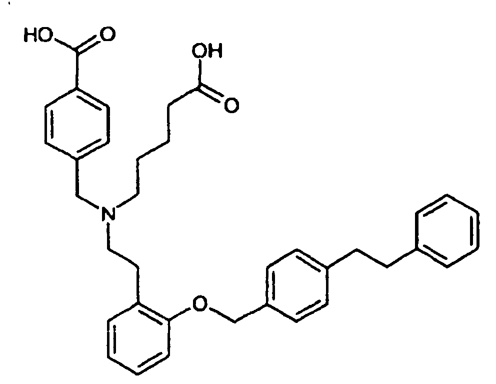

6. Procédé de production de composés de formule générale (I), **caractérisé en ce que** :
[A] des composés de formule (II) 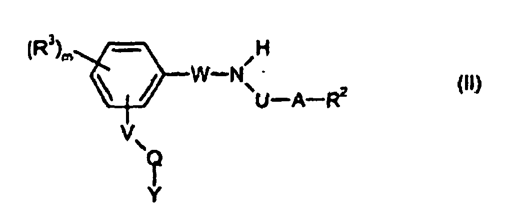 sont amenés à réagir avec des composés de formule (III)
E-X-R¹ (III)
formules dans lesquelles
R¹, R², R³, V, Q, Y, W, X, U, A et m ont les mêmes définitions que dans la revendication 1,
E désigne un groupe partant qui est substitué en présence d'une base, ou une fonction hydroxy éventuellement activée ;
ou bien
[B] des composés de formule (IV) 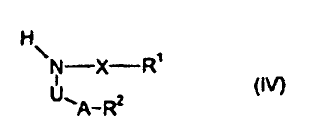 sont amenés à réagir avec des composés de formule (V) 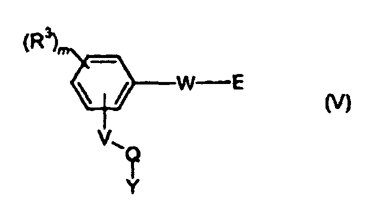 formules dans lesquelles
R¹, R², R³, V, Q, Y, W, X, U, A et m ont les mêmes définitions que dans la revendication 1,
E désigne un groupe partant qui est substitué en présence d'une base, ou une fonction hydroxy éventuellement activée ;
ou bien
[C] des composés de formule (VI) 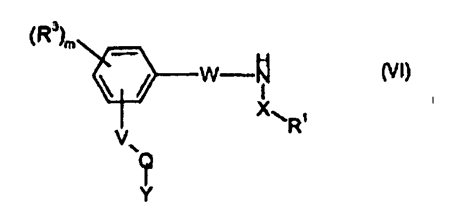 sont amenés à réagir avec des composés de formule (VII)
E-U-A-R² (VII)
formules dans lesquelles
R¹, R², R³, V, Q, Y, W, X, U, A et m ont les mêmes définitions que dans la revendication 1,
E désigne un groupe partant qui est substitué en présence d'une base, ou désigne une fonction hydroxy éventuellement activée ;
ou bien
[D] des composés de formule (VIII) 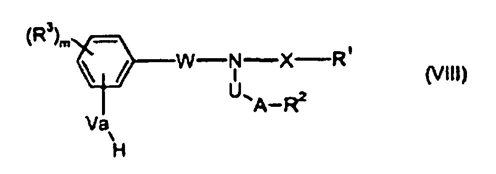 dans laquelle
Va représente O ou S et
W, A, X, U, R¹, R², R³ et m ont la définition indiquée dans la revendication 1,
sont amenés à réagir avec des composés de formule (IX) 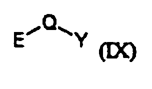 dans laquelle
Q, Y ont les mêmes définitions que dans la revendication 1,
E désigne un groupe partant qui est substitué en présence d'une base, ou désigne une fonction hydroxy éventuellement activée ;
ou bien
[E] des composés de formule (X) 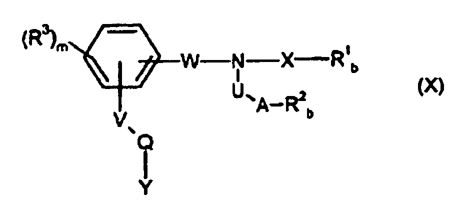 dans laquelle
R³, V, Q, Y, W, X, U, A et m ont les mêmes définitions que dans la revendication 1,
R¹ _{b} et R² _{b} représentent chacun indépendamment un groupe CN ou COOAlk, Alk désignant un reste alkyle linéaire ou ramifié ayant jusqu'à 6 atomes de carbone,
sont transformés en les acides carboxyliques libres correspondants avec des solutions aqueuses d'acides forts ou de bases fortes,
ou bien
[F] des composés de formule (XI) 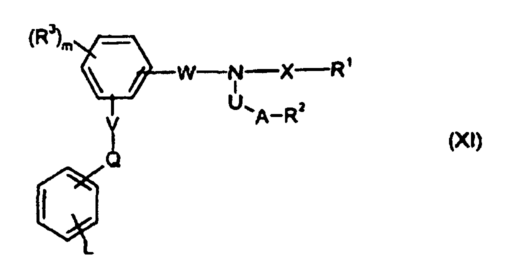 dans laquelle
R¹, R², R³, V, Q, Y, W, X, U, A et m ont les mêmes définitions que dans la revendication 1,
L représente Br, I ou le groupe CF₃SO₂-O,
sont amenés à réagir avec des composés de formule (XII)
M-Z (XII)
dans laquelle
M désigne un reste aryle ou hétéroaryle, un reste alkyle, alcényle ou alcynyle linéaire ou ramifié ou un reste cycloalkyle ou un reste arylalkyle, un reste arylalcényle ou un reste arylalcynyle,
Z représente les groupements -B(OH)₂, -CH≡CH, -CH=CH₂ ou -Sn(nBu)₃,
en présence d'un composé de palladium, en outre éventuellement en présence d'un agent réducteur et d'autres additifs et en présence d'une base ;
ou bien
[G] des composés de formule (XIII) 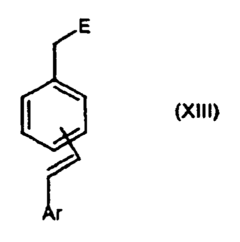 dans laquelle
Ar représente un reste aryle ou hétéroaryle,
E désigne un groupe partant qui est substitué en présence d'une base,
sont amenés à réagir selon le procédé D avec des composés de formule (VIII) et les composés ainsi obtenus de formule (XIV) 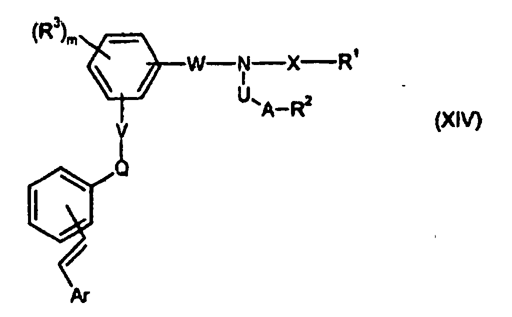 sont hydrogénés avec de l'hydrogène en présence d'un catalyseur.

7. Composés de formule (II) 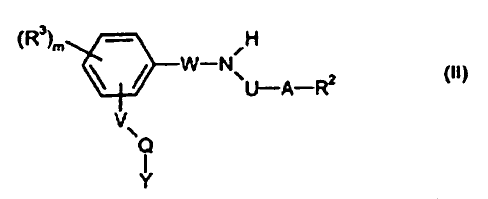 dans laquelle
V, Q, Y, R³, m, W, N, U, A et R² ont la définition indiquée dans la revendication 1.

8. Composés de formule (IV) 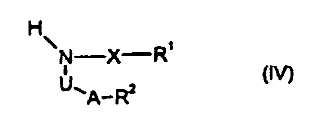 dans laquelle
U, A, X, R¹ et R² ont la définition indiquée dans la revendication 1.

9. Composés de formule (VI) 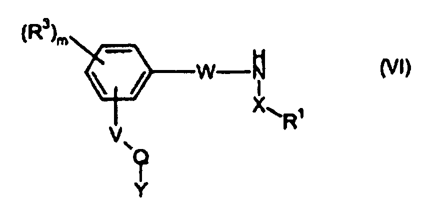 dans laquelle V, Q, Y, R³, m, W, X et R¹ ont la définition indiquée dans la revendication 1.

10. Médicament contenant au moins un composé de formule générale (I) suivant l'une des revendications 1 à 5.

11. Utilisation de composés de formule (I) suivant l'une des revendications 1 à 5 pour la préparation d'un médicament destiné au traitement de maladies du système cardiovasculaire.

12. Utilisation de composés de formule générale (I) suivant l'une des revendications 1 à 5 pour la préparation de médicaments destinés au traitement de l'angine de poitrine, d'ischémies et de l'insuffisance cardiaque.

13. Utilisation de composés de formule générale (I) suivant l'une des revendications 1 à 5 pour la préparation de médicaments destinés au traitement de l'hypertonie, de maladies thromboemboliques, de l'artériosclérose et de maladies veineuses.

14. Utilisation de composés de formule générale (I) suivant l'une des revendications 1 à 5 pour la préparation de médicaments destinés au traitement de fibroses.

15. utilisation suivant la revendication 14, **caractérisée en ce que** la fibrose est une fibrose hépatique.
